(19) Europäisches Patentamt
European Patent Office
Office européen des brevets

(11) EP 2 945 957 B1

(12) # FASCICULE DE BREVET EUROPEEN

(45) Date de publication et mention de la délivrance du brevet:
**08.11.2023 Bulletin 2023/45**

(21) Numéro de dépôt: **14705819.2**

(22) Date de dépôt: **16.01.2014**

(51) Classification Internationale des Brevets (IPC):
***C07F 9/30*** (2006.01) ***C07D 255/02*** (2006.01)
***C07F 9/6558*** (2006.01) ***C07D 473/18*** (2006.01)
***C07F 5/00*** (2006.01) ***A61K 49/00*** (2006.01)
***G01N 33/533*** (2006.01)

(52) Classification Coopérative des Brevets (CPC):
**C07D 255/02; C07D 213/79; C07D 473/18; C07F 9/58; C07F 9/65583; C07H 23/00; C09K 11/06**

(86) Numéro de dépôt international:
**PCT/FR2014/050085**

(87) Numéro de publication internationale:
**WO 2014/111661 (24.07.2014 Gazette 2014/30)**

(54) **NOUVEAUX AGENTS COMPLEXANTS HYDROSOLUBLES ET COMPLEXES DE LANTHANIDE CORRESPONDANTS**

NEUARTIGE WASSERLÖSLICHE KOMPLEXBILDNER UND ZUGEHÖRIGE LANTHANIDKOMPLEXE

NOVEL WATER-SOLUBLE COMPLEXING AGENTS AND CORRESPONDING LANTHANIDE COMPLEXES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.01.2013 FR 1350374**

(43) Date de publication de la demande:
**25.11.2015 Bulletin 2015/48**

(73) Titulaire: **Cisbio Bioassays**
**30200 Codolet (FR)**

(72) Inventeurs:
- **LAMARQUE, Laurent**
  **F-30290 Saint Victor La Coste (FR)**
- **PARKER, David**
  **Durham DH1 5WA (GB)**
- **BUTLER, Stephen J.**
  **Durham DH 1 5BW (GB)**
- **DELBIANCO, Martina**
  **I-20013 Magenta (IT)**

(74) Mandataire: **Cabinet Beau de Loménie**
**158, rue de l'Université**
**75340 Paris Cedex 07 (FR)**

(56) Documents cités:
**EP-A1- 0 203 047** **EP-A1- 0 321 353**
**WO-A1-2005/021538** **WO-A1-2005/058877**
**WO-A1-2007/128874** **WO-A1-2013/011236**

- **Harri Takalo ET AL: "Synthesis and Luminescence of Novel Eu"' Complexing Agents and Labels with 4-(Phenylethyny1)pyridine Subunits", HELVETICA CHIMICA ACTA, 1 janvier 1996 (1996-01-01), pages 789-802, XP055018996, Extrait de l'Internet: URL:http://onlinelibrary.wiley.com/store/10.1002/hlca.19960790321/asset/19960790321_ftp.pdf?v=1&t=gyg00f71&s=fcb313ca9776bd67f7628bd4ab68fdf79847fb01 [extrait le 2012-02-09]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- Martti Latva ET AL: "Evaluation of solution structures of highly luminescent europium(III) chelates by using laser induced excitation of the 7F0 5D0 transition", Inorganica Chimica acta, 1 janvier 1998 (1998-01-01), pages 63-72, XP055019096, Extrait de l'Internet: URL:http://pdn.sciencedirect.com/science?_ob=MiamiImageURL&_cid=271401&_user=987766&_pii=S0020169397055539&_check=y&_origin=browse&_zone=rslt_list_item&_coverDate=1998-01-03&wchp=dGLzVBA-zSkzV&md5=c429925e29cf1ac1852b56e9bcc6c192/1-s2.0-S0020169397055539-main.pdf [extrait le 2012-02-10]
- ANTHONY D'ALÉO ET AL: "Ytterbium-Based Bioprobes for Near-Infrared Two-Photon Scanning Laser Microscopy Imaging", ANGEWANDTE CHEMIE INTERNATIONAL EDITION, vol. 51, no. 27, 2 juillet 2012 (2012-07-02), pages 6622-6625, XP055060978, ISSN: 1433-7851, DOI: 10.1002/anie.201202212
- VIRGINIE PLACIDE ET AL: "Design and synthesis of europium luminescent bio-probes featuring sulfobetaine moieties", TETRAHEDRON LETTERS, vol. 55, no. 7, 13 janvier 2014 (2014-01-13), pages 1357-1361, XP055107089, ISSN: 0040-4039, DOI: 10.1016/j.tetlet.2014.01.025
- JAMES W. WALTON ET AL: "Very bright europium complexes that stain cellular mitochondria", CHEMICAL COMMUNICATIONS, vol. 49, no. 16, 21 janvier 2013 (2013-01-21), page 1600, XP055060987, ISSN: 1359-7345, DOI: 10.1039/c2cc35247h

## Description

La présente invention a pour objet des agents complexants ou ligands hydrosolubles, des complexes de lanthanides obtenus à partir de ces agents complexants, et des conjugués fluorescents obtenus à partir de ces complexes de lanthanides.

## Etat de la technique

Les complexes de lanthanide ont vu leur utilisation augmenter de manière très importante depuis une vingtaine d'années dans le domaine des sciences de la vie. Ces composés fluorescents présentent en effet des caractéristiques spectroscopiques intéressantes, qui en font des marqueurs de choix pour détecter des molécules biologiques. Ces composés fluorescents sont particulièrement appropriés pour être utilisés en conjonction avec des fluorophores compatibles pour effectuer des mesures de FRET (acronyme de l'expression anglaise « Förster resonnance energy transfer »), dont l'application pour étudier les interactions entre biomolécules est exploitée de manière commerciale par plusieurs sociétés, dont Cisbio Bioassays et sa gamme de produits HTRF®. La durée de vie relativement longue des complexes de lanthanides permet également d'effectuer des mesures de fluorescence en temps résolu, c'est-à-dire avec un délai après excitation des fluorophores, ce qui permet de limiter les interférences de fluorescence dues au milieu de mesure. Cette dernière caractéristique est d'autant plus utile que le milieu de mesure se rapproche d'un milieu biologique, qui comprend de nombreuses protéines dont la fluorescence pourrait interférer avec celle des composés étudiés.

De nombreux complexes de lanthanides ont été décrits. Latva et al par exemple, ont divulgué 41 complexes d'Eu(III) et de Tb(III), dont ils ont étudié la luminescence (Journal of Luminescence Volume 75, n° 2, Septembre 1997, Pages 149-169). Le composé **39** en particulier, est constitué d'un cycle 1,4,7-triazacyclononane (ci-après « TACN »), dont les atomes d'azote sont substitués par des chromophores dérivés de phényléthynylpyridine. Bien que le rendement quantique du complexe constitué de ce chromophore et d'Eu(III) soit considéré comme bon par les auteurs, ce complexe n'est pas adapté au couplage avec une biomolécule. Par ailleurs, l'utilisation de ce composé en milieu aqueux peut être problématique puisque il est très hydrophobe. Enfin, l'absorption de ce complexe est optimale à 315 nm, alors que les lampes laser souvent utilisées dans les dosages biologiques émettent à la longueur d'onde de 337 nm.

D'Aléo et al ont décrit la synthèse de complexes de lanthanides constitués de trois ligands dérivés de l'acide dipicolonique (Inorg Chem. 2008 Nov 17;47(22):10258-68). L'un de ces ligands (L1) est constitué d'une molécule d'acide dipicolinique substituée par un groupe phényléthynyl, lui-même portant un éther-oxyde de polyéthylèneglycol (ci-après « PEG ») sur le groupe phényle. D'après les auteurs, le groupe PEG confère à ce produit une bonne solubilité en milieux aqueux et dans les solvants organiques. Toutefois, ces complexes ne sont pas suffisamment stables en milieu aqueux et ne sont pas utilisables dans une réaction de bioconjuguaison.

D'Aléo et al. (Angew. Chem. Int. Ed. 2012, 51, 6622-6625) rapportent l'utilisation de complexes d'Ytterbium comme sondes pour la microscopie à balayage laser. Les deux complexes testés, Yb1 et Yb2, comprennent des molécules d'acide dipicolinique substituées par un groupe phényléthynyl, lui-même portant un groupe $N(PEG)_2$.

Placide et al. (Tetrahedron Letters 2014, 55, 1357-1361) et Walton et al. (Chem. Comm. 2013, 49, 1600-1602) ont décrit la synthèse de complexes d'Europium à partir d'un ligand TACN dont les atomes d'azote sont substitués par des chromophores dérivés de phényléthynylpyridine.

La demande de brevet WO2005/058877 est relative à des complexes de lanthanides dont certains sont basés sur un cycle TACN dont trois atomes d'azote sont substitués par des chromophores constitués d'un dérivé de pyridine, notamment de phénylpyridine. Les inventeurs proposent par ailleurs d'inclure dans ces composés un groupe réactif pour pouvoir les conjuguer facilement avec des biomolécules. Il est ainsi proposé d'inclure ce groupe réactif *via* un bras d'espacement soit au niveau d'un carbone du cycle TACN, soit au niveau de la pyridine du chromophore.

Plusieurs autres complexes de lanthanides ont été divulgués et certains sont exploités de manière commerciale : on peut citer en particulier les cryptates macropolycycliques de lanthanides (EP 0 180 492, EP 0 321 353, EP 0 601 113 , WO2001/96877, WO2008/063721), les complexes de lanthanide comportant un motif dérivé de la coumarine lié à un motif diéthylènetriamine penta-acide (US 5,622,821), et ceux comprenant des dérivés de pyridine (US 4,920,195, US 4,761,481), de bipyridine (US 5,216,134), ou de terpyridine (US 4,859,777, US 5,202,423, US 5,324,825).

La demande de brevet WO89/01475 décrit la préparation de macrocycles triazotés dont l'un des atomes de carbone porte un groupe L-Z, et notamment du 2-(4-N-benzamidyl)butyl-1,4,7-triazacyclonane (intermédiaire 12). La synthèse d'hétéromacrocycles azotés dont l'un des atomes de carbone est substitué est également décrite par Cox et al (J. Chem. Soc., Perkin Trans. 1, 1990, 2567-2576) et Craig et al (J. Chem. Soc., Chem. Commun., 1989, 794-796).

La demande de brevet WO 2013/011236 décrit des agents complexants de formule :

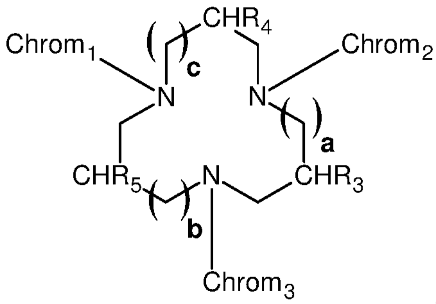

.

La présente invention vise à pallier aux inconvénients des composés de l'art antérieur, et à fournir des complexes de lanthanides fluorescents présentant une meilleure brillance que les composés de l'art antérieur lorsqu'ils sont excités aux alentours de 337 nm, une bonne solubilité en milieu aqueux, un spectre d'émission adapté à leur utilisation dans des expériences de FRET, ainsi qu'une bonne praticité pour le marquage de biomolécules.

**Description de l'invention**

Les problèmes mentionnés précédemment ont été résolus grâce à des agents complexants constitués d'un macrocycle triazoté (1,4,7-triazacyclononane, ci-après TACN) dont les atomes d'azote sont substitués par des chromophores de type phényléthynylpyridine, ces chromophores comprenant un groupes affectant la densité électronique de la molécule (ci-après groupe « O-donneur ») directement lié au groupe phényle, et lesdits agents complexant comportant par ailleurs au moins un groupe conférant à la molécule un caractère relativement hydrophile. Ces composés peuvent également comporter un groupe réactif permettant leur conjugaison avec une molécule à marquer. Les agents complexants selon l'invention forment des complexes stables avec les lanthanides, et peuvent être utilisés pour produire des conjugués fluorescents de molécules d'intérêt. Les complexes de lanthanides selon l'invention présentent d'excellentes propriétés photophysiques, en particulier en ce qui concerne leur rendement quantique, la durée de vie de leur luminescence et leur spectre d'excitation qui est très bien adapté à une excitation laser à environ 337 nm. La présence de trois chromophores augmente de façon significative le coefficient d'absorption molaire (epsilon) et par conséquent la brillance du complexe. La brillance (rendement quantique x coefficient d'absorption molaire) de ces complexes dans des milieux biologiques est également meilleure que celle des composés de l'art antérieur, et leur solubilité en milieu aqueux les rend très adaptés à une utilisation en milieu biologique.

**AGENTS COMPLEXANTS**

Les agents complexants selon l'invention sont les composés de formule (I') :

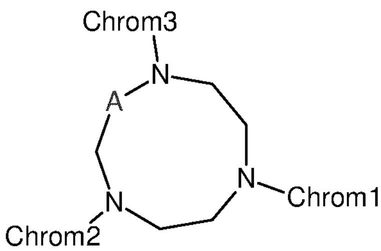

(I')

dans laquelle :

- A représente $-CH_2-$ ou $-CH(L_2-G)-$
- chrom1, chrom2 sont identiques et sont choisis parmi les groupes de formule :

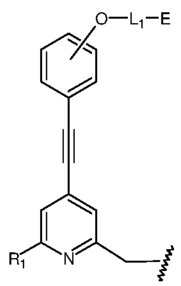

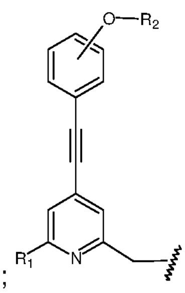

;

- chrom 3 est soit identique à chrom1 et chrom2, soit un groupe de formule :

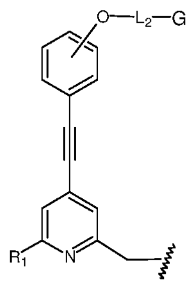

;

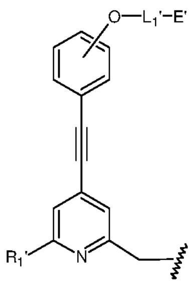

;

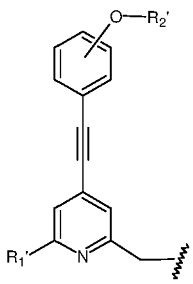

$R_2$, $R_2$', qui peuvent être identiques ou différents, sont choisis parmi : H ; -Alk ; -phényle ; - $CH_2$-CO-N-Alk ; -$CH_2$-CO-O-Alk ; -$CH_2$-CO-$NH_2$ ; -$CH_2$-CO-OH ;

◦ $L_1$, $L_1$', $L_2$ sont choisis parmi les groupes divalents de formules suivantes :

-(CH2),- ;-$(CH_2)_n$-O-$(CH_2)_m$-O-$(CH_2)_p$-;

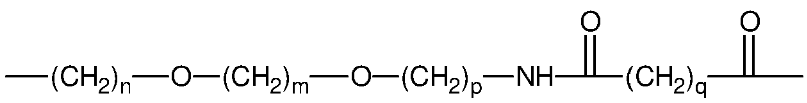

;

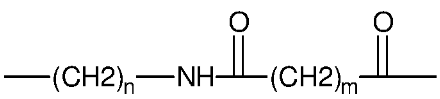

;

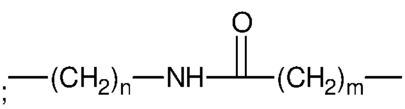

;

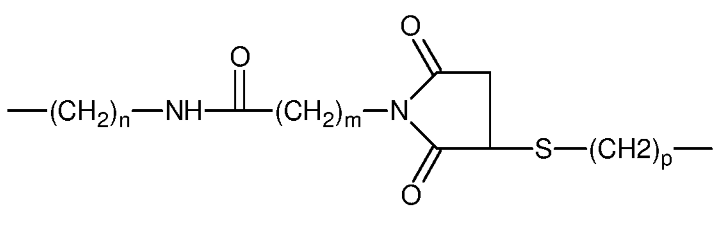

;

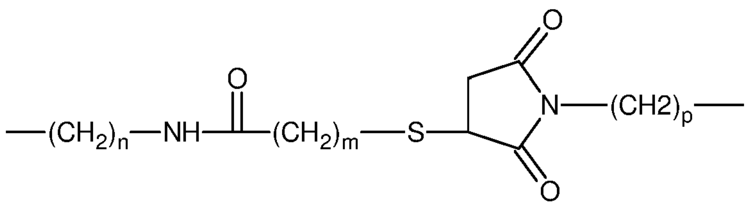

:

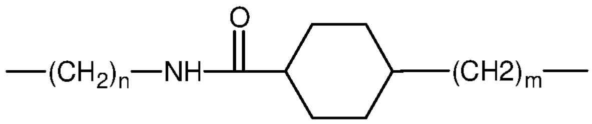

;

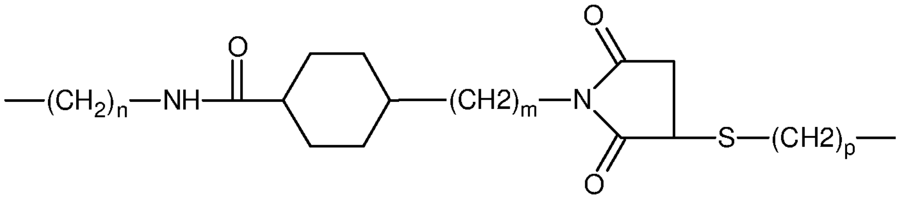

;-$(CH2)_n$-NH-; -$(CH2)_n$-NH-$(CH2)_m$-

; 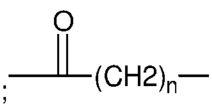 ; 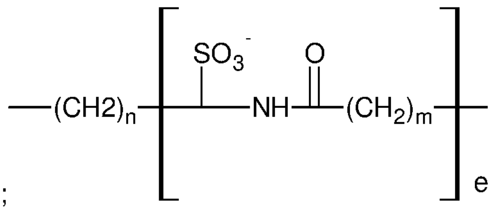

dans lesquelles n, m, p, q sont des nombres entiers de 1 à 16, de préférence de 1 à 5 et e est un nombre entier allant de 1 à 6, de préférence de 1 à 4 ;

- E, E', qui peuvent être identiques ou différents, sont des groupes augmentant l'hydrosolubilité de l'agent complexant, choisis parmi : -$SO_3H$, -$PO(OH)_2$, -COOH, -$N^+Alk_1Alk_2Alk_3$, un résidu carbohydrate de formule -$(CHOH)_k$-$CH_2OH$, k étant un nombre entier allant de 3 à 12, de préférence, égal à 4 ;
- $R_1$, $R_1$', qui peuvent être identiques ou différents, sont choisis parmi : -COOH, -$PO(OH)R_6$, $R_6$ étant choisi parmi les groupes : phényle, phényle substitué par un groupe -$SO_3H$, de préférence en position ortho ou para, benzyle, méthyle, éthyle, propyle, n-butyle, sec-butyle, isobutyle, tert-butyle ;
- G est une amine éventuellement protégée sous forme -NHBoc, un ester de succinimidyle, un haloacétamide, une hydrazine, un isothiocyanate, un groupe maléimide, ou un acide carboxylique éventuellement protégé sous la forme d'un groupe -COOMe ou, -COOtBu ;
- Alk, $Alk_1$; $Alk_2$, $Alk_3$, qui peuvent être identiques ou différents, représentent un ($C_1$-$C_6$)alkyle ;

sous réserve que :

- lorsque $R_1$ ou $R_1$' représentent un groupe -COOH, E ou E' ne représentent pas un groupe -COOH ;
- l'agent complexant comporte au moins un groupe E ou -$SO_3H$ ;
- lorsque chrom1, chrom2 et chrom3 comportent chacun un groupe $R_2$ ou $R_2$', alors A est un groupe -CH($L_2$-G)- dans lequel $L_2$ comprend au moins un groupe -$SO_3H$ ou bien $R_6$ est un groupe phényle substitué par un groupe -$SO_3H$.

En fonction du pH, les groupes -$SO_3H$, -COOH et -$PO(OH)_2$ sont sous forme déprotonée ou pas . Ces groupes désignent donc dans la suite du texte également les groupes -$SO3^-$, -$COO^-$ et -$PO(OH)O^-$, et vice-versa.

Une première sous-famille d'agents complexant préférés est celle constituée de composés selon la formule (I'), dans laquelle A est le groupe -$CH_2$- et chrom1, chrom2, chrom3 sont identiques et représentent un groupe de formule :

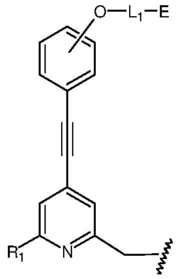

Une deuxième sous-famille d'agents complexant préférés est celle constituée de composés selon la formule (I'), dans laquelle A est le groupe $-CH_2-$ et chrom3 est différent de chrom1 et chrom2. Une quatrième sous-famille d'agents complexant préférés est celle constituée de composés selon la formule (I'), dans laquelle A est le groupe $-CH(L_2-G)-$ et chrom3 est différent de chrom1 et chrom2.

Les différentes combinaisons de chromophores chrom1, chrom2 et chrom3 appartenant à ces différentes sous-familles préférées sont résumées dans la table ci-après :

| | | |
|---|---|---|
| | 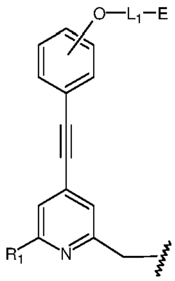 chrom1 = chrom2 | 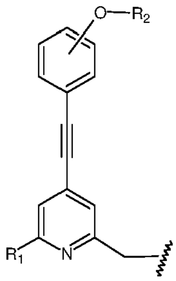 chrom1 = chrom2 |
| 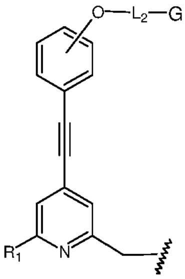 chrom3 | Deuxième sous-famille préférée :<br>A: $-CH_2-$<br>Quatrième sous-famille préférée<br>A : $-CH(L_2-G)-$ | Deuxième sous-famille préférée :<br>A : $-CH_2-$<br>Quatrième sous-famille préférée<br>A : $-CH(L_2-G)-$ |
| 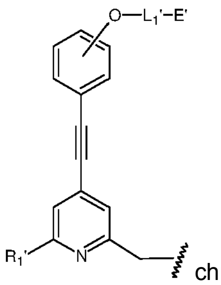 chrom3 | Composés symétriques<br>Première sous-famille préférée :<br>A: $-CH_2-$<br>Troisième sous- famille préférée :<br>A : $-CH(L_2-G)-$ | Deuxième sous-famille préférée :<br><br>A : $-CH_2-$<br>Quatrième sous-famille préférée<br>A : $-CH(L_2-G)-$ |
| 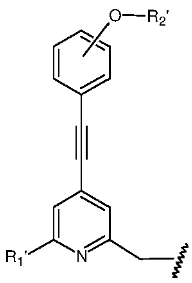 chrom3 | Deuxième sous-famille préférée :<br>A: $-CH_2-$<br>Quatrième sous-famille préféré<br>A : $-CH(L_2-G)-$ | Composés symétriques<br>Première sous-famille préférée :<br>A: $-CH(L_2-G)-$<br>L comprend au moins un groupe $SO_3H$ |

Pour chacune de ces sous-familles de composés selon la formule (I'), les composés dont les groupes $R_1$ ou

$R_1$' sont des groupes -COOH ou -PO(OH)$R_6$ lorsque $R_6$ représente un groupe phényle, benzyle, méthyle, éthyle, propyle, *n*-butyle, *sec*-butyle, isobutyle, ou *tert*-butyle, sont préférés. Parmi ces composés, on préfère tout particulièrement ceux dont les groupes $R_1$ ou R'$_1$ sont des groupes -PO(OH)$R_6$ lorsqu $R_6$ représente un groupe méthyle, éthyle, propyle, *n*-butyle, sec-butyle, isobutyle, ou tert-butyle, le groupe méthyle étant particulièrement préféré.

Pour chacune de ces sous-familles de composés selon la formule (I'), les composés dont le groupe A est le groupe -CH($L_2$-G)- et chrom1, chrom2, chrom3 sont identiques sont préférés.

Pour chacune de ces sous-familles de composés selon la formule (I'), les composés dont les groupes E ou E' sont des groupes -$SO_3H$ sont préférés.

Les composés selon l'invention dont les groupes $R_1$ ou $R_1$' sont des groupes -PO(OH)$R_6$ et dont les groupes E ou E' sont des groupes -$SO_3H$ constituent également des composés préférés.

Les composés selon l'invention comportant un groupe -$L_2$-G, qui peut être utilisé pour coupler le chélate ou complexe selon l'invention avec une molécule que l'on souhaite marquer, sont particulièrement utiles et sont donc également préférés.

Le groupe réactif G porté par un bras d'espacement $L_2$, permet de coupler les composés selon l'invention avec une espèce que l'on souhaite rendre fluorescente, par exemple une molécule organique, un peptide ou une protéine. Les techniques de conjugaison de deux molécules organiques sont basées sur l'utilisation de groupes réactifs et relèvent des connaissances générales de l'homme du métier. Ces techniques classiques sont décrites par exemple dans Bioconjugate Techniques, G.T. Hermanson, Academic Press, Second Edition 2008, p. 169-211. Typiquement, le groupe réactif est un groupe électrophile ou nucléophile qui peut former une liaison covalente lorsqu'il est respectivement mis en présence d'un groupe nucléophile ou électrophile approprié. La réaction de conjugaison entre un composé selon l'invention comportant un groupe réactif et une molécule organique, un peptide ou une protéine portant un groupe fonctionnel entraîne la formation d'une liaison covalente comportant un ou plusieurs atomes du groupe réactif.

Le groupement réactif G est une amine (éventuellement protégée sous forme -NHBoc), un ester de succinimidyle, un haloacétamide, une hydrazine, un isothiocyanate, un groupe maléimide, une amine aliphatique, ou un acide carboxylique (éventuellement protégé sous la forme d'un groupe - COOMe, -COOtBu). Dans ce dernier cas, l'acide devra être activé sous forme d'ester pour pouvoir réagir avec une espèce nucléophile.

Les groupes réactif G et les groupes solubilisant E et E' peuvent être directement liés à l'agent complexant par une liaison covalente ou bien via un bras d'espacement constitué de manière avantageuse par un radical organique bivalent. Ainsi, les bras d'espacement $L_2$, $L_1$ et $L_1$' sont choisis parmi :

- un groupe choisi parmi les groupes divalents de formules suivantes :

$-(CH2)_n-$ ;$-(CH_2)_n-O-(CH_2)_m-O-(CH_2)_p-$ ;

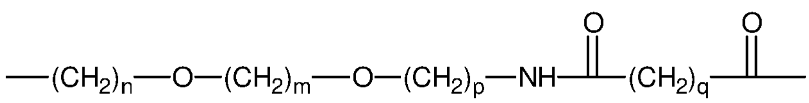 ;

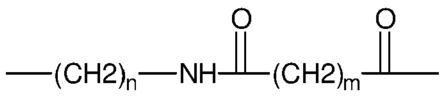 ; 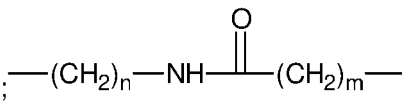 ;

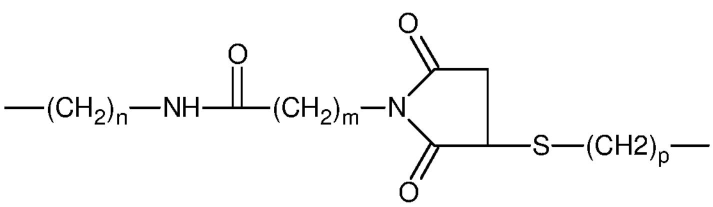 ;

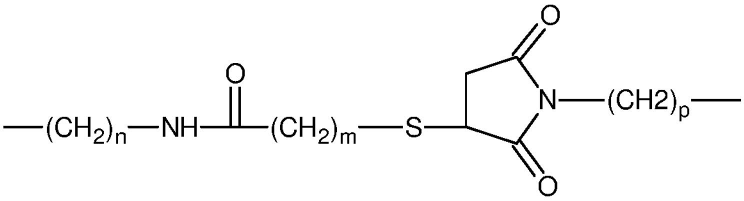

;

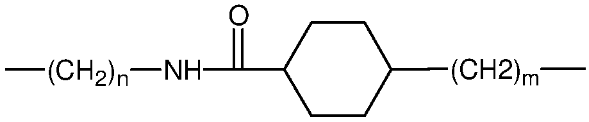

;

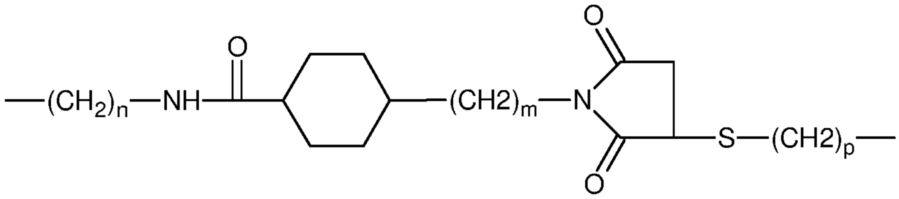

;

$-(CH_2)_n-NH-$ ; $-(CH2)_n-NH-(CH2)_m-$ ; 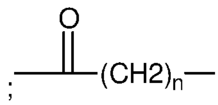 ;

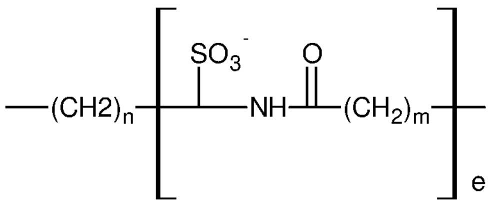

dans lesquelles n, m, p, q sont des nombres entiers de 1 à 16, de préférence de 1 à 5 et e est un nombre entier allant de 1 à 6, de préférence de 1 à 4.

De manière préférée, le groupe $-L_2$-G est constitué d'un groupement réactif G choisi parmi : un acide carboxylique (éventuellement protégé sous la forme d'un groupe -COOMe, -COOtBu), une amine (éventuellement protégée sous forme -NHBoc), un ester de succinimidyle, un haloacétamide, une hydrazine, un isothiocyanate, un groupe maléimide, une amine aliphatique, et d'un bras d'espacement $L_2$ constitué d'une chaîne alkylène comprenant de 1 à 5 atomes de carbone ou d'un groupe choisi parmi les groupes de formule :

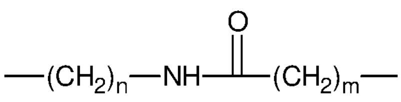 ; 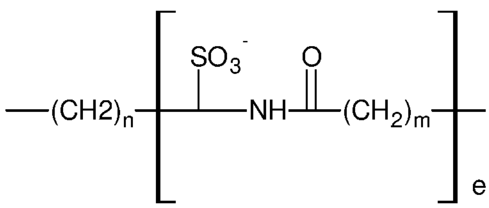

où n, m, sont des nombres entiers de 1 à 16, de préférence de 1 à 5 et e est un nombre entier allant de 1 à 6, de préférence de 1 à 4, le groupe G étant lié à l'une ou l'autre extrémité de ces groupes divalents.

De la même façon, le bras d'espacement $L_1$ ou $L_1$', conférant le caractère hydrosoluble aux composés selon l'invention, est de manière préférée constitué d'une chaîne alkylène comprenant de 1 à 5 atomes de carbone ou d'un groupe choisi parmi les groupes de formule :

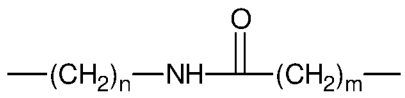 ; 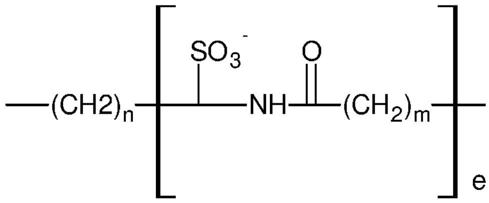

où n, m, sont des nombres entiers de 1 à 16, de préférence de 1 à 5 et e est un nombre entier allant de 1 à 6, de préférence de 1 à 4, le groupe E ou E' étant lié à l'une ou l'autre extrémité de ces groupes divalents.

## COMPLEXES

L'invention concerne également les complexes de lanthanides constitués d'un atome de lanthanide complexé par un agent complexant tel que décrit ci-dessus, le lanthanide étant choisi parmi : $Eu^{3+}$, $Tb^{3+}$, $Gd^{3+}$, $Dy^{3+}$, $Nd^{3+}$, $Er^{3+}$. De préférence, le lanthanide est $Tb^{3+}$ ou $Eu^{3+}$ et de manière encore plus préférée $Eu^{3+}$.

Ces complexes sont préparés en mettant en contact les agents complexants selon l'invention et un sel de lanthanide. Ainsi la réaction entre un équivalent d'agent complexant et 1 à 5 équivalents de sel de lanthanide (europium ou terbium sous forme de chlorures, d'acétates ou de triflates) dans un solvant (acétonitrile, méthanol ou autre solvant compatible avec ces sels) à reflux pendant plusieurs heures conduit au complexe correspondant.

Comme indiqué précédemment, les complexes fluorescents obtenus présentent d'excellentes propriétés photophysiques, en particulier en ce qui concerne leur rendement quantique, la durée de vie de leur luminescence et leur spectre d'excitation qui est très bien adapté à une excitation laser à environ 337 nm. De plus la répartition des bandes de leurs spectres d'émission est centrée autour de 620 nm conférant ainsi aux complexes des propriétés exceptionnelles et très favorables dans une utilisation de FRET avec des accepteurs de type cyanine ou allophycocyanine (telle que la XL665 commercialisée par Cisbio Bioassays). Du fait de la grande stabilité de ces complexes dans les milieux biologiques contenant des cations divalents ($Mn^{2+}$, $Ca^{2+}$, $Mg^{2+}$...) ou de l'EDTA, leur luminescence reste excellente comparée aux complexes de l'art antérieur.

## CONJUGUES

Les agents complexants et complexes de lanthanides selon l'invention comportant un groupe $-L_2$-G, sont particulièrement adaptés au marquage de molécules organiques ou biologiques comportant un groupe fonctionnel susceptible de réagir avec le groupe réactif pour former une liaison covalente. Ainsi l'invention concerne aussi l'utilisation des complexes de lanthanides pour le marquage de molécules d'intérêt (protéines, anticorps, enzymes, hormones etc...).

En particulier, les conjugués selon l'invention comportent un complexe selon l'invention et une molécule choisie parmi : un acide aminé, un peptide, une protéine, un anticorps, un sucre, une chaîne glucidique, un nucléoside, un nucléotide, un oligonucléotide, un substrat d'enzyme (en particulier un substrat d'enzyme suicide telle qu'une benzylguanine ou une benzylcytosine (substrats des enzymes commercialisées sous les dénominations Snaptag et Cliptag)), un chloroalcane (substrat de l'enzyme commercialisée sous la dénomination Halotag), le coenzyme A (substrat de l'enzyme commecialisée sous le nom ACPtag ou MCPtag).

## SYNTHESE

La préparation des agents complexants (ligands) et des complexes selon l'invention est décrite de manière schématique ci-après, et de manière plus détaillée dans la partie expérimentale.

Dans les schémas et la partie expérimentale qui suivent, les composés référencés 21 ; 22 ; 23 ; 39i-j ; 40i-j ; 42e ; 43e ; 44i-j ; 44o-p ; 45i-j ; 45o-p ; 46i-j ; 46o-p ; 52e-f ; 53e-f ; 54m-r ; 55m-r ; 56m-r ; 60e-f ; 61e-f ; 62e-f ; 67e-f ; 68i-l ; 69i-l ; 70i-l ; 79-91 ; 107a-b ; 108a-b ; 109a-b ; 110a ; 111a ; 112a et 121-125 sont des composés de référence qui ne font pas partie de l'invention.

### *Synthèse des macrocycles*

## Schéma 1 : Synthèse du TACN substitué

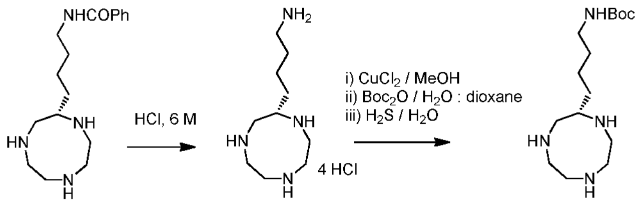


1 2 3

Le macrocycle triazacyclononane comportant un bras latéral dont l'extrémité est une fonction NHBoc protégée a été obtenu selon la séquence réactionnelle décrite dans le schéma 1. Le composé 1, obtenu en utilisant les procédures décrites par Parker et al. (J. Chem. Soc. Perkin Trans 1, 1990-2567), est hydrolysé en utilisant une solution 6 M HCl conduisant au composé 2 qui est immédiatement complexé avec des sels de cuivre. Cette approche permet de protéger sélectivement les atomes d'azotes intracycliques et de laisser libre la fonction amine primaire qui est ensuite protégée par un groupement Boc. La décomplexation des sels de cuivre est réalisée en présence de sulfure d'hydrogène conduisant ainsi au composé 3.

## Schéma 2 : Synthèse du TACN-Mono protégé

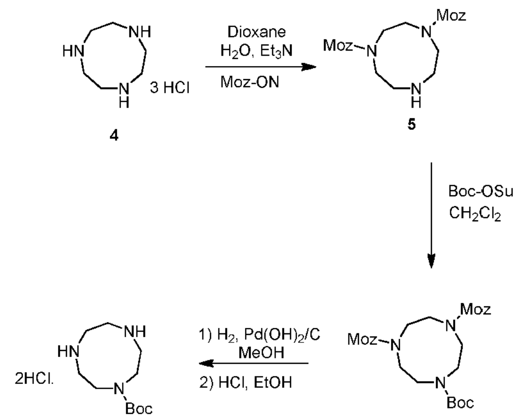


4 5 6 7a

Le triazacyclononane Boc monoprotégé **7a** n'est pas disponible commercialement. Il a été préparé selon la suite réactionnelle décrite dans le schéma 2. Deux équivalents de Moz-ON (((2-(4-méthoxybenzyloxycarbonyloxy imino)-2-phénylacétonitrile)) ont été condensés sur le macrocycle triazoté **4** pour donner le composé disubstitué avec des rendements convenables. La purification de ces produits s'est avérée délicate car le groupement Moz est sensible à l'acidité. Même celle de la silice est suffisante pour entraîner une dégradation des produits lors de la purification sur colonne de chromatographie. Pour éviter cette dégradation, les milieux réactionnels ont été purifiés sur colonne d'alumine neutre ce qui a permis de conduire au composé **5** avec un rendement de 74%. Dans l'étape suivante, le groupement Boc est introduit à l'aide du *N*-(*tert*-butoxycarbonyloxy)succinimide (Boc-OSu) pour donner le macrocycle triazoté trisubstitué 6. Enfin les amines portant les groupements Moz sont déprotégées par hydrogénolyse. L'utilisation du Pd/C à 10% comme catalyseur n'a pas permis de déprotéger les amines lorsque le milieu réactionnel est hydrogéné pendant 48 h sous 3,45 bars de pression. En revanche, l'utilisation du catalyseur de Pearlman ($Pd(OH)_2/C$) a permis l'obtention du macrocycle triazoté monoprotégé. Pour isoler facilement ce produit, les sels d'hydrochlorure ont été formés par addition d'une petite quantité d'acide chlorhydrique à froid, ce dernier étant recueilli par précipitation dans l'éther diéthylique. Cette méthodologie permet de préparer à l'échelle de plusieurs grammes le composé monoprotégé avec un groupement Boc **7a**.

*Synthèse des alcynes*

## Schéma 3 : Synthèse des alcynes O-propylénique-fonctionnalisées

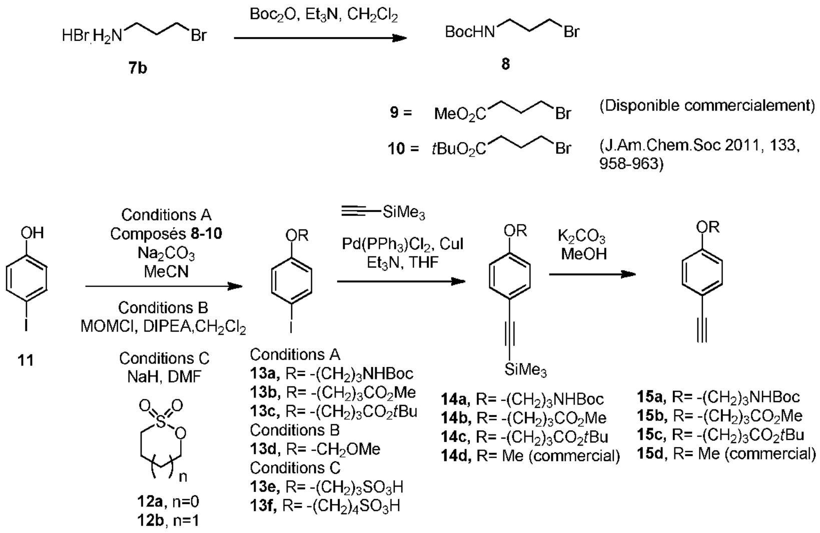


Les alcynes (vrais ou protégés par un groupement TMS) qui ne sont pas disponibles dans le commerce, ont été synthétisés en utilisant un couplage de Sonogashira avec le triméthylsilylacétylène qui a largement été décrit dans la littérature (Sonogashira et al Tetrahedron Letters, 50 (1975) 4467-4470, Rossi et al Organic Préparation and Procedure International 27 (1995) 129-160). Cette réaction permet de coupler un alcyne vrai avec un halogénure aromatique (de préférence un dérivé iodé ou bromé) ou un groupe tosyle. Leur synthèse est décrite dans les schémas 3 et 4 et détaillée dans la partie expérimentale. La protection par un groupement protecteur Boc de l'amine **7b** a été nécessaire pour éviter la polyalkylation. Le dérivé bromé **10** a été préparé en suivant les procédures décrites dans la littérature (J. Am. Chem. Soc 2011, 133, 958). Les réactions d'alkylations de l'iodophénol **11** ont conduits aux éthers phénoliques **13a-c** avec des rendements convenables. Le phénol **13d** est protégé avec du méthylchlorométhyl éther dans le dichlorométhane en présence de diisopropyléthylamine. Les composés **13e-f** ont été obtenus en effectuant une réaction entre l'iodophénol 11 et la sultone correspondante (1,3 propane sultone **12a** et 1,4-butane sultone 12b). Les couplages de Sonogashira ont été réalisés dans les conditions classiques ce qui a permis d'obtenir les alcynes triméthylsilylés **14a-c** qui ont été ensuite, suivant les besoins, déprotégés pour conduire aux alcynes vrais **15a-d.** Les alcynes **14d** et **15d** sont disponibles dans le commerce.

**Schéma 4 : Synthèse des alcynes O-$CH_2R$**

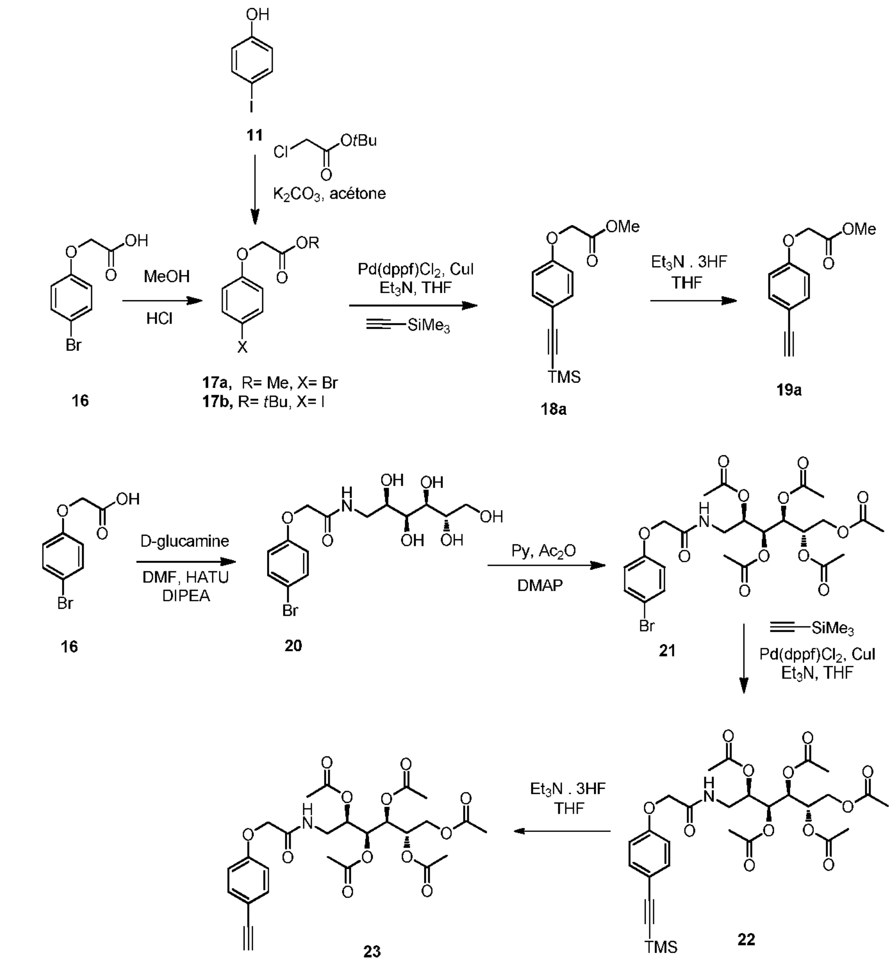


11

16

17a, R= Me, X= Br

17b, R= *t*Bu, X= I

18a

19a

16

20

21

22

23

En ce qui concerne les alcynes **19a** et **23,** ils ont été obtenus selon les suites réactionnelles décrites dans le schéma 4. Les composés **17a** et **17b** ont été respectivement obtenus par réaction d'estérification ou réaction d'alkylation à partir des précurseurs **16** et **11.** La triple liaison a été introduite par un couplage de Sonogashira comme précédemment (voir schéma 3). En ce qui concerne le composé **23**, il a été obtenu à partir de l'acide 4-bromophénoxyacétique **16** via une réaction de couplage avec la D-glucamine. Les groupements hydroxyles ont ensuite été protégés sous forme d'acétates pour diminuer la polarité de la molécule, faciliter les extractions et les purifications. L'introduction de la triple liaison puis la déprotection du TMS a conduit au composé **23**.

*Synthèse des pyridines trisubstituées*

### Schéma 5 : Synthèse de la pyridine 2-carbométhoxyester

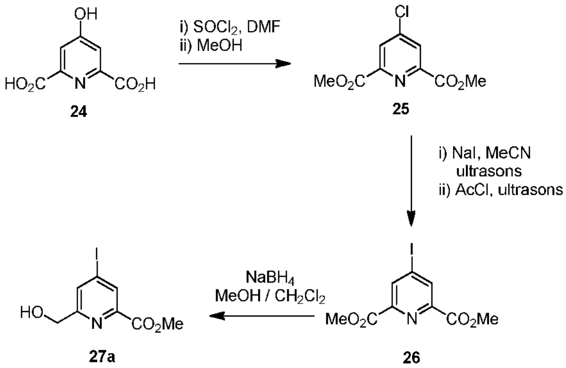


L'acide chélidamique **24** a été converti en diester chloré **25** comme décrit par Maury et al (Inorganic Chemistry 50 (2011) 4987-4999). L'échange chlore-iode a été réalisé en présence d'iodure de sodium sous ultrasons, pour conduire au composé **26**. La réduction sélective du diester **26** en présence de borohydrure de sodium à 0°C a permis d'obtenir le monoalcool monoester **27a** avec un rendement de 60%.

### Schéma 6 : Pyridine 2-phosphinoxyester

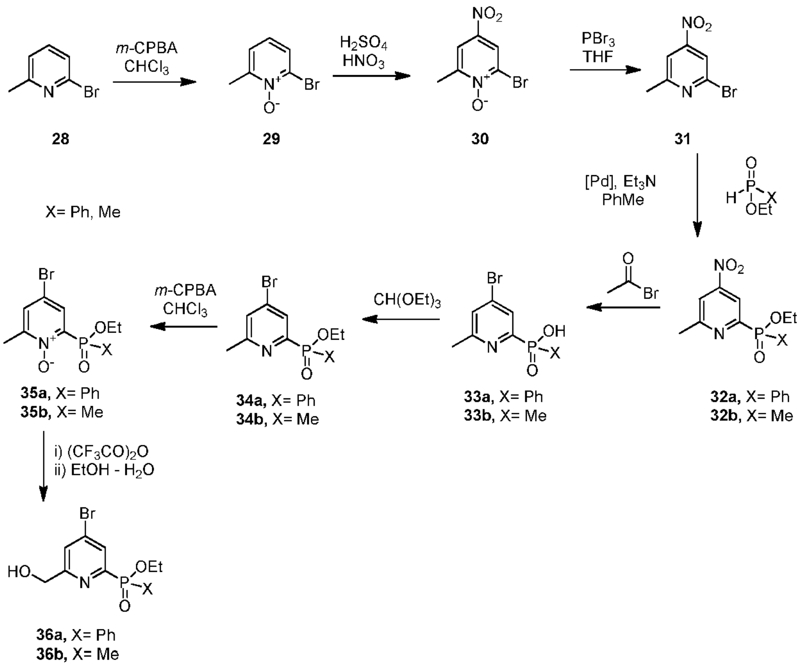


L'objectif de la synthèse est d'obtenir un dérivé de la pyridine trisubstitué par des groupes différents. Pour cela, la 2-bromo-6-méthylpyridine disponible commercialement a été oxydée en analogue N-oxyde **29** simplement par oxydation en présence d'acide métachloroperbenzoïque (m-CPBA). L'activation de cet hétérocycle azoté a permis d'introduire facilement le groupement nitro en position 4, correspondant au troisième groupement fonctionnel. Le N-oxyde a ensuite été supprimé en utilisant le tribromure de phosphore, pour conduire au dérivé pyridinyle libre. L'introduction du groupement phosphinate a été réalisée en utilisant un couplage au palladium en présence d'acide phényl phosphinique commercial. En ce qui concerne la pyridine méthylphosphinate, elle a été obtenue par couplage du dérivé éthylméthyl phosphinate, lui-même obtenu par hydrolyse du diéthylméthyl phosphonite en utilisant 1 équivalent d'eau. La mise au point de ces couplages s'est avérée particulièrement difficile mais a finalement permis d'obtenir les composés souhaités **32a** et **32b.** La substitution du groupement nitro par un atome de brome a été réalisée en présence de bromure d'acétyle. Bien que ce réactif soit efficace dans cette réaction, il engendre la formation d'acide bromhydrique qui hydrolyse le groupement phosphinate. Celui-ci a été réintroduit immédiatement après en utilisant l'orthoformiate d'éthyle. La fonctionnalisation du méthyle en position 6 de la pyridine a été réalisée en utilisant un réarrangement décrit par Ginsburg et al (Journal American Chemical Society 79 (1957) 481-485). La pyridine a été oxydée une seconde fois en utilisant

les mêmes conditions que celles décrites précédemment. Cette fonction N-oxyde est suffisamment nucléophile pour réagir avec l'anhydride trifluoroacétique qui subit intermédiairement un réarrangement permettant ainsi d'introduire un groupement trifluoroacétate en position 6 conduisant aux acétates correspondants. Ces derniers ne sont pas isolés mais directement hydrolysés pour conduire ainsi aux intermédiaires clés de la synthèse **36a** et **36b.**

***Synthèse** des chromophores*

**Schéma 7 : Synthèse des chromophores de type « carboxylate »**

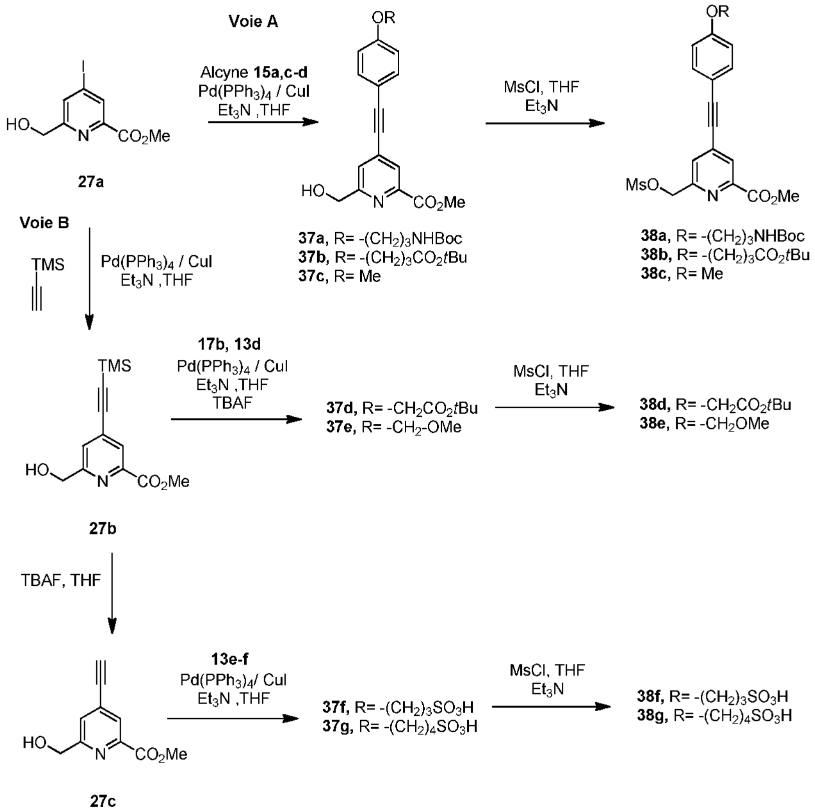


Les chromophores de type carboxylate **38a-g** ont été synthétisés selon les suites réactionnelles décrites dans le schéma 7. Pour les chromophores **38a-c** (voie A), une seule réaction de Sonogashira est mise en jeu ce qui permet d'obtenir les squelettes des chromophores en une seule étape. Pour éviter de préparer les alcynes dont les substituants sont des O-alkyl carboxylates, O-alkyl-sulfonates ou MOM, une stratégie de synthèse linéaire a été utilisée : le dérivé iodé a été couplé avec l'acétylène triméthylsilylé grâce à un couplage de Sonogashira ce qui permet d'obtenir l'intermédiaire clé **27b.** Une réaction de Sila-Sonogashira (déprotection *in situ* du groupement triméthylsilyle) a conduit aux squelettes des chromophores **37d-e** qui ont été activés comme précédemment sous forme de dérivés mésylés. Dans le cas des dérivés O-alkyle sulfonates, il s'est avéré utile de se débarrasser du TMS de **27b** car les sels de fluorure de tétrabutylammonium ont été difficiles à éliminer lors de la purification par chromatographie sur colonne de silice ou par HPLC préparative. Ainsi l'alcyne vrai **27c** a été couplé avec les différents iodophényl sulfonates (**13e-f**) pour conduire aux composés **37f-g** qui ont ensuite été mésylés de manière classique.

## Schéma 8 : Synthèse des chromophores de type « phosphinate »

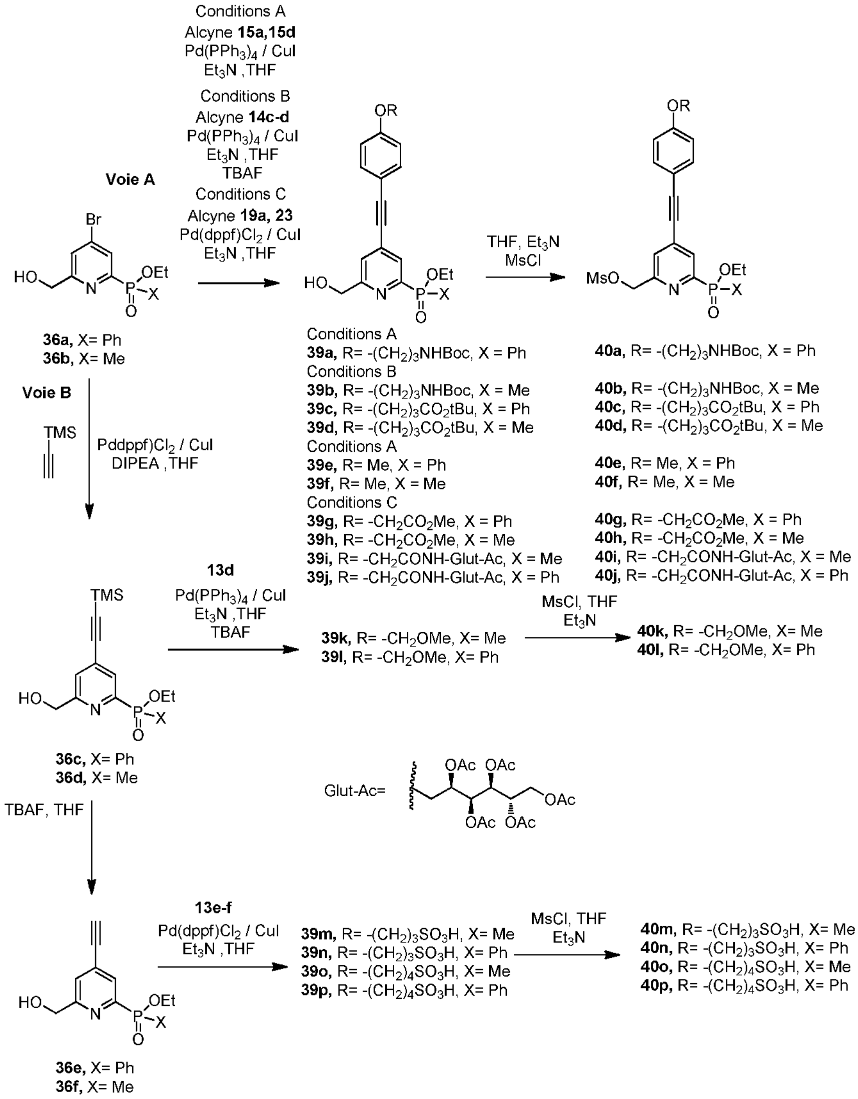


Les chromophores de type phosphinate (P-Me et P-Ph) **40a-p** ont été préparés selon les mêmes stratégies que celles décrites pour les chromophores de type carboxylate. Les séquences réactionnelles sont reportées dans le schéma 8 et les synthèses sont détaillées dans la partie expérimentale.

*Synthèse des complexes*

**Schéma 9 : Synthèse des complexes symétriques de type « carboxylate » dérivés du TACN**

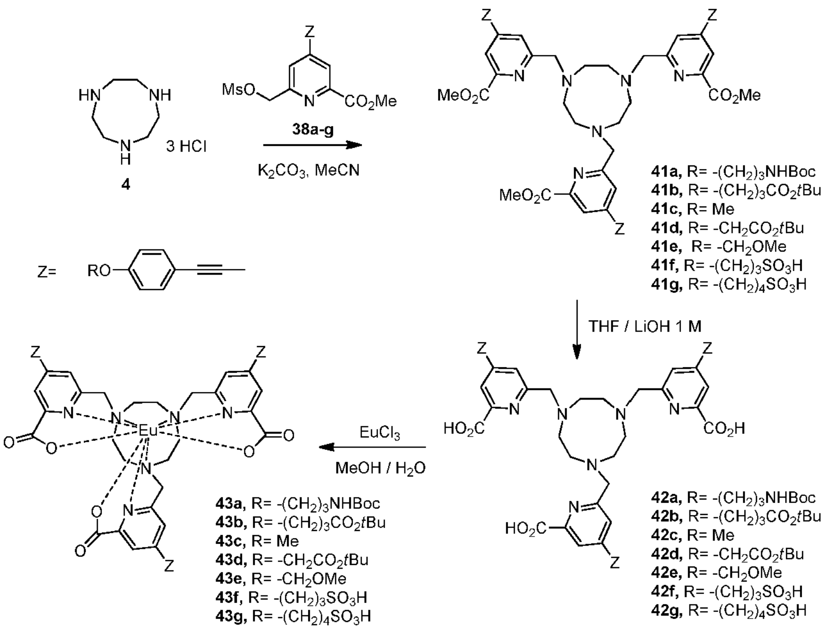


Les complexes symétriques de type carboxylate ont été synthétisés selon la suite réactionnelle décrite dans le schéma 9. La première étape a consisté à alkyler le triazacyclononane commercial avec les différents chromophores mésylés 38a-g. Les fonctions esters méthyliques ont ensuite été hydrolysées pour conduire aux carboxylates correspondants, et les produits obtenus ont été mis en contact avec les sels d'europium pour donner lieu à la formation des complexes d'europium 43a-g.

**Schéma 10 : Synthèse des complexes symétriques de type « phosphinate » dérivés du TACN**

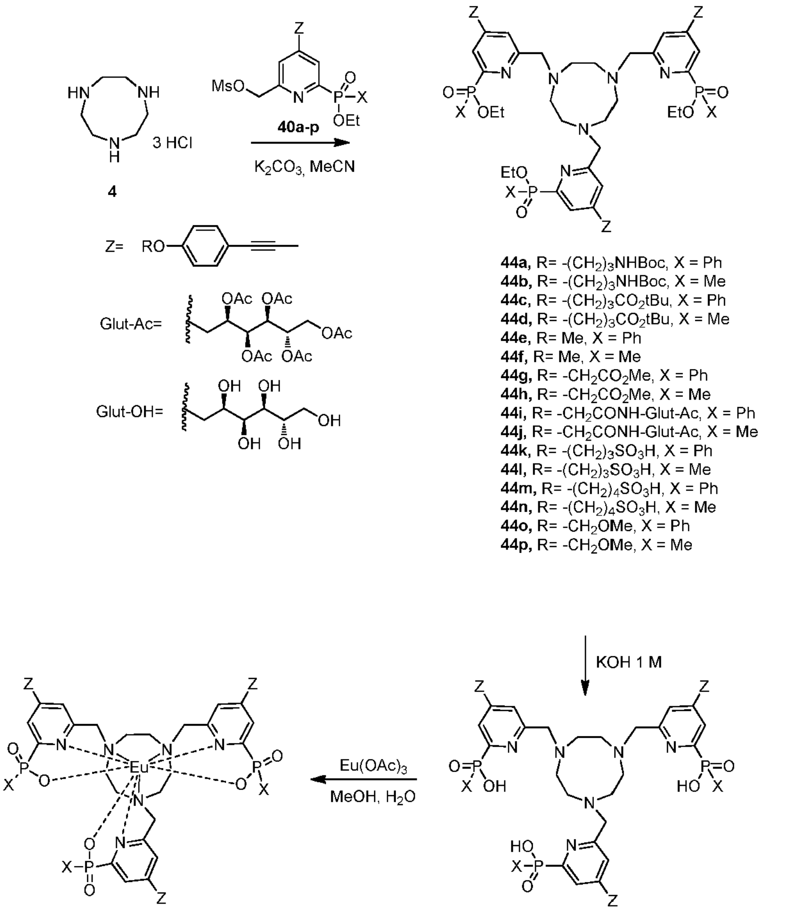


4

**44a,** R= $-(CH_2)_3NHBoc$, X = Ph
**44b,** R= $-(CH_2)_3NHBoc$, X = Me
**44c,** R= $-(CH_2)_3CO_2tBu$, X = Ph
**44d,** R= $-(CH_2)_3CO_2tBu$, X = Me
**44e,** R= Me, X = Ph
**44f,** R= Me, X = Me
**44g,** R= $-CH_2CO_2Me$, X = Ph
**44h,** R= $-CH_2CO_2Me$, X = Me
**44i,** R= $-CH_2CONH$-Glut-Ac, X = Ph
**44j,** R= $-CH_2CONH$-Glut-Ac, X = Me
**44k,** R= $-(CH_2)_3SO_3H$, X = Ph
**44l,** R= $-(CH_2)_3SO_3H$, X = Me
**44m,** R= $-(CH_2)_4SO_3H$, X = Ph
**44n,** R= $-(CH_2)_4SO_3H$, X = Me
**44o,** R= $-CH_2OMe$, X = Ph
**44p,** R= $-CH_2OMe$, X = Me

**46a,** R= $-(CH_2)_3NHBoc$, X = Ph
**46b,** R= $-(CH_2)_3NHBoc$, X = Me
**46c,** R= $-(CH_2)_3CO_2tBu$, X = Ph
**46d,** R= $-(CH_2)_3CO_2tBu$, X = Me
**46e,** R= Me, X = Ph
**46f,** R= Me, X = Me
**46g,** R= $-CH_2CO_2H$, X = Ph
**46h,** R= $-CH_2CO_2H$, X = Me
**46i,** R= $-CH_2CONH$-Glut-OH, X = Ph
**46j,** R= $-CH_2CONH$-Glut-OH, X = Me
**46k,** R= $-(CH_2)_3SO_3H$, X = Ph
**46l,** R= $-(CH_2)_3SO_3H$, X = Me
**46m,** R= $-(CH_2)_4SO_3H$, X = Ph
**46n,** R= $-(CH_2)_4SO_3H$, X = Me
**46o,** R= $-CH_2OMe$, X = Ph
**46p,** R= $-CH_2OMe$, X = Me

**45a,** R= $-(CH_2)_3NHBoc$, X = Ph
**45b,** R= $-(CH_2)_3NHBoc$, X = Me
**45c,** R= $-(CH_2)_3CO_2tBu$, X = Ph
**45d,** R= $-(CH_2)_3CO_2tBu$, X = Me
**45e,** R= Me, X = Ph
**45f,** R= Me, X = Me
**45g,** R= $-CH_2CO_2H$, X = Ph
**45h,** R= $-CH_2CO_2H$, X = Me
**45i,** R= $-CH_2CONH$-Glut-OH, X = Ph
**45j,** R= $-CH_2CONH$-Glut-OH, X = Me
**45k,** R= $-(CH_2)_3SO_3H$, X = Ph
**45l,** R= $-(CH_2)_3SO_3H$, X = Me
**45m,** R= $-(CH_2)_4SO_3H$, X = Ph
**45n,** R= $-(CH_2)_4SO_3H$, X = Me
**45o,** R= $-CH_2OMe$, X = Ph
**45p,** R= $-CH_2OMe$, X = Me

Les complexes symétriques de type phosphinates ont été synthétisés selon la suite réactionnelle décrite dans le schéma 10. La première étape a consisté à alkyler le triazacyclononane avec les différents chromophores mésylés **40a-p**. Les esters phosphinates ont ensuite été hydrolysés pour conduire aux acide phosphiniques correspondants et les produits obtenus ont été mis en contact sans purification préalable avec les sels d'europium, donnant lieu à la formation des complexes d'europium **46a-p**.

**Schéma 11 : Synthèse des complexes dissymétriques de type « carboxylate » dérivés du TACN mono-protégé**

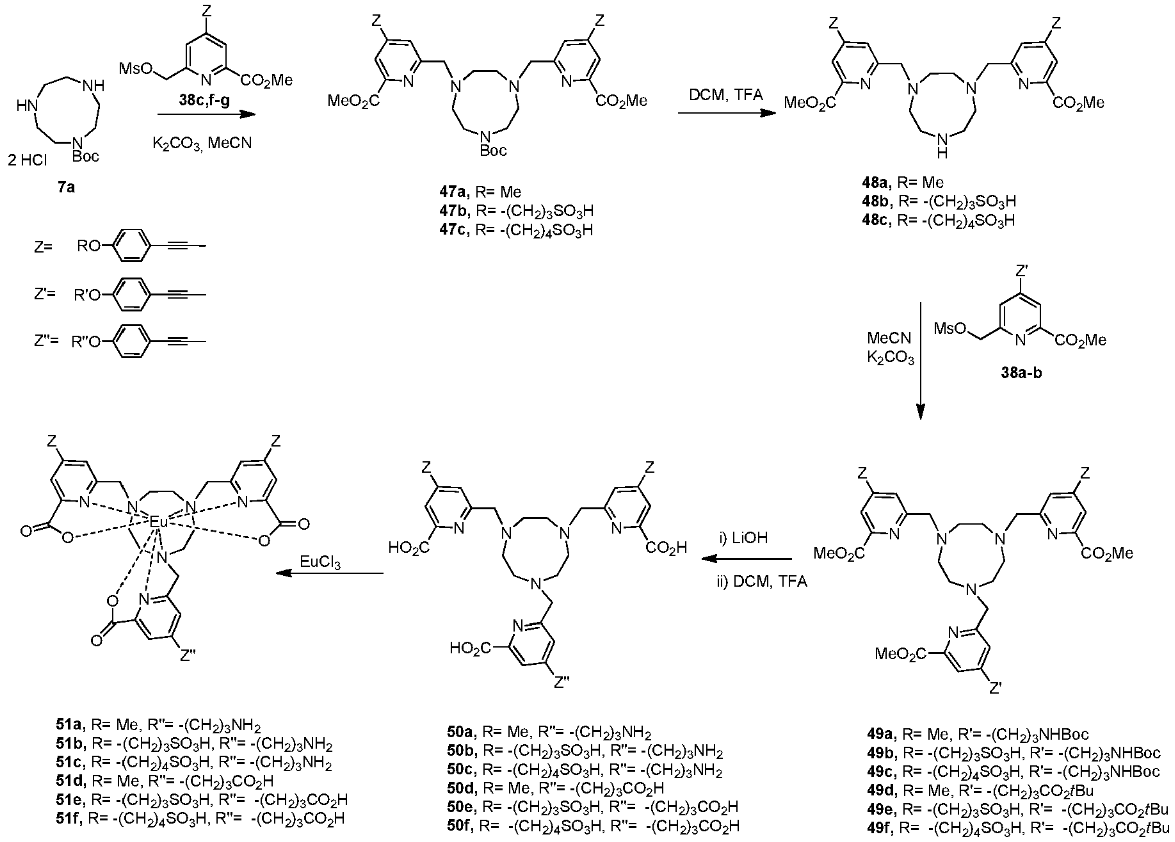


Les complexes d'europium ont été préparés selon les schémas 11 et 12. A partir du macrocycle mono-substitué, deux unités « chromophores carboxylates » ou « phosphinates » ont été condensées pour conduire aux dérivés **47a-c** ou **52a-f.** Le groupement protecteur Boc a été éliminé en présence d'acide trifluoroacétique, et le troisième chromophore comportant un groupement amino ou carboxyle masqué a été alkylé pour permettre la conjugaison ultérieure à une biomolécule. Les trois fonctions esters méthyliques (carboxylates) ou éthyliques (phosphinates) ont ensuite été hydrolysées et le groupement Boc ou tBu éliminé en milieu acide. La formation du complexe de lanthanide a été réalisée en faisant réagir les ligands 50 et 55 avec les sels de lanthanides correspondants et en l'occurrence le chlorure ou l'acétate d'europium, pour conduire aux complexes **51** et **56**.

**Schéma 12 : Synthèse des complexes dissymétriques de type « phosphinate » dérivés du TACN mono-protégé**

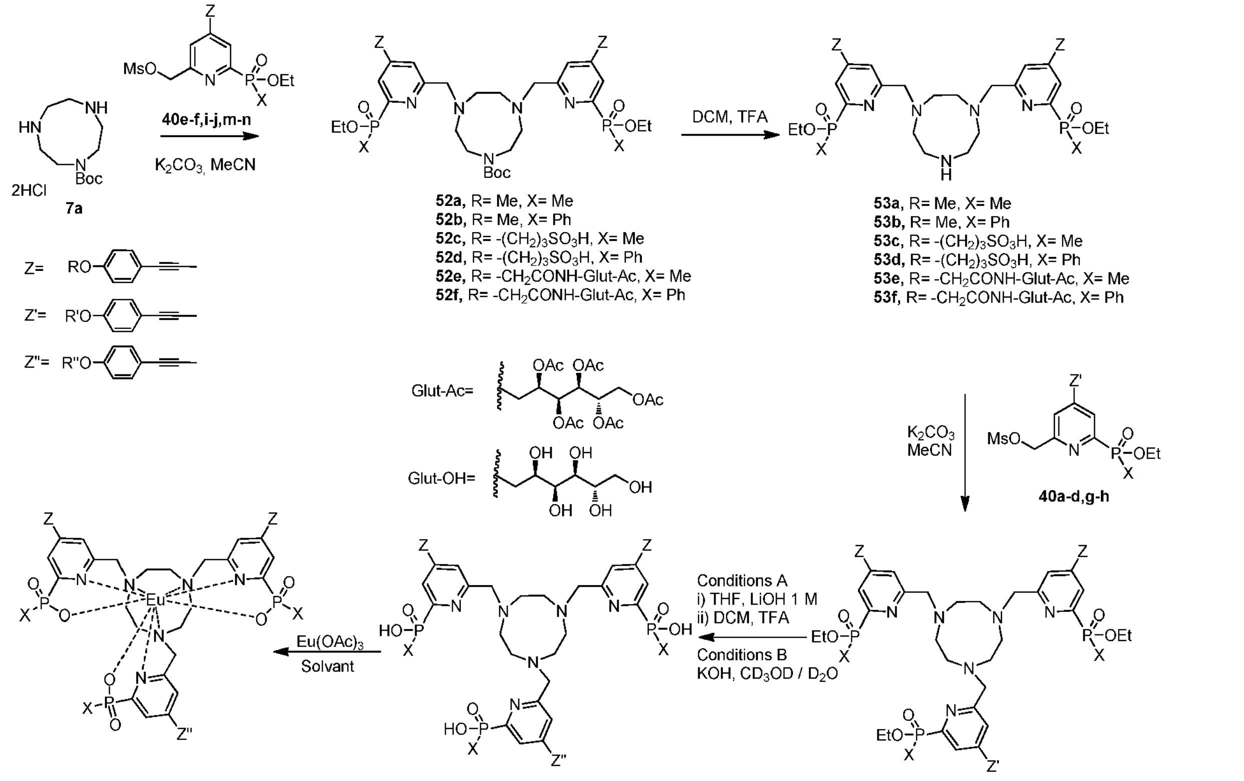


**52a,** R= Me, X= Me
**52b,** R= Me, X= Ph
**52c,** R= $-(CH_2)_3SO_3H$, X= Me
**52d,** R= $-(CH_2)_3SO_3H$, X= Ph
**52e,** R= $-CH_2CONH$-Glut-Ac, X= Me
**52f,** R= $-CH_2CONH$-Glut-Ac, X= Ph

**53a,** R= Me, X= Me
**53b,** R= Me, X= Ph
**53c,** R= $-(CH_2)_3SO_3H$, X= Me
**53d,** R= $-(CH_2)_3SO_3H$, X= Ph
**53e,** R= $-CH_2CONH$-Glut-Ac, X= Me
**53f,** R= $-CH_2CONH$-Glut-Ac, X= Ph

**56a,** R= Me, X= Me, R"= $-(CH_2)_3-NH_2$
**56b,** R= Me, X= Ph, R"= $-(CH_2)_3-NH_2$
**56c,** R= Me, X= Me, R"= $-(CH_2)_3-CO_2H$
**56d,** R= Me, X= Ph, R"= $-(CH_2)_3-CO_2H$

**56e,** R= Me, X= Me, R"= $-CH_2-CO_2H$
**56f,** R= Me, X= Ph, R"= $-CH_2-CO_2H$

**56g,** R= $-(CH_2)_3SO_3H$, X= Me, R"= $-(CH_2)_3-NH_2$
**56h,** R= $-(CH_2)_3SO_3H$, X= Ph, R"= $-(CH_2)_3-NH_2$
**56i,** R= $-(CH_2)_3SO_3H$, X= Me, R"= $-(CH_2)_3-CO_2H$
**56j,** R= $-(CH_2)_3SO_3H$, X= Ph, R"= $-(CH_2)_3-CO_2H$

**56k,** R= $-(CH_2)_3SO_3H$, X= Me, R"= $-CH_2-CO_2H$
**56l,** R= $-(CH_2)_3SO_3H$, X= Ph, R"= $-CH_2-CO_2H$

**56m,** R= $-CH_2CONH$-Glut-OH, X= Me, R"= $-(CH_2)_3-NH_2$
**56n,** R= $-CH_2CONH$-Glut-OH, X= Ph, R"= $-(CH_2)_3-NH_2$
**56o,** R= $-CH_2CONH$-Glut-OH, X= Me, R'"= $-(CH_2)_3-CO_2H$
**56p,** R= $-CH_2CONH$-Glut-OH, X= Ph, R'= $-(CH_2)_3-CO_2H$

**56q,** R= $-CH_2CONH$-Glut-OH, X= Me,R"= $-CH_2-CO_2H$
**56r,** R= $-CH_2CONH$-Glut-OH, X= Ph, R"= $-CH_2-CO_2H$

Conditions A
**55a,** R= Me, X= Me, R"= $-(CH_2)_3-NH_2$
**55b,** R= Me, X= Ph, R"= $-(CH_2)_3-NH_2$
**55c,** R= Me, X= Me, R"= $-(CH_2)_3-CO_2H$
**55d,** R= Me, X= Ph, R"= $-(CH_2)_3-CO_2H$
Conditions B
**55e,** R= Me, X= Me, R"= $-CH_2-CO_2H$
**55f,** R= Me, X= Ph, R"= $-CH_2-CO_2H$
Conditions A
**55g,** R= $-(CH_2)_3SO_3H$, X= Me, R"= $-(CH_2)_3-NH_2$
**55h,** R= $-(CH_2)_3SO_3H$, X= Ph, R"= $-(CH_2)_3-NH_2$
**55i,** R= $-(CH_2)_3SO_3H$, X= Me, R"= $-(CH_2)_3-CO_2H$
**55j,** R= $-(CH_2)_3SO_3H$, X= Ph, R"= $-(CH_2)_3-CO_2H$
Conditions B
**55k,** R= $-(CH_2)_3SO_3H$, X= Me, R"= $-CH_2-CO_2H$
**55l,** R= $-(CH_2)_3SO_3H$, X= Ph, R"= $-CH_2-CO_2H$
Conditions A
**55m,** R= $-CH_2CONH$-Glut-OH, X= Me, R"= $-(CH_2)_3-NH_2$
**55n,** R= $-CH_2CONH$-Glut-OH, X= Ph, R"= $-(CH_2)_3-NH_2$
**55o,** R= $-CH_2CONH$-Glut-OH, X= Me, R"= $-(CH_2)_3-CO_2H$
**55p,** R= $-CH_2CONH$-Glut-OH, X= Ph, R"= $-(CH_2)_3-CO_2H$
Conditions B
**55q,** R= $-CH_2CONH$-Glut-OH, X= Me,R"= $-CH_2-CO_2H$
**55r,** R= $-CH_2CONH$-Glut-OH, X= Ph, R"= $-CH_2-CO_2H$

**54a,** R= Me, X= Me, R'= $-(CH_2)_3$-NHBoc
**54b,** R= Me, X= Ph, R'= $-(CH_2)_3$-NHBoc
**54c,** R= Me, X= Me, R'= $-(CH_2)_3-CO_2tBu$
**54d,** R= Me, X= Ph, R'= $-(CH_2)_3-CO_2tBu$

**54e,** R= Me, X= Me, R'= $-CH_2-CO_2Me$
**54f,** R= Me, X= Ph, R'= $-CH_2-CO_2Me$

**54g,** R= $-(CH_2)_3SO_3H$, X= Me, R'= $-(CH_2)_3$-NHBoc
**54h,** R= $-(CH_2)_3SO_3H$, X= Ph, R'= $-(CH_2)_3$-NHBoc
**54i,** R= $-(CH_2)_3SO_3H$, X= Me, R'= $-(CH_2)_3-CO_2tBu$
**54j,** R= $-(CH_2)_3SO_3H$, X= Ph, R'= $-(CH_2)_3-CO_2tBu$

**54k,** R= $-(CH_2)_3SO_3H$, X= Me, R'= $-CH_2-CO_2Me$
**54l,** R= $-(CH_2)_3SO_3H$, X= Ph, R'= $-CH_2-CO_2Me$

**54m,** R= $-CH_2CONH$-Glut-Ac, X= Me, R'= $-(CH_2)_3$-NHBoc
**54n,** R= $-CH_2CONH$-Glut-Ac, X= Ph, R'= $-(CH_2)_3$-NHBoc
**54o,** R= $-CH_2CONH$-Glut-Ac, X= Me, R'= $-(CH_2)_3-CO_2tBu$
**54p,** R= $-CH_2CONH$-Glut-Ac, X= Ph, R'= $-(CH_2)_3-CO_2tBu$

**54q,** R= $-CH_2CONH$-Glut-Ac, X= Me,R'= $-CH_2-CO_2Me$
**54r,** R= $-CH_2CONH$-Glut-Ac, X= Ph, R'= $-CH_2-CO_2Me$

**Schéma 13 : Synthèse des complexes symétriques de type « carboxylate » dérivés du TACN C-substitué**

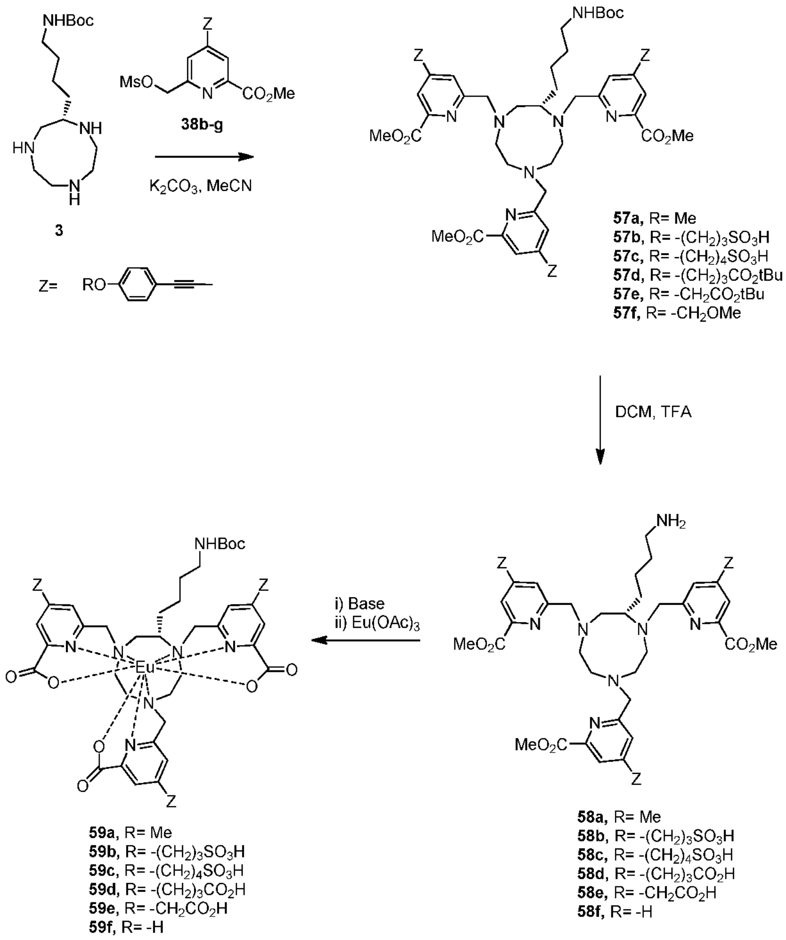


Les synthèses des complexes symétriques comportant la chaîne Boc alkyle sur le macrocycle ont été décrites dans les schémas 13 (version carboxylate) et 14 (version phosphinate). Cette fois le groupe fonctionnel permettant la bioconjugaison est placé sur le macrocycle. Ce nouveau système rend la synthèse plus aisée dans la mesure où les trois chromophores peuvent être identiques. Comme décrit dans la synthèse des complexes symétriques carboxylates ou phosphinates, le triazacyclononane **3** a été alkylé par les différents chromophores **38** et **40**, puis les fonctions esters (carboxylates et phosphinates) ont été hydrolysées. Enfin la complexation avec les sels d'europium a conduit aux complexes **59a-f** et **62a-l**.

**Schéma 14 : Synthèse des complexes symétriques de type « phosphinate » dérivés du TACN C-substitué**

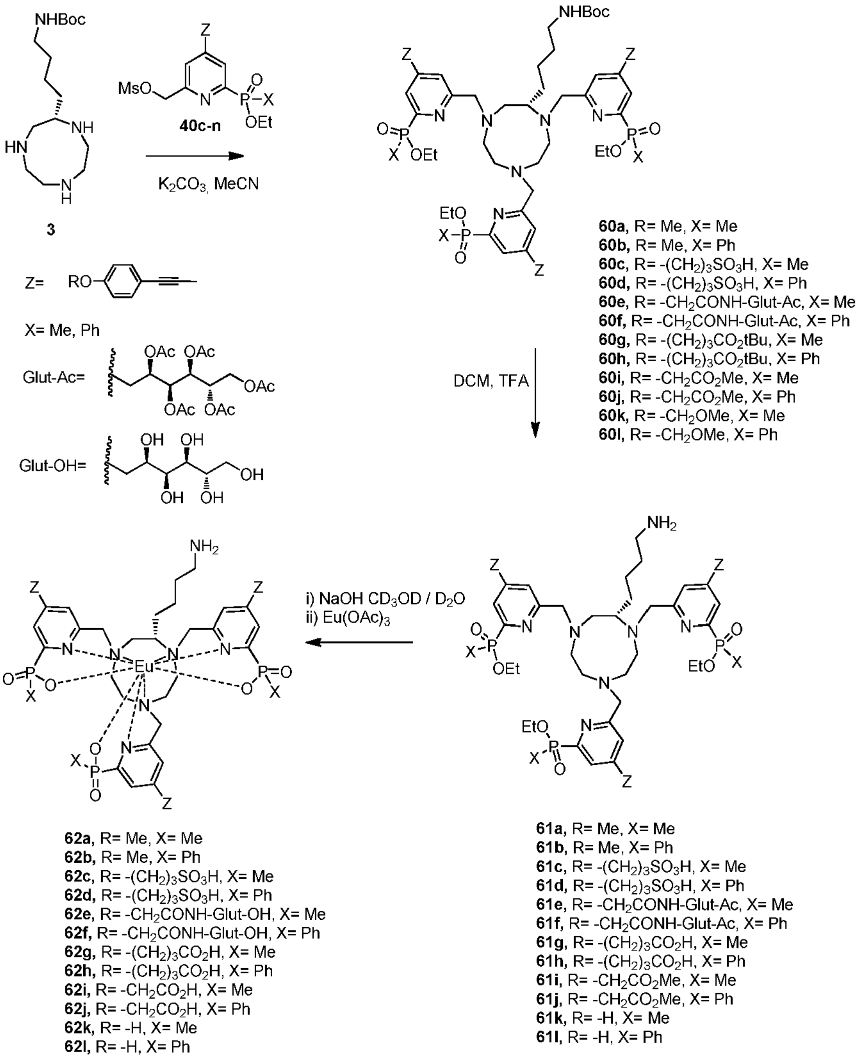

**Schéma 15 : Synthèse des complexes dissymétriques de type « carboxylate » dérivés du TACN C-substitué**

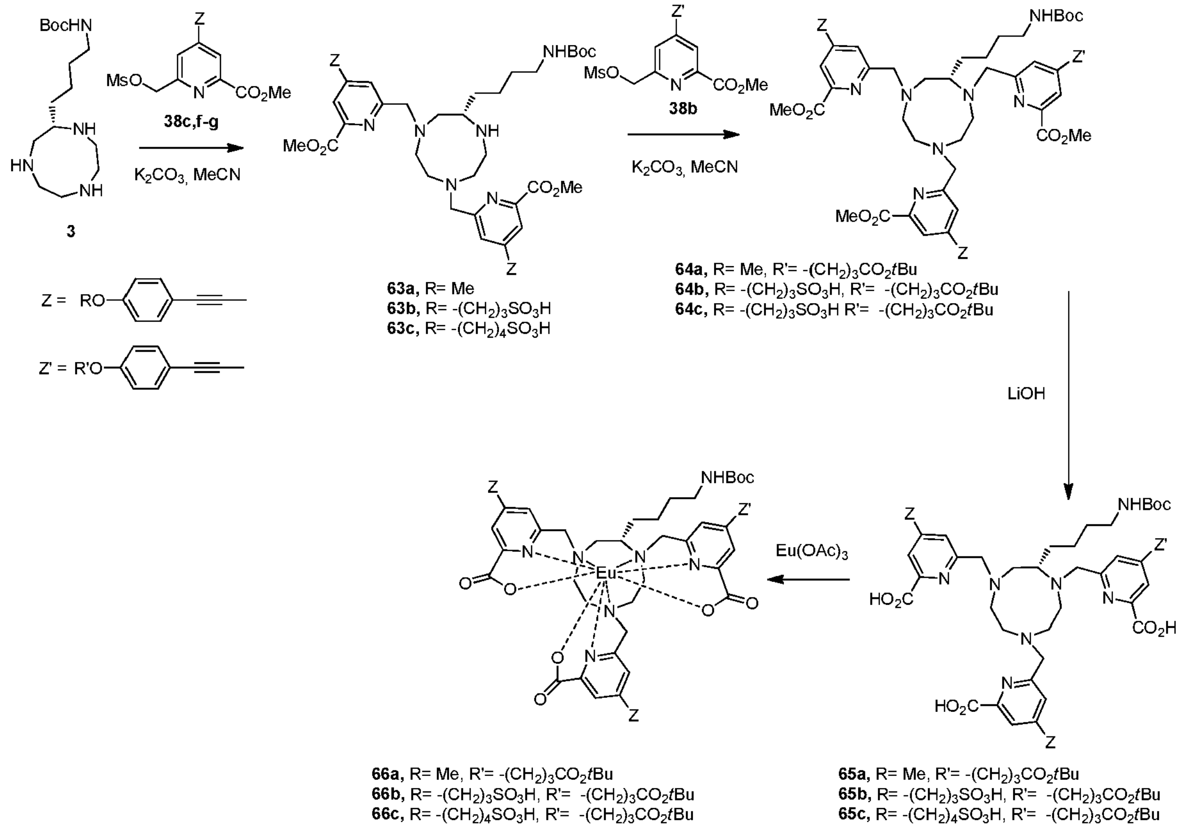


3

Z = RO–

Z' = R'O–

**63a,** R= Me
**63b,** R= $-(CH_2)_3SO_3H$
**63c,** R= $-(CH_2)_4SO_3H$

**64a,** R= Me, R'= $-(CH_2)_3CO_2\mathit{t}Bu$
**64b,** R= $-(CH_2)_3SO_3H$, R'= $-(CH_2)_3CO_2\mathit{t}Bu$
**64c,** R= $-(CH_2)_3SO_3H$ R'= $-(CH_2)_3CO_2\mathit{t}Bu$

**66a,** R= Me, R'= $-(CH_2)_3CO_2\mathit{t}Bu$
**66b,** R= $-(CH_2)_3SO_3H$, R'= $-(CH_2)_3CO_2\mathit{t}Bu$
**66c,** R= $-(CH_2)_4SO_3H$, R'= $-(CH_2)_3CO_2\mathit{t}Bu$

**65a,** R= Me, R'= $-(CH_2)_3CO_2\mathit{t}Bu$
**65b,** R= $-(CH_2)_3SO_3H$, R'= $-(CH_2)_3CO_2\mathit{t}Bu$
**65c,** R= $-(CH_2)_4SO_3H$, R'= $-(CH_2)_3CO_2\mathit{t}Bu$

L'encombrement stérique (présence d'un méthylène) autour de l'atome d'azote en position alpha du substituant du cycle triazacyclononane **3** diminue la réactivité de cet atome. Ainsi lorsque le triazacyclononane **3** est alkylé dans les conditions classiques ($K_2CO_3$, MeCN), les deux premiers atomes d'azote sont alkylés en moins de 24h. En revanche la troisième substitution est cinétiquement plus longue ce qui permet, d'un point de vue pratique, d'isoler par purification les composés disubstitués **63a-c** et **67a-f.** Ce résultat permet d'obtenir des macrocycles portant deux types de chromophores (deux chromophores identiques et un troisième différent) sans utiliser de groupements protecteurs orthogonaux comme cela a été décrit dans les expériences précédentes (schémas 11 et 12). Le choix des troisièmes chromophores s'est porté sur des substituants dont la déprotection est orthogonale au groupement Boc. Les troisièmes chromophores ont été introduits en présence de $K_2CO_3$ dans l'acétonitrile ce qui a conduit après 48h aux composés trialkylés. La suite réactionnelle est identique à ce qui a été décrit dans les expériences précédentes, à savoir hydrolyse des fonctions ester (carboxylate ou phosphinate) puis traitement du ligand par des sels d'europium conduisant aux différents complexes **66a-c** et **70a-l** (schémas 15 et 16).

**Schéma 16 : Synthèse des complexes dissymétriques de type « phosphinate » dérivés du TACN C-substitué**

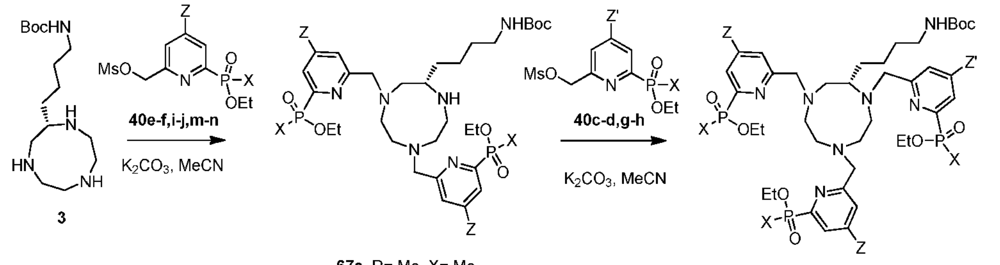


3

67a, R= Me, X= Me
67b, R= Me, X= Ph
67c, R= $-(CH_2)_3SO_3H$, X= Me
67d, R= $-(CH_2)_3SO_3H$, X= Ph
67e, R= $-CH_2CONH$-Glut-Ac, X= Me
67f, R= $-CH_2CONH$-Glut-Ac, X= Ph

68a, R= Me, X= Me, R'= $-(CH_2)_3-CO_2tBu$
68b, R= Me, X= Ph, R'= $-(CH_2)_3-CO_2tBu$
68c, R= Me, X= Me, R'= $-CH_2-CO_2Me$
68d, R= Me, X= Ph, R'= $-CH_2-CO_2Me$
68e, R= $-(CH_2)_3SO_3H$, X= Me, R'= $-(CH_2)_3-CO_2tBu$
68f, R= $-(CH_2)_3SO_3H$, X= Ph, R'= $-(CH_2)_3-CO_2tBu$
68g, R= $-(CH_2)_3SO_3H$, X= Me, R'= $-CH_2-CO_2Me$
68h, R= $-(CH_2)_3SO_3H$, X= Ph, R'= $-CH_2-CO_2Me$
68i, R= $-CH_2CONH$-Glut-Ac, X= Me, R'= $-(CH_2)_3-CO_2tBu$
68j, R= $-CH_2CONH$-Glut-Ac, X= Ph, R'= $-(CH_2)_3-CO_2tBu$
68k, R= $-CH_2CONH$-Glut-Ac, X= Me,R'= $-CH_2-CO_2Me$
68l, R= $-CH_2CONH$-Glut-Ac, X= Ph, R'= $-CH_2-CO_2Me$

X= Me, Ph

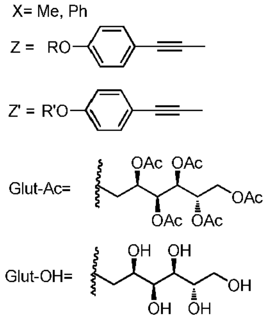


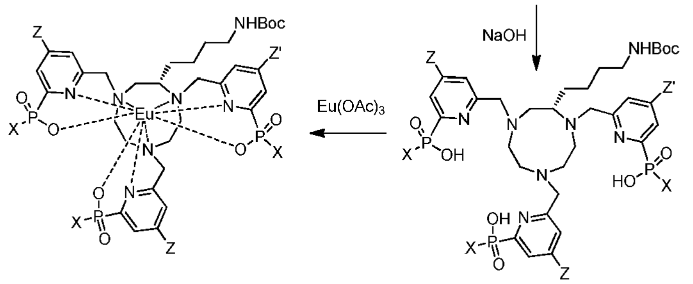


70a, R= Me, X= Me, R'= $-(CH_2)_3-CO_2tBu$
70b, R= Me, X= Ph, R'= $-(CH_2)_3-CO_2tBu$
70c, R= Me, X= Me, R'= $-CH_2-CO_2H$
70d, R= Me, X= Ph, R'= $-CH_2-CO_2H$
70e, R= $-(CH_2)_3SO_3H$, X= Me, R'= $-(CH_2)_3-CO_2tBu$
70f, R= $-(CH_2)_3SO_3H$, X= Ph, R'= $-(CH_2)_3-CO_2tBu$
70g, R= $-(CH_2)_3SO_3H$, X= Me, R'= $-CH_2-CO_2H$
70h, R= $-(CH_2)_3SO_3H$, X= Ph, R'= $-CH_2-CO_2H$
70i, R= $-CH_2CONH$-Glut-OH, X= Me, R'= $-(CH_2)_3-CO_2tBu$
70j, R= $-CH_2CONH$-Glut-OH, X= Ph, R'= $-(CH_2)_3-CO_2tBu$
70k, R= $-CH_2CONH$-Glut-OH, X= Me,R'= $-CH_2-CO_2H$
70l, R= $-CH_2CONH$-Glut-OH, X= Ph, R'= $-CH_2-CO_2H$

69a, R= Me, X= Me, R'= $-(CH_2)_3-CO_2tBu$
69b, R= Me, X= Ph, R'= $-(CH_2)_3-CO_2tBu$
69c, R= Me, X= Me, R'= $-CH_2-CO_2H$
69d, R= Me, X= Ph, R'= $-CH_2-CO_2H$
69e, R= $-(CH_2)_3SO_3H$, X= Me, R'= $-(CH_2)_3-CO_2tBu$
69f, R= $-(CH_2)_3SO_3H$, X= Ph, R'= $-(CH_2)_3-CO_2tBu$
69g, R= $-(CH_2)_3SO_3H$, X= Me, R'= $-CH_2-CO_2H$
69h, R= $-(CH_2)_3SO_3H$, X= Ph, R'= $-CH_2-CO_2H$
69i, R= $-CH_2CONH$-Glut-OH, X= Me, R'= $-(CH_2)_3-CO_2tBu$
69j, R= $-CH_2CONH$-Glut-OH, X= Ph, R'= $-(CH_2)_3-CO_2tBu$
69k, R= $-CH_2CONH$-Glut-OH, X= Me,R'= $-CH_2-CO_2H$
69l, R= $-CH_2CONH$-Glut-OH, X= Ph, R'= $-CH_2-CO_2H$

***Caractéristiques des complexes d'europium :***

Les caractéristiques des différents complexes d'europium préparés ci-dessus sont spécifiées dans le tableau 1 suivant :

**Tableau 1**

| Comp. | Possède un ou plusieurs groupes solubilisants | Le complexe hydrosoluble possède un groupe (amine ou COOH) permettant la conjugaison avec une autre molécule | Pour améliorer la solubilité, doit être modifié par introduction de groupes solubilisant via un groupe amine, COOH (après activation) ou phénol | Peut-être conjugué avec une autre molécule, par introduction d'un bras portant un groupe solubilisant ET un groupe réactionnel, via un groupe amine, COOH (après activation) |
|---|---|---|---|---|
| **43a** | - | - | + | - |
| **43b** | + | - | - | - |
| **43c** | - | - | - | - |
| **43d** | + | - | - | - |

(suite)

| Comp. | Possède un ou plusieurs groupes solubilisants | Le complexe hydrosoluble possède un groupe (amine ou COOH) permettant la conjugaison avec une autre molécule | Pour améliorer la solubilité, doit être modifié par introduction de groupes solubilisant via un groupe amine, COOH (après activation) ou phénol | Peut-être conjugué avec une autre molécule, par introduction d'un bras portant un groupe solubilisant ET un groupe réactionnel, via un groupe amine, COOH (après activation) |
|---|---|---|---|---|
| **43e** | - | - | + | - |
| **43f** | + | - | - | - |
| **43g** | + | - | - | - |
| **51a** | - | - | + | + |
| **51b** | + | + | - | - |
| **51c** | + | + | - | - |
| **51d** | + | - | + | + |
| **51e** | + | + | - | - |
| **51f** | + | + | - | - |
| **59a** | - | - | + | + |
| **59b** | + | + | - | - |
| **59c** | + | + | - | - |
| **59d** | + | + | - | - |
| **59e** | + | + | | |
| **59f** | - | + | + | - |
| **66a** | + | + | - | - |
| **66b** | + | + | - | - |
| **66c** | + | + | - | - |
| **46a** | - | - | + | - |
| **46b** | - | - | + | - |
| **46c** | + | - | - | - |
| **46d** | + | - | - | - |
| **46e** | - | - | - | - |
| **46f** | - | - | - | - |
| **46g** | + | - | - | - |
| **46h** | + | - | - | - |
| **46i** | + | - | - | - |
| **46j** | + | - | - | - |
| **46k** | + | - | - | - |
| **461** | + | - | - | - |
| **46m** | + | - | - | - |
| **46n** | + | - | - | - |
| **46o** | - | - | + | - |
| **46p** | - | - | + | - |

(suite)

| Comp. | Possède un ou plusieurs groupes solubilisants | Le complexe hydrosoluble possède un groupe (amine ou COOH) permettant la conjugaison avec une autre molécule | Pour améliorer la solubilité, doit être modifié par introduction de groupes solubilisant via un groupe amine, COOH (après activation) ou phénol | Peut-être conjugué avec une autre molécule, par introduction d'un bras portant un groupe solubilisant ET un groupe réactionnel, via un groupe amine, COOH (après activation) |
|---|---|---|---|---|
| **56a** | - | - | + | + |
| **56b** | - | - | + | + |
| **56c** | + | - | - | + |
| **56d** | + | - | - | + |
| **56e** | + | - | - | + |
| **56f** | + | - | - | + |
| **56g** | + | + | - | - |
| **56h** | + | + | - | - |
| **56i** | + | + | - | - |
| **56j** | + | + | - | - |
| **56k** | + | + | - | - |
| **56l** | + | + | - | - |
| **56m** | + | + | - | - |
| **56n** | + | + | - | - |
| **56o** | + | + | - | - |
| **56p** | + | + | - | - |
| **56q** | + | + | - | - |
| **56r** | + | + | - | - |
| **62a** | - | - | + | + |
| **62b** | - | - | + | + |
| **62c** | + | + | - | - |
| **62d** | + | + | - | - |
| **62e** | + | + | - | - |
| **62f** | + | + | - | - |
| **62g** | + | + | - | - |
| **62h** | + | + | - | - |
| **62i** | + | + | - | - |
| **62j** | + | + | - | - |
| **62k** | - | + | + | - |
| **621** | - | + | + | - |
| **70a** | + | + | - | - |
| **70b** | + | + | - | - |
| **70c** | + | + | - | - |
| **70d** | + | + | - | - |

(suite)

| Comp. | Possède un ou plusieurs groupes solubilisants | Le complexe hydrosoluble possède un groupe (amine ou COOH) permettant la conjugaison avec une autre molécule | Pour améliorer la solubilité, doit être modifié par introduction de groupes solubilisant via un groupe amine, COOH (après activation) ou phénol | Peut-être conjugué avec une autre molécule, par introduction d'un bras portant un groupe solubilisant ET un groupe réactionnel, via un groupe amine, COOH (après activation) |
|---|---|---|---|---|
| **70e** | + | + | - | - |
| **70f** | + | + | - | - |
| **70g** | + | + | - | - |
| **70h** | + | + | - | - |
| **70i** | + | + | - | - |
| **70j** | + | + | - | - |
| **70k** | + | + | - | - |
| **70l** | + | + | - | - |

- Les composés satisfaisant (+) au critère de la première colonne sont des composés selon l'invention comportant des groupes augmentant leur hydrosolubilité. Ils ne peuvent pas être conjugués à une autre molécule mais peuvent être utilisés comme composés fluorescents classiques.
- Les composés satisfaisant (+) au critère de la deuxième colonne possèdent un groupe réactif permettant la conjugaison du complexe d'europium avec une molécule que l'on souhaite marquer. Dans les exemples de cette table, ces groupes réactifs sont des groupes amine, protégées ou pas, ou des groupes carboxylates, protégés ou pas, ces derniers devant être activés, par exemple sous forme d'ester de N-hydrosuccinimide. L'homme du métier peut mettre en oeuvre les techniques classiques de chimie pour utiliser ces groupes amines ou carboxylates pour conjuguer ces complexes avec une molécule d'intérêt. Lorsque le groupe réactif est une amine, des réactifs de couplage peuvent être utilisés, comme par exemple le DCC (dicyclohexylcarbodiimide) et le NHS (N-Hydroxysuccinimide), pour former une espèce capable de réagir avec une amine présente sur une molécule que l'on souhaite marquer. D'autres réactifs connus de l'homme du métier peuvent être utilisés, comme le TSTU (O-(N-succinimidyl)-1,1,3,3-tetramethyluronium tetrafluoroborate), le HATU ou encore le TBTU
- Les composés satisfaisant (+) au critère de la troisième colonne ne sont pas des composés selon l'invention mais des intermédiaires conduisant à ces composés : ils possèdent en effet des groupes réactifs carboxylates (qui doivent être activés, par ex. sous forme d'esters de NHS), amines ou phénols (chacun éventuellement protégés) permettant l'introduction de groupes qui augmenteront l'hydrosolubilité des complexes concernés. Après déprotection des groupements Boc, $CO_2tBu$ ou MOM, l'homme du métier peut utiliser les techniques classiques pour obtenir les composés selon l'invention à partir de ces complexes d'europium. En particulier, lorsque les groupes réactionnels sont des amines, il pourra les faire réagir par exemple avec les composés électrophiles suivants, comportant une fonction ester de NHS et un groupe augmentant le caractère hydrophile (ammonium quaternaire, phosphonate ou sulfonate) :

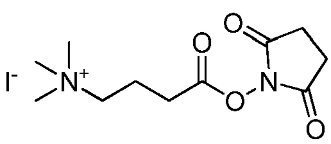 ; 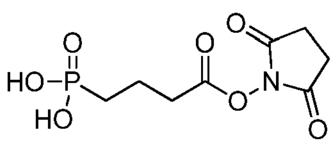 ; 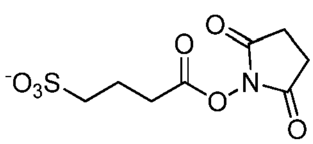

La synthèse du premier composé est décrite dans Analytical Chemistry, 2006, 78, 4175-4183, celle du second composé est décrite dans European Journal of Chemistry, 2001, 349-352, et celle du troisième composé est décrite dans la demande de brevet WO 02/095412.

Lorsque les groupes réactionnels sont des carboxylates activés sous forme d'ester de NHS, les groupes nucléophiles suivants peuvent par exemple être utilisés pour introduire les groupes conférant une meilleure solubilité :

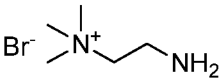 ; 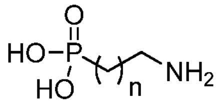

n=0,1,2 ; 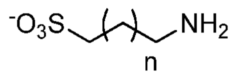 n= 1, 2

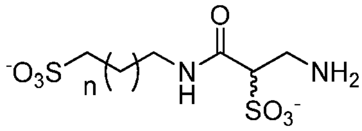

n= 0,1

Le premier réactif est disponible dans le commerce (Toronto Research Chemicals), comme le second et le troisième (Sigma). Le quatrième a été synthétisé selon le schéma suivant en condensant l'ester de NHS préparé selon les procédures décrites dans Bioconjugate Chemistry, 2008, 19, 279-289, sur la taurine (n = 0) ou l'homotaurine (n= 1). Ces derniers composés ont été ensuite déprotégés (groupement Fmoc) dans les conditions classiques connues de l'homme du métier.

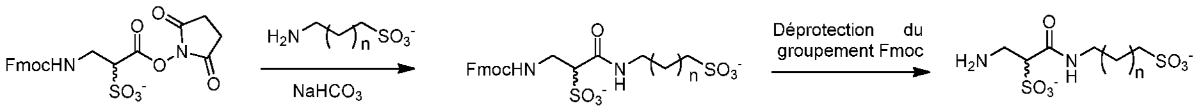


Enfin lorsque le groupe réactionnel est un phénol ou une amine primaire, secondaire ou tertiaire, un groupe sulfonate peut être introduit sur le complexe par réaction avec une sultone de formule suivante (disponible dans le commerce, Sigma):

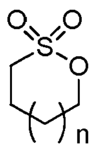 n=0,1

- Les complexes satisfaisant (+) au critère de la quatrième colonne ne comportent qu'un seul groupe réactif amine ou carboxylate, protégé ou pas. Des complexes selon l'invention avec une hydrosolubilité améliorée et pouvant être conjugués à une autre molécule peuvent être préparés en faisant réagir les complexes comprenant une amine (éventuellement après déprotection des groupes Boc) avec des composés de formule :

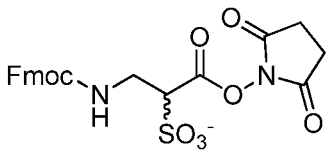 ou 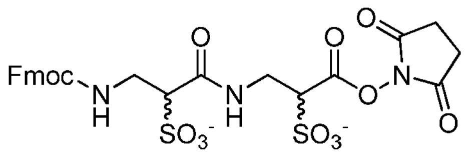

La synthèse de ces deux composés est décrite dans Bioconjugate Chemistry, 2008, 19, 279-289.
Ces différents aspects sont représentés dans les schémas 17 et 18.

**Schéma 17 : Synthèse des complexes dissymétriques de type « carboxylate » hydrosolubles fonctionnalisés**

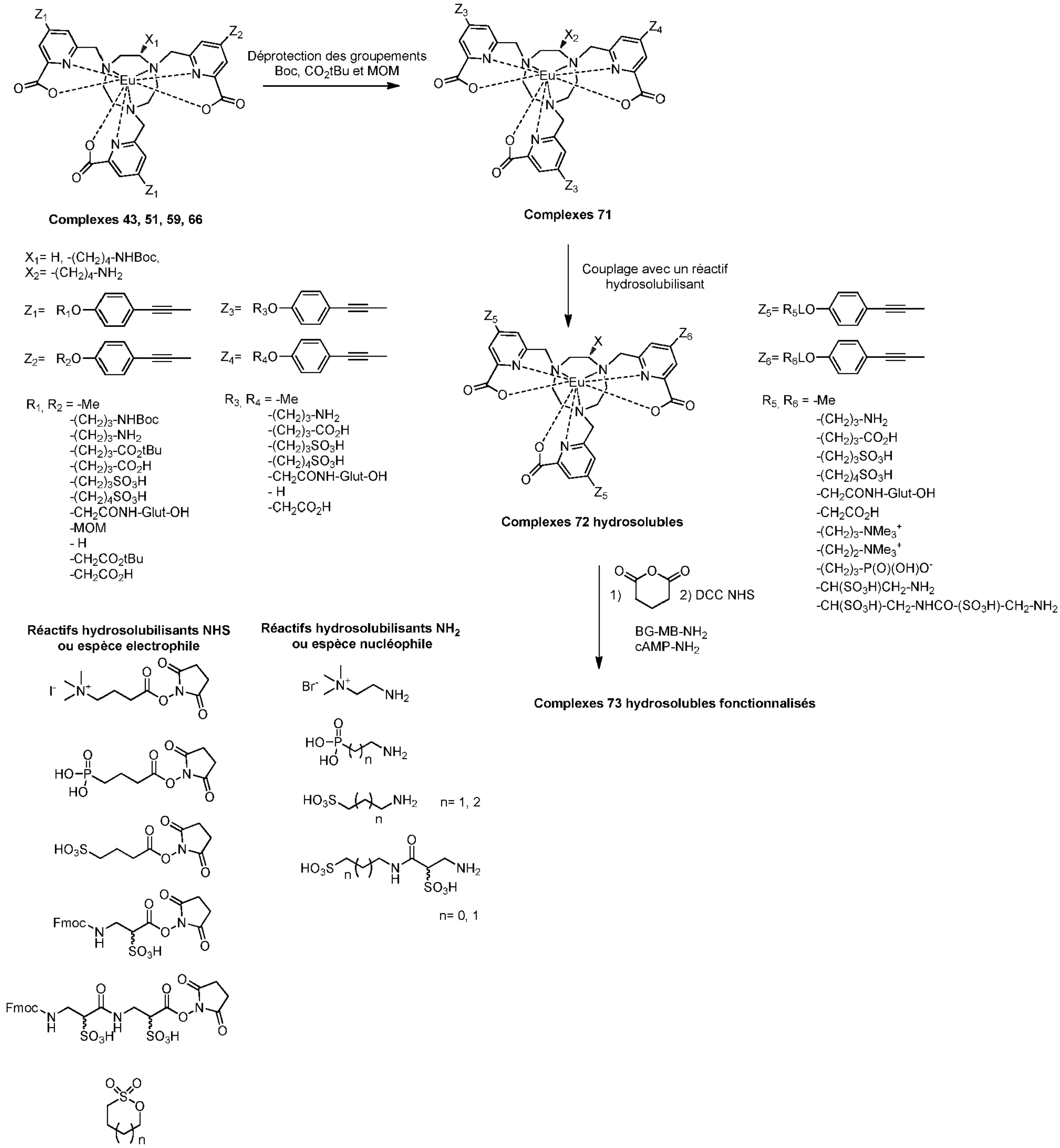

**Schéma 18 : Synthèse des complexes dissymétriques de type « phosphinate » hydrosolubles fonctionnalisés**

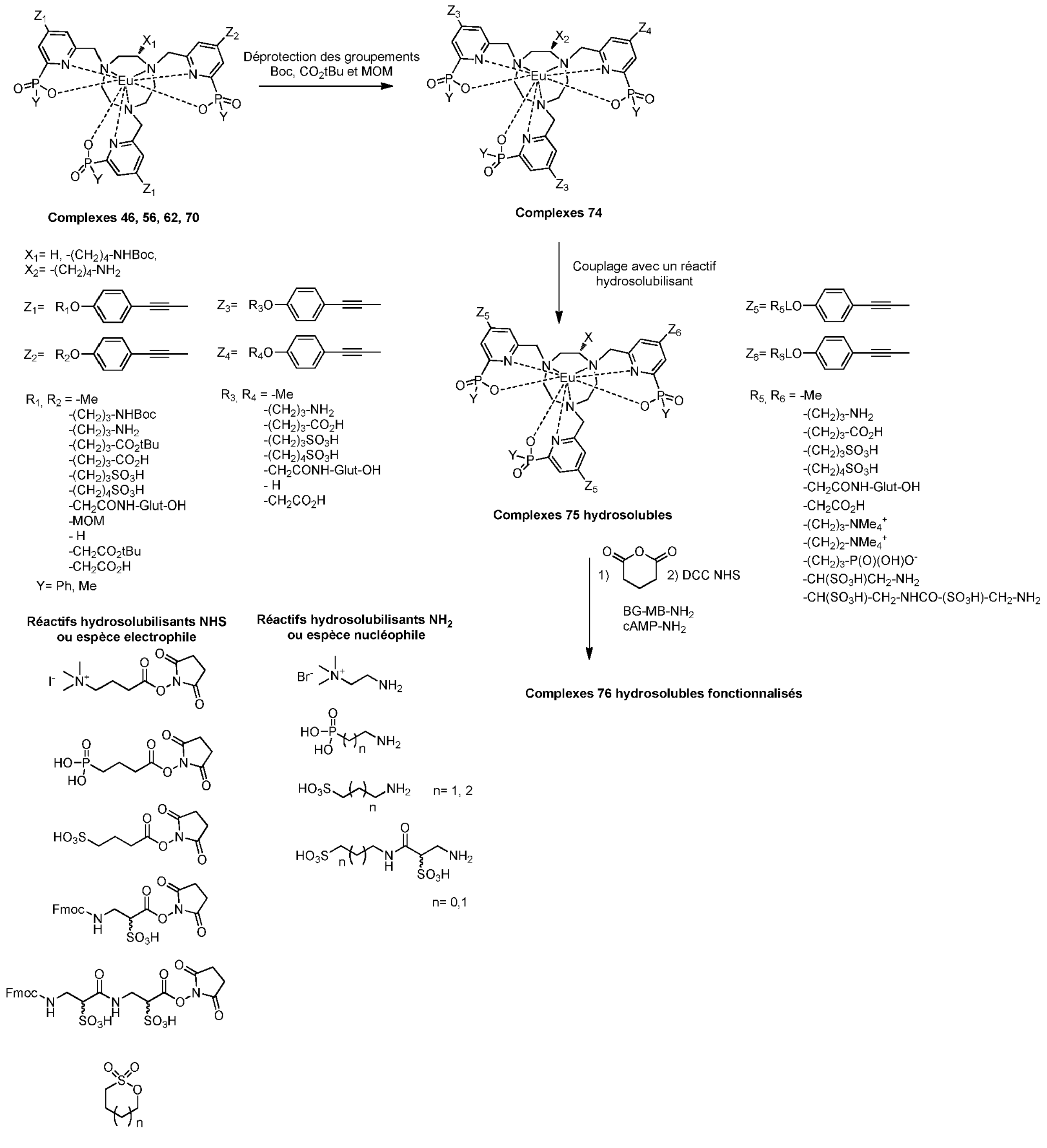


Dans une mise en oeuvre particulière de l'invention, le bras d'espacement L ($L_1$, $L_1$' ou $L_2$) comprend un ou plusieurs motifs sulfonates. La préparation de tels composés à partir de complexes d'europium décrits plus hauts, ainsi que celle de conjugués de ces complexes avec l'AMP cyclique ou un dérivé de la benzylguanine est résumée dans les schémas 19 et 20.

## Schéma 19 : exemples de fonctionnalisation avec linker mono et disulfonate

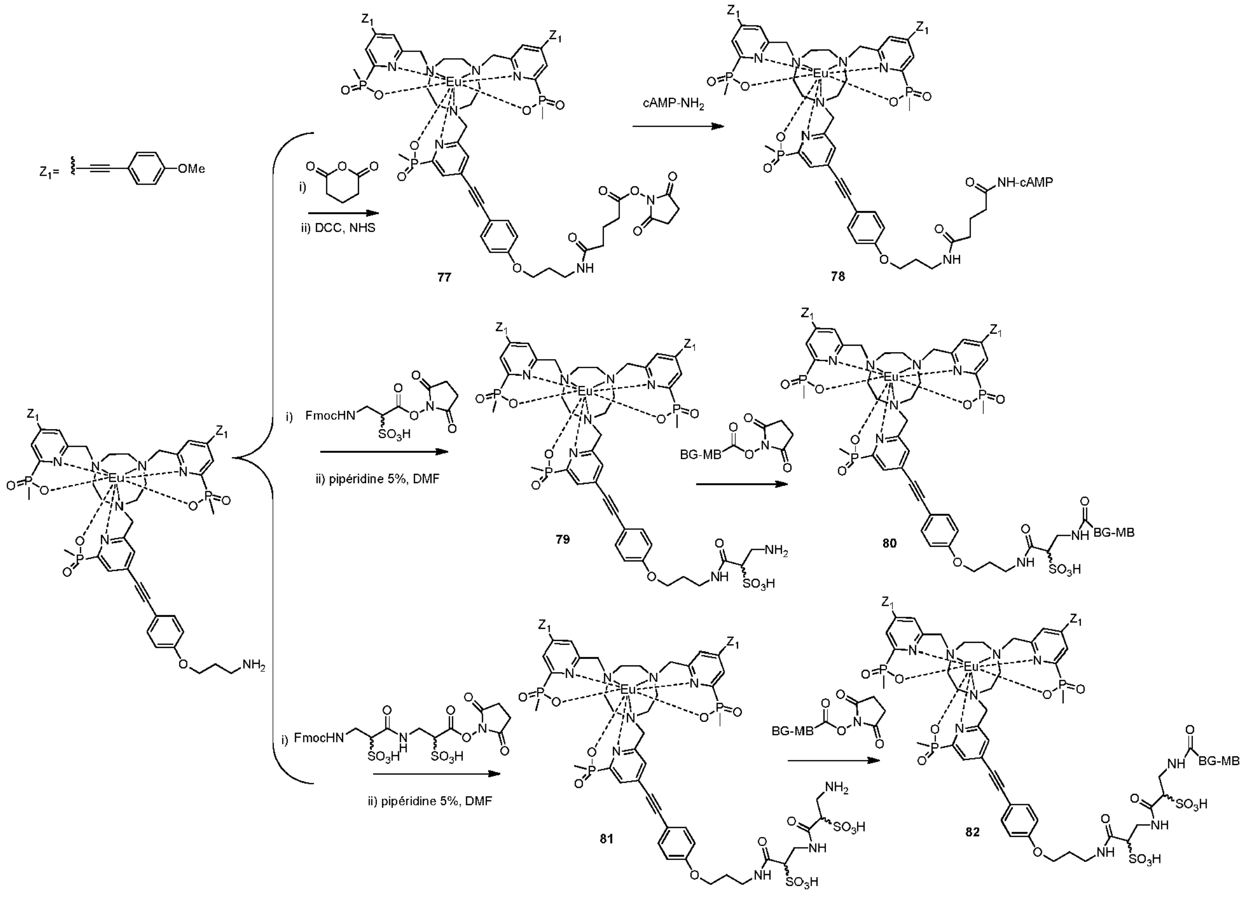


cAMP-NH$_2$ = 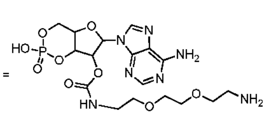 BG-MB- = 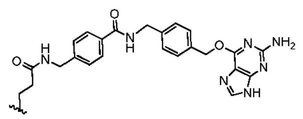

**Schéma 20 : exemples de fonctionnalisation avec linker mono et disulfonate**

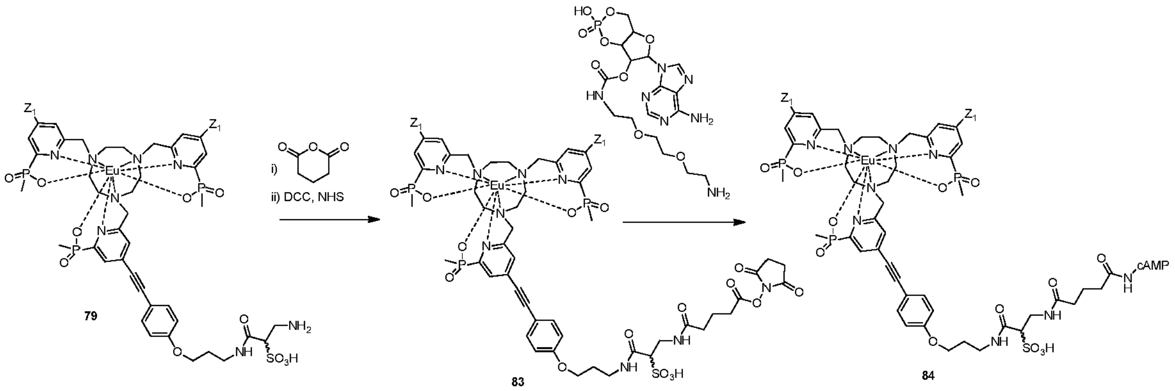


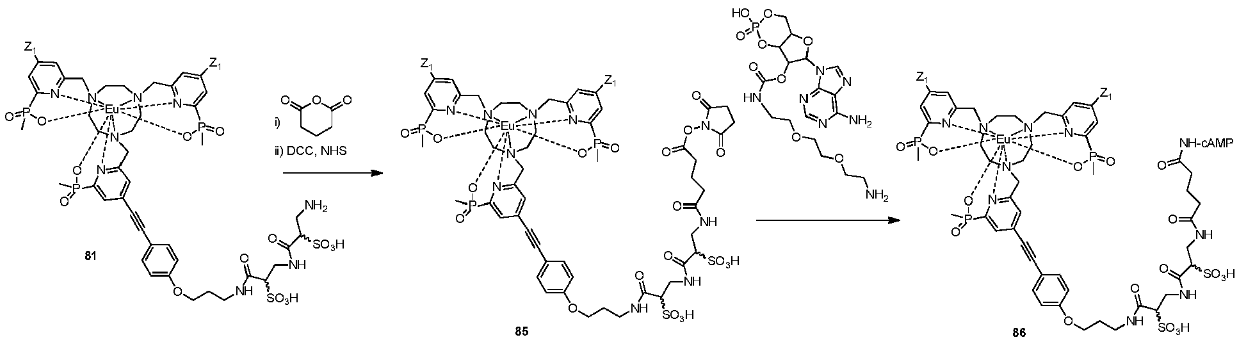


cAMP-$NH_2$ = 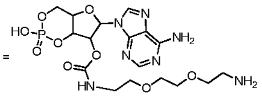

Z1= 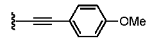

**Schéma 21 : exemples de fonctionnalisation maléimide, biotine et peptidique**

La préparation de conjugués de complexes d'europium selon l'invention avec des peptides cationiques (polyarginine ou polylysine) et avec la biotine est représentée dans le schéma 21.

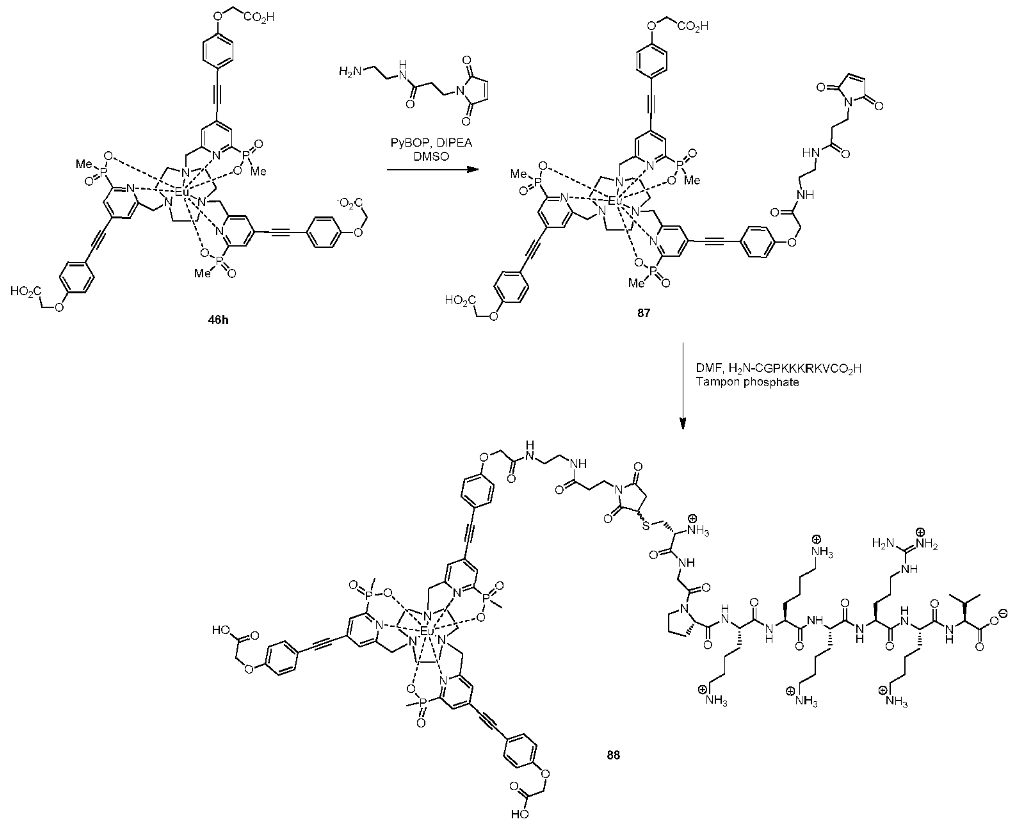


46h

87

88

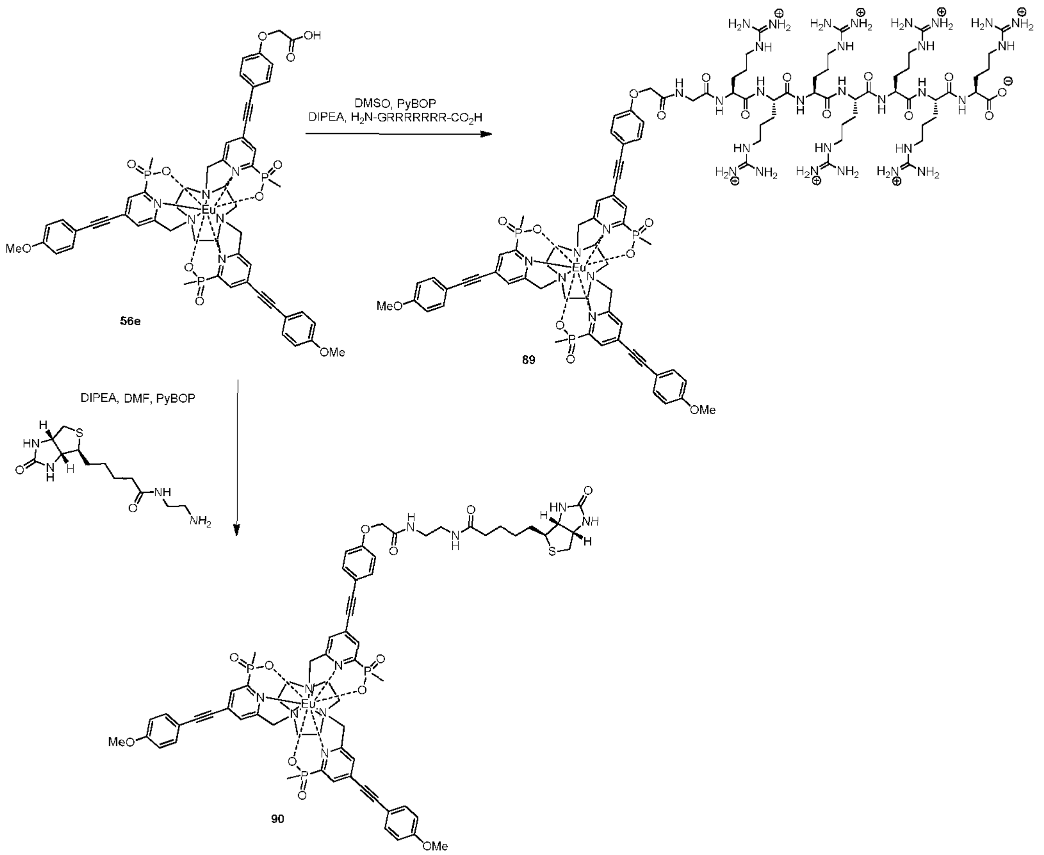


56e

89

90

**Schéma 22 : exemple de fonctionnalisation avec une structure TACN substituée**

L'utilisation de complexes selon l'invention portant un groupe LG au niveau du macrocylce TACN pour la préparation de conjugués est illustrée dans le schéma 22, avec l'exemple d'un conjugué avec l'AMPc.

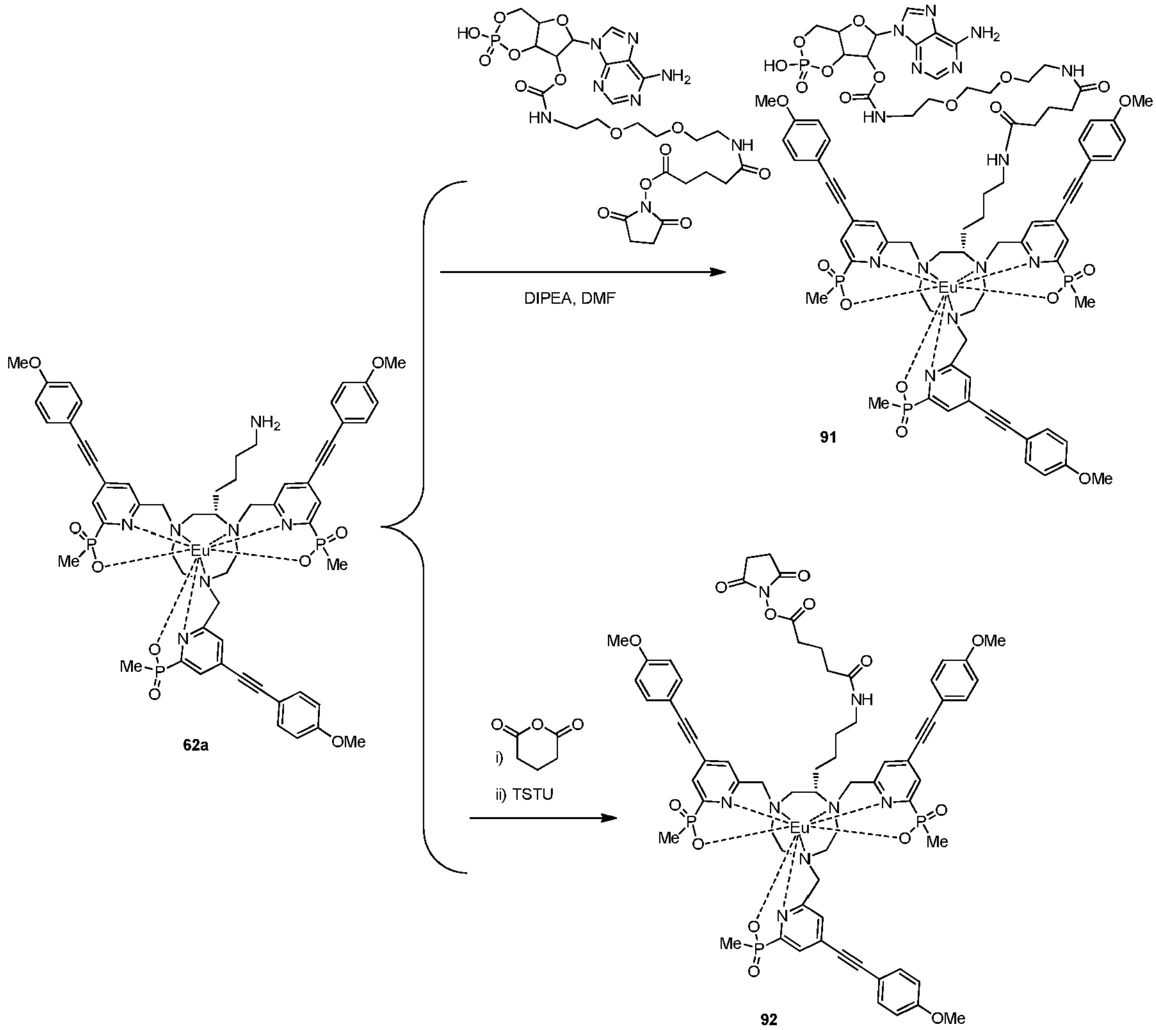


62a

91

92

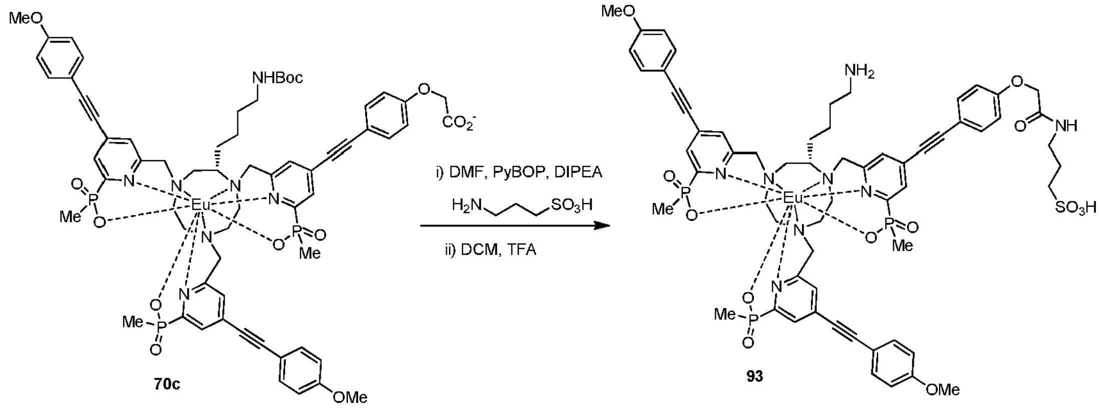


70c

93

**Schéma 23 : synthèse des complexes symétriques de type « phényl-sulfonate-phosphinate » dérivés du TACN C-substitué**

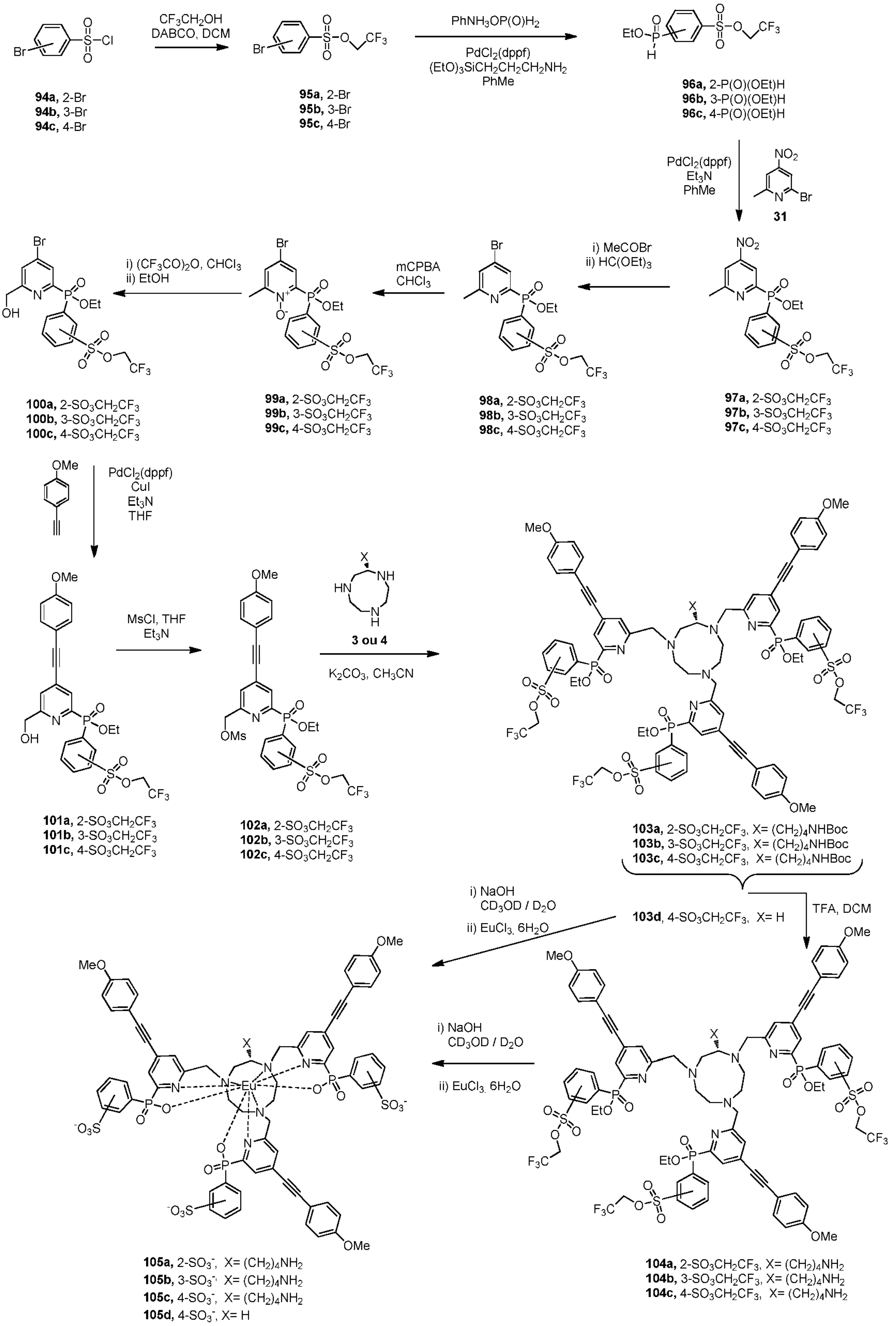


**94a**, 2-Br
**94b**, 3-Br
**94c**, 4-Br

**95a**, 2-Br
**95b**, 3-Br
**95c**, 4-Br

**96a**, 2-P(O)(OEt)H
**96b**, 3-P(O)(OEt)H
**96c**, 4-P(O)(OEt)H

**31**

**97a**, 2-$SO_3CH_2CF_3$
**97b**, 3-$SO_3CH_2CF_3$
**97c**, 4-$SO_3CH_2CF_3$

**98a**, 2-$SO_3CH_2CF_3$
**98b**, 3-$SO_3CH_2CF_3$
**98c**, 4-$SO_3CH_2CF_3$

**99a**, 2-$SO_3CH_2CF_3$
**99b**, 3-$SO_3CH_2CF_3$
**99c**, 4-$SO_3CH_2CF_3$

**100a**, 2-$SO_3CH_2CF_3$
**100b**, 3-$SO_3CH_2CF_3$
**100c**, 4-$SO_3CH_2CF_3$

**101a**, 2-$SO_3CH_2CF_3$
**101b**, 3-$SO_3CH_2CF_3$
**101c**, 4-$SO_3CH_2CF_3$

**102a**, 2-$SO_3CH_2CF_3$
**102b**, 3-$SO_3CH_2CF_3$
**102c**, 4-$SO_3CH_2CF_3$

**3 ou 4**

**103a**, 2-$SO_3CH_2CF_3$, X= $(CH_2)_4NHBoc$
**103b**, 3-$SO_3CH_2CF_3$, X= $(CH_2)_4NHBoc$
**103c**, 4-$SO_3CH_2CF_3$, X= $(CH_2)_4NHBoc$

**103d**, 4-$SO_3CH_2CF_3$, X= H

**104a**, 2-$SO_3CH_2CF_3$, X= $(CH_2)_4NH_2$
**104b**, 3-$SO_3CH_2CF_3$, X= $(CH_2)_4NH_2$
**104c**, 4-$SO_3CH_2CF_3$, X= $(CH_2)_4NH_2$

**105a**, 2-$SO_3^-$, X= $(CH_2)_4NH_2$
**105b**, 3-$SO_3^-$, X= $(CH_2)_4NH_2$
**105c**, 4-$SO_3^-$, X= $(CH_2)_4NH_2$
**105d**, 4-$SO_3^-$, X= H

Les synthèses des complexes **105** sont décrites dans le schéma 23. Les dérivés commerciaux **94** (chlorures

de sulfonyle) sont protégés en utilisant du trifluoroéthanol. Un premier couplage en utilisant des sels de palladium permet d'introduire la fonction phosphinate sur le noyau aromatique puis cette fonction phosphinate est couplée sur le motif pyridine **31** dont la synthèse a été décrite précédemment. La suite de la synthèse est identique aux complexes de type phosphinate : substitution du groupement nitro par un atome de brome en utilisant du bromure d'acétyle et reestérification de l'acide phosphinique correspondant conduisant aux composés **98**, fonctionnalisation du groupe méthyle en hydroxylméthylène via l'intermédiaire N-oxyde, préparation des chromophores **101** par un couplage de Sonogashira, activation de la fonction alcool en dérivés mésylés **102** qui sont ensuite condensés sur le macrocycle 3 ou 4 (7a peut également être envisagé), puis en fonction du macrocycle utilisé, déprotection en milieu acide du groupe protecteur orthogonal Boc permettant la fonctionnalisation, déprotection des groupements sulfonates et phosphinates en milieu basique et enfin formation des complexes en utilisant les sels d'europium.

**Schéma 24 : synthèse des complexes symétriques de type « carboxylate alkylsulfonate»**

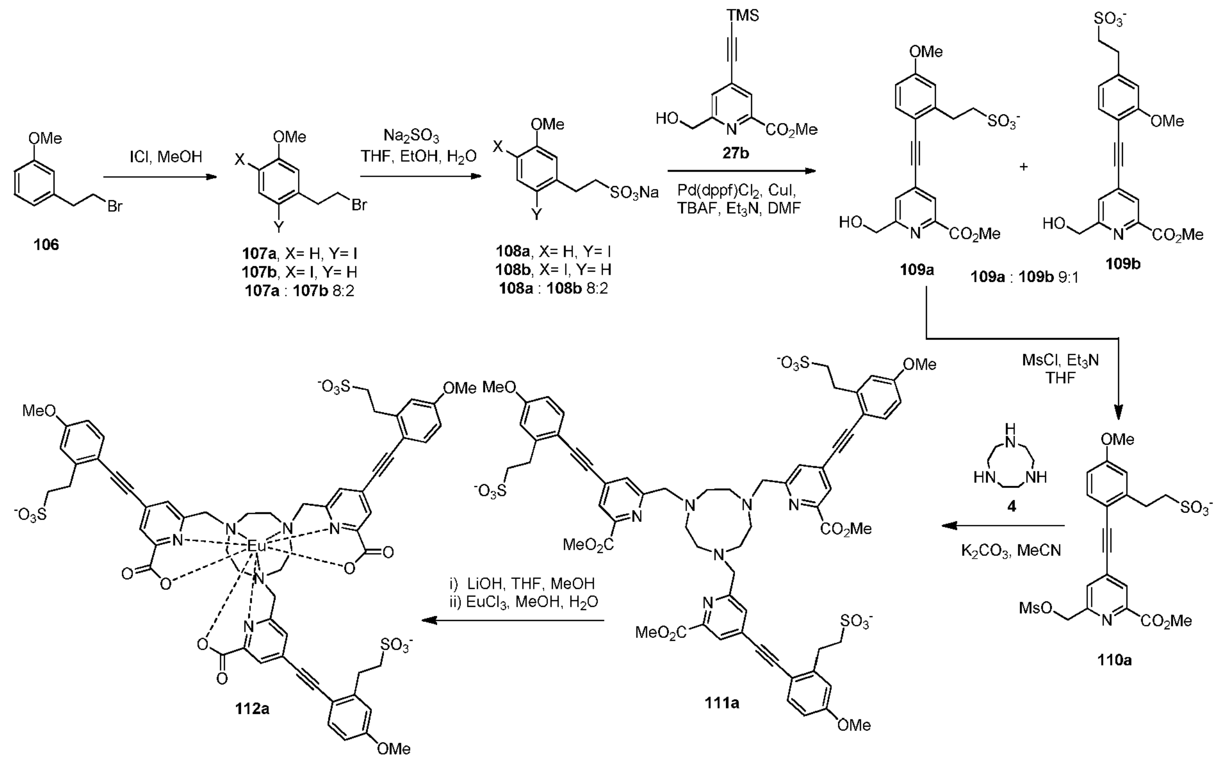


Pour introduire le groupement sulfonate sur le noyau aromatique donnant lieu à la série de complexe **112a**, le composé **106** commercial est d'abord iodé en présence de chlorure d'iode dans le méthanol (mélange d'isomères de position **107a-b**) puis sulfoné en traitant les composés **107** avec du sulfite de sodium. La synthèse est ensuite identique aux synthèses précédentes à savoir réaction de Sonogashira entre le composé **27b** et **108**, activation de l'alcool sous forme de dérivé mésylé, alkylation du macrocycle **4**, hydrolyse et formation du complexe **112a**. L'exemple du schéma 24 illustre la méthologie permettant d'introduire un groupement sulfonate sur une chaîne aliphatique portée par le noyau aromatique du chromophore. Il faut noter que des complexes analogues peuvent être obtenus avec des dérivés dont la chaîne aliphatique comporte un ou trois atomes de carbone et ceci sans changer les propriétés photo-physiques.

**Schéma 25 : synthèse des complexes symétriques de type « méthyl-phosphinate » dérivés du TACN-C substitué**

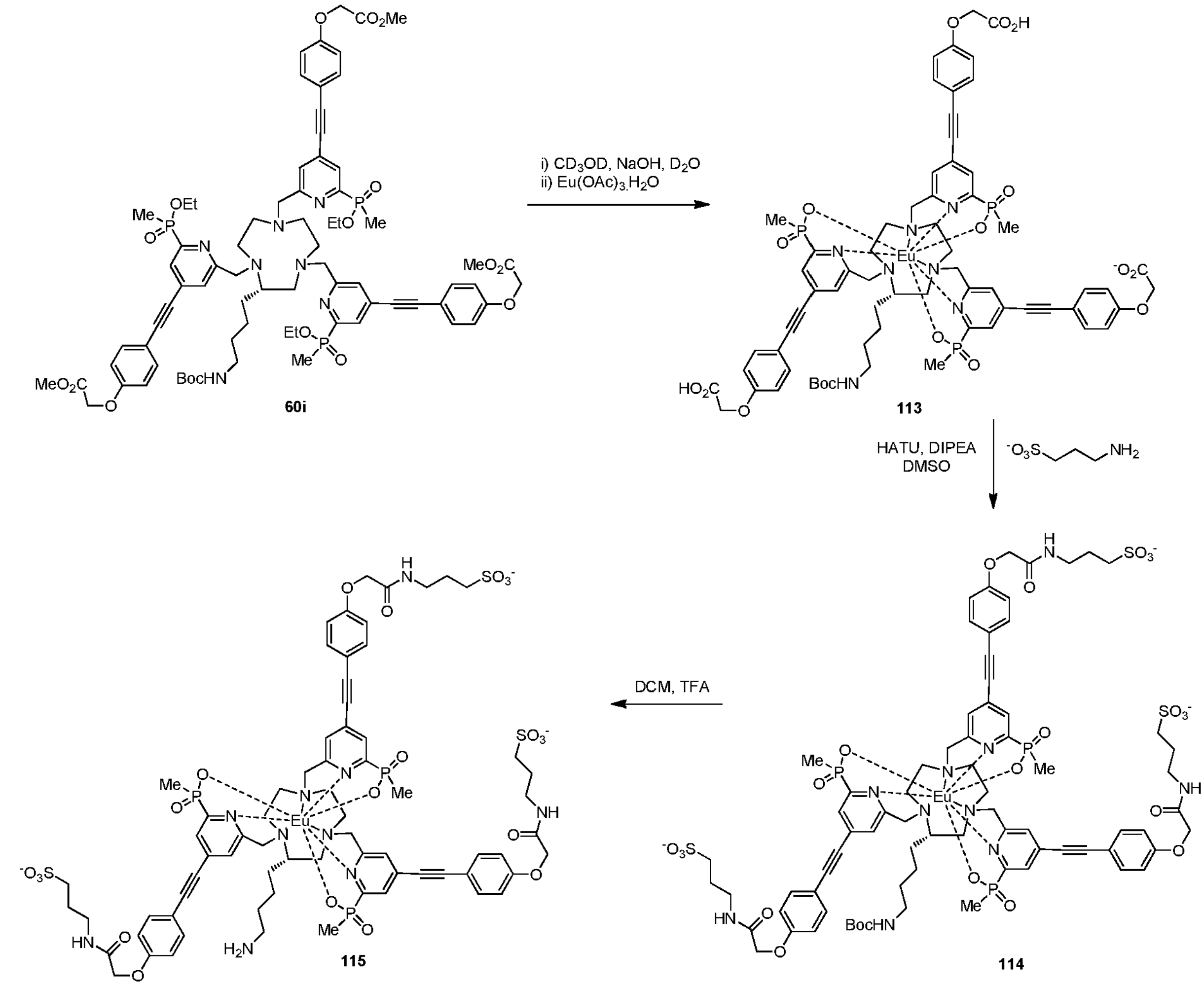


60i 113 114 115

La synthèse du complexe 115 comportant trois groupements sulfonates est représentée dans le schéma 25. Le ligand 60i est saponifié puis complexé avec des sels d'europium. Les groupements carboxylates sont alors libres et peuvent être condensés avec l'homotaurine en utilisant le HATU comme agent de couplage. Enfin la déprotection du groupement Boc permet de libérer l'amine qui pourra ensuite être utilisée dans une réaction de bioconjugaison.

PARTIE EXPERIMENTALE

**Abréviations utilisées :**

Boc : tert-butyloxycarbonyle
Boc-OSu: N-(tert-butoxycarbonyloxy)succinimide
cAMP : adénosyl monophosphate cyclique
CCM : chromatographie en couche mince
δ: déplacement chimique
DABCO : 1,4-diazabicyclo[2.2.2]octane
DCC : dicyclohexylcarbodiimide
DCM : dichlorométhane
DCU: dicyclohexylurée
DIPEA: diisopropyléthylamine
DMAP : diméthylaminopyridine

DMF : diméthylformamide
DMSO : diméthylsulfoxyde
ESI - : ionisation par électronébulisation en mode négatif
ESI + : ionisation par électronébulisation en mode positif
EtOH : éthanol
h : heure
HATU : (O-(7-azabenzotriazol-1-yl)-N,N,N',N'-tétraméthyluronium hexafluorophosphate)
HPLC : chromatographie liquide à haute performance
HRMS : spectroscopie de masse à haute résolution
HTRF: Fluorescence Homogène en Temps Résolu
Hz : Hertz
LC-MS : chromatographie liquide à haute performance couplée à la spectrométrie de masse
LRMS : Spectre de masse basse résolution
m-CPBA : acide métachloroperbenzoïque
MeCN : acétonitrile
MeOH : méthanol
min : minute
MOM : méthyl méthyl éther
Mops : acide 3-(N-morpholino)propanesulfonique
Moz : p-méthoxybenzyloxycarbonyle
Moz-ON : ((2-(4-méthoxybenzyloxycarbonyloxy imino)-2-phénylacétonitrile)
Ms : mésyle
NBS: N-bromosuccinimide
NHS : N-hydroxysuccinimide
NIS : N-iodosuccinimide
Pd(dppf)$Cl_2$ : bis(diphénylphosphino)ferrocène]dichloropalladium(II)
PEG : polyéthylène glycol
Ph: phényle
ppm : partie par million
PtF: point de fusion
Py : pyridine
PyBOP : hexafluorophosphate de benzotriazol-1-yl-oxytripyrrolidinophosphonium
RMN : résonance magnétique nucléaire
SM : spectrométrie de masse
TBAF : fluorure de tétrabutylammonium
TEA / $Et_3N$: triéthylamine
TEAAc : tampon acétate de triéthylammonium
TFA : acide trifluoro acétique
THF : tétrahydrofurane
TMS = triméthylsilyle
Ts : tosyle
TSTU: tétrafluoroborate de O-(N-Succinimidyl)-1,1,3,3-tétraméthyluronium

**Chromatographie**

Les chromatographies sur couches minces (CCM) ont été effectuées sur des plaques de gel de silice Merck 60 $F_{254}$ sur feuille d'aluminium ou sur des plaques d'oxyde d'aluminium neutre Merck 60 $F_{254}$ (type E) sur feuille d'aluminium.

Les chromatographies liquides à haute performance (HPLC) analytiques et préparatives ont été effectuées sur deux appareils :

- HPLC Analytique : ThermoScientific, pompe quaternaire P4000, Détecteur UV 1000 à lampe au deutérium (190-350 nm), colonne analytique Waters XBridge C18, 3,5 μm, 4,6 × 100 mm.
- HPLC Préparative : Shimadzu, 2 pompes LC-8A, détecteur UV à barrette de diodes Varian ProStar, colonne préparative Waters XBridge prép. C18, 5 μm: 19 × 100 mm ou 50 × 150 mm.

**Spectroscopie**

• Résonance Magnétique Nucléaire (RMN)

Les spectres RMN ($^1$H, $^{13}$C et $^{31}$P) ont été réalisés à l'aide d'un spectromètre Bruker Avance 400 MHz NanoBay (aimant de 9,4 Teslas), muni d'une sonde de mesure BBFO, multi noyaux de diamètre de 5 mm, de gradient Z et de lock $^2$H. Les déplacements chimiques ($\delta$) sont exprimés en partie par million (ppm). Les abréviations suivantes sont utilisées :
s : singulet, s l : singulet large, s app : singulet apparent, d : doublet, t : triplet, q : quadruplet, m : multiplet, dd : doublet dédoublé, td : triplet dédoublé, qd : quadruplet dédoublé, ddd : doublet de doublet dédoublé, AB : système AB.

• Spectrométrie de masse (LRMS)

Les spectres de masse (LC-MS) ont été réalisés à l'aide d'un spectromètre Waters ZQ 2000 simple quadipôle à source multimode ESI/APCI équipé de colonne Waters XBridge C18, 3,5$\mu$m, 4,6 $\times$ 100 mm.

• Spectrométrie de masse haute résolution (HRMS)

Les analyses ont été effectuées avec un spectromètre de masse QStar Elite (Applied Biosystems SCIEX) équipé d'une source d'ionisation à pression atmosphérique (API) assistée pneumatiquement. L'échantillon a été ionisé en mode electrospray positif dans les conditions suivantes : tension electrospray (ISV) : 5500 V ; tension d'orifice (OR) : 20 V ; pression du gaz de nébulisation (air) : 20 psi. Le spectre de masse haute résolution (HRMS) a été obtenu avec un analyseur temps de vol (TOF). La mesure de masse exacte a été effectuée en triplicat avec un double étalonnage interne.

**Point de fusion**

Appareil à point de fusion : les points de fusion ont été réalisés en utilisant un appareil BUCHI melting point B-540.

**Composé 2**

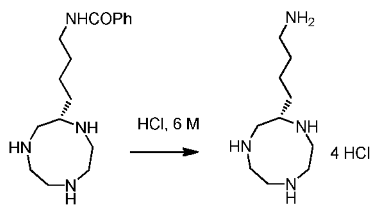


1 2

Le compose 1 (182 mg, 0,6 mmol) (J. Chem. Soc Perkin Trans 1, 1990, 2567) a été dissous dans une solution aqueuse hydrochlorhydrique 6 M (3 mL) qui a ensuite été agitée à 100°C pendant 72 h. L'avancement de la réaction a été suivi par CCM. Après cette période la réaction était complète. La solution a été refroidie à température ambiante et diluée avec de l'eau (3 mL) puis a été extraite avec du diéthyl éther (3 $\times$ 10 mL). La phase aqueuse a été concentrée sous pression réduite pour conduire au composé 2 sous forme de sels d'hydrochlorure (100 mg, quantitatif). RMN $^1$H (400 MHz, $D_2O$) $\delta$ : 3,42-3,13 (m, 10H), 3,96-2,87 (m, 3H), 1,64-1,37 (m, 6H). RMN $^{13}$C (100 MHz, $D_2O$) $\delta$ : 53,3; 46,1; 43,2; 41,8; 39,2; 39,1; 39,0; 30,6; 26,5; 22,1. HRMS (ESI+) calculée pour $C_{10}H_{25}N_4$ $[M+H]^+$, *m/z* 201,2079, trouvée: 201,2068.

**Composé 3**

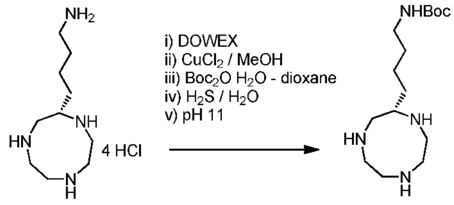


2 3

La tétraamine 2 a été convertie en amine libre (66 mg, 0,33 mmol) par chromatographie échange d'ions en utilisant de la résine DOWEX 1x2-200. L'amine libre a été dissoute dans du méthanol (4 mL) et à cette solution a été ajouté du chlorure de cuivre monohydraté (56 mg, 1,33 mmol). La solution initialement incolore a virée au vert intense. Le mélange a été agité à température ambiante pendant 2 h puis le solvant a été éliminé sous pression réduite pour conduire à un solide verdâtre qui a été redissous dans de l'eau (2 mL). A cette solution a été ajoutée une solution di-tert-butyl bicarbonate (140 mg, 0,66 mmol) dans du dioxane (2 mL) puis a été agitée à température ambiante pendant 3 h. Après cette période, la réaction n'était pas complète. Un deuxième ajout d'une solution de di-tert-butyl bicarbonate (70 mg, 0,33 mmol) dans du dioxane (1 mL) a été réalisé puis cette solution a été agitée à température ambiante pendant 16 h supplémentaires. L'avancement de la réaction a été suivi par LC-MS. Après cette période la réaction était complète. La solution verte a été traitée en faisant buller du sulfure d'hydrogène sur une période de 5 min et le mélange a été centrifugé pour éliminer le précipité noir. Le surnageant a été lavé avec du dichlorométhane puis la solution aqueuse a été ajusté à pH 11 avant de réaliser une extraction intensive avec du dichlorométhane (8 $\times$ 10 mL). Les phases organiques ont été réunies et séchées sur sulfate de magnésium puis filtrées et concentrées sous pression réduite pour conduire à une huile incolore (27 mg, 27%). RMN $^1H$ (400 MHz, $CDCl_3$) $\delta$: 6,46 (s l, 1H), 3,13 (m, 2H), 2,89-2,67 (m, 10H), 2,47 (m, 1H), 2,35 (s l, 3H), 1,47 (s, 9H), 1,49-1,35 (m, 6H); RMN $^{13}C$ (100 MHz, $CDCl_3$) $\delta$ : 156,3; 79,3; 55,2; 49,9; 46,2; 45,3; 44,3; 40,6; 40,3; 33,9; 30,4; 23,7; 28,6. HRMS (ESI+) calculée pour $C_{15}H_{33}N_4O_2$ [M+H]$^+$, *m/z* 301,2604, trouvée: 301,2603.

**Composé 5**

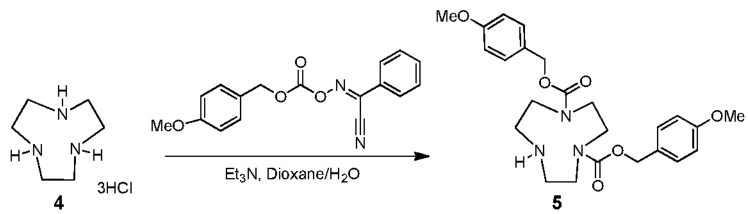


4 5

A une solution d'hydrochlorure de 1,4,7-triazacyclononane **4** (188 mg, 0,78 mmol) dans un mélange dioxane / eau (10 mL, 8:2) a été ajouté le 2-(4-méthoxybenzyloxycarbonyloxyimino)-2-phénylacétonitrile (Moz-ON) (477 mg, 1,5 mmol). Le mélange réactionnel a été homogénéisé par une agitation vigoureuse puis à ce mélange a été ajoutée une solution de triéthylamine (540 $\mu$L, 3,8 mmol) dans un mélange dioxane / eau (10 mL, 8:2). L'avancement de la réaction a été suivi par CCM. Après 24 h, la réaction était totale. Le solvant a été éliminé sous pression réduite. Le résidu a été dissous dans du dichlorométhane. Cette solution a été lavée avec de l'eau saturée en chlorure de sodium (3 $\times$ 10 mL). La phase organique a été séchée sur sulfate de magnésium, filtrée et concentrée sous pression réduite. Le brut réactionnel a été purifié par chromatographie sur colonne d'alumine (dichlorométane / méthanol 98:2 jusqu'à 90:10 par incréments de 1%) pour conduire au composé souhaité 5 sous la forme d'une huile (266 mg, 74 %). RMN $^1H$ (400 MHz, $CDCl_3$) $\delta$ : 7,32-7,25 (m, 4H), 6,88-6,85 (m, 4H), 5,09 (m, 4H), 3,80 (s, 6H), 3,56-3,47 (m, 4H), 3,31-3,25 (m, 4H), 2,87 (m, 4H). RMN $^{13}C$ (100 MHz, $CDCl_3$) $\delta$ : 159,68; 156,76; 130,05; 128,92; 114,03; 67,15; 55,41; 52,20; 51,98; 51,26; 50,31; 50,07; 49,19; 48,18; 47,73; 47,66. LRMS (ESI+) calculée pour $C_{24}H_{32}N_3O_6$ [M+H]$^+$, *m/z* 458,2291, trouvée: 458,08. $R_f$ = 0,48 (silice; dichlorométhane - méthanol 90:10).

## Composé 6

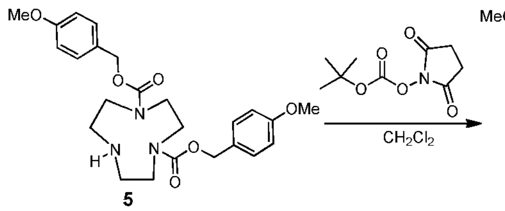


5 6

A une solution de macrocycle diprotégé 5 (50 mg, 0,1 mmol) dans du dichlorométhane (7 mL), a été ajouté le N-(tert-butoxycarbonyloxy)succinimide (Boc-OSu) (36 mg, 0,15 mmol). La solution a été agitée à température ambiante sous atmosphère inerte. L'avancement de la réaction a été suivi par CCM. Après 24 h, la réaction était totale. La solution a été directement lavée avec de l'eau saturée en chlorure de sodium (3 $\times$ 10 mL). La phase organique a été séchée sur sulfate de magnésium, filtrée et concentrée sous pression réduite. Le produit brut a été purifié par chromatographie sur colonne d'alumine (dichlorométhane / méthanol 90:10 jusqu'à 70:30 par incrément de 5%) pour conduire au composé 6 sous la forme d'une huile de couleur brune (44 mg, 72%). RMN $^1$H (400 MHz, $CDCl_3$) $\delta$ : 7,30-7,19 (m, 4H), 6,88-6,83 (m, 4H), 5,06-4,92 (m, 4H), 3,77 (s, 6H), 3,46-3,39 (m, 12H), 1,55 (s, 9H). RMN $^{13}$C (100 MHz, $CDCl_3$) $\delta$ : 159,65; 156,56; 155,76; 130,04; 128,98; 114,00; 80,01; 67,15; 55,38; 49,74; 49,64; 49,47;49,37; 49,12; 48,92; 48,80; 28,53; 27,75; 25,60. HRMS (ESI+) calculée pour $C_{29}H_{39}N_3O_8$ $[M+H]^+$, *m/z* 558,2815, trouvée: 558,2808. $R_f$= 0,28 (silice; cyclohexane - acétate d'éthyle 50:50).

## Composé 7a

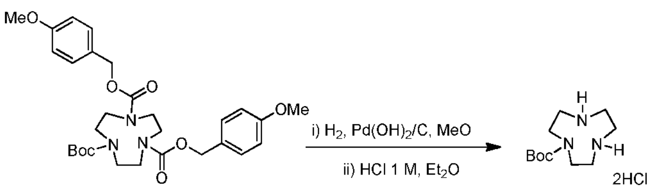


6 7a

A une solution de macrocycle diMOZ-monoBoc 6 (300 mg, 0,5 mmol) dans le méthanol (25 mL) a été ajouté de l'hydroxyde de palladium adsorbé sur charbon (environ 100 mg). Le milieu réactionnel a été placé dans un hydrogénateur et a été maintenu sous une vive agitation et sous atmosphère d'hydrogène (3,45 bars soit 50 PSI). L'avancement de la réaction a été suivi par CCM d'alumine. Après une nuit, la réaction était totale. Le milieu réactionnel a été filtré sur Celite puis concentré sous pression réduite. L'huile incolore obtenue a été dissoute dans du méthanol (3 mL). A cette solution a été ajoutée une solution aqueuse d'acide chlorhydrique à 1 M (pH 2-3). Le mélange a été concentré sous pression réduite et a été redissous dans un minimum de méthanol (2 mL). A cette solution, a été ajouté de l'éther diéthylique (35 mL). Le précipité blanc obtenu a été recueilli par filtration pour donner un solide blanc correspondant à l'hydrochlorure 7a (102 mg, 62%). PtF: 172-174°C. RMN $^1$H (500 MHz, $D_2O$) $\delta$ : 3,74 (t, *J*= 4,5 Hz, 4H), 3,63 (s, 4H), 3,48 (t, *J* = 4,5 Hz, 4H), 1,47 (s, 9H). RMN $^{13}$C (125 MHz, $CDCl_3$) $\delta$:136,0; 62,9; 25,9; 22,2 ; 21,7; 6,7. HRMS (ESI+) calculée pour $C_{11}H_{23}N_3O_2$ $[M+H]^+$, *m/z* 230,1869, trouvée: 230,1863. $R_t$ = 10,92 min (colonne Waters XBridge RP-$C_{18}$, 3,5 $\mu$m, 4,6 $\times$ 100 mm) avec une solution aqueuse d'acide trifluoroacétique à 0,2% (pH 1 - MeCN (v/v)) comme éluant, gradient linéaire de 15 à 100% MeCN (19 min), avec un débit de 1 mL $min^{-1}$ et une détection UV de 280 nm.

## Composé 8

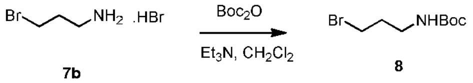


7b 8

A une solution d'hydrobromure de 3-bromopropylamine **7b** (3,72 g, 16,9 mmol) dans du dichlorométhane (100 mL) ont été ajoutés du dicarbonate de di-*tert*-butyle (3,71 g, 16,9 mmol) et de la triéthylamine (10 mL). Le mélange réactionnel a été agité à température ambiante pendant 12 h. L'avancement de la réaction a été suivi par CCM. Après cette période la réaction était totale. Le solvant a été éliminé sous pression réduite. A ce résidu a été additionnée une solution saturée de chlorure de sodium (100 mL) et le mélange a été extrait avec de l'éther diéthylique (2 $\times$ 50 mL). Les phases organiques ont été réunies, lavées par une solution saturée de chlorure de sodium (3 $\times$ 50 mL) et séchées sur

sulfate de sodium. Après filtration, le solvant a été éliminé sous pression réduite pour conduire au composé 8 sous forme d'un solide légèrement brun. Le composé était suffisamment pur pour être utilisé dans la suite de la synthèse sans purification supplémentaire (3,50 g, 87%). PtF: 32-33°C. RMN $^1$H (200 MHz, $CDCl_3$) $\delta$ : 4,63 (s, 1H), 3,42 (t, *J* = 6,5 Hz, 2H), 3,26 (td, *J* = 6,5; 6,5 Hz, 2H), 2,03 (m, *J* = 6,5 Hz, 2H), 1,43 (s, 9H) ; RMN $^{13}$C (125 MHz, $CDCl_3$) $\delta$ : 156,09; 79,54; 39,96; 32,82; 30,90; 28,48. HRMS (ESI+) calculée pour $C_8H_{16}NO_2Br$ $[M+H]^+$, *m/z* 255,0703, trouvée: 255,0695. $R_f$ = 0,59 (silice; cyclohexane - acétate d'éthyle 50:50).

**Composé 10**

La synthèse de ce composé a été décrite par S. Machida et al. (Journal American Chemical Society 2011-133-958-963, supplementary material).

## Composé 13a

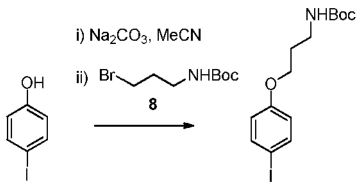


11 13a

A une solution de 4-iodophénol **11** (3,00 g, 13,6 mmol) dans de l'acétonitrile anhydre (100 mL) a été ajouté le carbonate de sodium (5,65 g, 40,9 mmol). La réaction a été chauffée à reflux pendant 1 h puis à cette suspension a été ajouté le dérivé bromé **8** (2,60 g, 10,9 mmol). Le mélange réactionnel a été chauffé à reflux pendant 12 h. L'avancement de la réaction a été suivi par CCM. Après cette période la réaction était totale. Le mélange réactionnel a été refroidi à température ambiante et le solvant a été éliminé sous pression réduite. A ce résidu a été additionnée de l'eau (100 mL) et le mélange a été extrait avec du dichlorométhane (2 $\times$ 50 mL). Les phases organiques ont été réunies, séchées sur sulfate de magnésium et filtrées. Le solvant a été éliminé sous pression réduite et le résidu a été purifié par chromatographie sur colonne de silice en utilisant du dichlorométhane pour conduire au composé **13a** sous forme d'un solide blanc (3,10 g, 75%). PtF : 79-80°C. RMN $^1$H (500 MHz, $CDCl_3$) $\delta$: 7,52 (d, *J* = 8,9 Hz, 2H), 6,64 (d, *J* = 8,9 Hz, 2H), 4,69 (s, 1H), 3,95 (t, *J* = 6,2 Hz, 2H), 3,29 (td, *J* = 6,2; 6,2 Hz, 2H), 1,94 (m, *J* = 6,2 Hz, 2H), 1,41 (s, 9H), RMN $^{13}$C (125 MHz, $CDCl_3$) $\delta$: 158,7; 155,9; 138,2; 116,9; 82,9; 65,9; 37,9. HRMS (ESI+) calculée pour $C_{14}H_{20}NO_3I$ $[M+H]^+$, *m/z* 378,0561, trouvée: 378,0559. $R_f$ = 0,58 (silice; cyclohexane - acétate d'éthyle 50:50).

**Composés 13b et 13c**

Ces composés ont été synthétisés selon la même procédure que celle utilisée pour la préparation du composé 13a.

## Composé 13d

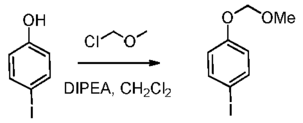


11 13d

A une solution de iodophénol **11** (606 mg, 3 mmol) dans du dichlorométhane (8 mL) refroidie à 5°C, ont été ajoutés de la diisopropyléthylamine (1,045 mL, 6 mmol) puis goutte à goutte du chloro(méthoxy)méthane (MOMCl) (607 $\mu$L, 8 mmol) sur une période de 5 min. Après la fin de l'addition, le mélange a été réchauffé à température ambiante puis à 60°C pendant 4 h. L'avancement de la réaction a été suivi par CCM. Après cette période la réaction était totale. Le mélange a été refroidi à température ambiante puis à cette solution a été ajouté du dichlorométhane (20 mL). La phase organique a été lavée avec de l'eau (2 $\times$ 20 mL), séchée avec du sulfate de magnésium, filtrée et concentrée sous pression réduite. Le résidu a été purifié par chromatographie flash sur colonne de silice avec du dichlorométhane comme éluant pour conduire au produit souhaité **13d** (605 mg, 76%). RMN $^1$H (400 MHz, $CDCl_3$) $\delta$ : 7,57 (d, *J* = 8,9 Hz, 2H), 6,83 (d, *J* = 8,9 Hz, 2H), 5,14 (s, 2H), 3,46 (s, 3H). RMN $^{13}$C (100 MHz, $CDCl_3$) $\delta$ :157,09; 138,28; 118,61; 94,33; 84,39; 56,05. LRMS (ESI+) calculée pour $C_8H_{10}IO_2$ $[M+H]^+$, *m/z* 264,97, trouvée: 264,03. $R_f$ = 0,73 (silice;

cyclohexane - acétate d'éthyle 30:70).

**Composé 13e**

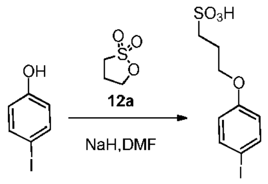


11 13e

A une solution de iodophénol 11 (200 mg, 1 mmol) dans du diméthylformamide anhydre (3 mL) a été ajouté de l'hydrure de sodium à 60% en dispersion dans de l'huile minérale par petites portions (52 mg, 2,1 mmol) sur une période de 5 min et sous courant d'azote. Le mélange a été agité à température ambiante pendant 30 min puis à cette suspension a été ajouté du 1,3-propane sultone **12a** (115 µL, 1,3 mmol). Le mélange a ensuite été agité pendant 2 h 30 à température ambiante sous atmosphère inerte. L'avancement de la réaction a été suivi par HPLC. Après cette période la réaction était complète. La suspension a été filtrée, et le solide a été lavé avec du dichlorométhane (30 mL) puis séché sous pression réduite pour conduire à un solide blanc qui était suffisamment pur pour être utilisé dans l'étape suivante sans purification complémentaire (309 mg, 91%). PtF : 350°C (déc). RMN $^1H$ (400 MHz, DMSO-$d_6$) δ : 7,56 (d, *J* = 8,8 Hz, 2H), 6,76 (d, *J* = 8,8 Hz, 2H), 4,03 (t, *J* = 6,5 Hz, 2H), 2,59 (t, *J* = 7,4 Hz, 2H), 2,00 (q, *J* = 6,5 Hz, 7,4 Hz, 2H). RMN $^{13}C$ (100 MHz, DMSO-$d_6$) δ : 158,55; 137,96; 117,33; 82,86; 66,81; 47,81; 25,11. HRMS (ESI+) calculée pour $C_9H_{15}NO_4IS$ $[M+NH_4]^+$, *m/z* 359,9766, trouvée: 359,9760. $R_t$ = 12,28 min (colonne Waters XBridge RP-$C_{18}$, 3,5 µm, 4,6 × 100 mm) avec une solution aqueuse d'acide trifluoroacétique à 0,2% (pH 1 - MeCN (v/v)) comme éluant,[isocratique 5% MeCN (2 min)], gradient linéaire de 5 à 100% MeCN (19 min), avec un débit de 1 mL $min^{-1}$ et une détection UV de 280 nm.

**Composé 13f**

Ce composé a été synthétisé selon la même procédure que celle utilisée pour la préparation du composé **13e,** en utilisant la sultone **12b**

**Composé 14a**

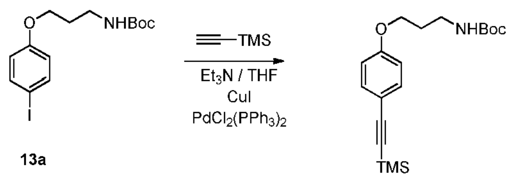


13a 14a

Une solution de dérivé iodé **13a** (2,50 g, 6,6 mmol) dans un mélange de solvant de tétrahydrofurane (20 mL) et de triéthylamine (20 mL) a été dégazée sous agitation pendant 20 min. A cette solution ont été additionnés du triméthylsilylacétylène (1,95 g, 19,8 mmol) suivi du bis-chlorure bis-triphénylphosphine de palladium (II) (46 mg, 0,06 mmol) et de l'iodure de cuivre (I) (25 mg, 0,13 mmol). La réaction a été agitée à température ambiante pendant 12 h. L'avancement de la réaction a été suivi par CCM. Après cette période la réaction était totale. Les solvants ont été éliminés sous pression réduite. Au résidu a été additionnée une solution de chlorure d'ammonium saturée (50 mL) et le mélange a été extrait avec du dichlorométhane (2 × 25 mL). Les phases organiques ont été réunies et lavées par une solution de chlorure d'ammonium saturée (50 mL) puis par une solution de saturée de chlorure de sodium (2 × 50 mL) et ensuite séchées sur sulfate de sodium. Après filtration, le solvant a été éliminé sous pression réduite et le résidu a été purifié par chromatographie sur colonne de silice (dichlorométhane / pentane 1/1 puis dichlorométhane) pour conduire au composé **14a** sous forme d'un solide blanc (1,50 g, 65%). PtF : 92-93°C. RMN $^1H$ (200 MHz, $CDCl_3$) δ : 7,37 (d, *J* = 8,7 Hz, 2H), 6,78 (d, *J* = 8,7 Hz, 2H), 4,69 (s, 1H), 3,99 (t, *J* = 6,2 Hz, 2H), 3,29 (td, *J* = 6,2; 6,2 Hz, 2H), 1,95 (m, *J* = 6,2 Hz, 2H), 1,42 (s, 9H), 0,22 (s, 9H). RMN $^{13}C$ (100 MHz, $CDCl_3$) δ : 159,08; 156,13; 133,61; 115,54; 114,44; 105,28; 92,63; 79,41; 65,92; 38,06; 29,66; 28,54; 0,20. HRMS (ESI+) calculée pour $C_{19}H_{29}NO_3Si$ $[M+H]^+$, *m/z* 348,1989, trouvée: 348,1993. $R_f$ = 0,77 (silice; dichlorométhane - méthanol 95:5).

**Composés 14b et 14c**

Ces composés ont été synthétisés selon la même procédure que celle utilisée pour la préparation du composé **14a**, en utilisant les précurseurs correspondants.

## Composé 15a

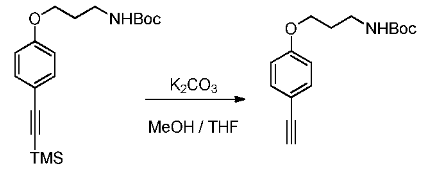


**14a** **15a**

A une solution de dérivé silylé **14a** (1,27 g, 3,7 mmol) dans un mélange de tétrahydrofurane (30 mL) et de méthanol (30 mL) a été ajouté du carbonate de potassium (1,52 g, 11,0 mmol). Le milieu réactionnel a été agité à température ambiante pendant 2 h. L'avancement de la réaction a été suivi par CCM. Après cette période la réaction était totale. Le mélange réactionnel a été filtré et le filtrat a été concentré sous pression réduite. Le résidu a été purifié par chromatographie sur colonne de silice (pentane / dichlorométhane 50/50 jusqu'à 100% par incréments de 10%) pour conduire au composé **15a** sous forme d'un solide blanc (0,86 g, 85%). RMN $^1H$ (200 MHz, $CDCl_3$) $\delta$ : 7,39 (d, $J$ = 8,7 Hz, 2H), 6,81 (d, $J$ = 8,7 Hz, 2H), 4,69 (s, 1H), 3,99 (t, $J$= 6,2 Hz, 2H), 3,29 (td, $J$ = 6,2 Hz et $J$ = 6,2 Hz, 2H), 2,97 (s, 1H), 1,95 (t, $J$ = 6,2 Hz, 2H), 1,42 (s, 9H). LRMS (ESI+) calculée pour $C_{16}H_{22}NO_3$ $[M+H]^+$, $m/z$ 276,1600, trouvée: 276,18. $R_f$ = 0,90 (silice; dichlorométhane - méthanol 90:10).

**Composés 15b et 15c**

Ces composés ont été synthétisés selon la même procédure que celle utilisée pour la préparation du composé **15a** en utilisant les précurseurs correspondants.

## Composé 17a

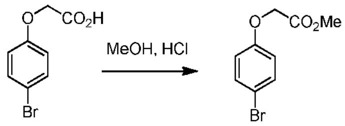


**16** **17a**

A une solution méthanolique d'hydrochlorure 3 M (3 mL) a été additionné l'acide 2-(4-bromophénoxy)-acétique **16** (1,0 g, 4,3 mmol). Le mélange a été agité pendant 20 h à température ambiante sous atmosphère inerte. Le solvant a été éliminé sous pression réduite et l'huile résiduelle a été dissoute dans du dichlorométhane (15 mL) et a été lavée successivement avec de l'eau (10 mL), une solution de bicarbonate saturée (10 mL) puis à nouveau avec de l'eau (10 mL). La phase organique a été séchée sur sulfate de magnésium, filtrée puis concentrée sous pression réduite pour conduire au composé **17a** sous forme d'une huile incolore qui était suffisamment pure pour être utilisée dans la suite de la synthèse sans purification supplémentaire (1 g, 95%). RMN $^1H$ (400 MHz, $CDCl_3$) $\delta$ : 7,31 (d, $J$ = 8,5 Hz, 2H), 6,78 (d, $J$ = 8,5 Hz, 2H), 4,55 (s, 2H), 3,80 (s, 3H); RMN $^{13}C$ (176 MHz, $CDCl_3$) $\delta$ : 169,3; 157,1; 132,7; 116,7; 114,3; 65,6; 52,6; HRMS (ESI+) calculée pour $C_9H_9O_3BrNa$ $[M+Na]^+$, $m/z$ 266,9633 trouvée: 266,9622; $R_f$ = 0,65 (silice; cyclohexane - acétate d'éthyle 1:1).

## Composé 17b

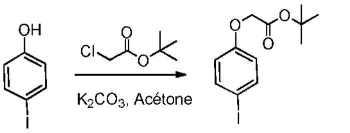


**11** **17b**

A une solution de iodophénol 11 (3,0 g, 15 mmol) dans de l'acétone (100 mL) ont été ajoutés du carbonate de

potassium (6,3 g, 45 mmol) et du tert-butyl chloroacétate (2,37 mL, 16,5 mmol). La suspension a été chauffée à reflux pendant 12 h. L'avancement de la réaction a été suivi par CCM. Après cette période la réaction était totale. Le mélange a été refroidi à température ambiante et le solvant a été éliminé sous pression réduite. Le résidu a été dissous dans du dichlorométhane (60 mL) et a été lavé avec de l'eau (2 × 40 mL). La phase organique a été séchée sur sulfate de magnésium, filtrée et concentrée sous pression réduite. Le produit brut a été purifié par chromatographie sur colonne de silice en utilisant du dichlorométhane comme éluant pour conduire à une huile jaunâtre identifiée comme le composé souhaité **17b** (4,8 g, 81%). RMN $^1H$ (400 MHz, $CDCl_3$) $\delta$ : 7,55 (d, *J* = 9,0 Hz, 2H), 6,66 (d, *J*= 9,0 Hz, 2H), 4,47 (s, 2H), 1,47 (s, 9H). RMN $^{13}C$ (100 MHz, $CDCl_3$) $\delta$ : 167,70; 157,91; 138,36; 117,06; 83,89; 82,66; 65,73; 28,13. HRMS (ESI+) calculée pour $C_{12}H_{19}INO_3$ $[M+NH_4]^+$, *m/z* 352,0410, trouvée: 352,0400. $R_f$ = 0,57 (silice; dichlorométhane 100%).

**Composé 19a**

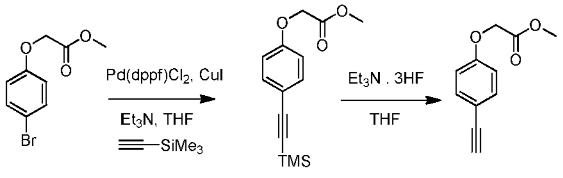


17a 18a 19a

A une solution du compose **17a** (1,20 g, 4,89 mmol) dans du tétrahydrofurane anhydre (2 mL) dégazée préalablement par trois cycles de congélation-décongélation sous vide ont été additionnés de l'éthynyltriméthylsilane (0,83 mL, 5,87 mmol) et de la triéthylamine (3,40 mL, 24,5 mmol). Le mélange a été dégazé à nouveau par trois cycles de congélation-décongélation sous vide puis on été additionnés du [1,1-Bis(diphénylphosphino)ferrocène]dichloropalladium(II) (41 mg, 0,49 mmol) et de l'iodure de cuivre (93 mg, 0,49 mmol). La solution marron a été chauffée à 65°C pendant 24 h sous atmosphère inerte. L'avancement de la réaction a été suivi par CCM. Après cette période la réaction était totale. Le solvant a été éliminé sous pression réduite et l'huile marron a été purifiée par chromatographie sur colonne de silice en utilisant un gradient d'éluant (cylohexane - acétate d'éthyle 30-70 jusquà 50-50 par incrément de 5%) conduisant au composé **18a** sous forme d'huile jaunâtre (760 mg, 60%). $R_f$ = 0,47 (silice; cyclohexane - acetate d'éthyle 75:25). Ce composé a été directement utilisé dans la suite de la synthèse. A cette huile (140 mg, 0,53 mmol) dissoute dans du tétrahydrofurane anhydre (2 mL) a été additionné de la triéthylamine trihydrofluorure (0,87 mL, 5,35 mmol) puis a été chauffée à 35 °C pendant 24 h sous atmosphère inerte. L'avancement de la réaction a été suivi par CCM. Après cette période la réaction était totale. Le solvant a été éliminé sous pression réduite et le produit réactionnel brut a été purifié par chromatographie sur colonne de silice en utilisant un éluant cyclohexane - acétate d'éthyle 70-30 pour conduire après évaporation des fractions à une huile incolore identifiée comme le composé **19a** (89 mg, 87%). RMN $^1H$ (600 MHz, $CDCl_3$) $\delta$ : 7,43 (d, *J* = 8,9 Hz, 2H), 6,85 (d, *J* = 8,9 Hz, 2H), 4,64 (s, 2H), 3,80 (s, 3H), 3,00 (s, 1H); RMN $^{13}C$ (151 MHz, $CDCl_3$) $\delta$ : 169,1; 158,2; 133,8; 115,6; 114,7; 83,4; 76,3; 65,3; 52,5. LRMS (ESI+) calculée pour $C_{11}H_9O_3$ $[M-H]^+$, *m/z* 189,06, trouvée: 189,00. $R_f$ = 0,39 (silice; cyclohexane - acétate d'éthyle 75:25).

**Composé 21**

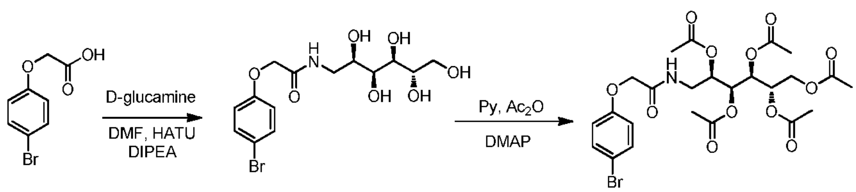


16 20 21

A une solution de d'acide 4-bromophénoxyacétique **16** (300 mg, 1,30 mmol) dans du diméthylformamide anhydre (20 mL) ont été additionnés successivement de la D-glucamine (282 mg, 1,56 mmol), du O-(7-azabenzotriazole-1-yl)-1,1,3,3-tétraméthyluronium hexafluorophosphate (HATU) (592 mg, 1,56 mmol) et de la diisopropyléthylamine (0,542 mL, 3,90 mmol). Le mélange a été agité à température ambiante pendant 16 h sous atmosphère inerte. L'avancement de la réaction a été suivi par CCM. Après cette période la réaction était totale. A ce mélange a été ajouté une solution aqueuse d'acide chlorhydrique 0,1 M (10 mL) puis le solvant a été éliminé sous pression réduite un solide jaune pâle correspondant au composé **20**. A ce solide dissous dans de la pyridine anhydre (40 mL) ont été ajoutés de l'anhydride acétique (6,18 mL, 64,9 mmol) et de la 4-diméthylaminopyridine (16 mg, 0,13 mmol). La solution a été agitée à 35°C pendant 16 h sous atmosphère inerte. L'avancement de la réaction a été suivi par CCM. Après cette période la réaction

était totale. Le solvant a été éliminé sous pression réduite et les dernières traces de réactifs ont été éliminées par distillation azéotropique pour donner une huile jaune qui a été répartie dans du dichlorométhane (30 mL) et une solution aqueuse de bicarbonate de sodium 0,5 M (30 mL). La phase aqueuse a été extraite avec du dichlorométhane (3 × 30 mL) et les phases organiques ont été réunies, lavées avec de l'eau (100 mL), séchées sur sulfate de magnésium, filtrées et concentrées sous pression réduite pour conduire à huile jaune. Le produit brut a été purifié par chromatographie sur colonne de silice en utilisant du dichlorométhane comme éluant pour conduire au composé **22** sous forme de solide blanc (481 mg, 60%). PtF : 125-126°C. RMN $^1H$ (700 MHz, $CDCl_3$) δ: 7,42 (d, *J* = 9,0 Hz, 2H), 6,85 (t, *J* = 6,0 Hz, 1H), 6,81 (d, *J* = 9,0 Hz, 2H), 5,49 (dd, *J* = 6,5; 4,7 Hz, 1H), 5,32 (dd, *J* = 5,8; 4,7 Hz, 1H), 5,17 (m, 1H), 5,02 (ddd, *J* = 6,5; 5,5; 3,3 Hz, 1H), 4,45 (q AB, 2H), 4,24 (dd, *J* = 12,5; 3,3 Hz, 1H), 4,11 (dd, *J* = 12.5; 5,5 Hz, 1H), 3,55 (m, 2H), 2,13 (s, 3H), 2,09 (s, 3H), 2,07 (s, 3H), 2,04 (s, 3H), 2,03 (s, 3H); RMN $^{13}C$ (176 MHz, $CDCl_3$) δ 170,7; 170,5; 170,2; 170,0; 169,9; 168,3; 156,4; 132,8; 116,7; 114,7; 70,2; 69,2; 69,1; 69,0; 67,6; 61,6; 39,4; 20,9; 20,8; 20,8; 20,7. HRMS (ESI+) calculée pour $C_{24}H_{30}BrNNaO_{12}$ [M+Na]$^+$, *m/z* 626,0849, trouvée: 626,0848. $R_f$ = 0,29 (silice, dichlorométhane).

## Composé 22

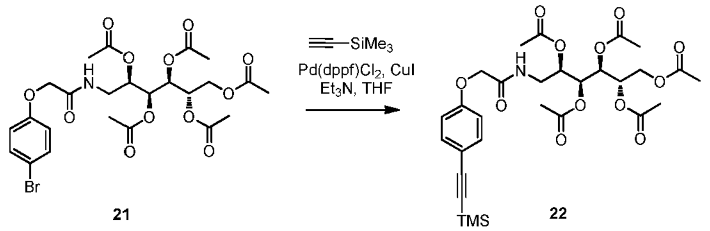


21 22

A une solution du composé **21** (290 mg, 0,48 mmol) dans du tétrahydrofurane anhydre (6 mL) dégazée préalablement par trois cycles de congélation-décongélation sous vide ont été additionnés de l'éthynyltriméthylsilane (82 mg, 0,58 mmol) et de la triéthylamine (0,67 mL, 4,81 mmol). Le mélange a été dégazé à nouveau par trois cycles de congélation-décongélation sous vide puis ont été additionnés du [1,1-Bis(diphénylphosphino)ferrocène]dichloropalladium(II) (39 mg, 0,048 mmol) et de l'iodure de cuivre (9 mg, 0,048 mmol). La solution marron a été chauffée à 65°C pendant 18 h sous atmosphère inerte. L'avancement de la réaction a été suivi par CCM. Après cette période la réaction était totale. Le solvant a été éliminé sous pression réduite et l'huile marron a été purifiée par chromatographie sur colonne de silice en utilisant un éluant (cylohexane - acétate d'éthyle 30-70) conduisant au composé **22** comme un solide blanc (182 mg, 63%). PtF : 152-154°C. RMN $^1H$ (700 MHz, $CDCl_3$) δ : 7,42 (d, *J* = 8,9 Hz, 2H), 6,86 (t, *J* = 6,1 Hz, 1H), 6,84 (d, *J* = 8,9 Hz, 2H), 5,48 (dd, *J* 6,5; 4,8 Hz, 1H), 5,33 (dd, *J* = 5,8; 4,8 Hz, 1H), 5,17 (ddd, *J* = 6,8; 5,8; 4,8 Hz, 1H), 5,02 (ddd, *J* = 6,5; 5,5; 3,4 Hz, 1H), 4,47 (q, AB, 2H), 4,25 (dd, *J* = 12,5; 3,4 Hz, 1H), 4,11 (dd, *J* = 12,5; 5,5 Hz, 1H), 3,55 (m, 2H), 2,09 (s, 3H), 2,08 (s, 3H), 2,07 (s, 3H), 2,04 (s, 3H), 2,03 (s, 3H), 0,24 (s, 9H); RMN $^{13}C$ (176 MHz, $CDCl_3$) δ : 170,7; 170,5; 170,2; 170,0; 169,9; 168,3; 157,2; 133,9; 117,2; 114,7; 104,6; 93,5; 70,2; 69,2; 69,1; 69,0; 67,3; 61,6; 39,4; 20,9; 20,8(4); 20,8(3); 20,7; 0,15. HRMS (ESI+) calculée pour $C_{29}H_{39}NNaO_{12}Si$ [M+Na]$^+$, *m/z* 644,2139, trouvée: 644,2158. $R_f$ = 0,63 (silice; cyclohexane - acétate d'éthyle 75:25).

## Composé 23

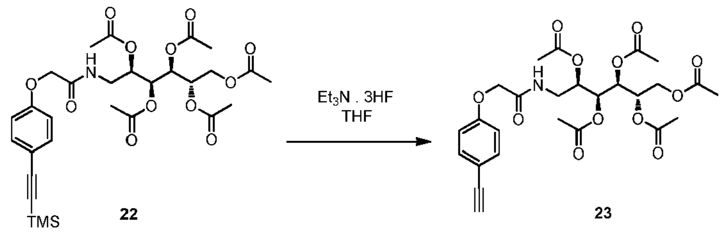


22 23

A une solution de composé 22 (109 mg, 0,17 mmol) dans du tétrahydrofurane anhydre (2 mL) a été additionnée de la triéthylamine trihydrofluorure (0,29 mL, 0,17 mmol) qui a ensuite été chauffée à 35 °C pendant 24 h sous atmosphère inerte. L'avancement de la réaction a été suivi par CCM. Après cette période la réaction était totale. Le solvant a été éliminé sous pression réduite et le produit brut a été purifié par chromatographie sur colonne de silice en utilisant un éluant cyclohexane- acétate d'éthyle 50-50 pour conduire après évaporation des fractions à un solide blanc identifié comme le composé **23** (75 mg, 78%). PtF : 148-150°C. HRMS (ESI+) calculée pour $C_{26}H_{31}NO_{12}Na$ [M+Na]$^+$, *m/z* 572,1744, trouvée: 572,1757. $R_f$ = 0,45 (silice, cyclohexane - acétate d'éthyle 70:30).

## Composé 25

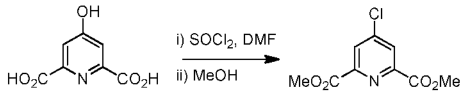


24 25

A une solution d'acide chélidamique **24** (17 g, 93 mmol) dans du chlorure de thionyle (95 mL) ont été additionnées quelques gouttes de diméthylformamide anhydre. Cette solution a été chauffée à 100°C pendant 48 h. Le chlorure de thionyle a été éliminé sous pression réduite. A ce résidu a été additionné du dichlorométhane (50 mL) et le mélange a été refroidi à 0°C. A ce mélange, a été additionné goutte à goutte sur une période de 10 min du méthanol anhydre (40 mL) et le mélange a été réchauffé à température ambiante puis a été agité pendant une nuit. Les solvants ont été éliminés sous pression réduite et à ce résidu a été additionnée une solution saturée de bicarbonate de sodium (100 mL). Le mélange a été filtré et le filtrat a été extrait avec du dichlorométhane (3 × 50 mL), les phases organiques ont été regroupées, séchées sur sultate de magnésium, filtrées et évaporées sous pression réduite pour donner un solide blanc identifié comme le composé **25**. Le produit était suffisament pur pour être utilisé dans la suite de la synthèse sans purification supplémentaire (13,3 g, 63%). PtF : 141-142°C. RMN $^1H$ (300 MHz, $CDCl_3$) $\delta$ : 8,32 (s, 2H), 4,06 (s, 6H). RMN $^{13}C$ (75 MHz, $CDCl_3$) $\delta$ : 163,59; 149,06; 145,45; 127,78; 52,95. HRMS (ESI+) calculée pour $C_9H_9NO_4Cl$ $[M+H]^+$, *m/z* 230,0215, trouvée: 230,0216. $R_f$ = 0,75 (silice, dichlorométhane - méthanol 90:10).

## Composé 26

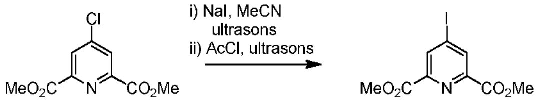


25 26

A une solution du composé 25 (6,0 g, 26,2 mmol) dans de l'acétonitrile anhydre (140 mL) a été additionné de l'iodure de sodium (39 g, 262 mmol) et le mélange a été soumis à des ultrasons pendant 20 min. A ce mélange a été additionné du chlorure d'acétyle (5,55 mL, 78,6 mmol) et le mélange a été soumis à des ultrasons pendant 5 h. A cette solution refroidie à 0°C a été additionnée une solution saturée de bicarbonate de sodium (75 mL) puis de l'eau (100 mL). Le mélange a été extrait avec de l'acétate d'éthyle (2 × 50 mL), et les phases organiques ont été réunies, lavées avec une solution de thiosufalte 0,2 M et enfin séchées sur sulfate de magnésium, filtrées puis concentrées sous pression réduite. A ce résidu a été additionné du méthanol (40 mL), le mélange a été agité pendant 20 min puis a été filtré pour donner un solide blanc identifié comme le composé **26**. Le produit était suffisament pur pour être utilisé dans la suite de la synthèse sans purification supplémentaire (6,9 g, 82%). PtF : 165-167°C. RMN $^1H$ (300 MHz, $CDCl_3$) $\delta$: 8,68 (s, 2 H), 4,05 (s, 6H). RMN $^{13}C$ (75 MHz, $CDCl_3$) $\delta$ 163,48; 149,06; 147,83; 136,29; 108,76; 52,86. HRMS (ESI+) calculée pour $C_9H_9NO_4I$ $[M+H]^+$, *m/z* 321 ,9571, trouvée: 321,9567. $R_f$ = 0,36 (silice, cyclohexane - acétate d'éthyle 50:50).

## Composé 27a

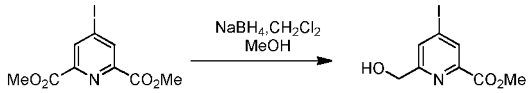


26 27a

A une solution de diester **26** (7,0 g, 21,8 mmol) dans du dichlorométhane (50 mL) refroidie à 0°C ont été additionnés du méthanol (35 mL) puis du borohydrure de sodium (540 mg, 14,2 mmol). Le mélange a été agité pendant 2 h à 0°C. L'avancement de la réaction a été suivi par CCM. Après cette période la réaction était totale. A ce mélange a été additionnée une solution aqueuse d'acide chlorhydrique 1 M (20 mL). Le solvant a été éliminé sous pression réduite et au résidu a été additionnée une solution saturée de bicarbonate de sodium (100 mL), puis le mélange a été extrait avec de l'acétate d'éthyle (4 × 30 mL). Les phases organiques ont été réunies, séchées sur sulfate de magnésium, filtrées et concentrées sous pression réduite. Le résidu a été purifié par chromatographie sur colonne de silice (cyclohexane / acétate d'éthyle 60/40 jusqu'à 0/100 par incrément de 10%) pour donner le composé **27a** comme un solide blanc (3,8 g, 60%). PtF : 119-122°C. RMN $^1H$ (300 MHz, $CDCl_3$) $\delta$ : 8,41 (s, 1H), 7,99 (s, 1H), 4,85 (s, 2H), 4,02 (s, 3H). RMN $^{13}C$ (75 MHz, $CDCl_3$) $\delta$ : 164,44; 161,44; 147,31; 133,34; 133,06; 106,97; 64,31; 53,29. HRMS (ESI+) calculée pour $C_8H_9NO_3I$ $[M+H]^+$, *m/z* 293,9622, trouvée: 293,9615. $R_f$ = 0,54 (silice, dichlorométhane - méthanol 90:10).

## Composé 27b

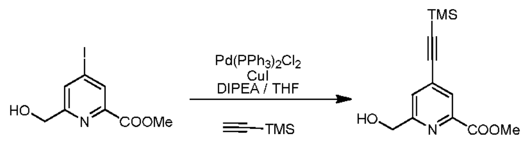


27a 27b

A la pyridine iodée **27a** (3,22 g, 11 mmol) a été ajouté du tétrahydrofurane anhydre (10 mL) et la solution a été dégazée par trois cycles de congélation-décongélation. A cette solution ont été ajoutés du triméthylsilyl acétylène (7,8 mL, 55 mmol) et de la diisopropyléthylamine (9,6 mL, 50 mmol) et la solution a été à nouveau dégazée. A cette solution ont été ajoutés du tétrakis(triphénylphosphine)palladium(0) (385 mg, 0,55 mmol) et de l'iodure de cuivre (209 mg, 0,11 mmol). Cette nouvelle solution a été à nouveau dégazée trois fois puis a été agitée à température ambiante sous atmosphère inerte. Un changement de couleur du jaune très clair au marron foncé a été observé et le mélange a été agité à 65 °C sous atmosphère inerte. L'avancement de la réaction a été suivi par CCM. Après 3 h la réaction était totale. Le mélange réactionnel a été refroidi à température ambiante et le solvant a été éliminé sous pression réduite. Le produit brut a été purifié par chromatographie sur colonne de silice (dichlorométhane /méthanol 0 à 4 % par incréments de 0,5 %) pour obtenir le composé **27b** (2,61 g, 90 %) ; RMN $^1H$ (400 MHz, $CDCl_3$) $\delta$ : 8,01 (s, 1H), 7,56 (s, 1H), 4,82 (s, 2H), 3.97 (s, 3H), 0,25 (s, 9H); RMN $^{13}C$ (100 MHz, $CDCl_3$) $\delta$ : 165,2; 160,8; 147,2; 133,4; 126,2; 126,0; 101,9; 101,3; 64,6; 53,1;- 0,3. HRMS (ESI+) calculée pour $C_{13}H_{18}NO_3Si$ $[M+H]^+$, *m/z* 264,1056 trouvée: 264,1050. R*f* = 0,68 (silice, dichlorométhane - méthanol, 90:10).

## Composé 27c

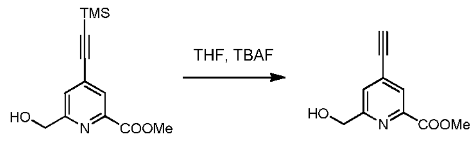


27b 27c

A une solution de composé **27b** (263 mg, 1 mmol) dans du tétrahydrofurane (10 mL) a été additionné une solution de fluorure de tétrabutylammonium dans du THF 1 M (1 mL). Le mélange a été agité à température ambiante pendant 1 h. L'avancement de la réaction a été suivi par CCM. Après cette période la réaction était totale. Le solvant a été éliminé sous pression réduite. Le résidu a été dissous dans du dichlorométhane (20 mL) et la phase organique a été lavée avec de l'eau (4 × 10 mL), séchée sur sulfate de magnésium, filtrée et concentrée sous pression réduite pour conduire au composé **27c** (162 mg, 85%).

## Composé 29

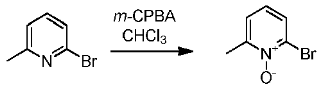


28 29

A une solution de 2-bromo-6-méthylpyridine 28 (40 g, 232,5 mmol) dans du chloroforme (400 mL) a été additionné à température ambiante de l'acide m-chloroperbenzoïque (82 g, 475 mmol). Le mélange a été chauffé à 65°C pendant 20 h puis a été refroidi à 0°C pendant 3 h. Après filtration du précipité, le filtrat a été concentré sous pression réduite. A ce résidu a été additionnée une solution aqueuse d'hydroxyde de sodium 2 M (100 mL) et cette solution a été extraite avec du dichlorométhane (4 × 100 mL). Les phases organiques ont été regroupées, séchées sur sulfate de magnésium, filtrées et concentrées sous pression réduite pour conduire au composé **29** sous forme d'un solide jaune utilisé dans la suite de la synthèse sans purification supplémentaire (35,48 g, 81%). PtF: 48-55 °C. RMN $^1H$ (400 MHz, $CDCl_3$) $\delta$ : 7,55 (dd, *J* = 7,9; 1,6 Hz, 1H), 7,23 (dd, *J* = 7,9; 1,6 Hz, 1H), 7,01 (t, *J* = 7,9 Hz, 1H), 2,57 (s, 3H); RMN $^{13}C$ (100 MHz, $CDCl_3$) $\delta$ : 151,2; 133,5; 128,7; 125,3; 125,2; 19,3. HRMS (ESI+) calculée pour $C_6H_7NOBr$ $[M+H]^+$, *m/z* 187,9711, trouvée: 187,9698. $R_f$ = 0,33 (silice, dichlorométhane - méthanol 96:4).

## Composé 30

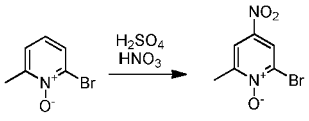


29 30

A une solution de composé N-oxyde 29 (13,2 g, 69,8 mmol) dans de l'acide sulfurique concentré à 96% (18,5 mL, 347,7 mmol) refroidie à 0°C a été additionné goutte à goutte de l'acide nitrique fumant à 52,5% (13,6 mL, 329,5 mmol). Le mélange a été agité à température ambiante pendant 15 min puis a été chauffé à 85°C pendant 16 h. La solution a été ensuite refroidie à température ambiante et a été versée dans de la glace pilée (80 g). Après 15 min d'agitation, le précipité a été filtré et a été lavé avec de l'eau (50 mL). Le solide jaune a été dissous dans du dichlorométhane et la solution a été séchée sur sulfate de magnésium, filtrée et concentrée sous pression réduite pour conduire au composé 30 sous forme d'un solide jaune qui a été utilisé dans la suite de la synthèse sans purification supplémentaire (15 g, 92%). PtF: 137-138°C. RMN $^1H$ (400 MHz, $CDCl_3$) $\delta$ : 8,40 (d, J = 2,8 Hz, 1H), 8,09 (d, *J* = 2,8 Hz, 1H), 2,62 (s, 3H); RMN $^{13}C$ (100 MHz, $CDCl_3$) $\delta$ : 151,8; 140,7; 134,0; 122,8; 118,9; 19,6. HRMS (ESI+) calculée pour $C_6H_6BrN_2O_3$ $[M+H]^+$, *m/z* 232,9562, trouvée: 232,9564. *Rf* = 0,53 (silice, dichlorométhane - méthanol 98:2).

## Composé 31

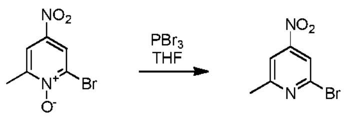


30 31

A une solution de composé N-oxyde **30** (16 g, 68,66 mmol) dans le tétrahydrofurane anhydre (500 mL), a été additionné du tribromure de phosphore (22 mL, 234 mmol) et le mélange a été chauffé à 60°C sous atmosphère inerte pendant 16 h. La solution a été refroidie à température ambiante et a été concentrée sous pression réduite puis a été versée dans une solution glacée d'hydroxyde de sodium 2 M (300 mL). Le mélange a été extrait avec du dichlorométhane (3 × 100 mL), les phases organiques ont été regroupées, séchées sur du sulfate de magnésium et concentrées sous pression réduite. L'huile résultante a été purifiée par chromatographie sur colonne de silice en utilisant le dichlorométhane comme éluant conduisant ainsi au composé **31** sous forme d'un solide jaune (5,4 g, 36%). PtF 51-52°C. RMN $^1H$ (400 MHz, $CDCl_3$) $\delta$ : 8,02 (d, *J* = 2,8 Hz, 1H), 7,83 (d, *J* = 2,8 Hz, 1H), 2,70 (s, 3H); RMN $^{13}C$ (100 MHz, $CDCl_3$) $\delta$ : 162,9; 154,9; 142,4; 118,4; 115,0; 24,6. HRMS (ESI+) calculée pour $C_6H_6BrN_2O_2$ $[M+H]^+$, *m/z* 216,9613, trouvée: 232,9621. *Rf* = 0,75 (silice, dichlorométhane - méthanol 98:2).

## Composé 32a

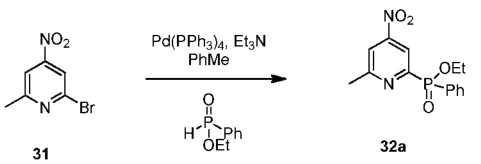


31 32a

A une solution de 2-bromo-6-méthyl-4-nitropyridine **31** (1,01 g, 4,68 mmol) dans du toluène anhydre (10 mL), ont été additionnés du phénylphosphinate d'éthyle (0,95 g, 5,60 mmol) et de la triéthylamine (2,6 mL, 19,0 mmol). Le mélange a été dégazé par trois cycles de congélation-décongélation. A cette solution a été ajouté du tétrakis(triphénylphosphine)palladium(0) (83 mg, 0,07 mmole) et le mélange a été à nouveau dégazé trois fois avant d'être agité à reflux pendant 16 h sous atmosphère inerte. L'avancement de la réaction a été suivi par CCM. Après cette période, la réaction était totale. La solution a été refroidie et a été diluée avec du dichlorométhane (20 mL). Le mélange a été lavé avec une solution aqueuse d'acide chlorhydrique 1 M (2 × 15 mL) suivi d'un lavage avec de l'eau (3 × 15 mL). La phase organique a été séchée sur du sulfate de magnésium, filtrée et le solvant a été éliminé sous pression réduite pour obtenir un résidu sombre qui a été purifié par chromatographie sur colonne de silice (dichlorométhane / méthanol 0,5%) conduisant ainsi au composé **32a** sous la forme d'une huile jaune (645 mg, 45 %). RMN $^1H$ (400 MHz, $CDCl_3$) $\delta$ : 8,55 (dd, *J* = 5,6; 1,4 Hz, 1H), 7,97 (ddd, *J* = 11,2; 7,7 Hz, 1,4 Hz, 2H), 7,90 (d, *J* = 1,4 Hz, 1H), 7,55 (td, *J* = 7,7; 1,4 Hz, 1H), 7,46 (td, *J* =7,7; 3,5 Hz, 2H), 4,15 (qd, *J* = 7,0; 4,2 Hz, 2H), 2,72 (s, 3H), 1,38 (t, *J* = 7,0 Hz, 3H) ; RMN $^{13}C$ (100 MHz, $CDCl_3$) $\delta$ : 163,0

(d, *J* = 21 Hz), 158,1 (d, *J* = 167 Hz), 154,0 (d, *J* =13 Hz), 132,9 (d, *J* = 3 Hz), 132,5 (d, *J* = 10 Hz), 129,1 (d, *J* = 140 Hz), 128,5 (d, *J* = 13 Hz), 117,6 (d, *J* = 24 Hz), 117,5 (d, *J* = 3 Hz), 62,2 (d, *J* = 6 Hz), 24,9, 16,4; RMN $^{31}P$ (162 MHz, $CDCl_3$) δ: 23,7. HRMS (ESI+) calculée pour $C_{14}H_{16}N_2O_4P$ $[M+H]^+$, *m/z* 307,0848, trouvée: 307,0851. *Rf* = 0,47 (silice, dichlorométhane - méthanol 95:5).

## Composé 32b

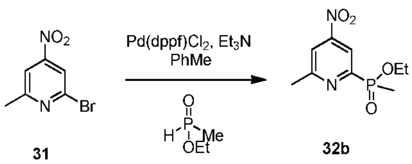


31 32b

A du diéthylméthyl phosphonite (2 g, 14,7 mmol) a été ajoutée à 0°C de l'eau (264 μL, 14,7 mmol) sans précaution particulière. Le mélange réactionnel a été réchauffé à température ambiante en 1 h puis laissé sous agitation pendant 16 h à température ambiante. A ce mélange ont été ajoutés une solution de 2-bromo-6-méthyl-4-nitropyridine **31** (2,65 g, 12,3 mmol) dans du toluène anhydre (35 mL) puis de la triéthylamine (6 mL, 43,0 mmol). Le mélange a été dégazé par trois cycles de congélation-décongélation. A cette solution a été ajouté du bis(diphénylphosphino)ferrocène]dichloropalladium(II) (800 mg, 1,1 mmol) et le mélange a été à nouveau dégazé trois fois avant d'être agité à reflux pendant 16 h sous atmosphère inerte. L'avancement de la réaction a été suivi par CCM. Après cette période, la réaction était totale. La solution a été refroidie et concentrée sous pression réduite puis diluée avec du dichlorométhane (20 mL). Le mélange a été lavé avec une solution aqueuse d'acide chlorhydrique 1 M (2 × 15 mL) puis avec de l'eau (3 × 15 mL). La phase organique a été séchée sur du sulfate de magnésium, filtrée et le solvant a été éliminé sous pression réduite pour obtenir un résidu sombre qui a été purifié par chromatographie sur colonne sur silice (dichlorométhane / méthanol 1,6% par incréments de 0,1%) conduisant ainsi au composé **32b** sous la forme d'une huile incolore (2,1 g, 59%). RMN $^1H$ (400 MHz, $CDCl_3$) δ : 8,54-8,56 (m, 1H), 7,96-8,01 (m, 1H), 3,68-4,44 (m, 2H), 2,79 (s, 3H), 1,83 (d, *J* = 15 Hz, 3H), 1,31 (t, *J* = 7,1 Hz, 3H); RMN $^{13}C$ (100 MHz, $CDCl_3$) δ: 163,2 (d, *J* = 21 Hz), 158,1 (d, *J* = 157 Hz), 154,3 (d, *J* = 157 Hz), 117,9 (d, *J* = 3 Hz), 117,2 (d, *J* = 23 Hz), 61,6 (d, *J* = 7 Hz), 24,9; 16,6 (d, *J* = 6 Hz), 13,5 (d, *J* = 105 Hz); RMN $^{31}P$ (162 MHz, $CDCl_3$) δ : + 37,2. HRMS (ESI+) calculée pour $C_9H_{13}N_2O_4P$ $[M+H]^+$, *m/z* 244,0613, trouvée: 244,0607. $R_f$ = 0,26 (silice, dichlorométhane - méthanol 95:5).

## Composé 33a

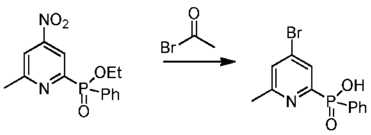

32a 33a

Au dérivé nitré **32a** (2,00 g, 6,54 mmol) a été ajouté du bromure d'acétyle (15 mL, 0,2 mol) et le mélange a été agité à 70°C pendant 16 h sous argon. Au cours de cette période un précipité brun pâle s'était formé. La suspension a été versée lentement dans du méthanol (100 mL) refroidi à 0°C sur une période de 10 min. Le mélange a été agité pendant 10 min à température ambiante. Le solvant a été éliminé sous pression réduite pour conduire au composé souhaité sous la forme d'un solide brun pâle qui a été utilisé directement dans la suite de la synthèse sans purification (1,81 g, 90 %) ; RMN $^1H$ (400 MHz, $CD_3OD$) δ : 8,33 (dd, *J* = 7,2 Hz, 2,0 Hz, 1H), 8,23 (d, *J* = 2,0 Hz, 1H), 7,95 (ddd, *J* =13,2 Hz, 7,6 Hz, 1,6 Hz, 2H), 7,63 (1H, td, *J* = 7,6 Hz, 1,6 Hz, 1H), 7,55 (2H, td, *J* = 7,6 Hz, 3,6 Hz), 2,77 (3H, s) ; RMN $^{13}C$ (100 MHz, $CD_3OD$) δ : 159,4 (d, *J* = 20 Hz), 151,7 (d, *J* = 160 Hz), 145,1 (d, *J* = 10 Hz), 134,8 (d, *J* = 3 Hz), 133,3 (d, *J* = 10 Hz), 131,0 (d, *J* = 24 Hz), 130,6 (d, *J* = 3 Hz), 130,2 (d, *J* = 140 Hz), 129,6 (d, *J* = 12 Hz), 20,4 ; RMN $^{31}P$ (162 MHz, $CD_3OD$) δ : 14,3. HRMS (ESI+) calculée pour $C_{12}H_{10}NO_2P$ $[M+H]^+$, *m/z* 309,9633, trouvée: 309,9648. $R_f$ = 0,01 (silice, dichlorométhane - méthanol 95:5).

## Composé 33b

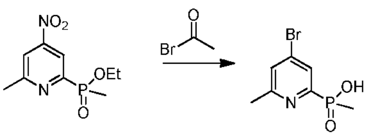

32b 33b

Au dérivé nitré **32b** (5 g, 20,5 mmol) a été ajouté du bromure d'acétyle (40 mL, 541 mmol) et le mélange a été agité à 70°C pendant 16 h sous argon. Au cours de cette période un précipité brun pâle s'était formé. La suspension a été versée lentement dans du méthanol (100 mL) refroidi à 0°C sur une période de 10 min. Le mélange a été agité à température ambiante pendant 10 min. Le solvant a été éliminé sous pression réduite pour conduire au composé souhaité sous la forme d'un solide brun pâle qui a été utilisé directement dans la suite de la synthèse sans purification (4,57 g, 90%) ; RMN $^1$H (400 MHz, $CD_3OD$) δ : 8,25-8,28 (m, 1H), 8,17-8,18 (m, 1H), 2,76 (s, 3H), 1,80 (d, *J* = 16 Hz, 3H) ; RMN $^{31}$P (162 MHz, $CD_3OD$) δ : + 28,6. HRMS (ESI+) calculée pour $C_7H_{10}BrNO_2P$ $[M+H]^+$, *m/z* 249,9633, trouvée: 249,9627. $R_f$ = 0,01 (silice, dichlorométhane - méthanol 95 :5).

## Composé 34a

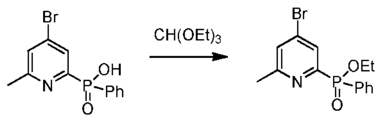


33a 34a

A l'acide phosphinique **33a** (1,80 g, 5,80 mmol) a été ajouté de l'orthoformiate d'éthyle (50 mL) et le mélange a été agité à 140°C pendant 72 h sous atmosphère inerte. L'avancement de la réaction a été suivi par CCM. Après cette période, la réaction était totale. La solution a été refroidie à température ambiante et le solvant a été éliminé sous pression réduite. Le résidu a été purifié par chromatographie sur colonne de silice (dichlorométhane / méthanol 0,5 %) pour conduire au composé **34a** sous la forme d'une huile jaune (1,08 g, 55 %). RMN $^1$H (400 MHz, $CDCl_3$) δ : 8,04 (dd, *J* = 6,3 Hz, 1,4 Hz, 1H), 7,95 (ddd, *J* = 11,2; 7,0; 1,4 Hz, 2H), 7,51 (1H, td, 7,0; 1,4 Hz, 1H), 7,43 (td, *J* = 7,0; 3,5 Hz, 2H), 7,37 (d, *J* = 1,4 Hz, 1H), 4,11 (qd, *J* = 7,0; 4,2 Hz, 2H), 2,52 (s, 3H), 1,34 (t, *J* = 7,0 Hz, 3H) ; RMN $^{13}$C (100 MHz, $CDCl_3$) δ : 161,2 (d, *J* = 22 Hz), 155,7 (d, *J* = 165 Hz), 133,5 (d, *J* = 15 Hz), 132,7 (d, *J* = 3 Hz), 132,6 (d, *J* = 10 Hz), 130,0 (d, *J* = 139 Hz), 128,5 (d, *J* = 3 Hz), 128,4 (d, *J* = 23 Hz), 128,3 (d, *J* = 13 Hz), 62,1 (d, *J* = 6 Hz), 24,5, 16,7 ; RMN $^{31}$P (162 MHz, $CDCl_3$) δ : 25,5. HRMS (ESI+) calculée pour $C_{14}H_{16}NO_2BrP$ $[M+H]^+$, *m/z* 340,0102, trouvée: 340,0102. $R_f$ = 0,3 (silice, cyclohexane - acétate d'éthyle 30:70).

## Composé 34b

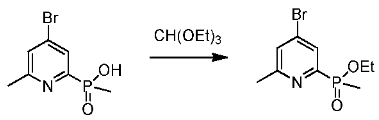


33b 34b

A l'acide phosphinique **33b** (4,54 g, 18,4 mmol) a été ajouté de l'orthoformiate d'éthyle (46 mL) et le mélange a été agité à 140°C pendant 42 h sous argon. L'avancement de la réaction a été suivi par CCM. Après cette période, la réaction était totale. La solution a été refroidie à température ambiante et le solvant a été éliminé sous pression réduite. Le résidu a été purifié par chromatographie sur colonne de silice (dichlorométhane / méthanol 1 %) pour conduire au composé **34b** sous la forme d'une huile jaune (3,9 g, 68 %) ; RMN $^1$H (400 MHz, $CDCl_3$) δ : 8,04 (dd, *J* = 5,9; 1,8 Hz, 1H), 7,46 (s app, 1H), 3,82-4,16 (m, 2H), 2,59 (s, 3H), 1,77 (d, *J* = 15 Hz, 3H), 1,28 (t, *J* = 7,1 Hz, 3H); RMN $^{13}$C (100 MHz, $CDCl_3$) δ : 161,1 (d, *J* = 21 Hz), 155,4 (d, *J* = 156 Hz), 133,6 (d, *J* = 14 Hz), 128,9 (d, *J* = 3 Hz), 128,2 (d, *J* = 22 Hz), 61,3 (d, *J* = 6 Hz), 24,4; 16,5 (d, *J* = 6 Hz), 13,6 (d, *J* = 104 Hz); RMN $^{31}$P (162 MHz, $CDCl_3$) δ : + 38,0; HRMS (ESI+) calculée pour $C_9H_{13}NO_2BrP$ $[M+H]^+$, *m/z* 276,9867, trouvée: 276,9862. $R_f$ = 0,52 (silice, dichlorométhane-méthanol 95:5).

## Composé 35a

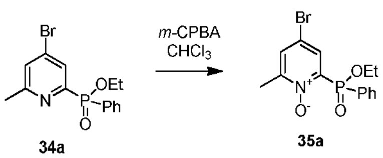


34a 35a

A une solution de dérivé pyridinyle **34a** (1,25 g, 3,68 mmol) dans du chloroforme (20 mL) a été ajouté de l'acide méta-chloroperbenzoïque (1,27 g, 7,35 mmol). La solution résultante a été agitée à 65°C pendant 16 h sous atmosphère

inerte. L'avancement de la réaction a été suivi par CCM. Après cette période, la réaction était totale. Le mélange a été refroidi à température ambiante et le solvant a été éliminé sous pression réduite pour obtenir une huile jaune. Cette huile a été dissoute dans du dichlorométhane (50 mL) et a été lavée avec une solution aqueuse de bicarbonate de sodium 0,5 M (50 mL). Les phases ont été séparées et la phase aqueuse a été extraite avec du dichlorométhane (3 × 30 mL). Les phases organiques ont été réunies, ont été séchées sur sulfate de magnésium, filtrées et le solvant a été éliminé sous pression réduite. L'huile jaune résultante a été purifiée par chromatographie sur colonne de silice (dichlorométhane / méthanol 0 à 2 % par incréments de 0,1 %) pour obtenir une huile jaune correspondant au composé **35a** (1,11 g, 75 %). RMN $^{1}H$ (400 MHz, $CDCl_3$) δ : 8,05 (dd, *J* = 7,7 Hz, 2,1 Hz, 1H), 7,98 (dd, *J* = 7,7 Hz, 13,3 Hz, 2H), 7,50 (t, 7,7 Hz, 1H), 7,44 (d, *J* = 2,1 Hz, 1H), 7,41 (td, *J* = 7,7 Hz, 4,2 Hz, 2H), 4,13 (qd, *J* = 5,6 Hz, 4,9 Hz, 2H), 2,32 (s, 3H), 1,34 (t, *J* = 5,6 Hz, 3H) ; RMN $^{13}C$ (100 MHz, $CDCl_3$) δ : 151,0 (d, *J* = 4 Hz), 144,2 (d, *J* = 149 Hz), 133,2 (d, *J* = 11 Hz), 133,1 (d, *J* = 4 Hz), 133,0 (d, *J* = 11 Hz), 132,2 (d, *J* = 4 Hz), 129,0 (d, *J* = 152 Hz), 128,4 (d, *J* = 14 Hz), 117,4 (d, *J* = 12 Hz), 62,3 (d, *J* = 6 Hz), 17,5, 16,7 ; RMN $^{31}P$ (162 MHz, $CDCl_3$) δ : +21,2. HRMS (ESI+) calculée pour $C_{14}H_{16}NO_3BrP$ $[M+H]^+$, *m/z* 356,0051, trouvée: 356,0061. $R_f$ = 0,5 (silice, dichlorométhane - méthanol 90:10).

## Composé 35b

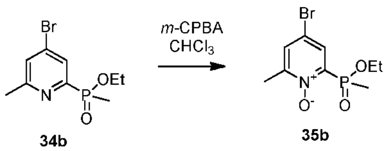


**34b** **35b**

A une solution de dérivé pyridinyle **34b** (3,5 g, 11,87 mmol) dans du chloroforme (40 mL) a été ajouté de l'acide méta-chloroperbenzoïque (4,1 g, 23.8 mmol). La solution résultante a été agitée à 65°C pendant 2 h sous argon. L'avancement de la réaction a été suivi par CCM. Après cette période, la réaction était totale. Le mélange a été refroidi à température ambiante et le solvant a été éliminé sous pression réduite pour obtenir une huile jaune. Cette huile a été dissoute dans du dichlorométhane (50 mL) et a été lavée avec une solution de bicarbonate de sodium 0,5 M (100 mL). Les phases ont été séparées et la phase aqueuse a été extraite avec du dichlorométhane (3 × 100 mL). Les phases organiques ont été réunies, ont été séchées sur sulfate de magnésium et le solvant a été éliminé sous pression réduite. L'huile jaune résultante a été purifiée par chromatographie sur colonne de silice (dichlorométhane / méthanol 0 à 2 % par incréments de 0,1 %) pour obtenir une huile jaune correspondant au composé **35b** (2,6 g, 75 %). RMN $^{1}H$ (400 MHz, $CDCl_3$) δ : 8,04 (dd, *J* = 7,9; 2,9 Hz, 1H), 7,54 (dd, *J* = 2,9 Hz, 1H), 3,89-4,22 (m, 2H), 2,48 (s, 3H), 1,97 (d, *J* = 17 Hz, 3H), 1,31 (t, *J* = 7,1 Hz, 3H); RMN $^{13}C$ (100 MHz, $CDCl_3$) δ : 151,1 (d, *J* = 4 Hz), 143,8 (d, *J* = 136 Hz), 133,4 (d, *J* = 11 Hz), 132,3 (d, *J* = 2 Hz), 118,0 (d, *J* = 12 Hz), 62,1 (d, *J* = 7 Hz), 17,5; 16,6 (d, *J* = 6 Hz), 14,5 (d, *J* = 111 Hz); RMN $^{31}P$ (162 MHz, $CDCl_3$) δ : + 32,2; *m/z* HRMS (ESI+) calculée pour $C_9H_{13}BrNO_3P$ $[M+H]^+$, *m/z* 292,9816, trouvée: 292,9811. $R_f$= 0,27 (silice, dichlorométhane - méthanol 95:5).

## Composé 36a

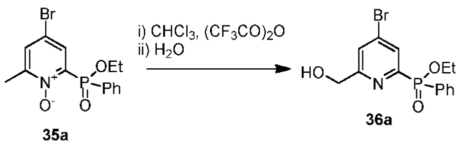


**35a** **36a**

Au dérivé pyridinyl N-oxyde **35a** (1,7 g, 4,8 mmol) ont été ajouté du chloroforme (35 mL) et de l'anhydride trifluoroacétique (25 mL). La solution a été chauffée à 60°C pendant 2 h sous agitation. L'avancement de la réaction a été suivi par CCM. Après cette période, la réaction était totale. La solution a été refroidie à température ambiante et le solvant a été éliminé sous pression réduite. Le résidu a été purifié par chromatographie sur colonne de silice (dichlorométhane / méthanol de 0 à 5 % par incréments de 1 %) pour obtenir un solide blanc correspondant au composé **36a** (1,6 g, 97%). PtF 98-100°C. RMN $^{1}H$ (400 MHz, $CDCl_3$) δ : 8,16 (dd, *J* = 6,0; 1,3 Hz, 1H), 7,95 (ddd, *J* = 12,3; 8,3;1,4 Hz, 2H), 7,62 (s, 1H); 7,60-7,56 (m, 1H); 7,53-7,46 (m, 2H); 4,78 (s, 2H); 4,67 (s l, 1H); 4,24-4,10 (m, 2H); 1,40 (t, J = 7,0 Hz, 3H). HRMS (ESI+) calculée pour $C_{14}H_{16}NO_3BrP$ $[M+H]^+$, *m/z* 356,0051, trouvée: 356,0049. $R_f$ = 0,44 (silice, dichlorométhane - méthanol 90:10).

## Composé 36b

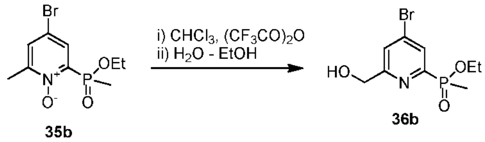

35b
36b

Au dérivé pyridinyl N-oxyde **35b** (3,0 g, 10,23 mmol) ont été ajouté du chloroforme (60 mL) et de l'anhydride trifluoroacétique (28 mL). La solution a été chauffée à 60°C pendant 2 h sous agitation. L'avancement de la réaction a été suivi par CCM. Après cette période, la réaction était totale. La solution a été refroidie à température ambiante et le solvant a été éliminé sous pression réduite. A ce résidu ont été ajouté de l'éthanol (5 mL) et de l'eau (5 mL). La solution a été agitée à température ambiante pendant 12 h puis concentrée sous pression réduite. A ce résidu ont été ajoutés du dichlorométhane (70 mL) et de l'eau (70 mL). La phase organique a été séparée puis séchée sur sulfate de magnésium, filtrée et évaporée sous pression réduite pour conduire au composé brut qui a été purifié par chromatographie sur colonne de silice (dichlorométhane / méthanol de 0 à 5 % par incréments de 1 %) pour obtenir une huile jaunâtre correspondant au composé **36b** (2,2 g, 67 %). RMN $^{1}H$ (400 MHz, $CDCl_3$) δ : 8,13 (dd *J* = 5,9; 1,8 Hz, 1H), 7,63 (s app, 1H), 4,80 (s, 2H), 3,70-4,20 (m, 2H), 3,26 (si, 1H), 1,76 (d, *J* = 15 Hz, 3H), 1,27 (t, *J* = 7,1 Hz, 3H); RMN $^{13}C$ (100 MHz, $CDCl_3$) δ : 162,8 (d, *J* = 20 Hz), 154,8 (d, *J* = 154 Hz), 134,5 (d, *J* = 13 Hz), 129,5 (d, *J* = 22 Hz), 126,3 (d, *J* = 3 Hz), 64,2; 61,5 (d, *J* = 6 Hz), 16,5 (d, *J* = 6 Hz), 13,6 (d, *J* = 105 Hz); RMN $^{31}P$ (162 MHz, $CDCl_3$) δ : + 38,0; HRMS (ESI+) calculée pour $C_9H_{13}NO_3BrP$ $[M+H]^+$, *m/z* 292,9816, trouvée: 292,9813. $R_f$ = 0,24 (silice, dichlorométhane - méthanol 95:5).

## Composé 36c

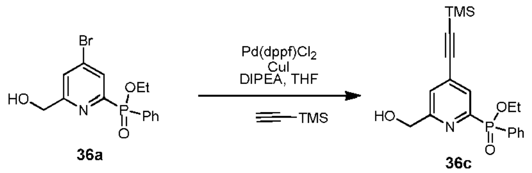

36a
36c

A la pyridine bromée **36a** (178 mg, 0,5 mmol) a été ajouté du tétrahydrofurane anhydre (5 mL) et la solution a été dégazée par trois cycles de congélation-décongélation. A cette solution ont été ajoutés le triméthylsilyl acétylène (355 µL, 2,5 mmol) et de la diisopropyléthylamine (227 µL, 2,5 mmol) et la solution a été à nouveau dégazée. A cette solution ont été ajoutés du [1,1-Bis(diphénylphosphino)ferrocène]dichloropalladium(II) (37 mg, 0,05 mmol) et de l'iodure de cuivre (10 mg, 0,05 mmol). Cette nouvelle solution a été à nouveau dégazée trois fois puis a été agitée sous atmosphère inerte. Un changement de couleur du jaune très clair au marron foncé a été observé et le mélange a été agité à 65 °C sous atmosphère inerte. L'avancement de la réaction a été suivi par CCM. Après 1 h la réaction était totale. Le mélange réactionnel a été refroidi à température ambiante et le solvant a été éliminé sous pression réduite. Le résidu a été dilué dans du dichlorométhane (60 mL) et lavé avec une solution aqueuse saturée de chlorure d'ammonium (60 mL) puis avec de l'eau (60 mL). La phase organique a été séchée sur sulfate de magnésium, filtrée puis concentrée sous pression réduite pour conduire à un résidu. Le produit brut a été purifié par chromatographie sur colonne de silice (dichlorométhane /méthanol 0 à 6 % par incréments de 1 %) pour obtenir le composé **36c** (163 mg, 87 %). HMRS (ESI+) calculée pour $C_{19}H_{25}NO_3PSi$ $[M+H]^+$, *m/z* 374,1336 trouvée: 374,1336. $R_f$ = 0,44 (silice; dichlorométhane - méthanol: 90 :10).

## Composé 36d

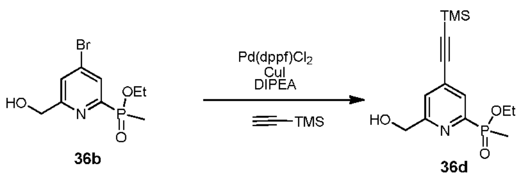

36b
36d

A la pyridine bromée **36b** (250 mg, 0,85 mmol) a été ajouté du tétrahydrofurane anhydre (5 mL) et la solution a été dégazée par trois cycles de congélation-décongélation. A cette solution ont été ajoutés le triméthylsilyl acétylène (600 µL, 4,3 mmol) et de la diisopropyléthylamine (750 µL, 4,3 mmol) et la solution a été à nouveau dégazée. A cette

solution ont été ajoutés du [1,1-Bis(diphénylphosphino)ferrocène]dichloropalladium(II) (63 mg, 0,85 mmol) et de l'iodure de cuivre (16 mg, 0,085 mmol). Cette nouvelle solution a été à nouveau dégazée trois fois puis a été agitée sous atmosphère inerte et le mélange a été agité à 65 °C. L'avancement de la réaction a été suivi par CCM. Après 1 h la réaction était totale. Le mélange réactionnel a été refroidi à température ambiante et le solvant a été éliminé sous pression réduite. Le résidu a été dilué dans du dichlorométhane (50 mL) et lavé avec une solution aqueuse saturée de chlorure d'ammonium (50 mL) puis avec de l'eau (50 mL). La phase organique a été séchée sur sulfate de magnésium, filtrée puis concentrée sous pression réduite pour conduire à un résidu. Le produit brut a été purifié par chromatographie sur colonne de silice (dichlorométhane /méthanol 0 à 4 % par incréments de 1 %) pour obtenir le composé **36d** (265 mg, 98 %). LMRS (ESI+) calculée pour $C_{14}H_{23}NO_3PSi$ $[M+H]^+$, *m/z312,1185* trouvée: 312,30. $R_f$= 0,48 (silice; dichlorométhane - méthanol: 90 :10).

**Composé 36e**

Ce composé a été préparé selon la même procédure que celle utilisée pour le composé 36f, en utilisant le précurseur correspondant.

**Composé 36f**

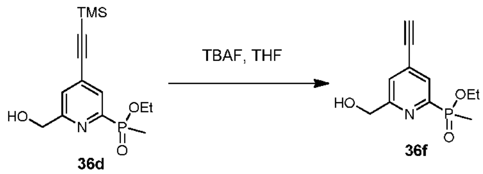


36d 36f

A une solution du dérivé sililé **36d** (200 mg, 643 µmol) dans du tétrahydrofurane anhydre (10 mL) a été ajoutée une solution de fluorure de tétrabutyle ammonium dans du tétrahydrofurane anhydre 1 M (500 µL, 643 µmol). La solution a été agitée à température ambiante pendant 1 h sous atmosphère inerte. L'avancement de la réaction a été suivi par CCM. Après cette période la réaction était totale. Le solvant a été éliminé sous pression réduite et le résidu a été dilué dans du dichlorométhane (50 mL) et a été lavé avec de l'eau (2 × 50 mL). La phase organique a été séchée sur sulfate de magnésium et a été filtrée puis concentrée sous pression réduite pour conduire au composé **36f** désiré qui a été utilisé dans la suite de la synthèse sans purification supplémentaire (146 mg, 95%). LRMS (ESI+) calculée pour $C_{11}H_{15}NO_3P$ $[M+H]^+$, *m/z* 240,0790 trouvée: 240,33. $R_f$ = 0,42 (silice; dichlorométhane - méthanol 90:10).

**Composé 37a**

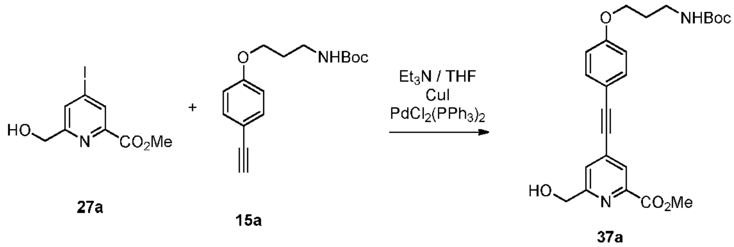


27a 15a 37a

Une solution de dérivé acétylènique **15a** (0,864 g, 3,1 mmol) et de dérivé iodé **27a** (0,735 g, 2,5 mmol) dans un mélange de tétrahydrofurane anhydre (20 mL) et de triéthylamine (20 mL) a été dégazée sous agitation pendant 20 min. A cette solution ont été additionnés du bis-chlorure bis-triphénylphosphine de palladium (II) (22 mg, 0,031 mmol) et de l'iodure de cuivre (I) (12 mg, 0,063 mmol). La réaction a été agitée à température ambiante pendant 12 h. L'avancement de la réaction a été suivi par CCM. Après cette période la réaction était totale. Les solvants ont été éliminés sous pression réduite. Au résidu a été additionnée une solution de chlorure d'ammonium saturée (50 mL) et le mélange a été extrait avec du dichlorométhane (2 × 25 mL). Les phases organiques ont été réunies, lavées par une solution de chlorure d'ammonium saturée (50 mL) puis avec une solution saturée de chlorure de sodium (2 × 50 mL) et ensuite séchées sur sulfate de magnésium. Après filtration le solvant a été éliminé sous pression réduite et le résidu a été purifié par chromatographie sur colonne de silice (dichlorométhane / méthanol 98/2) pour conduire au composé **37a** sous forme d'un solide blanc (0,91 g, 82%). PtF : 143-144°C. RMN $^1H$ (500 MHz, $CDCl_3$) δ: 8,04 (s, 1H), 7,58 (s, 1H), 7,45 (d, *J* = 8,7 Hz, 2H), 6,86 (d, *J* = 8,7 Hz, 2H), 4,83 (d, *J* = 5,4 Hz, 2H), 4,75 (s, 1H), 4,01 (t, *J* = 6,1 Hz, 2H), 3,97 (s, 3H), 3,31 (td, *J* = 6,1; 6,1 Hz, 2H), 1,96 (m, *J* = 6,1 Hz, 2H), 1,41 (s, 9H). RMN $^{13}C$ (125 MHz, $CDCl_3$) δ: 165,4; 160,8; 160,1;

156,2; 147,3; 134,1; 133,8; 125,8; 125,4; 114,9; 113,9; 95,9; 85,4; 66,1; 64,8; 53,2; 38,1; 29,7; 28,6. HRMS (ESI+) calculée pour $C_{24}H_{28}N_2O_6$ [M+H]+, *m/z* 441,2020, trouvée: 441,2021. $R_f$ = 0,32 (silice, dichlorométhane - méthanol 96:4).

**Composé 37b**

Ce composé a été préparé selon la même procédure que celle utilisée pour le composé **37c** en utilisant le précurseur correspondant.

**Composé 37c**

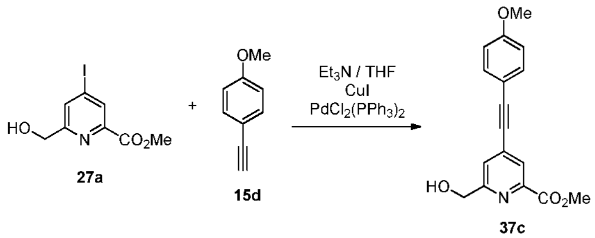


27a 15d 37c

Une solution de dérivé acétylènique **15d** (133 mg, 1 mmol) et de dérivé iodé **27a** (294 mg, 1 mmol) dans un mélange de tétrahydrofurane anhydre (8 mL) et de triéthylamine (5 mL) a été dégazée sous agitation pendant 20 min. A cette solution ont été additionnés du bis-chlorure bis-triphénylphosphine de palladium (II) (68 mg, 0,1 mmol) et de l'iodure de cuivre (I) (37 mg, 0,2 mmol). La réaction a été agitée à 75°C pendant 12 h. L'avancement de la réaction a été suivi par CCM. Après cette période la réaction était totale. Les solvants ont été éliminés sous pression réduite. Au résidu a été additionnée une solution de chlorure d'ammonium saturée (50 mL) et le mélange a été extrait avec du dichlorométhane (2 × 25 mL). Les phases organiques ont été réunies, lavées par une solution de chlorure d'ammonium saturée (50 mL) puis avec une solution saturée de chlorure de sodium (2 × 50 mL) et ensuite séchées sur sulfate de magnésium. Après filtration le solvant a été éliminé sous pression réduite et le résidu a été purifié par chromatographie sur colonne de silice en utilisant un gradient d'éluant (dichlorométhane/méthanol 100/0 jusqu'à 95/5 par incrément de 0.5%) pour conduire au composé **37c** (241 mg, 68%). RMN $^1H$ (400 MHz, $CDCl_3$) $\delta$ : 8,07 (s, 1H), 7,59 (s, 1H), 7,49 (d, *J* = 8,9 Hz, 2H), 6,90 (d, *J* = 8,9 Hz, 2H), 4,85 (s, 2H), 4,00 (s, 3H), 3,84 (s, 3H). RMN $^{13}C$ (100 MHz, $CDCl_3$) $\delta$ : 165,4; 160,7; 160,6; 147,3; 134,0; 133,8; 125,8; 125,4; 114,4; 113,8; 96,0; 85,3; 64,7; 55,5; 53,1. HRMS (ESI+) calculée pour $C_{17}H_{16}NO_4$ [M+H]+, *m/z* 298,1079, trouvée: 298,1075. R*f* = 0,6 (silice, dichlorométhane - méthanol, 90:10).

**Composé 37d**

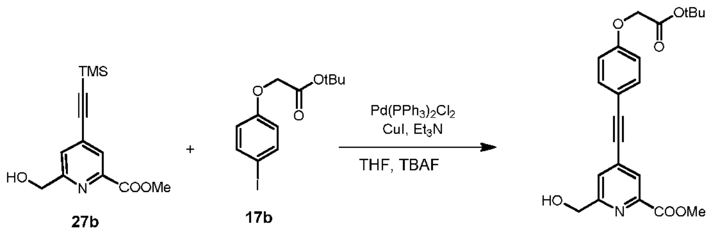


27b 17b 37d

A une solution de dérivé acétylénique **27b** (263 mg, 1 mmol) dans du tétrahydrofurane anhydre (10 mL) et de la triéthylamine anhydre (5 mL) a été additionné du dérivé iodé **17b** (370 mg, 1 mmol). Le mélange a été dégazé sous agitation pendant 1 h. A cette solution ont été additionnés une solution de fluorure de tétrabutylammonium dans du tétrahydrofurane 1 M (1,5 mL, 1,5 mmol), du bis-chlorure bis-triphénylphosphine de palladium (II) (72 mg, 0,1 mmol) et de l'iodure de cuivre (I) (40 mg, 0,2 mmol). Le milieu réactionnel a été agité à 75 °C pendant 3 h à l'abri de la lumière. L'avancement de la réaction a été suivi par CCM. Après cette période la réaction était totale. Les solvants ont été éliminés sous pression réduite. Au résidu noir a été additionnée de l'eau (10 mL) et le mélange a été extrait avec du dichlorométhane (3 × 20 mL). Les phases organiques ont été réunies et séchées sur sulfate de magnésium. Après filtration, le solvant a été éliminé sous pression réduite. Le produit brut obtenu a été purifié par chromatographie flash sur colonne de silice (dichlorométhane / méthanol 0,5% jusqu'à 5 % par incréments de 1%) pour conduire au composé **37d** sous forme d'une huile jaunâtre (203 mg, 54%). RMN $^1H$ (400 MHz, $CDCl_3$) $\delta$ : 7,95 (s, 1H), 7,59 (s, 1H), 7,40 (d, *J* = 8,8 Hz, 2H), 6,81 (d, *J* = 8,8 Hz, 2H), 4,80 (s, 2H), 4,47 (s, 2H), 3,89 (s, 3H), 1,41 (s, 9H). RMN $^{13}C$ (100 MHz, $CDCl_3$) $\delta$ : 167,47; 165,16; 161,32; 158,69; 133,54; 131,93; 131,83; 128,43; 125,31; 114,72; 114,56; 95,26; 85,41; 82,61; 65,43; 65,54; 52,87;

27,91. HRMS (ESI+) calculée pour $C_{18}H_{18}NO_5$ $[M+H]^+$, *m/z* 398,1604 trouvée: 398,1597. R*f* = 0,74 (silice, dichlorométhane - méthanol, 90:10).

### Composé 37e

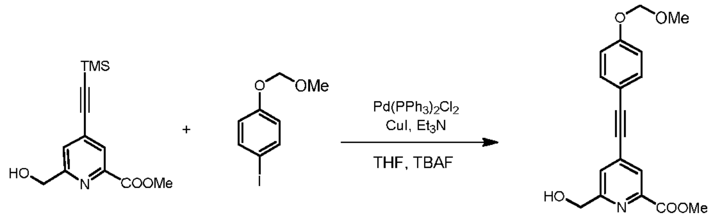


27b 13d 37e

A une solution de dérivé acétylénique **27b** (124 mg, 0,4 mmol) dans du tétrahydrofurane anhydre (8 mL) et de la triéthylamine anhydre (5 mL) a été additionné du dérivé iodé **13d** (105 mg, 0,4 mmol). Le mélange a été dégazé sous agitation pendant 1 h. A cette solution ont été additionnés une solution de fluorure de tétrabutylammonium dans du tétrahydrofurane 1 M (600 μL, 0,6 mmol), du bis-chlorure bis-triphénylphosphine de palladium (II) (28 mg, 0,04 mmol) et de l'iodure de cuivre (I) (15 mg, 0,1 mmol). Le milieu réactionnel a été agité à 75 °C pendant 2 h 30 à l'abri de la lumière. L'avancement de la réaction a été suivi par CCM. Après cette période la réaction était totale. Les solvants ont été éliminés sous pression réduite. Au résidu noir a été additionnée de l'eau (10 mL) et le mélange a été extrait avec du dichlorométhane (3 × 20 mL). Les phases organiques ont été réunies et séchées sur sulfate de magnésium. Après filtration, le solvant a été éliminé sous pression réduite. Le produit brut obtenu a été purifié par chromatographie flash sur colonne de silice (dichlorométhane / méthanol 0,5% jusqu'à 5 % par incréments de 1%) pour conduire au composé **37e** sous forme d'une huile jaunâtre (137 mg, quantitatif). RMN $^1H$ (400 MHz, $CDCl_3$) δ: 8,05 (s, 1H), 7,22 (s, 1H), 7,47 (d, *J* = 8,8 Hz, 2H), 7,02 (d, *J* = 8,8 Hz, 2H), 5,19 (s, 2H), 4,86 (s, 2H), 3,98 (s, 3H), 3,47 (s, 3H). HRMS (ESI+) calculée pour $C_{18}H_{18}NO_5$ $[M+H]^+$, *m/z* 328,1185 trouvée: 328,1177. R*f* = 0,66 (silice, dichlorométhane - méthanol, 90:10).

### Composé 37f

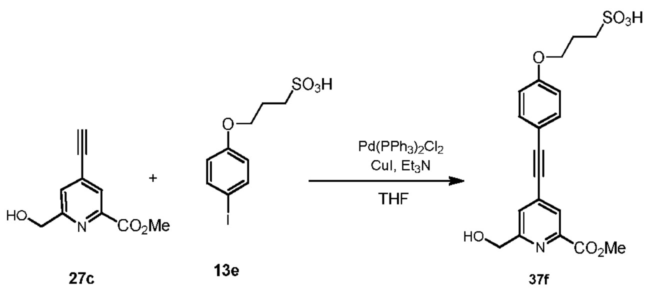


27c 13e 37f

A une solution de dérivé acétylénique **27c** (145 mg, 0,76 mmol) dans du tétrahydrofurane anhydre (20 mL) et de la triéthylamine anhydre (3,5 mL) a été additionné du dérivé iodé **13e** (273 mg, 0,8 mmol). Le mélange a été dégazé sous agitation pendant 1 h. A ce mélange a été additionné du bis-chlorure bis-triphénylphosphine de palladium (II) (68 mg, 0,1 mmol) et de l'iodure de cuivre (I) (36 mg, 0,2 mmol). Le milieu réactionnel a été agité à 65 °C pendant 3 h. L'avancement de la réaction a été suivi par HPLC et CCM. Après cette période la réaction était totale. Les solvants ont été éliminés sous pression réduite. Le produit brut obtenu a été purifié par chromatographie flash sur colonne de silice (dichlorométhane / méthanol 0% jusqu'à 10 % par incrément de 1%) pour conduire au composé **37f** sous forme d'une huile jaunâtre (122 mg, 40%). LRMS (ESI+) calculée pour $C_{19}H_{20}NO_7S$ $[M+H]^+$, *m/z* 406,0960 trouvée: 406,30. $R_f$ = 0,24 (silice, dichlorométhane - méthanol - triéthylamine 96:3:1). $R_t$ = 11,1 min (colonne Waters XBridge RP-$C_{18}$, 3,5 μm, 4,6 × 100 mm) avec une solution aqueuse d'acide trifluoroacetique à 0,2% (pH 1 - MeCN (v/v) comme éluant [gradient linéaire de 5 à 100% MeCN (21 min), avec un débit de 1 mL $min^{-1}$ et une détection UV à 320 nm.

**Composé 37g**

Ce composé a été préparé selon la même procédure que celle utilisée pour le composé **37f** en utilisant le précurseur correspondant.

**Composé 38a**

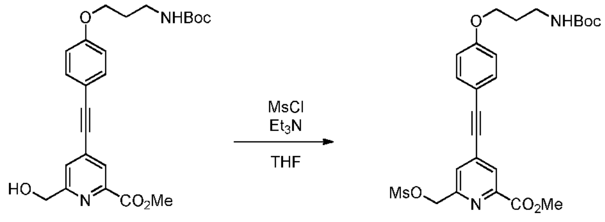


37a 38a

A une solution d'alcool **37a** (195 mg, 0,44 mmol) dans du tétrahydrofurane anhydre (7 mL), a été ajoutée goutte à goutte de la triéthylamine (0,2 mL, 148 μmol) sous atmosphère inerte. A ce mélange refroidi à 4°C, a été ajoutée goutte à goutte du chlorure de mésyle (67 μL, 0,84 mmol). L'avancement de la réaction a été suivi par CCM. Après 5 min, la réaction était totale. Le solvant a été éliminé sous pression réduite. Le résidu a été dissous dans du dichlorométhane (10 mL) et cette solution a été lavée avec de l'eau (2 × 10 mL). La phase organique a été séchée sur sulfate de magnésium, filtrée et concentrée sous pression réduite pour conduire à une huile de couleur jaune-verte (240 mg, quantitatif). Le produit **38a** était suffisamment pur pour être utilisé dans la suite de la synthèse sans purification supplémentaire. LRMS (ESI+) calculée pour $C_{25}H_{31}N_2O_8S$ $[M+H]^+$, *m/z* 519,1801, trouvée: 519,13. R*f* = 0,6 (silice, dichlorométhane - méthanol 96:4).

**Composé 38b**

Ce composé a été préparé selon la même procédure que celle utilisée pour le composé **38a** en utilisant le précurseur correspondant.

**Composé 38c**

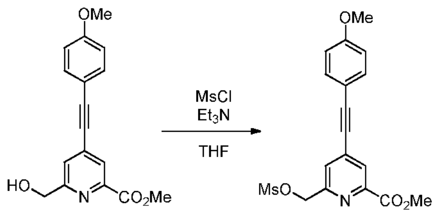


37c 38c

A une solution d'alcool **37c** (241 mg, 0,8 mmol) dans du tétrahydrofurane anhydre (10 mL), a été ajoutée goutte à goutte de la triéthylamine (339 mg, 2,8 mmol) sous atmosphère inerte. A ce mélange refroidi à 4°C, a été ajoutée goutte à goutte une solution de chlorure de mésyle (94 μL, 1,2 mmol) dans du tétrahydrofurane anhydre (0,2 mL). L'avancement de la réaction a été suivi par CCM. Après 20 min, la réaction était totale. Le solvant a été éliminé sous pression réduite. Le résidu a été dissous dans du dichlorométhane (20 mL) et cette solution a été lavée avec de l'eau (3 × 10 mL). La phase organique a été séchée sur sulfate de magnésium, filtrée et concentrée sous pression réduite pour conduire à une huile de couleur jaune-verte (314 mg, quantitatif). Le produit **38c** était suffisamment pur pour être utilisé dans la suite de la synthèse sans purification supplémentaire. RMN $^1H$ (400 MHz, $CDCl_3$) δ : 8,16 (d, *J* = 1,3 Hz, 1H); 7,72 (d, *J* = 1,3 Hz, 1H); 7,53 (d, *J* = 8,9 Hz, 2H); 6,93 (d, *J* = 8,9 Hz, 2H); 5,43 (s, 2H); 4,03 (s, 3H); 3,86 (s, 3H); 3,18 (s, 3H). RMN $^{13}C$ (100 MHz, $CDCl_3$) δ : 165,00; 160,88; 154,68; 147,95, 134,75; 133,86; 126,77; 126,46; 114,40; 113,54; 96,98; 84,97; 70,76; 55,49; 53,25; 38,16. LRMS (ESI+) calculée pour $C_{18}H_{18}NO_6S$ $[M+H]^+$, *m/z* 376,0855, trouvée: 376,04. R*f* = 0,52 (silice, cyclohexane - acétate d'éthyle 30:70).

**Composé 38d**

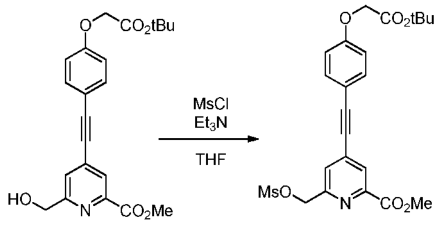


37d 38d

A une solution d'alcool **37d** (114 mg, 0,29 mmol) dans du tétrahydrofurane anhydre (5 mL), a été ajoutée goutte à goutte de la triéthylamine (120 μL, 0,86 mmol) sous atmosphère inerte. A ce mélange refroidi à 4°C, a été ajoutée goutte à goutte une solution de chlorure de mésyle (33 μL, 0,43 mmol) dans du tétrahydrofurane anhydre (1 mL). L'avancement de la réaction a été suivi par CCM. Après 10 min, la réaction était totale. Le solvant a été éliminé sous pression réduite. Le résidu a été dissous dans du dichlorométhane (15 mL) et cette solution a été lavée avec de l'eau (3 × 5 mL). La phase organique a été séchée sur sulfate de magnésium, filtrée et concentrée sous pression réduite pour conduire à une huile de couleur jaune-verte (138 mg, quantitatif). Le produit **38d** était suffisamment pur pour être utilisé dans la suite de la synthèse sans purification supplémentaire. RMN $^1H$ (400 MHz, $CDCl_3$) δ : 8,14 (s, 1H), 7,70 (s, 1H), 7,50 (d, *J* = 8,8 Hz, 2H), 6,90 (d, *J* = 8,8 Hz, 2H), 5,40 (s, 2H), 4,55 (s, 2H), 4,00 (s, 3H), 3,16 (s, 3H), 1,48 (s, 9H). RMN $^{13}C$ (100 MHz, $CDCl_3$) δ : 167,56; 164,98; 159,12; 154,71; 147,97; 133,62; 133,85; 126,79; 126,46; 114,99; 114,49; 96,60; 85,13; 82,85; 70,73; 65,67; 53,26; 38,17; 27,91. LRMS (ESI+) calculée pour $C_{23}H_{26}NO_8S$ $[M+H]^+$, *m/z* 476,14 trouvée: 476,19. R*f* = 0,74 (oxyde d'alumine neutre, cyclohexane - acétate d'éthyle 30:70).

**Composé 38e**

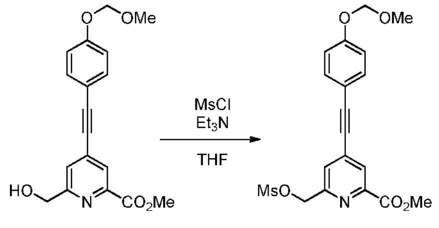


37e 38e

A une solution d'alcool **37e** (140 mg, 0,25 mmol) dans du tétrahydrofurane anhydre (4 mL), a été ajoutée goutte à goutte de la triéthylamine (132 μL, 0,95 mmol) sous atmosphère inerte. A ce mélange refroidi à 4°C, a été ajoutée goutte à goutte une solution de chlorure de mésyle (37 μL, 0,48 mmol) dans du tétrahydrofurane anhydre (1 mL). L'avancement de la réaction a été suivi par CCM. Après 10 min, la réaction était totale. Le solvant a été éliminé sous pression réduite. Le résidu a été dissous dans du dichlorométhane (15 mL) et cette solution a été lavée avec de l'eau (3 × 5 mL). La phase organique a été séchée sur sulfate de magnésium, filtrée et concentrée sous pression réduite pour conduire à une huile de couleur jaune-verte (152 mg, quantitatif). Le produit **38e** était suffisamment pur pour être utilisé dans la suite de la synthèse sans purification supplémentaire. RMN $^1H$ (400 MHz, $CDCl_3$) δ : 8,14 (s, 1H), 7,70 (s, 1H), 7,50 (d, *J* = 8,8 Hz, 2H), 6,90 (d, *J* = 8,8 Hz, 2H), 5,40 (s, 2H), 4,55 (s, 2H), 4,00 (s, 3H), 3,16 (s, 3H), 1,48 (s, 9H). RMN $^{13}C$ (100 MHz, $CDCl_3$) δ : 167,56; 164,98; 159,12; 154,71; 147,97; 133,62; 133,85; 126,79; 126,46; 114,99; 114,49; 96,60; 85,13; 82,85; 70,73; 65,67; 53,26; 38,17; 27,91. HRMS (ESI+) calculée pour $C_{19}H_{20}NO_7S$ $[M+H]^+$, *m/z* 406,0960 trouvée: 406,0954. R*f* = 0,61 (oxyde d'alumine neutre, cyclohexane - acétate d'éthyle, 30:70).

## Composé 38f

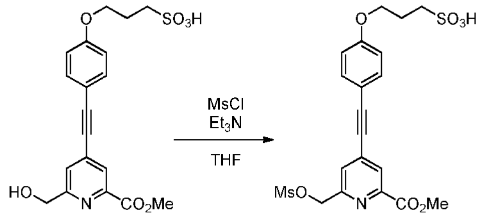


37f 38f

A une solution d'alcool **37f** (39 mg, 84 μmol) dans du tétrahydrofurane anhydre (4 mL), a été ajoutée goutte à goutte de la triéthylamine (40 μL, 0,29 mmol) sous atmosphère inerte. A ce mélange refroidi à 4°C, a été ajoutée goutte à goutte une solution de chlorure de mésyle (14 μL, 0,17 mmol) dans du tétrahydrofurane anhydre (1 mL). L'avancement de la réaction a été suivi par HPLC. Après 10 min, la réaction était totale. Le solvant a été éliminé sous pression réduite et le résidu a été dissous dans du tampon aqueux d'acétate de triéthylammonium 25 mM pH 6 (5 mL) et cette solution a été purifiée par HPLC préparative en utilisant le gradient suivant : colonne Waters XBridge RP-$C_{18}$, 5 μm, 50 × 150 mm) avec tampon aqueux d'acétate de triéthylammonium 25 mM pH 6 - MeCN (v/v) comme éluant [isocratique 5% MeCN (2 min)], gradient linéaire de 5 jusqu'à 100% MeCN (18 min) avec un débit de 100 mL $min^{-1}$ et une détection UV à 320 nm pour conduire au produit mésylé désiré **38f** (41 mg, quantitatif). LRMS (ESI+) calculée pour $C_{20}H_{22}NO_9S_2$ $[M+H]^+$, *m/z* 484,0736 trouvée: 484,34. $R_t$ = 11,05 min (colonne Waters XBridge RP-$C_{18}$, 3,5 μm, 4,6 × 100 mm) avec une solution aqueuse d'acide trifluoroacetique à 0,2% (pH 1 - MeCN (v/v) comme éluant [gradient linéaire de 5 à 80% MeCN (25 min)], avec un débit de 1 mL $min^{-1}$ et une détection UV à 320 nm.

## Composé 38g

Ce composé a été préparé selon la même procédure que celle utilisée pour le composé **38f** en utilisant le précurseur correspondant.

## Composé 39a

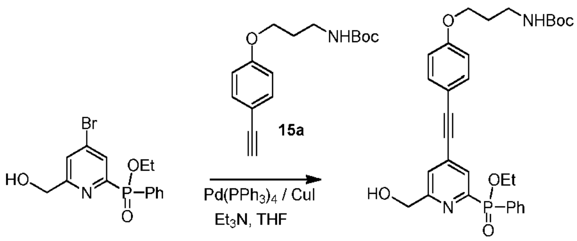


36a 39a

Au dérivé bromé **36a** (142 mg, 0,4 mmol) a été ajouté du tétrahydrofurane anhydre (10 mL) et la solution a été dégazée par trois cycles de congélation-décongélation. A cette solution ont été ajoutés du dérivé acétylénique **15a** (147 mg, 0,4 mmol) et de la triéthylamine (5 mL) et la solution a été à nouveau dégazée. A cette solution ont été ajoutés du tétrakis(triphénylphosphine)palladium(0) (46 mg, 0,04 mmol) et de l'iodure de cuivre (7,6 mg, 0,04 mmol). Cette nouvelle solution a été à nouveau dégazée trois fois puis a été agitée à 65 °C sous atmosphère inerte. L'avancement de la réaction a été suivi par CCM. Après 1 h la réaction était totale. Le mélange réactionnel a été refroidi à température ambiante et le solvant a été éliminé sous pression réduite. Le résidu a été dilué dans du dichlorométhane (25 mL) et lavé avec une solution aqueuse saturée de chlorure d'ammonium (25 mL) puis avec de l'eau (25 mL). La phase organique a été séchée sur sulfate de magnésium, filtrée puis concentrée sous pression réduite pour conduire à un résidu qui a été purifié par chromatographie sur colonne de silice (dichlorométhane / méthanol 0 à 3 % par incréments de 0,5 %) pour obtenir une huile jaune correspondant au composé **39a** (154 mg, 70 %). RMN $^1H$ (400 MHz, $CDCl_3$) δ : 8,01 (dd, *J* = 6,4; 2,0 Hz, 1H), 7,89 (dd, *J* = 8,4; 12,4 Hz, 2H), 7,48 (t, *J* = 8,4 Hz, 1H), 7,40 (d, *J* = 8,8 Hz, 2H), 7,39 (td, *J* = 8,4; 4,2 Hz, 2H), 7,33 (d, *J* = 2,0 Hz, 1H), 6,81 (d, *J* = 8,8 Hz, 2H), 4,73 (s, 1H), 4,69 (s, 2H), 4,08 (qd, *J* = 5,6; 4,8 Hz, 2H), 3,97 (t, *J* = 6 Hz, 2H), 3,26 (m, 2H), 1,92 (q, *J* = 6 Hz, 2H), 1,37 (s, 9H, 1,31 (t, *J* = 5,6 Hz, 3H) ; RMN $^{13}C$ (100 MHz, $CDCl_3$) δ : 160,3 (d, *J* = 18 Hz), 159,8; 156,0; 153,2 (d, *J* = 164 Hz); 133,7; 133,0 (d, *J* = 11 Hz); 132,6 (d, *J* = 5 Hz); 132,3 (d, *J* = 10 Hz); 129,6 (d, *J* = 138 Hz); 128,6 (d, *J* = 18 Hz); 128,5 (d, *J* = 9 Hz); 123,8 (d, *J* = 3 Hz); 114,7; 113,8; 96,0; 85,3 (d, *J* = 2 Hz); 79,3; 65,9; 63,8; 61,9 (d, *J* = 6 Hz); 37,9; 29,5; 28,4; 16,6; RMN $^{31}P$ (162 MHz, $CDCl_3$) δ: +25,6. HRMS (ESI+) calculée pour $C_{36}H_{36}N_2O_6P$ $[M+H]^+$, *m/z* 551,2306 trouvée: 551,2305. R*f* = 0,24 (silice, dichlorométhane

- méthanol, 95:5).

## Composé 39b

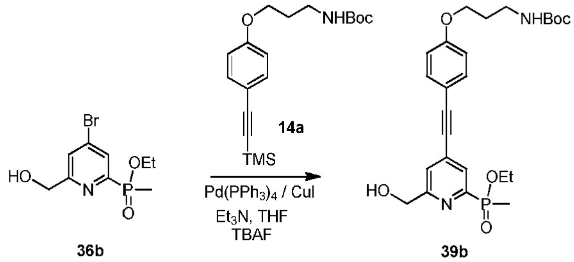


36b 39b

Au dérivé bromé **36b** (200 mg, 0,68 mmol) a été ajouté du tétrahydrofurane anhydre (10 mL) et la solution a été dégazée par trois cycles de congélation-décongélation. A cette solution ont été ajoutés du dérivé acétylénique **14a** (260 mg, 0,75 mmol) et de la triéthylamine (5 mL) et la solution a été à nouveau dégazée. A cette solution ont été ajoutés du tétrakis(triphénylphosphine)palladium(0) (79 mg, 0,068 mmol) et de l'iodure de cuivre (13 mg, 0,068 mmol). Cette nouvelle solution a été à nouveau dégazée trois fois puis a été agitée à 65 °C sous atmosphère inerte. L'avancement de la réaction a été suivi par CCM. Après 1 h la réaction était totale. Le mélange réactionnel a été refroidi à température ambiante et le solvant a été éliminé sous pression réduite. Le résidu a été dilué dans du dichlorométhane (25 mL) et lavé avec une solution aqueuse saturée de chlorure d'ammonium (25 mL) puis avec de l'eau (25 mL). La phase organique a été séchée sur sulfate de magnésium, filtrée puis concentrée sous pression réduite pour conduire à un résidu qui a été purifié par chromatographie sur colonne de silice (dichlorométhane / méthanol 0 à 5 % par incréments de 1 %) pour obtenir une huile jaune correspondant au composé **39b** (220 mg, 66 %). HRMS (ESI+) calculée pour $C_{25}H_{34}N_2O_6P$ $[M+H]^+$, *m/z* 489,2149 trouvée: 489,2152. R*f* = 0,35 (silice, dichlorométhane - méthanol, 95:5).

**Composés 39c-d**

Ces composés ont été préparés selon la même procédure que celle utilisée pour le composé **39b** en utilisant les précurseurs correspondants.

## Composé 39f

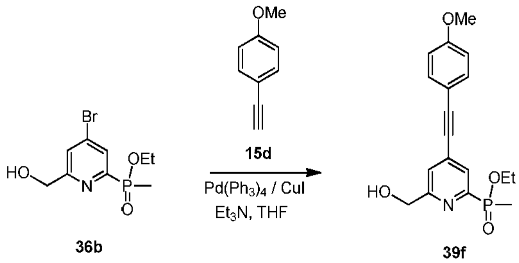


36b 39f

Au dérivé bromé **36b** (400 mg, 1,36 mmol) a été ajouté du tétrahydrofurane anhydre (10 mL) et la solution a été dégazée par trois cycles de congélation-décongélation. A cette solution ont été ajoutés à l'éthynylanisole **15d** (180 mg, 1,36 mmol) et de la triéthylamine (5 mL) et la solution a été à nouveau dégazée. A cette solution ont été ajoutés du tétrakis(triphénylphosphine)palladium(0) (158 mg, 0,136 mmol) et de l'iodure de cuivre (26 mg, 0,136 mmol). Cette nouvelle solution a été à nouveau dégazée trois fois puis a été agitée à 65 °C sous argon. L'avancement de la réaction a été suivi par CCM. Après 1 h la réaction était totale. Le mélange réactionnel a été refroidi à température ambiante et le solvant a été éliminé sous pression réduite. Le résidu a été dilué dans du dichlorométhane (40 mL) et lavé avec une solution aqueuse saturée de chlorure d'ammonium (40 mL) puis avec de l'eau (40 mL). La phase organique a été séchée sur sulfate de magnésium, filtrée puis concentrée sous pression réduite. Le produit brut a été purifié par chromatographie sur colonne de silice (dichlorométhane / méthanol 0 à 8 % par incréments de 1 %) pour obtenir une huile correspondant au composé **39f** (480 mg, 96 %). RMN $^1H$ (400 MHz, $CDCl_3$) $\delta$ : 7,95 (s l, 1H), 7,51 (s l, 1H), 7,42 (d, *J* = 8,8 Hz, 2H), 6,84 (d, *J* = 8,8 Hz, 2H), 4,78 (s, 2H), 4,10 - 4,04 (m, 1H), 3,86 - 3,81 (m, 1H), 3,78 (s, 3H), 1,73 (d, *J* = 14,9 Hz, 3H), 1,23 (t, *J* = 7 Hz, 3H); RMN $^{13}C$ (100 MHz, $CDCl_3$) $\delta$ : 161,2 (d, *J* = 18 Hz), 160,6; 153,1 (d, *J* = 156 Hz), 133,6; 132,9 (d, *J* = 12 Hz), 127,7 (d, *J* = 19 Hz), 124,1 (d, *J* = 4 Hz), 114,2; 114,0; 96,0; 85,3; 64,2; 61,2 (d, *J* = 6 Hz), 55,3; 16,4 (d,

*J* = 6 Hz), 13,4 (d, *J* = 104 Hz); RMN $^{31}P$ (162 MHz, $CDCl_3$) δ: +38,6. HRMS (ESI+) calculée pour $C_{18}H_{21}NO_4P$ $[M+H]^+$, *m*/*z* 346,1203 trouvée: 346,1202. $R_f$ = 0,26 (oxyde d'alumine neutre, dichlorométhane - méthanol 95:5).

**Composé 39g**

Ce composé a été préparé selon la même procédure que celle utilisée pour le composé **39h** en utilisant le précurseur correspondant.

## Composé 39h

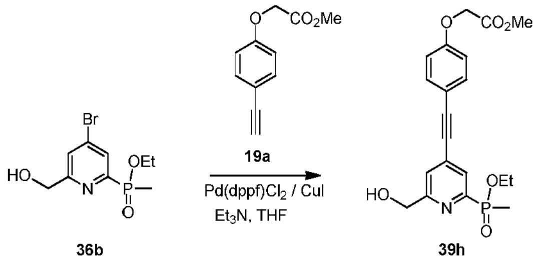


**36b** **39h**

Au dérivé bromé **36b** (103 mg, 0,35 mmol) a été ajouté du tétrahydrofurane anhydre (1 mL) et la solution a été dégazée par trois cycles de congélation-décongélation. A cette solution ont été ajoutés du dérivé acétylénique **19a** (80 mg, 0,42 mmol) et de la triéthylamine (0,24 mL, 1,75 mmol) et la solution a été à nouveau dégazée. A cette solution ont été ajoutés du [1,1-Bis(diphénylphosphino)ferrocène]dichloropalladium(II) (30 mg, 0,035 mmol) et de l'iodure de cuivre (7 mg, 0,035 mmol). Cette nouvelle solution a été à nouveau dégazée trois fois puis a été agitée à 65 °C sous atmosphère inerte. L'avancement de la réaction a été suivi par CCM. Après 18 h la réaction était totale. Le mélange réactionnel a été refroidi à température ambiante et le solvant a été éliminé sous pression réduite. Le produit brut a été purifié par chromatographie sur colonne de silice (dichlorométhane / méthanol 0 à 3 % par incréments de 0,1 %) pour obtenir une huile jaune correspondant au composé **39h** (122 mg, 87 %). RMN $^1H$ (400 MHz, $CDCl_3$) δ: 7,98 (s l, 1H), 7,50 (s l, 1H), 7,45 (d, *J* = 8,9 Hz, 2H), 6,88 (d, *J* = 8,9 Hz, 2H), 4,80 (s, 2H), 4,65 (s, 2H), 4,09 (m, 1H), 3,99 (s l, 1H), 3,86 (m, 1H), 3,79 (s, 3H), 1,76 (d, *J* = 14,9 Hz, 3H), 1,26 (t, *J* = 6,9 Hz, 3H); RMN $^{13}C$ (100 MHz, $CDCl_3$) δ: 168,9; 161,0 (d, *J* = 19 Hz), 158,8; 153,3 (d, *J* = 155 Hz), 133,8; 132,9 (d, *J* = 12 Hz), 128,1 (d, *J* = 22 Hz), 124,2; 115,1; 115,0; 95,7; 85,6; 65,2; 64,3; 61,3 (d, *J* = 5 Hz), 52,5; 16,5 (d, *J* = 4 Hz), 13,5 (d, *J* = 104 Hz); RMN $^{31}P$ (162 MHz, $CDCl_3$) δ: +39,5. HRMS (ESI+) calculée pour $C_{20}H_{22}NNaO_6P$ $[M+Na]^+$, *m*/*z* 426,1082 trouvée: 426,1063. $R_f$ = 0,44 (silice; dichlorométhane - méthanol 90:10).

## Composé 39i

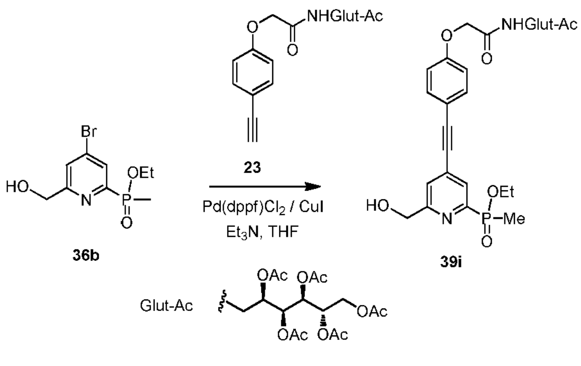


**36b** **39i**

A une solution de dérivé **36b** (40 mg, 0,14 mmol) dans du tétrahydrofurane anhydre (1 mL) dégazée par trois cycles de congélation-décongélation sous vide ont été ajoutés du dérivé acétylénique **23** (75 mg, 0,14 mmol) et de la triéthylamine (0,19 mL, 1,36 mmol) et la solution a été à nouveau dégazée. A cette solution ont été ajoutés du [1,1-Bis(diphénylphosphino)ferrocène]dichloropalladium(II) (11 mg, 0,014 mmol) et de l'iodure de cuivre (2,6 mg, 0,014 mmol). Cette nouvelle solution a été agitée à 65 °C sous argon. L'avancement de la réaction a été suivi par CCM. Après 16 h la réaction était totale. Le mélange réactionnel a été refroidi à température ambiante et le solvant a été éliminé sous pression réduite. Le produit brut a été purifié par chromatographie sur colonne de silice (dichlorométhane / méthanol 0 à 3 % par incréments de 0,5 %) pour obtenir une huile incolore correspondant au composé **39i** (71 mg, 68 %). RMN $^1H$ (700 MHz, $CDCl_3$) δ: 8,02 (d, *J* = 4,6 Hz, 1H), 7,56 (m, 1H), 7,49 (d, *J* = 8,6 Hz, 2H), 7,38 (t, *J* = 6,9 Hz, 1H), 6,93

(d, $J$ = 8,6 Hz, 2H), 5,48 (dd, $J$ = 6,4; 4,8 Hz, 1H), 5,32 (dd, $J$ = 5,8; 4,8 Hz, 1H), 5,17 (m, 1H), 5,02 (ddd, $J$ = 6,5; 5,6; 3,4 Hz, 1H), 4,81 (s, 2H), 4,50 (q AB, 2H), 4,25 (dd, $J$ = 12,4; 3,4 Hz, 1H), 4,11 (dd, $J$ = 12,4; 5,5 Hz, 1H), 3,86 (m, 2H), 3,79 (si, 1H), 3,55 (m, 2H), 2,12 (s, 3H), 2,09 (s, 3H), 2,05 (s, 3H), 2,04 (s, 3H), 2,02 (s, 3H), 1,77 (d, $J$ = 14,9 Hz, 3H), 1,27 (t, $J$ = 6,9 Hz, 3H); RMN $^{13}C$ (176 MHz, $CDCl_3$) $\delta$: 170,7; 170,5; 170,2; 170,0; 169,9; 168,1; 160,9 (d, $J$ = 19 Hz), 158,1; 153,5 (d, $J$ = 156 Hz), 134,0; 132,9 (d, $J$ = 12 Hz), 127,7 (d, $J$ = 10 Hz), 128,4 (d, $J$ = 12 Hz), 115,5; 115,1; 95,4; 85,8; 70,2; 69,2; 69,1; 69,0; 67,3; 64,2; 61,6; 61,3 (d, $J$ = 6 Hz), 39,4; 20,8(4); 20,8(3); 20,8(0); 20,7(9); 20,6(6); 16,6 (d, $J$ = 6 Hz), 13,6 (d, $J$ = 105 Hz); RMN $^{31}P$ (284 MHz, $CDCl_3$) $\delta$ +39.0; HRMS (ESI+) calculée pour $C_{35}H_{43}N_2NaO_{15}P$ $[M+Na]^+$, $m/z$ 785,2299 trouvée: 785,2285. $R_f$ = 0,24 (silice; dichlorométhane - méthanol 95:5).

**Composé 39j** : Ce composé a été préparé selon la même procédure que celle utilisée pour le composé **39i** en utilisant le précurseur correspondant.

## Composé 39m

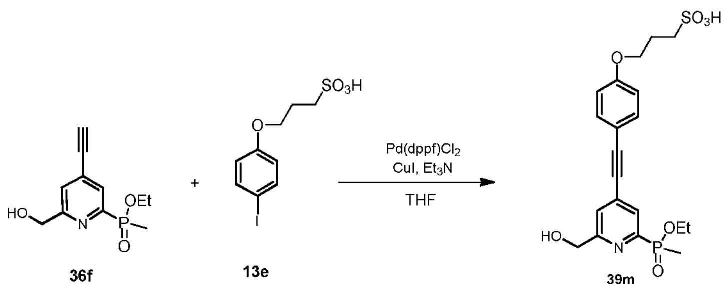


36f 13e 39m

A une solution de dérivé acétylénique **36f** (146 mg, 610 µmol) dans du tétrahydrofurane anhydre (12 mL) et de la triéthylamine anhydre (6 mL) a été additionné du dérivé iodé **13e** (208 mg, 610 µmol). Le mélange a été dégazé sous agitation pendant 1 h. A cette solution ont été additionnés du bis(diphénylphosphino)ferrocène]dichloropalladium(II) (45 mg, 61 µmol) et de l'iodure de cuivre (I) (12 mg, 61 µmol). Le milieu réactionnel a été agité à 65 °C pendant 2 h à l'abri de la lumière. L'avancement de la réaction a été suivi par CCM. Après cette période la réaction était totale. Les solvants ont été éliminés sous pression réduite. Le produit brut obtenu a été purifié par : colonne Waters XBridge RP-$C_{18}$, 5 µm, 50 × 150 mm) avec tampon aqueux d'acétate de triéthylammonium 25 mM pH 6 - MeCN (v/v) comme éluant [isocratique 5% MeCN (2 min), gradient linéaire de 5 jusqu'à 100% MeCN (18 min) avec un débit de 100 mL $min^{-1}$ et une détection UV à 320 nm composé **39m.** LRMS (ESI+) calculée pour $C_{20}H_{25}NNaO_7PS$ $[M+H+Na]^+$, $m/z$ 477,0987 trouvée: 477,21. $R_t$ = 6,55 min (colonne Waters XBridge RP-$C_{18}$, 3,5 µm, 4,6 × 100 mm) avec une solution aqueuse d'acide formique à 0,1% (pH 2 - MeCN (v/v)) comme éluant, [isocratique 5% MeCN (1 min)], gradient linéaire de 5 à 100% MeCN (14 min), avec un débit de 1 mL $min^{-1}$.

## Composé 39n

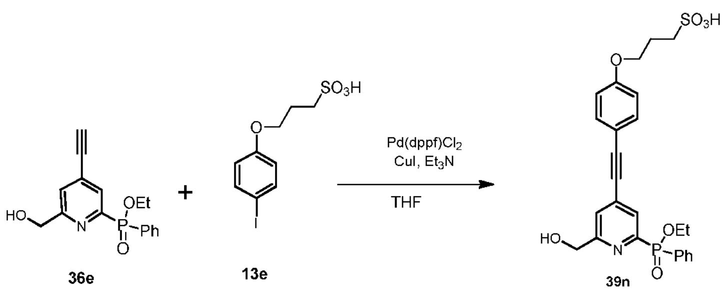


36e 13e 39n

A une solution de dérivé acétylénique **36e** (50 mg, 134 µmol) dans du tétrahydrofurane anhydre (10 mL) et de la triéthylamine anhydre (5 mL) a été additionné du dérivé iodé **13e** (50 mg, 147 µmol). Le mélange a été dégazé sous agitation pendant 1 h. A cette solution ont été additionnés du bis(diphénylphosphino)ferrocène]dichloropalladium(II) (10 mg, 13 µmol) et de l'iodure de cuivre (I) (2,5 mg, 13 µmol). Le milieu réactionnel a été agité à 65 °C pendant 1 h à l'abri de la lumière et sous atmosphère inerte. L'avancement de la réaction a été suivi par LC-MS. Après cette période la réaction était totale. Les solvants ont été éliminés sous pression réduite. Au résidu noir a été additionnée une solution saturée de chlorure d'ammonium (40 mL) et le mélange a été extrait avec du dichlorométhane (2 × 40 mL). Les phases organiques ont été réunies et séchées sur sulfate de magnésium. Après filtration, le solvant a été éliminé sous pression réduite. Le produit brut obtenu a été purifié par chromatographie flash sur colonne de silice (dichlorométhane - méthanol - triéthylamine : 99,75: 0 :0,25 jusqu'à 89,75 : 10 : 0,25 par incréments de 1% de méthanol) pour conduire au composé

**39n** sous forme d'une huile jaunâtre (9 mg, 13%). LRMS (ESI+) calculée pour $C_{25}H_{27}NO_7PS$ $[M+H]^+$, *m/z* 516,1246 trouvée: 516,34. $R_f$ = 0,51 (silice, dichlorométhane - méthanol - triéthylamine 99:9:1). $R_t$ = 7,6 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composés 39o-p**

Ces composés ont été préparés selon la même procédure que celle utilisée pour les composés **39m-n** respectivement en utilisant les précurseurs correspondants.

## Composé 40a

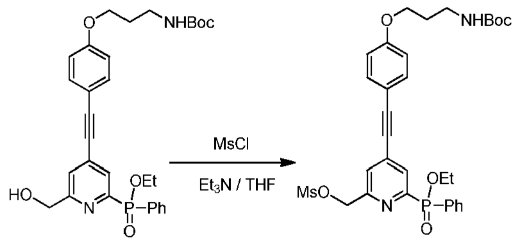


39a 40a

A une solution d'alcool **39a** (118 mg 0,21 mmol) dans du tétrahydrofurane anhydre (7 mL) refroidie à 5°C ont été ajoutés de la triéthylamine (87 μL, 0,64 mmol) puis du chlorure de mésyle (24 μL, 0,32 mmol) sous atmosphère inerte. Le milieu réactionnel a été agité à température ambiante pendant 15 min. L'avancement de la réaction a été suivi par CCM. Après cette période, la réaction était totale. Le solvant a été éliminé sous pression réduite. Au résidu a été additionnée de l'eau saturée en chlorure de sodium (30 mL) et le mélange a été extrait avec du dichlorométhane (2 × 30 mL). Les phases organiques ont été regroupées et séchées sur sulfate de magnésium. Après filtration, le solvant a été éliminé sous pression réduite pour conduire à une huile marron correspondant au composé **40a** (125 mg, 95%) qui était suffisamment pur pour être utilisé dans l'étape suivante sans purification complémentaire. HRMS (ESI+) calculée pour $C_{31}H_{38}N_2O_8PS$ $[M+H]^+$, *m/z* 629,2081 trouvée: 629,2081. R*f* = 0,15 (oxyde d'alumine neutre, cyclohexane - acétate d'éthyle 30:70).

## Composé 40b

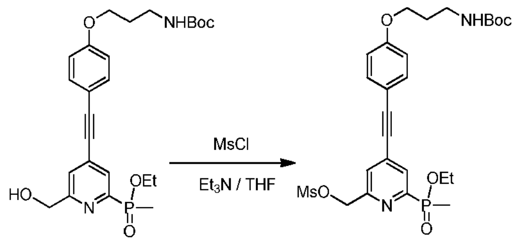


39b 40b

A une solution d'alcool **39b** (90 mg 0,18 mmol) dans du tétrahydrofurane anhydre (5 mL) refroidie à 5°C ont été ajoutés de la triéthylamine (77 μL, 0,55 mmol) puis du chlorure de mésyle (21 μL, 0,27 mmol) sous atmosphère inerte. Le milieu réactionnel a été agité à 5°C pendant 5 min. L'avancement de la réaction a été suivi par CCM. Après cette période, la réaction était totale. Le solvant a été éliminé sous pression réduite. Au résidu a été additionnée de l'eau (50 mL) et le mélange a été extrait avec du dichlorométhane (2 × 50 mL). Les phases organiques ont été regroupées et séchées sur sulfate de magnésium. Après filtration, le solvant a été éliminé sous pression réduite pour conduire à une huile marron correspondant au composé **40b** (97 mg, 95%) qui était suffisamment pur pour être utilisé dans l'étape suivante sans purification complémentaire. HRMS (ESI+) calculée pour $C_{26}H_{36}N_2O_8PS$ $[M+H]^+$, *m/z* 567,1925 trouvée: 567,1939. R*f* = 0,13 (oxyde d'alumine neutre, cyclohexane - acétate d'éthyle 30:70).

## Composé 40f

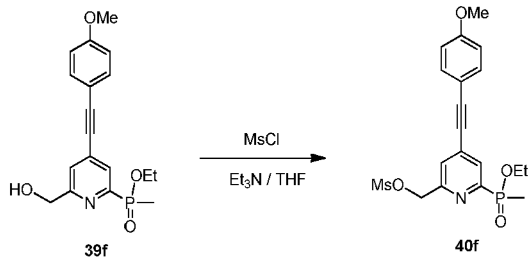


39f 40f

A une solution d'alcool **39f** (214 mg, 0,62 mmol) dans du tétrahydrofurane anhydre (5 mL) refroidie à 5°C ont été ajoutés de la triéthylamine (0,26 mL, 1,86 mmol) puis du chlorure de mésyle (71 µL, 0,93 mmol) sous atmosphère inerte. Le milieu réactionnel a été agité à température ambiante pendant 15 min. L'avancement de la réaction a été suivi par CCM. Après cette période, la réaction était totale. Le solvant a été éliminé sous pression réduite. Au résidu a été additionnée de l'eau saturée en chlorure de sodium (30 mL) et le mélange a été extrait avec du dichlorométhane (2 × 30 mL). Les phases organiques ont été regroupées et séchées sur sulfate de magnésium. Après filtration, le solvant a été éliminé sous pression réduite pour conduire à une huile incolore correspondant au composé **40f** (213 mg, 81%) qui était suffisamment pur pour être utilisé dans l'étape suivante sans purification complémentaire. RMN $^1H$ (400 MHz, $CDCl_3$) δ: 8,11 (dd, *J* = 6,0; 1,5 Hz, 1H), 7,65 (si, 1H), 7,52 (d, *J* = 8,9 Hz, 2H), 6,93 (d, *J* = 8,9 Hz, 2H), 5,39 (s, 2H), 4,20 - 4,10 (m, 1H), 3,95 - 3,88 (m, 1H), 3,86 (s, 3H), 3,16 (s, 3H), 1,80 (d, *J* = 15,1 Hz, 3H), 1,31 (t, *J* = 7,1 Hz, 3H); HRMS (ESI+) calculée pour $C_{19}H_{23}NO_6PS$ $[M+H]^+$, *m/z* 424,0978 trouvée: 424,0975. $R_f$ = 0,17 (oxyde d'alumine neutre, cyclohexane - acétate d'éthyle 30 : 70).

## Composé 40h

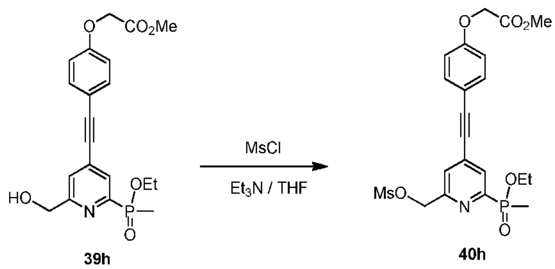


39h 40h

A une solution d'alcool **39h** (122 mg 0,3 mmol) dans du tétrahydrofurane anhydre (3 mL) refroidie à 5°C ont été ajoutés de la triéthylamine (0,15 mL, 1 mmol) puis du chlorure de mésyle (35 µL, 0,45 mmol) sous atmosphère inerte. Le milieu réactionnel a été agité à température ambiante pendant 15 min. L'avancement de la réaction a été suivi par CCM. Après cette période, la réaction était totale. Le solvant a été éliminé sous pression réduite. Au résidu a été additionnée de l'eau saturée en chlorure de sodium (30 mL) et le mélange a été extrait avec du dichlorométhane (2 × 30 mL). Les phases organiques ont été regroupées et séchées sur sulfate de magnésium. Après filtration, le solvant a été éliminé sous pression réduite pour conduire à une huile incolore correspondant au composé **40h** (115 mg, 80%) qui était suffisamment pur pour être utilisé dans l'étape suivante sans purification complémentaire. RMN $^1H$ (400 MHz, $CDCl_3$) δ : 8,08 (s l, 1H), 7,63 (s l, 1H), 7,48 (d, *J* = 8,9 Hz, 2H), 6,90 (d, *J* = 8,9 Hz, 2H), 5,35 (s, 2H), 4,66 (s, 2H), 4,12 (m, 1H), 3,87 (m, 1H), 3,80 (s, 3H), 3,10 (s, 3H), 1,76 (d, *J* = 14,9 Hz, 3H), 1,26 (t, *J* = 7,0 Hz, 3H); RMN $^{31}P$ (162 MHz, $CDCl_3$) δ :+38,2. HRMS (ESI+) calculée pour $C_{21}H_{24}NO_8NaPS$ $[M+Na]^+$, *m/z* 504,0858 trouvée: 504,0859. $R_f$ = 0,56 (silice; dichlorométhane - méthanol 90:10).

## Composé 40i

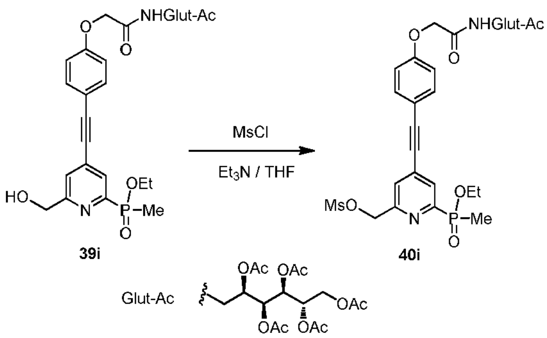


39i 40i

Glut-Ac

A une solution d'alcool **39i** (71 mg, 93 µmol) dans du tétrahydrofurane anhydre (1,5 mL) refroidie à 5°C ont été ajoutés de la triéthylamine (0,26 µL, 0,19 mmol) puis du chlorure de mésyle (11 µL, 0,14 mmol) sous atmosphère inerte. Le milieu réactionnel a été agité à température ambiante pendant 1 h. L'avancement de la réaction a été suivi par CCM. Après cette période, la réaction était totale. Le solvant a été éliminé sous pression réduite. Au résidu a été additionnée de l'eau saturée en chlorure de sodium (10 mL) et le mélange a été extrait avec du dichlorométhane (3 × 10 mL). Les phases organiques ont été regroupées et séchées sur sulfate de magnésium. Après filtration, le solvant a été éliminé sous pression réduite pour conduire à une huile incolore correspondant au composé **40i** (78 mg, quantitatif) qui était suffisamment pur pour être utilisé dans l'étape suivante sans purification complémentaire. RMN $^1$H (400 MHz, $CDCl_3$) $\delta$ 8,01 (d, *J* = 5,6 Hz, 1H), 7,64 (m, 1H), 7,52 (d, *J* = 8,6 Hz, 2H), 7,38 (m, 1H), 6,94 (d, *J* = 8,6 Hz, 2H), 5,49 (dd, *J* = 6,4; 4,8 Hz, 1H), 5,38 (m, 2H), 5,33 (dd, *J* = 5,8; 4,6 Hz, 1H), 5,17 (m, 1H), 5,02 (ddd, *J* = 6,6; 5.6; 3,4 Hz, 1H), 4,50 (m, 2H), 4,25 (dd, *J* = 12,5; 3,4 Hz, 1H), 4,11 (dd, *J* = 12,5; 5,6 Hz, 1H), 3,87 (m, 2H), 3,55 (m, 2H), 3,14 (s, 3H), 2,13 (s, 3H), 2,09 (s, 3H), 2,06 (s, 3H), 2,05 (s, 3H), 2,02 (s, 3H), 1,77 (d, *J* = 15 Hz, 3H), 1,28 (t, *J* = 7,0 Hz, 3H); LRMS (ESI+) calculée pour $C_{36}H_{46}N_2O_{17}PS$ $[M+H]^+$, *m/z* 841,2255 trouvée: 841,31. $R_f$ = 0,44 (silice; dichlorométhane - méthanol: 90 :10).

## Composé 40j

Ce composé a été préparé selon la même procédure que celle utilisée pour le composé **40i** en utilisant le précurseur correspondant.

## Composés 40m-p

Ces composés ont été préparés selon la même procédure que celle utilisée pour le composé **38f** en utilisant les précurseurs correspondants.

## Composé 41a

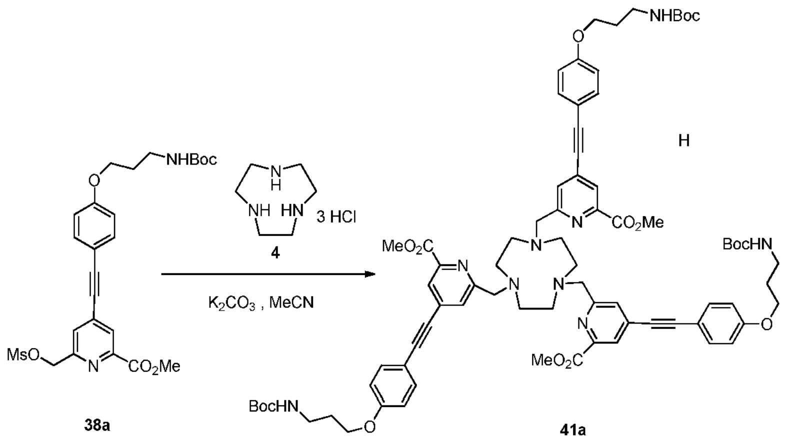


4 38a 41a

A une solution de dérivé mésylé **38a** (235 mg, 0,454 mmol) dans de l'acétonitrile anhydre (10 mL), ont été ajoutés sous atmosphère inerte du carbonate de potassium (65 mg, 0,471 mmol) et du 1,4,7-triazacyclononane **4** (30

mg, 0,126 mmol). Le milieu réactionnel a été chauffé à 65°C pendant 18 h. L'avancement de la réaction a été suivi par LC-MS. Après cette période, la réaction était totale. Le milieu réactionnel a été refroidi à température ambiante et le solvant a été éliminé sous pression réduite. Le résidu a été purifié par HPLC préparative en utilisant le gradient suivant : colonne Waters XBridge RP-$C_{18}$, 5 μm, 50 × 150 mm) avec une solution d'acide formique 0,1% pH 2 - MeCN (v/v) comme éluant [isocratique 5% MeCN (3 min)], gradient linéaire de 5 jusqu'à 100% MeCN (25 min) avec un débit de 100 mL $min^{-1}$ et une détection UV à 320 nm pour conduire au composé **41a** (52,7 mg, 30%). LMRS (ESI+) calculée pour $C_{78}H_{94}N_9O_{15}$ $[M+H]^+$, *m/z* 1396,6869, trouvée: 1396,31. $R_t$ = 14,03 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composé 41b**

Ce composé a été préparé selon la même procédure que celle utilisée pour le composé **41a** en utilisant le précurseur correspondant.

**Composé 41c**

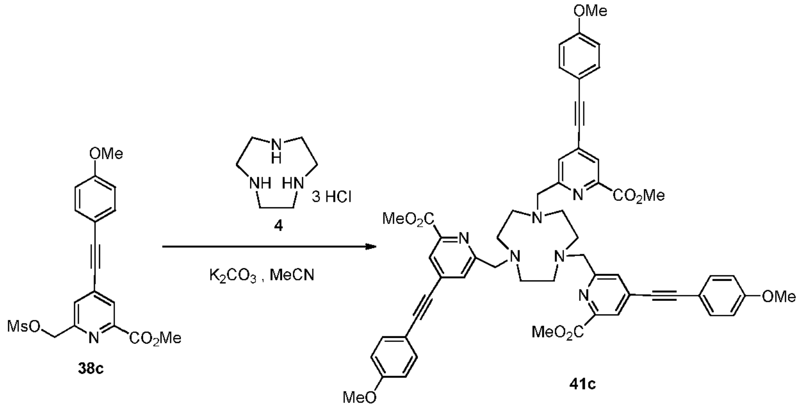


38c

41c

A une solution de dérivé mésylé **38c** (46,8 mg, 120 μmol) dans de l'acétonitrile anhydre (5 mL), ont été ajoutés sous atmosphère inerte du carbonate de potassium (34,7 mg, 250 μmol) et du 1,4,7-triazacyclononane **4** (9,6 mg, 40 μmol). Le milieu réactionnel a été chauffé à 60°C pendant 3 h sous atmosphère inerte. L'avancement de la réaction a été suivi par HPLC. Après cette période, la réaction était totale. Le milieu réactionnel a été refroidi à température ambiante et le solvant a été éliminé sous pression réduite. Le produit brut obtenu a été purifié par HPLC préparative : colonne Macherey Nagel (Hilic, 5 μm, 21 × 250 mm) avec une solution de tampon acétate d'ammonium 50 mM pH 5,3 - MeCN (v/v) comme éluant [isocratique 97% MeCN (3 min)], gradient linéaire de 97 jusqu'à 80% MeCN (20 min) avec un débit de 14 mL $min^{-1}$ et une détection UV à 330 nm pour conduire au composé **41c** sous forme d'une huile jaunâtre (32,7 mg, 87%). HMRS (ESI+) calculée pour $C_{57}H_{55}N_6O_9$ $[M+H]^+$, *m/z* 967,4025 trouvée: 967,4022. $R_t$ = 13,73 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composé 41d**

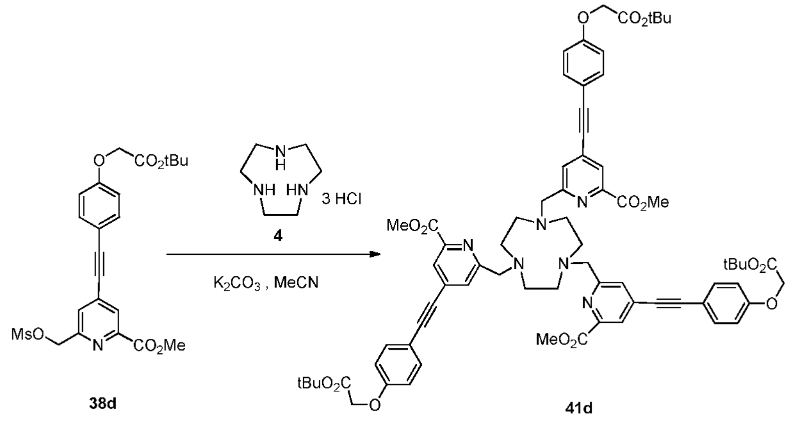


38d

41d

A une solution de dérivé mésylé **38d** (88,2 mg, 190 μmol) dans de l'acétonitrile anhydre (4 mL), ont été ajoutés sous atmosphère inerte du carbonate de potassium (53 mg, 370 μmol) et du 1,4,7-triazacyclononane **4** (14,7 mg, 62 μmol). Le milieu réactionnel a été chauffé à 60°C pendant 16 h. L'avancement de la réaction a été suivi par LC-MS. Après cette période, la réaction était totale. Le milieu réactionnel a été refroidi à température ambiante et le solvant a été éliminé sous pression réduite. Au résidu a été additionné du dichlorométhane (30 mL) et la solution a été lavée avec de l'eau (2 × 15 mL). La phase organique a été séchée sur sulfate de magnésium. Après filtration, le solvant a été éliminé sous pression réduite. Le produit brut obtenu a été purifié par HPLC préparative: colonne Macherey Nagel Hilic, (5 μm, 21 × 250 mm) avec une solution de tampon acétate d'ammonium 50 mM pH 5,3 - MeCN (v/v) comme éluant [isocratique 97% MeCN (3 min)], gradient linéaire de 97 jusqu'à 80% MeCN (20 min) avec un débit de 14 mL $min^{-1}$ et une détection UV à 330 nm pour conduire au composé **41d** sous forme (19 mg, 8%). LRMS (ESI+) calculée pour $C_{72}H_{79}N_6O_{15}$ $[M+H]^+$, *m/z* 1267,5603, trouvée: 1267,82. $R_t$ = 14,26 min (colonne Waters XBridge RP-$C_{18}$, 3,5 μm, 4,6 × 100 mm) avec une solution aqueuse d'acide formique à 0,1% pH 2 - MeCN (v/v) comme éluant [isocratique 5% MeCN (1 min)], gradient linéaire de 5 à 100% MeCN (14 min), avec un débit de 1 mL $min^{-1}$.

## Composé 41e

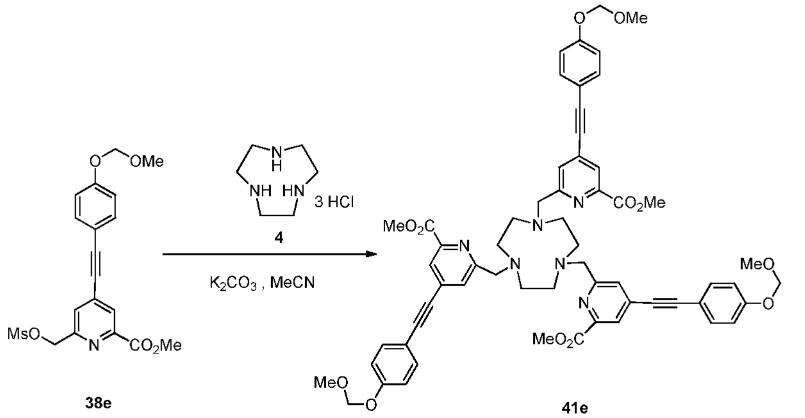


38e

41e

A une solution de dérivé bromé **38e** (76,3 mg, 190 μmol) dans de l'acétonitrile anhydre (5 mL), ont été ajoutés sous atmosphère inerte du carbonate de potassium (52,3 mg, 380 μmol) et du 1,4,7-triazacyclononane **4** (14,5 mg, 60 μmol). Le milieu réactionnel a été chauffé à 60°C pendant 16 h. L'avancement de la réaction a été suivi par LC-MS. Après cette période, la réaction était totale. Le milieu réactionnel a été refroidi à température ambiante et le volume de solvant a été réduit sous pression réduite à 1 mL et le mélange réactionnel a été purifié par HPLC préparative en utilisant le gradient suivant : colonne Waters XBridge RP-$C_{18}$, 5 μm, 50 × 150 mm) avec une solution d'acide trifluoroacétique 1% pH 1 - MeCN (v/v) comme éluant [isocratique 15% MeCN (2 min), gradient linéaire de 15 jusqu'à 100% MeCN (23 min) avec un débit de 100 mL $min^{-1}$ et une détection UV à 320 nm pour conduire au composé **41e** (10,2 mg, 16%). HMRS (ESI+) calculée pour $C_{60}H_{61}N_6O_{12}$ $[M+H]^+$, *m/z* 1057,4342, trouvée: 1057,4341. $R_t$ = 12,94 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min), gradient linéaire de 5 à 100% MeCN (14 min), avec un débit de 1 mL $min^{-1}$.

### Composés 41f-g

Ces composés ont été préparés selon la même procédure que celle utilisée pour le composé **41c** en utilisant les précurseurs correspondants. La purification a été réalisé selon la méthode décrite pour le composé **47b.**

## Composé 43c

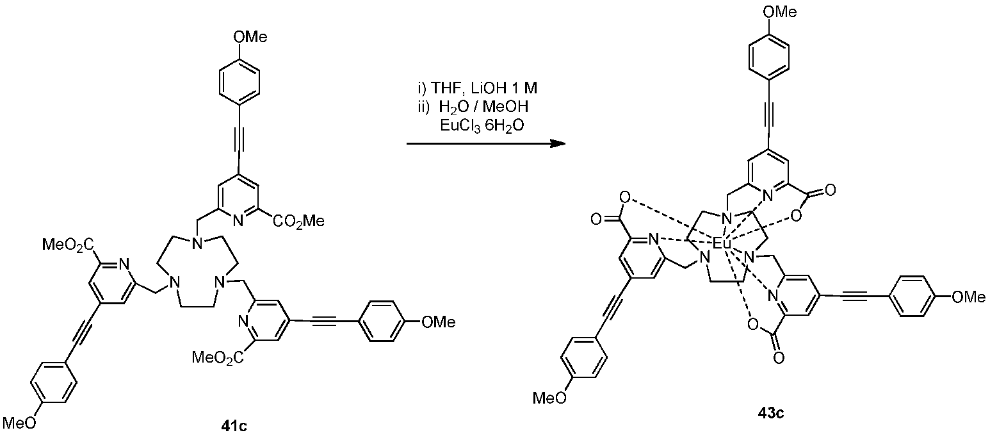


41c

43c

A une solution du ligand **41c** (32,7 mg, 33,8 μmol) dans du tétrahydrofurane (3 mL) a été additionnée une solution aqueuse d'hydroxyde de lithium 1 M (1,5 mL). La solution a été agitée à température ambiante pendant 20 min. L'avancement de la réaction a été suivi par HPLC analytique. Après cette période la réaction était totale. Le solvant a été éliminé sous pression réduite puis au résidu a été additionné de l'eau (2 mL) et du méthanol (3 mL). Le pH de la solution a été ajusté à 7 par addition d'acide chlorhydrique (4 M). A cette solution a été additionné du chlorure d'europium (36 mg, 98,1 μmol). Le mélange réactionnel a été agité à température ambiante pendant 30 min. L'avancement de la réaction a été suivi par LC-MS. Après cette période la réaction était totale. Le solvant a été éliminé sous pression réduite puis au résidu a été additionné de l'acétonitrile (4 mL) et cette solution a été purifiée par HPLC préparative: colonne Macherey Nagel Hilic, (5 μm, 21 × 250 mm) avec une solution de tampon acétate d'ammonium 50 mM pH 5,3 - MeCN (v/v) comme éluant [isocratique 97% MeCN (3 min)], gradient linéaire de 97 jusqu'à 80% MeCN (20 min) avec un débit de 14 mL $min^{-1}$ et une détection UV à 330 nm le composé désiré **43c** (4,8 mg, 13%). HMRS (ESI+) calculée pour $C_{54}H_{47}N_6O_9Eu$ $[M+2H]^{2+}$, *m/z* 537,1296 trouvée: 537,1299. $R_t$ = 10,55 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composés 43a-b, d-g**

Ces composés ont été préparés selon la même procédure que celle utilisée pour le composé **43c** en utilisant les précurseurs correspondants.

**Composés 44a-g, i, k-p**

Ces composés ont été préparés selon la même procédure que celle utilisée pour le composé **44h** en utilisant les précurseurs correspondants.

## Composé 44h

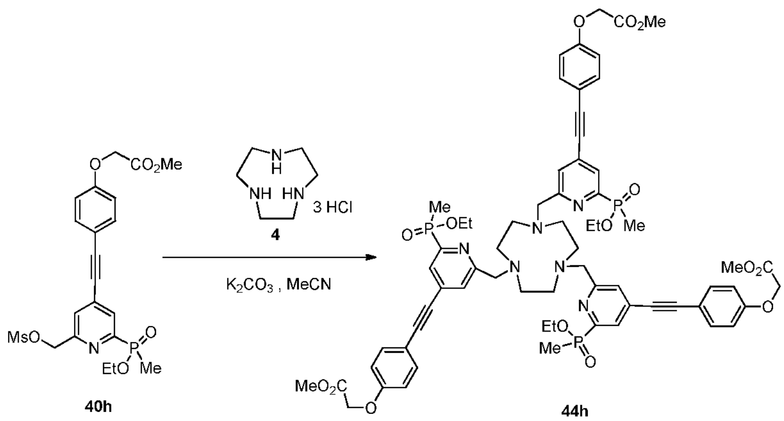


40h

44h

A une solution de dérivé mésylé **40h** (150 mg, 0,310 mmol) dans de l'acétonitrile anhydre (2 mL), ont été ajoutés sous atmosphère inerte du carbonate de potassium (44 mg, 0,321 mmol) et du 1,4,7-triazacyclononane **4** (13,8 mg, 0,107 mmol). Le milieu réactionnel a été chauffé à 60°C pendant 9 h. L'avancement de la réaction a été suivi par CCM. Après cette période, la réaction était totale. Le milieu réactionnel a été refroidi à température ambiante et le solvant a été éliminé sous pression réduite. Au résidu a été additionnée de l'eau (10 mL) et le mélange a été extrait avec de l'acétate d'éthyle (2 $\times$ 20 mL). Les phases organiques ont été regroupées et séchées sur sulfate de magnésium. Après filtration, le solvant a été éliminé sous pression réduite. Le produit brut obtenu a été purifié par chromatographie sur colonne de silice (dichlorométhane/méthanol, 0 jusqu'à 20% par incréments de 1%) pour conduire au composé **44h** sous forme d'une huile incolore (43 mg, 31%). RMN $^1H$ (700 MHz, $CDCl_3$) 7,96 (d $J$ = 5,5 Hz, 3H), 7,67 (s l, 3H), 7,45 (d, $J$ = 8,7 Hz, 6H), 6,87 (d, $J$ = 8,7 Hz, 6H), 4,63 (s, 6H), 4,25 (m, 6H), 4,09 (dq, $J$ = 17,3; 7,2 Hz, 3H), 3,87 (dq, $J$ = 17,3; 7,2 Hz, 3H), 3,78 (s, 9H), 3,24 (m, 12H), 1,73 (d, $J$ = 14,9 Hz, 9H), 1,23 (t, $J$ = 7,2 Hz, 9H); RMN $^{13}C$ (176 MHz, $CDCl_3$) $\delta$: 168,9; 158,9; 157,0; 154,6 (d, $J$ = 157 Hz); 133,8; 133,4 (d, $J$ = 11 Hz); 128,2 (d, $J$ = 21 Hz); 127,7; 115,1; 115,0; 95,6; 85,3; 65,2; 61,3 (d, $J$ = 6 Hz,); 60,7; 52,5; 52,2; 16,5 (d, $J$ = 4 Hz), 13,5 (d, $J$ = 104 Hz). RMN $^{31}P$ (284 MHz, $CDCl_3$) $\delta$: +39,2; $m/z$ HMRS (ESI+) calculée pour $C_{66}H_{76}N_6O_{15}P_3$ [M+H$^+$], $m/z$ 1285,4582, trouvée: 1285,4598. $R_f$ = 0,43 (silice; dichlorométhane - méthanol 87:13).

**Composés 46a-g, i, k-p**

Ces composés ont été préparés selon la même procédure que celle utilisée pour le composé **46h** en utilisant les précurseurs correspondants.

## Composé 46h

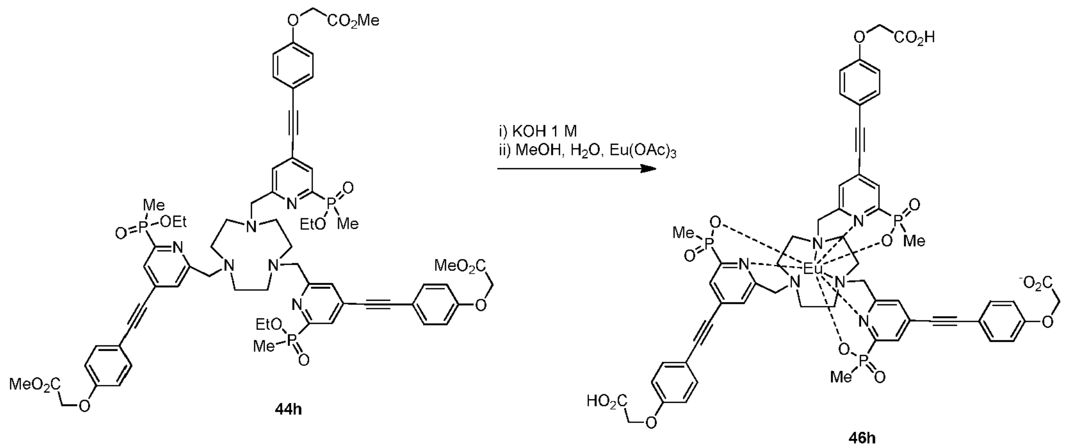


44h

46h

A une solution du ligand **44h** (10 mg, 7,8 $\mu$mol) dans un mélange de solvant $CD_3OD/D_2O$ (1,5 mL, 2:1 v/v) a été additionné de l'hydroxyde de potassium (6,6 mg, 118 $\mu$mol). La solution a été chauffée à 60°C pendant 18 h sous atmosphère inerte. L'avancement de la réaction a été suivi par RMN $^1H$ et $^{31}P$. Après cette période la réaction était totale. Le solvant a été éliminé sous pression réduite et le pH de la solution a été ajusté à 7 par addition d'une solution aqueuse d'acide chlorhydrique 1 M. Le mélange a été lyophilisé et le solide blanc obtenu a été dissous dans un mélange de solvant méthanol-eau (1 mL, 1:1 v/v). A cette solution a été additionné de l'acétate d'europium (3 mg, 9,4 $\mu$mol) et le pH de la solution a été ajusté à 5,8 par addition d'une solution aqueuse d'acide chlorhydrique 1 M. Le mélange réactionnel a été chauffé à 65°C sous atmosphère inerte pendant 18 h. Le mélange réactionnel a été refroidi à température ambiante et le pH de la solution a été ajusté à 7 par addition d'une solution aqueuse d'hydroxyde de potassium 1 M puis lyophilisé. Le produit brut ainsi obtenu a été purifié par HPLC semi-préparative pour donner le composé désiré **46h** sous la forme d'un solide blanc (4,5 mg, 45%). HMRS (ESI+) calculée pour $C_{57}H_{53}N_6O_{15}P_3Eu$ [M-H]$^-$, $m/z$ 1307,1999, trouvée: 1307,2030.

## Composé 44j

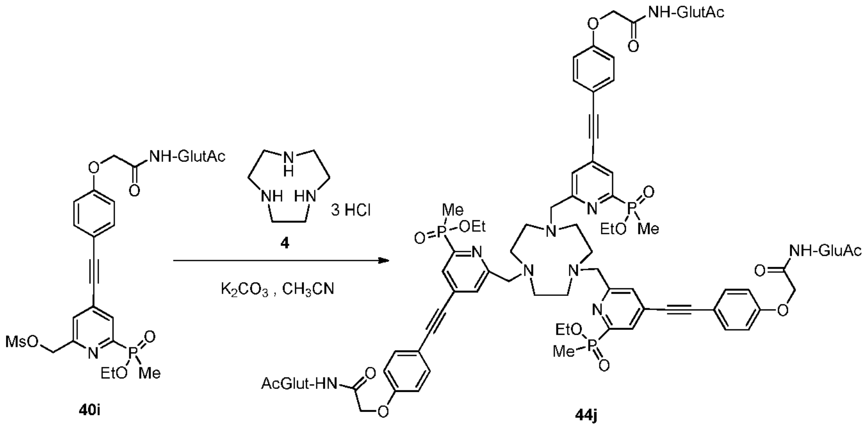


GlutAc= 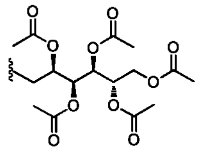

A une solution de dérivé mésylé **40i** (78 mg, 93 μmol) dans de l'acétonitrile anhydre (1 mL), ont été ajoutés sous atmosphère inerte du carbonate de potassium (14 mg, 96 μmol) et du 1,4,7-triazacyclononane **4** (4,1 mg, 33 μmol). Le milieu réactionnel a été chauffé à 60°C pendant 5 h sous atmosphère inerte. L'avancement de la réaction a été suivi par LC-MS. Après cette période, la réaction était totale. Le milieu réactionnel a été refroidi à température ambiante et le solvant a été éliminé sous pression réduite. Les sels de potassium ont été éliminés par décantation puis filtration. Le filtrat a été évaporé sous pression réduite et le produit brut obtenu a été purifié par chromatographie sur colonne de silice (dichlorométhane/méthanol, 0 jusqu'à 20% par incréments de 1%) pour conduire au composé **44j** sous forme d'une huile incolore (20 mg, 26%). RMN $^1$H (700 MHz, $CDCl_3$) δ : 8,00 (m, 3H), 7,74 (m, 3H), 7,55 (m, 3H), 7,50 (d, *J* = 8,6 Hz, 6H), 6,93 (d, *J* = 8,6 Hz, 6H), 5,48 (dd, *J* = 6,4; 4,8 Hz, 3H), 5,33 (dd, *J* = 5,8; 4,6 Hz, 3H), 5,18 (m, 3H), 5,02 (m, 3H), 4,49 (q AB, 6H), 4,25 (dd, *J* = 12,4; 3,4 Hz, 3H), 4,11 (dd, *J* = 12,4; 5,6 Hz, 3H), 3,95 (m, 6H), 3,87 (m, 6H), 3,55 (m, 6H), 2,93 (m, 6H), 2,69 (m, 6H), 2,12 (s, 9H), 2,09 (s, 9H), 2,06 (s, 9H), 2,05 (s, 9H), 2,02 (s, 9H), 1,74 (d, *J* = 15,0 Hz, 9H), 1,25 (t, *J* = 7,0 Hz, 9H); RMN $^{13}$C (176 MHz, $CDCl_3$) δ: 170,7; 170,5; 170,2; 170,0; 169,9; 168,0; 158,2; 157,0 (m l); 154,7 (d, *J* = 154 Hz), 134,1; 133,3; 128,3 (d, *J* = 21 Hz), 127,9 (m l); 115,3; 115,2; 96,3; 85,5; 70,2; 69,1(4); 69,1(1) 69,0; 67,3; 66,4; 61,6; 61,2; 52,0; 39,4; 20,9; 20,8(2); 20,8(1); 20,7; 16,6 (d, *J* = 6 Hz); 13,6 (d, *J* = 103 Hz); RMN $^{31}$P (284 MHz, $CDCl_3$) δ: +38,8; HMRS (ESI+) calculée pour $C_{111}H_{139}N_9O_{42}P_3Na$ $[M+H+Na]^{2+}$, *m/z* 1192,9060 trouvée: 1192,904. $R_f$ = 0,52 (silice; dichlorométhane - méthanol 85:15).

## Composé 46j

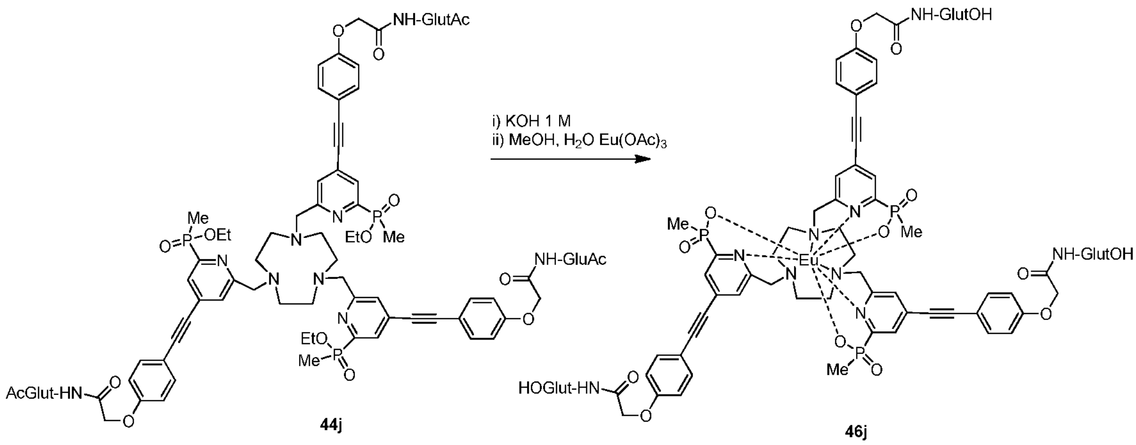


44j 46j

GlutAc= 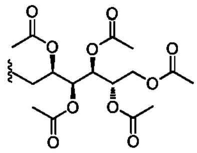 GlutOH= 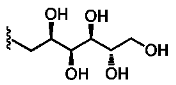

A une solution du ligand **44j** (17 mg, 7,2 μmol) dans un mélange de solvant $CD_3OD/D_2O$ (1,5 mL, 2:1 v/v) a été additionné de l'hydroxyde de potassium (8 mg, 150 μmol). La solution a été chauffée à 60°C pendant 7 h sous atmosphère inerte. L'avancement de la réaction a été suivi par RMN $^1H$ et $^{31}P$. Après cette période la réaction était totale. Le solvant a été éliminé sous pression réduite et le pH de la solution a été ajusté à 7 par addition d'une solution aqueuse d'acide chlorhydrique 1 M.

Le mélange a été lyophilisé et le solide blanc obtenu a été dissous dans un mélange de solvant méthanol-eau (2 mL, 1:1 v/v). A cette solution a été additionné de l'acétate d'europium (2,8 mg, 8,6 μmol). Le mélange réactionnel a été chauffé à 65°C sous atmosphère inerte pendant 18 h puis a été refroidi à température ambiante. La suspension a été centrifugée et le solide blanc a été séparé du surnageant puis lavé avec de l'eau (3 × 5 mL). Le surnageant et les filtrats ont été réunis, neutralisés avec une solution aqueuse d'hydroxyde de potassium (1 M) puis lyophilisés pour conduire à un solide blanc qui a été purifié par HPLC semi-préparative pour donner le composé désiré **46j** sous la forme d'un solide blanc (6 mg, 45%). LRMS (MALDI-TOF) calculée pour $C_{57}H_{93}N_9O_{27}P_3Eu$ $[M+H]^+$, *m/z* 1798,5; trouvée: 1799,8.

## Composé 47a

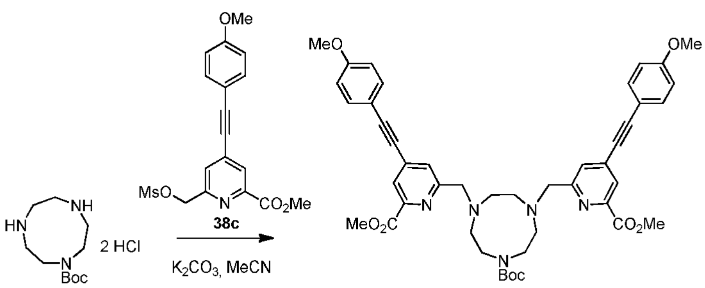


7a 47a

A une solution d'hydrochlorure de TACN monoBoc **7a** (48 mg, 160 μmol) dans l'acétonitrile anhydre (20 mL), a été ajouté du carbonate de potassium (87 mg, 640 μmol) sous atmosphère inerte. A cette suspension, a été ajoutée une solution de chromophore **38c** (150 mg, 300 μmol) dans l'acétonitrile anhydre (2 mL). Le mélange a été chauffé à 65°C pendant 12 h sous atmosphère inerte. L'avancement de la réaction a été suivi par LC-MS. Après cette période la réaction était totale. Le milieu réactionnel a été refroidi à température ambiante. Le solvant a été éliminé sous pression réduite. Le brut réactionnel a été purifié par chromatographie sur colonne de silice (dichlorométhane / méthanol 98 :2 jusqu'à 80 :20 par incréments de 2%) pour conduire au composé **47a** (95 mg, 75%). LMRS (ESI+) calculée pour $C_{45}H_{50}N_5O_8$ $[M+H]^+$, *m/z* 788,3659, trouvée: 788,78. $R_f$ = 0,53 (silice; dichlorométhane - méthanol : 90:10). $R_t$ = 12,89

min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composé 47b**

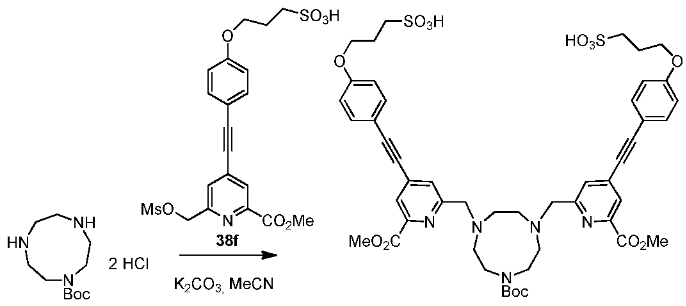


7a 47b

A une solution d'hydrochlorure de TACN monoBoc **7a** (14,6 mg, 48,3 μmol) dans l'acétonitrile anhydre (10 mL), a été ajouté du carbonate de potassium (20 mg, 144 μmol) sous atmosphère inerte. A cette suspension, a été ajoutée une solution de chromophore **38f** (41 mg, 84 μmol) dans l'acétonitrile anhydre (2 mL). Le mélange réactionnel a été chauffé à 65°C pendant 2 h sous atmosphère inerte. L'avancement de la réaction a été suivi par LC-MS. Après cette période la réaction était totale. Le milieu réactionnel a été refroidi à température ambiante. Le solvant a été éliminé sous pression réduite. Le brut réactionnel a été purifié par HPLC préparative en utilisant le gradient suivant : (colonne Waters XBridge RP-$C_{18}$, 5 μm, 50 × 150 mm) avec une solution aqueuse de tampon acétate de triéthylammonium 25 mM pH 6 - MeCN (v/v) comme éluant [isocratique 5% MeCN (2 min)], gradient linéaire de 5 jusqu'à 100% MeCN (18 min) avec un débit de 100 mL $min^{-1}$ et une détection UV à 320 nm pour conduire au composé **47b** sous forme d'un solide blanchâtre (17,7 mg, 36%). LMRS (ESI+) calculée pour $C_{49}H_{58}N_5O_{14}S_2$ $[M+H]^+$, *m/z* 1004,3422, trouvée: 1004,62. $R_t$ = 8,12 min (colonne Waters XBridge RP-$C_{18}$, 3,5 μm, 4,6 × 100 mm) avec une solution aqueuse d'acide trifluoroacétique à 0,2% (pH 1 - MeCN (v/v) comme éluant : gradient linéaire de 15 à 100% MeCN (19 min), avec un débit de 1 mL $min^{-1}$.

**Composé 47c**

Ce composé a été préparé selon la même procédure que celle utilisée pour le composé **47b** en utilisant le précurseur correspondant.

**Composé 48a**

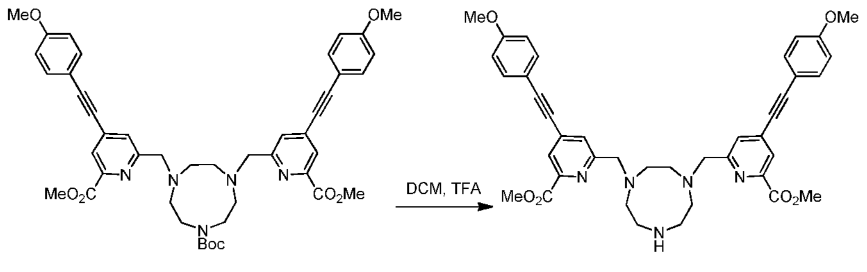


47a 48a

A une solution de **47a** (95 mg, 120 μmol) dans du dichlorométhane anhydre (4 mL) a été ajouté de l'acide trifluoroacétique (0,5 mL). La solution a été agitée sous argon à 5°C pendant 8 h. L'avancement de la réaction a été suivi par LC-MS. Après cette période la réaction était totale. Cette solution a été lavée avec une solution saturée de bicarbonate de sodium (2 × 5 mL). La phase organique a été séchée sur sulfate de magnésium, filtrée et concentrée sous pression réduite. Le résidu a été purifié par HPLC préparative en utilisant le gradient suivant : (colonne Waters XBridge RP-$C_{18}$, 5 μm, 50 × 150 mm) avec une solution aqueuse d'acide trifluoroacétique 0,2% pH 1 - MeCN (v/v) comme éluant [isocratique 15% MeCN (2 min)], gradient linéaire de 15 jusqu'à 100% MeCN (23 min) avec un débit de 100 mL $min^{-1}$ et une détection UV à 320 nm pour conduire au composé **48a** sous forme d'un solide blanchâtre (44 mg,

53%). LMRS (ESI+) calculée pour $C_{46}H_{42}N_5O_6$ $[M+H]^+$, m/z 688,3135 trouvée: 688,56. $R_t$ = 11,01 min (colonne Waters XBridge RP-$C_{18}$, 3,5 µm, 4,6 × 100 mm) avec une solution aqueuse d'acide trifluoroacétique à 0,2% (pH 1 - MeCN (v/v) comme éluant : gradient linéaire de 15 à 100% MeCN (19 min), avec un débit de 1 mL $min^{-1}$.

**Composé 48b**

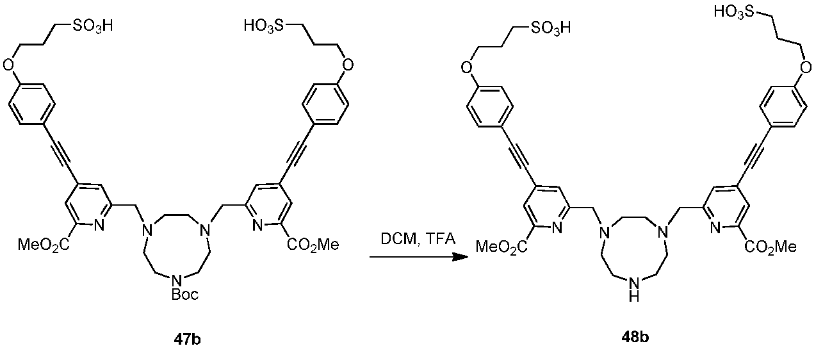


A une solution de **47b** (16,7 mg, 16,6 µmol) dans du dichlorométhane anhydre (5 mL) a été ajouté de l'acide trifluoroacétique (200 µL). La solution a été agitée sous argon à 5°C pendant 2 h puis à température ambiante pendant 2 h. L'avancement de la réaction a été suivi par LCMS. Après cette période la réaction était totale. Cette solution a été lavée avec une solution saturée de bicarbonate de sodium (2 × 5 mL). La phase aqueuse a été concentrée sous pression réduite. Le résidu a été purifié par HPLC préparative en utilisant le gradient suivant : (colonne Waters XBridge RP-$C_{18}$, 5 µm, 19 × 100 mm) avec une solution d'acétate de triéthylammonium 25 mM pH 6 - MeCN (v/v) comme éluant [isocratique 5% MeCN (2 min)], gradient linéaire de 5 jusqu'à 60% MeCN (17 min)] avec un débit de 20 mL $min^{-1}$ et une détection UV à 320 nm pour conduire au composé **48b.** LMRS (ESI+) calculée pour $C_{44}H_{50}N_5O_{12}S_2$ $[M+H]^+$, *m/z* 904,2897 trouvée: 904,61. $R_t$ = 6,85 min (colonne Waters XBridge RP-$C_{18}$, 3,5 µm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composé 48c**

Ce composé a été préparé selon la même procédure que celle utilisée pour le composé **48b** en utilisant le précurseur correspondant.

**Composé 49a**

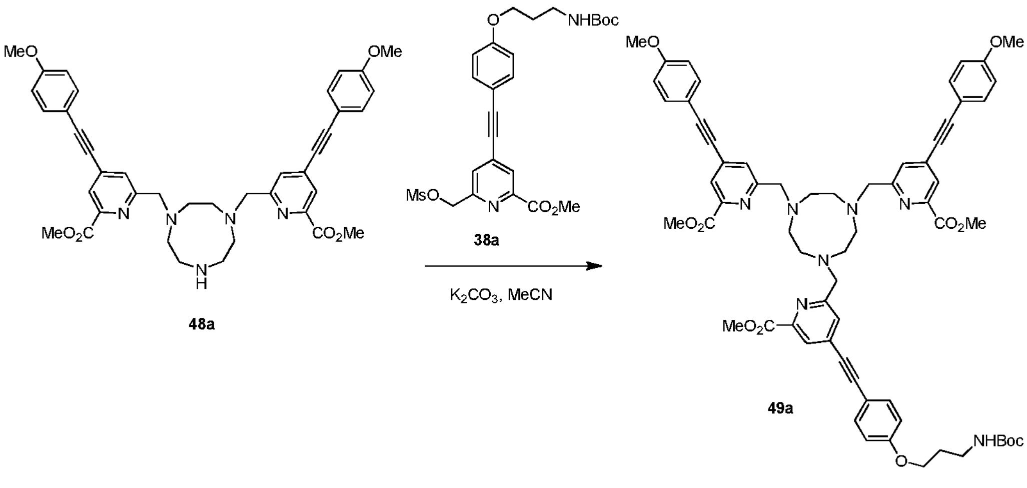


A une solution du composé **48a** (22 mg, 32 µmol) dans l'acétonitrile anhydre (7 mL), a été ajouté du carbonate de potassium (17 mg, 120 µmol) sous atmosphère inerte. A cette suspension, a été ajoutée une solution de chromophore **38a** (51,8 mg, 100 µmol) dans l'acétonitrile anhydre (2 mL). Le mélange réactionnel a été chauffé à 65°C pendant 2 h sous atmosphère inerte. L'avancement de la réaction a été suivi par HPLC. Après cette période la réaction était totale. Le milieu réactionnel a été refroidi à température ambiante. Le solvant a été éliminé sous pression réduite. Le brut

réactionnel a été purifié par HPLC préparative en utilisant le gradient suivant : colonne Waters XBridge RP-$C_{18}$, 5 μm, 50 × 150 mm) avec une solution aqueuse d'acide trifluoroacétique 0,2% pH 1 - MeCN (v/v) comme éluant [isocratique 5% MeCN (2 min), gradient linéaire de 5 jusqu'à 80% MeCN (43 min) avec un débit de 100 mL $min^{-1}$ et une détection UV à 320 nm pour conduire au composé **49a** sous forme d'un solide blanchâtre (7,5 mg, 21%). LMRS (ESI+) calculée pour $C_{64}H_{68}N_7O_{11}$ $[M+H]^+$, *m/z* 1110,4977, trouvée: 1111,22. $R_t$ = 23,74 min (colonne Waters XBridge RP-$C_{18}$, 3,5 μm, 4,6 × 100 mm) avec une solution aqueuse d'acide trifluoroacétique à 0,2% (pH 1 - MeCN (v/v) comme éluant : gradient linéaire de 5 à 80% MeCN (25 min), avec un débit de 1 mL $min^{-1}$.

**Composés 49b-f**

Ces composés ont été préparés selon la même procédure que celle utilisée pour le composé **49a** en utilisant les précurseurs correspondants.

**Composé 50a**

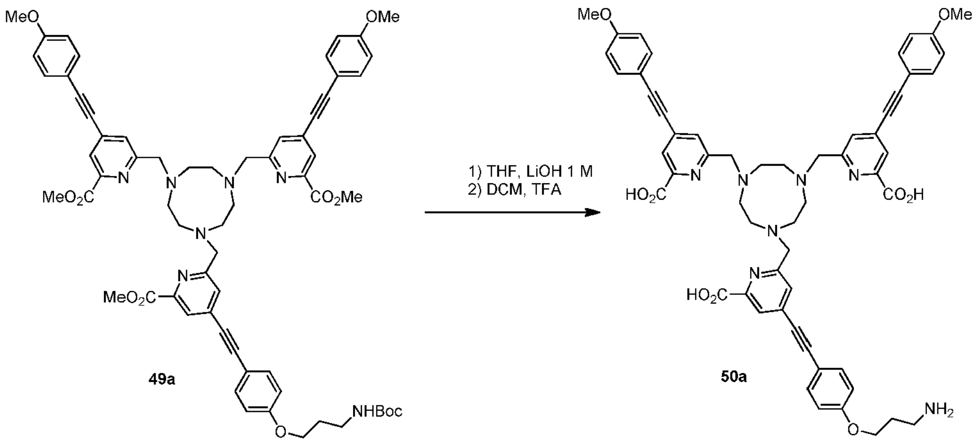


49a

50a

A une solution de ligand triester méthylique 49a (1 mg, 0,901 μmol) dans du THF (150 μL), ont été ajoutés de l'hydroxyde de lithium à 1 M (50 μmol) et de l'eau pure (100 μL). A cette suspension blanchâtre, a été ajouté du THF (1 mL) pour parfaire la solubilisation. Le mélange réactionnel a été agité à température ambiante pendant 2 h. L'avancement de la réaction a été suivi par HPLC. Après cette période, la réaction était totale. Le solvant a été éliminé sous pression réduite. Le milieu réactionnel a été alors repris dans de l'eau pure (500 μL) et a été acidifié par l'addition d'une solution aqueuse d'acide chlorhydrique (1 M) pour obtenir le pH du mélange compris entre 2-3. Le solvant a été éliminé sous pression réduite. Le résidu a été purifié par HPLC préparative conduisant au produit souhaité sous la forme d'une huile. A cette huile diluée dans du dichlorométhane (0,5 mL) ont été ajoutés de l'acide trifluoroacétique (150 μL) puis un volume supplémentaire de dichlorométhane (2,5 mL). Le mélange réactionnel a été agité à température ambiante pendant 10 min. L'avancement de la réaction a été suivi par HPLC. Après cette période, la réaction était totale. Le solvant a été éliminé sous pression réduite pour conduire au composé **50a** sous la forme d'une huile qui a été utilisée dans l'étape suivante sans purification complémentaire.

**Composés 50b-f**

Ces composés ont été préparés selon la même procédure que celle utilisée pour le composé **50a** en utilisant les précurseurs correspondants.

## Composé 51a

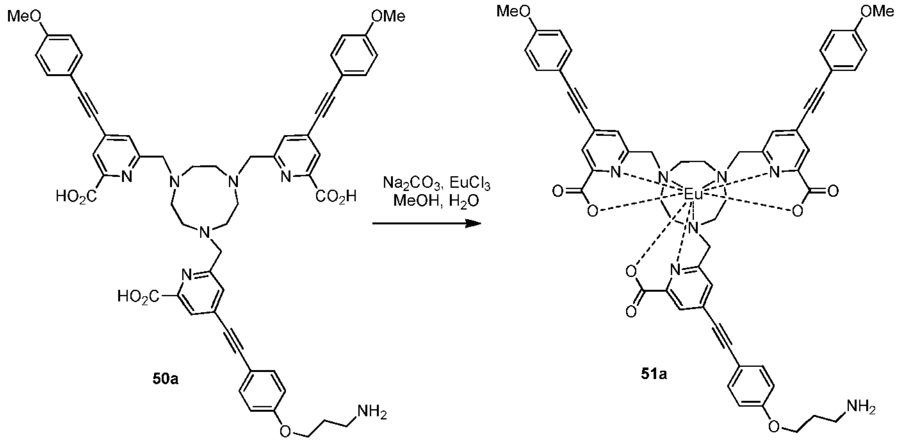


50a

51a

A une solution de ligand **50a** (967 $\mu$g, 1 $\mu$mol) dans du méthanol (1 mL) et de l'eau (0,5 mL), ont été ajoutés du carbonate de sodium (0,53 mg, 5 $\mu$mol) et du chlorure d'europium hexahydraté (732 $\mu$g, 2 $\mu$mol). Le mélange réactionnel a été chauffé à 50°C pendant une nuit. L'avancement de la réaction a été suivi par HPLC analytique. Après cette période la réaction était totale. Le milieu réactionnel a été refroidi à température ambiante puis le solvant a été éliminé sous pression réduite. Le résidu a été purifié par HPLC préparative conduisant au complexe **51a** sous la forme d'une huile incolore (560 nmol, 56%).

## Composés 51b-f

Ces composés ont été préparés selon la même procédure que celle utilisée pour le composé **51a** en utilisant les précurseurs correspondants.

## Composé 52a

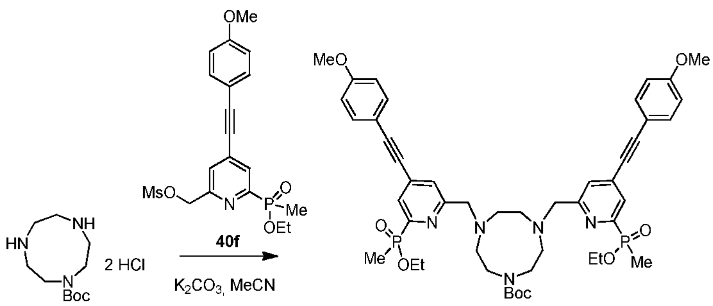


7a

52a

A une solution d'hydrochlorure de TACN monoBoc **7a** (43 mg, 140 $\mu$mol) dans l'acétonitrile anhydre (5 mL), a été ajouté du carbonate de potassium (117 mg, 850 $\mu$mol) sous atmosphère inerte. A cette suspension, a été ajoutée une solution de chromophore mésylé **40f** (120 mg, 280 $\mu$mol). L'avancement de la réaction a été suivi par HPLC analytique. Après agitation et chauffage à 65°C pendant 2 h, le milieu réactionnel a été refroidi à température ambiante. Le solvant a été éliminé sous pression réduite. Le résidu a été dissous dans du dichlorométhane (50 mL) et la solution résultante a été lavé avec de l'eau (2 $\times$ 50 mL), séchée sur sulfate de magnésium, filtrée et concentrée sous pression réduite. Le brut réactionnel a été purifié par chromatographie sur colonne de silice (dichlorométhane / méthanol 100 :0 jusqu'à 94:6 par incréments de 1%) pour conduire au composé **52a** sous forme d'une huile (86 mg, 70%). HMRS (ESI+) calculée pour $C_{47}H_{60}N_5O_8P_2$ $[M+H]^+$, *m/z* 884,3912, trouvée: 884,3919. $R_f$ = 0,56 (silice; dichlorométhane - méthanol : 90:10). $R_t$ = 10,65 min (colonne Waters XBridge RP-$C_{18}$, 3,5 $\mu$m, 4,6 $\times$ 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composés 52b-f**

Ces composés ont été préparés selon la même procédure que celle utilisée pour le composé **52a** en utilisant les précurseurs correspondants.

## Composé 53a

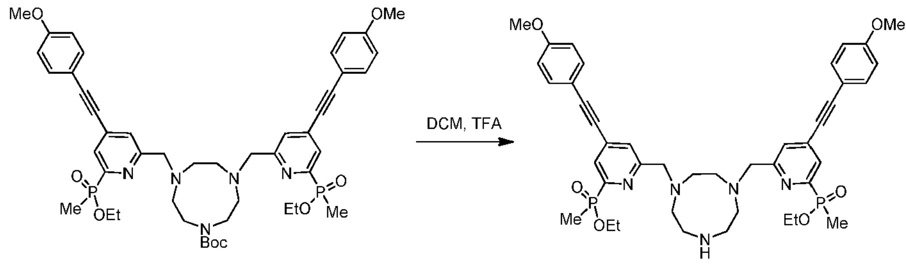


**52a** **53a**

A une solution de **52a** (70 mg, 0,079 mmol) dans du dichlorométhane anhydre (5 mL) a été ajouté de l'acide trifluoroacétique (0,5 mL). La solution a été agitée sous argon à 5 °C pendant 2 h 30. L'avancement de la réaction a été suivi par CCM. Après cette période la réaction était totale. Le solvant a été éliminé sous pression réduite et le résidu a été redissous dans du dichlorométhane (1 mL), qui a été à nouveau éliminé sous pression réduite. Cette procédure a été répétée 5 fois pour éliminer l'acide trifluoroacétique en excès. Le résidu a été purifié par HPLC préparative en utilisant le gradient suivant : (colonne Waters XBridge RP-$C_{18}$, 5 μm, 19 × 100 mm) avec une solution aqueuse de tampon d'acétate de triéthylammonium 25 mM pH 7 - MeCN (v/v) comme éluant [isocratique 5% MeCN (3,5 min)], gradient linéaire de 5 jusqu'à 100% MeCN (26,5 min) avec un débit de 20 mL $min^{-1}$ et une détection UV à 320 nm pour conduire au composé **53a** (26 mg, 42%). HMRS (ESI+) calculée pour $C_{42}H_{52}N_5O_6P_2$ $[M+H]^+$, *m/z* 784,3387, trouvée: 784,3388. $R_t$ = 9,88 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composés 53b-f**

Ces composés ont été préparés selon la même procédure que celle utilisée pour le composé **53a** en utilisant les précurseurs correspondants.

## Composé 54a

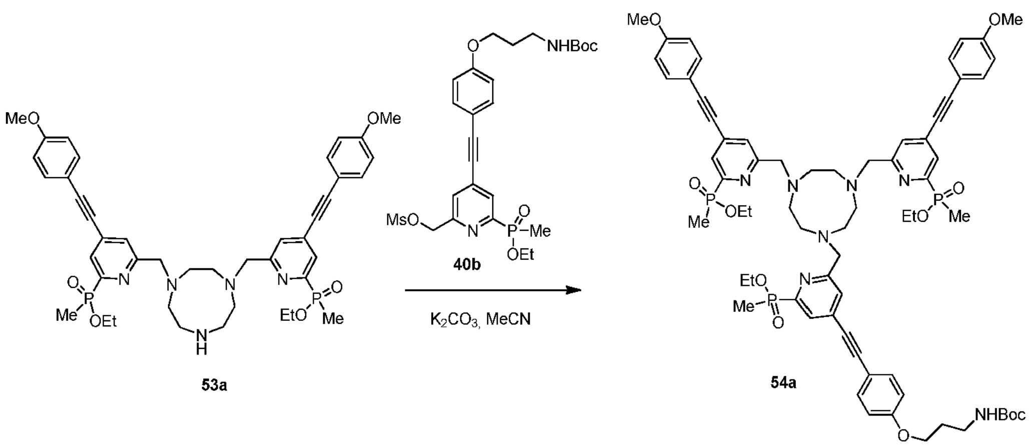


**53a** **54a**

A une solution de composé dialkylé **53a** (32 mg, 40 μmol) dans l'acétonitrile anhydre (5 mL), a été ajouté du carbonate de potassium (21 mg, 150 μmol) sous atmosphère inerte. A cette suspension, a été ajoutée une solution de chromophore NH-Boc mésylé **40b** (29 mg, 51 μmol) dans l'acétonitrile anhydre (5 mL). Le mélange réactionnel a été chauffé à 65°C pendant 2 h 30. L'avancement de la réaction a été suivi par HPLC. Après cette période la réaction était totale. Le milieu réactionnel a été refroidi à température ambiante et le solvant a été éliminé sous pression réduite. Le

résidu a été dissous dans du dichlorométhane (40 mL) et a été lavé avec de l'eau (2 × 40 mL), séché sur sulfate de magnésium, filtré et concentré sous pression réduite. Le brut réactionnel a été purifié par HPLC sur colonne préparative en utilisant le gradient suivant : (colonne Waters XBridge RP-$C_{18}$, 5 μm, 19 × 100 mm) avec une solution aqueuse de tampon d'acétate de triéthylammonium 25 mM pH 7 - MeCN (v/v) comme éluant [isocratique 5% MeCN (3,5 min)], gradient linéaire de 5 jusqu'à 100% MeCN (28,5 min) avec un débit de 20 mL $min^{-1}$ et une détection UV à 320 nm pour conduire au composé **54a** (36 mg, 43%). HMRS (ESI+) calculée pour $C_{67}H_{84}N_7O_{11}P_3$ $[M+2H]^{2+}$, *m/z* 627,7715, trouvée: 627,7718. $R_t$ = 11,08 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

### Composé 54e

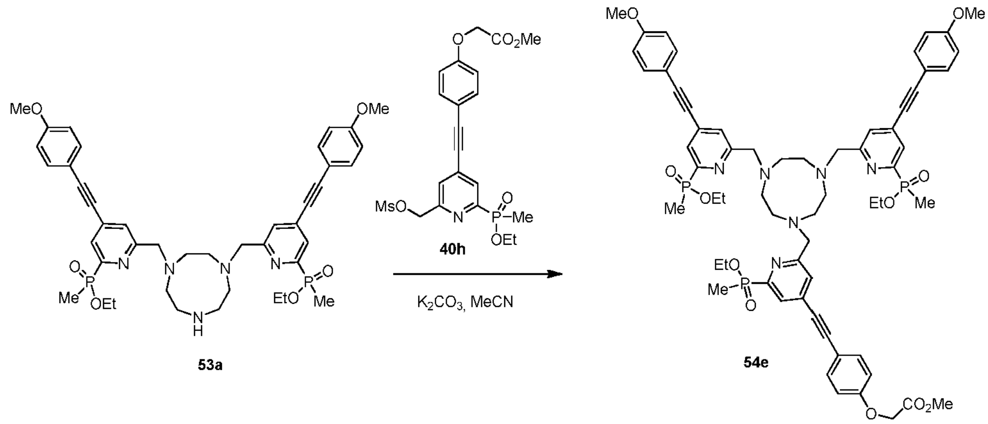


A une solution de TACN dibras **53a** (80 mg, 0,102 mmol) dans l'acétonitrile anhydre (4 mL), a été ajouté du carbonate de potassium (42 mg, 0,306 mmol) sous atmosphère inerte. A cette suspension, a été ajoutée une solution de chromophore **40h** (54 mg, 0,112 mmol) dans l'acétonitrile anhydre (2 mL). Le mélange réactionnel a été chauffé à 60°C pendant 15 h sous atmosphère inerte. L'avancement de la réaction a été suivi par HPLC. Après cette période la réaction était totale. La suspension a été refroidie à température ambiante et les sels ont été séparés par décantation. Le solvant du surnageant a été éliminé sous pression réduite et le brut réactionnel a été purifié par chromatographie sur colonne de silice (dichlorométhane / méthanol 0 à 20 % par incréments de 1 %) pour obtenir une huile incolore correspondant au composé **54e** (71 mg, 58 %). RMN $^{1}H$ (700 MHz, $CDCl_3$) δ:7,88 (d, *J* = 5,7 Hz, 3H), 7,52 (s l, 3H), 7,41 (d, *J* = 8,5 Hz, 2H), 7,40 (d, *J* = 8,6 Hz, 4H), 6,84 (d, *J* = 8,5 Hz, 2H), 6,83 (d, *J* = 8,6 Hz, 4H), 4,60 (s, 2H), 4,13 (m l, 6H), 4,05 (m, 3H), 3,86 (m, 3H), 3,77 (s, 6H), 3,75 (s, 3H), 3,29 (m l, 6H), 3,10 (m l, 6H), 1,71 (d, *J* = 14,9 Hz, 9H), 1,21 (t, *J* = 7,0 Hz, 9H); RMN $^{13}C$ (176 MHz, $CDCl_3$) δ: 169,0; 160,8; 158,8; 156,9 (m l); 154,2 (d, *J* = 159 Hz); 133,8; 133,7; 133,4 (m l); 127,9 (d, *J* = 22 Hz); 127,4; 114,9; 114,3; 113,4; 97,1; 96,6; 85,1; 84,9; 65,1; 61,5 (d, *J* = 6 Hz); 60,1 (s l); 55,4; 52,4; 51,3; 16,4 (d, *J* = 6 Hz), 13,5 (d, *J* = 104 Hz); RMN $^{31}P$ (284 MHz, $CDCl_3$) δ : +39,1. HRMS (ESI+) calculée pour $C_{62}H_{72}N_6O_{11}P_3$ $[M+H]^+$, *m/z* 1169,4470 trouvée: 1169,4430. $R_f$ = 0,28 (silice; dichlorométhane - méthanol 90:10).

**Composés 54f,k-l,p-r**

Ces composés ont été préparés selon la même procédure que celle utilisée pour le composé **54e** en utilisant les précurseurs correspondants.

**Composés 54b-d,g-j,m-p**

Ces composés ont été préparés selon la même procédure que celle utilisée pour le composé **54a** en utilisant les précurseurs correspondants.

## Composé 55a

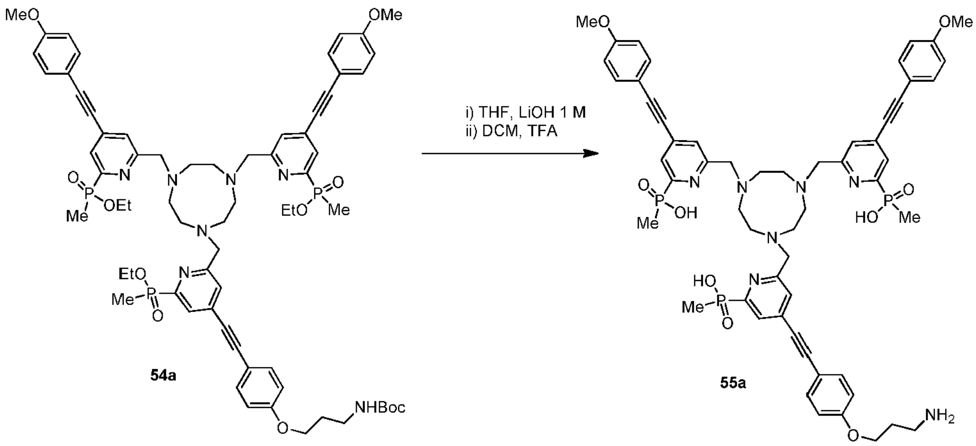


54a 55a

Au ligand **54a** (35,6 mg, 28,4 μmol) ont été additionnés du tétrahydrofurane (4 mL) et une solution aqueuse d'hydroxyde de lithium (3 mL, 1 M). La solution a été agitée à température ambiante pendant 2 h. L'avancement de la réaction a été suivi par LC-MS. Après cette période, la réaction était totale. Le mélange réactionnel a été concentré sous pression réduite et utilisé dans la suite de la synthèse sans purification complémentaire. LRMS (ESI+) calculée pour $C_{61}H_{71}N_7O_{11}P_3$ $[M+H]^+$, *m/z* 1170,4424, trouvée: 1170,99. $R_t$ = 9,17 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$. A une solution du composé obtenu précédemment dans du dichlorométhane (20 mL) a été ajouté de l'acide trifluoroacétique (1,5 mL). La solution a été agitée à 5°C pendant 6 h. L'avancement de la réaction a été suivi par LC-MS. Après cette période la réaction était totale. Le solvant a été éliminé sous pression réduite et le résidu a été redissous dans du dichlorométhane (6 mL). Le solvant a été à nouveau éliminé sous pression réduite. Cette procédure a été répétée 5 fois pour éliminer l'acide trifluoroacétique en excès. Le produit brut a été utilisé dans la suite de la synthèse sans purification complémentaire. LRMS (ESI+) calculée pour $C_{56}H_{63}N_7O_9P_3$ $[M+H]^+$, *m/z* 1070,3900, trouvée: 1070,79. $R_t$ = 6,82 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composés 55b-d,g-j,m-p**

Ces composés ont été préparés selon la même procédure que celle utilisée pour le composé **55a** en utilisant les précurseurs correspondants.

## Composé 56a

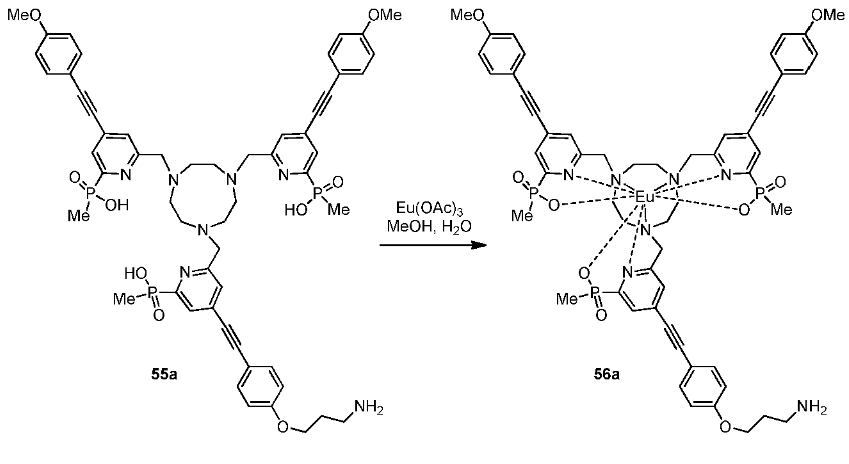


55a 56a

A une solution de TACN tribras asymétrique **55a** (28,4 μmol) dans du méthanol (15 mL) et de l'eau (2 mL), a été ajouté du chlorure d'europium hexahydraté (11,1 mg, 31 μmol). Le mélange réactionnel a été chauffé à 50°C pendant

12 h. L'avancement de la réaction a été suivi par LC-MS. Après cette période la réaction était totale. Le milieu réactionnel a été refroidi à température ambiante puis le solvant a été éliminé sous pression réduite. Le résidu a été purifié par HPLC sur colonne préparative en utilisant le gradient suivant : (colonne Waters XBridge RP-$C_{18}$, 5 μm, 19 × 100 mm) avec une solution aqueuse de tampon d'acétate de triéthyle ammonium 25 mM pH 7 - MeCN (v/v) comme éluant [isocratique 5% MeCN (3,5 min)], gradient linéaire de 5 jusqu'à 100% MeCN (23 min) avec un débit de 20 mL $min^{-1}$ et une détection UV à 320 nm pour conduire au composé **56a** (34 mg, 98%). HMRS (ESI+) calculée pour $C_{56}H_{61}EuN_7O_9P_3$ $[M+2H]^{2+}$, *m/z* 609,6466, trouvée: 609,6466. $R_t$ = 8,08 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

## Composé 56e

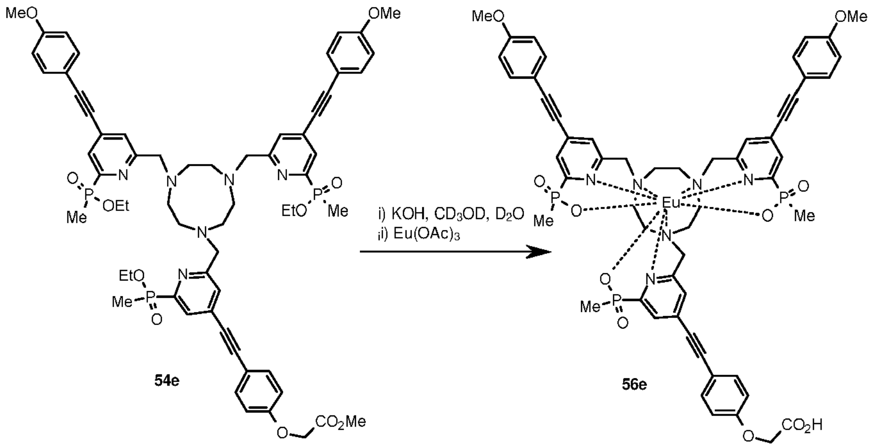


A une solution du ligand **54e** (30 mg, 26 μmol) dans un mélange de solvant $CD_3OD/D_2O$ (2,5 mL, 2:1 v/v) a été additionné de l'hydroxyde de potassium (5 mg, 100 μmol). La solution a été chauffée à 60°C pendant 24 h sous atmosphère inerte. L'avancement de la réaction a été suivi par RMN $^1H$ et $^{31}P$. Après cette période la réaction était totale. Le solvant a été éliminé sous pression réduite et le pH de la solution a été ajusté à 7 par addition d'une solution aqueuse d'acide chlorhydrique 1 M. A cette solution a été additionné de l'acétate d'europium (2,8 mg, 8,6 μmol). Le mélange réactionnel a été chauffé à 65°C sous atmosphère inerte pendant 18 h puis a été refroidi à température ambiante. La suspension a été centrifugée et le solide blanc a été séparé du surnageant, lavé avec du méthanol (3 × 2 mL). Le surgageant et les filtrats ont été réunis et concentrés sous pression réduite pour conduire à un solide blanchâtre qui a été purifié par HPLC semi-préparative pour donner le composé désiré **56e** sous la forme d'un solide blanc (13 mg, 45%). HRMS (ESI+) calculée pour $C_{55}H_{55}N_6O_{11}P_3Eu$ $[M+2H]^{2+}$, *m/z* 611,1220; trouvée: 611,1247.

## Composés 56f,k-l,p-r

Ces composés ont été préparés selon la même procédure que celle utilisée pour le composé **56e** en utilisant les précurseurs correspondants.

## Composés 57a-f

Ces composés ont été préparés selon la même procédure que celle utilisée pour le composé **60a** en utilisant les précurseurs correspondants.

## Composé 60a

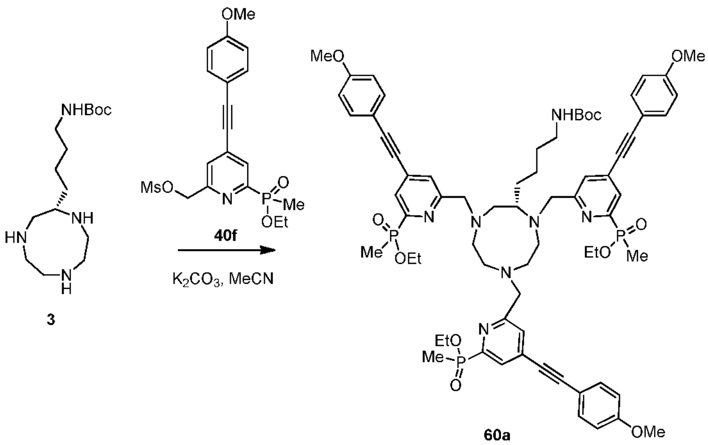


3

60a

A une solution du macrocycle 3 (21 mg, 0,071 mmol) dans l'acétonitrile anhydre (4 mL), a été ajouté du carbonate de potassium (30 mg, 0,21 mmol) sous atmosphère inerte. A cette suspension, a été ajoutée une solution de chromophore mésylé **40f** (70 mg, 0,14 mmol) dans l'acétonitrile anhydre (2 mL). Le mélange a été chauffé à 60°C pendant 48 h. L'avancement de la réaction a été suivi par LC-MS. Après cette période la réaction était complète. Le milieu réactionnel a été refroidi à température ambiante. Le solvant a été éliminé sous pression réduite. Le brut réactionnel a été purifié par chromatographie sur colonne de silice (dichlorométhane / méthanol 98 :2 jusqu'à 80 :20 par incréments de 2%) pour conduire au composé **60a** sous forme d'une huile (18 mg, 20%). RMN $^1H$ (400 MHz, $CDCl_3$) δ: 7,96 (m, 3H), 7,46 (m, 9H), 6,88 (m, 6H), 5,04 (s l, 1H), 4,11-3,84 (m, 12H), 3,80 (m, 9H), 3,09-2,74 (m, 13H), 1,77-1,70 (m, 9H), 1,39 (s, 9H), 1,42-1,23 (m, 7H), 1,25 (m, 9H); RMN $^{31}P$ (162 MHz, $CDCl_3$) δ: +39,2. HRMS (ESI+) calculée pour $C_{69}H_{87}N_7O_{11}P_3$ $[M+H]^+$, *m/z* 1282,5676, trouvée: 1282,5701. $R_f$ = 0,54 (silice; dichlorométhane - méthanol 80:20).

### Composés 58a-f

Ces composés ont été préparés selon la même procédure que celle utilisée pour le composé **61a** en utilisant les précurseurs correspondants.

### Composés 59a-f

Ces composés ont été préparés selon la même procédure que celle utilisée pour le composé **62a** en utilisant les précurseurs correspondants.

### Composés 60b-h, 60j-l

Ces composés ont été préparés selon la même procédure que celle utilisée pour le composé **60a** en utilisant les précurseurs correspondants.

**Composé 60i**

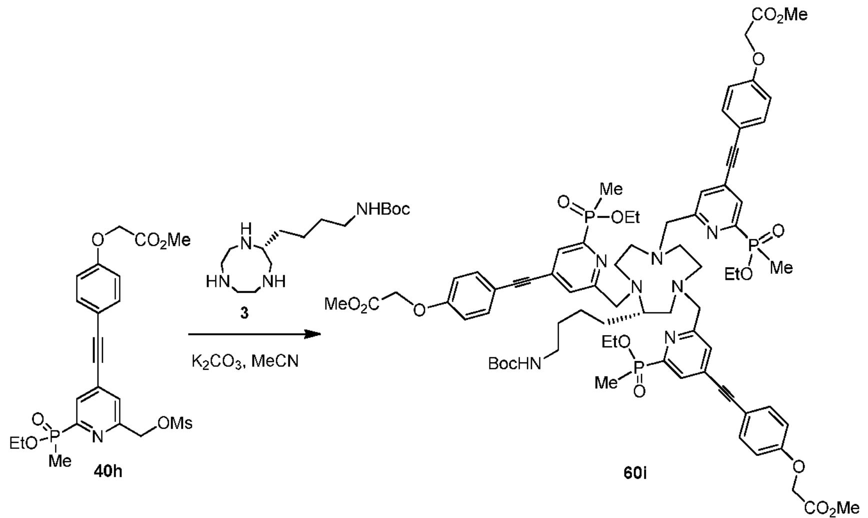


40h

60i

A une solution de composé **40h** (105 mg, 0,22 mmol) dans de l'acétonitrile anhydre (4 mL) ont été additionnés le macrocycle **3** (23 mg, 0,075 mmol) et du carbonate de potassium (32 mg, 0,23 mmol). Le mélange a été chauffé à 60°C pendant 18 h puis refroidi à température ambiante. Les sels en suspension ont été éliminés par décantation puis le solvant a été éliminé sous pression réduite. Le résidu a été purifié par chromatographie sur colonne de silice en utilisant un gradient de solvant (dichlorométhane - méthanol, 100-0 jusqu'à 80-20 par incrément de 1%) pour donner le composé **60i** sous forme d'une huile jaunâtre (45 mg, 41%). RMN $^1H$ (600 MHz, $CDCl_3$) δ: 7,99 (m, 3H), 7,49 (m, 9H), 6,90 (m, 6H), 5,10 (si, 1H), 4,67 (s, 6H), 4,12-3,86 (m, 12H), 3,82 (m, 9H), 3,12-2,90 (m, 13H), 1,85-1,70 (m, 9H), 1,41 (s, 9H), 1,45-1,28 (m, 6H), 1,27 (m, 9H); RMN $^{31}P$ (242 MHz, $CDCl_3$) δ: +40,4; HRMS (ESI+) calculée pour $C_{75}H_{94}N_7O_{17}P_3$ $[M+2H]^{2+}$, *m/z* 728,7960, trouvée: 728,7949. $R_f$ = 0,50 (silice; dichlorométhane - méthanol 80:20).

**Composé 61a**

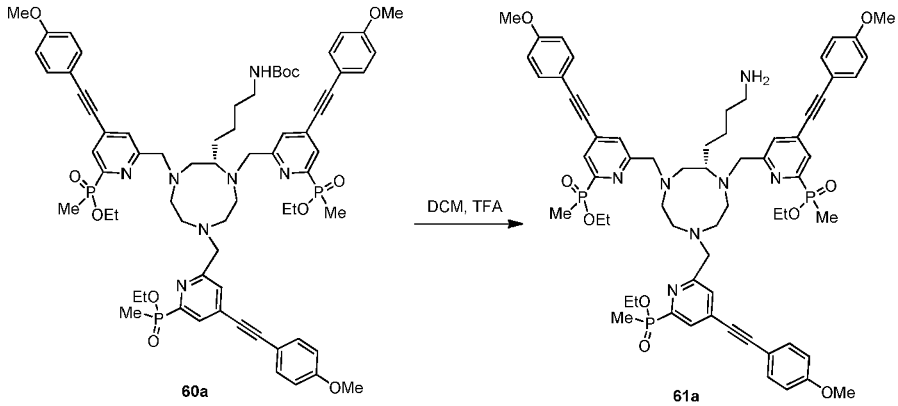


60a

61a

A une solution du composé **60a** (12 mg, 9,4 μmol) dans du dichlorométhane (0,8 mL) dégazée pendant 10 min sous atmosphère inerte, a été ajouté de l'acide trifluoroacétique (0,2 mL). La solution a été agitée à température ambiante pendant 15 min. L'avancement de la réaction a été suivi par LC-MS. Après cette période la réaction était totale. Le solvant a été éliminé sous pression réduite pour conduire au composé souhaité **61a** sous forme d'une huile jaune (12 mg, quantitatif). RMN $^1H$ (400 MHz, $CDCl_3$) δ: 7,93 (m, 3H), 7,46 (m, 9H), 6,88 (m, 6H), 4,15-3,84 (m, 12H), 3,76 (m, 9H), 3,00-2,71 (m, 13H), 1,80-1,75 (m, 9H), 1,42-1,23 (m, 7H), 1,25 (m, 9H); RMN $^{31}P$ (162 MHz, $CDCl_3$) δ : +39,7. HRMS (ESI+) calculée pour $C_{64}H_{79}N_7O_9P_3$ $[M+H]^+$, *m/z* 1182,5152, trouvée: 1182,5178.

**Composés 61b-h, 61j-l**

Ces composés ont été préparés selon la même procédure que celle utilisée pour le composé **61a** en utilisant les précurseurs correspondants.

## Composé 61i

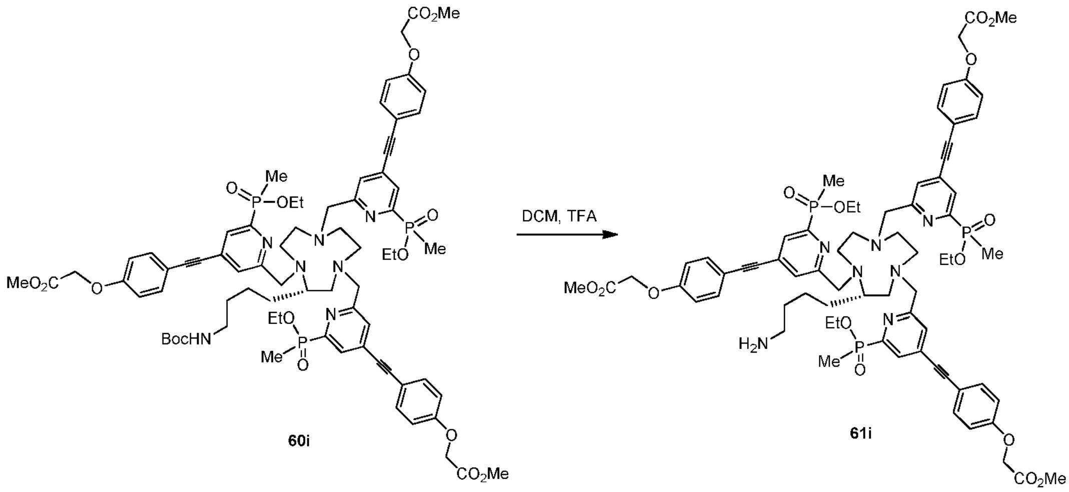


60i 61i

A une solution de composé **60i** (30 mg, 20 μmol) dans du dichlorométhane (1,8 mL) refroidie à 4°C et préalablement dégazée sous un courant d'argon pendant 10 min, a été additionné de l'acide trifluoroacétique (0,2 mL). La solution est réchauffée à température ambiante et agitée à cette température pendant 15 min. Le solvant a été éliminé sous pression réduite pour conduire à une huile jaune identifiée comme le composé **61i** (27 mg, quantitatif). RMN $^1H$ (600 MHz, $CDCl_3$) δ: 7,91 (m, 3H), 7,51 (m, 9H), 6,92 (m, 6H), 4,67 (s, 6H), 4,16-3,97 (m, 12H), 3,81 (m, 9H), 3,12-2,90 (m, 13H), 1,89-1,75 (m, 9H), 1,45-1,32 (m, 6H), 1,29 (m, 9H); RMN $^{31}P$ (242 MHz, $CDCl_3$) δ: +41,2; HRMS (ESI+) calculée pour $C_{70}H_{85}N_7O_{15}P_3$ $[M+H]^+$, *m/z* 1356,531, trouvée: 1356,533.

## Composé 62a

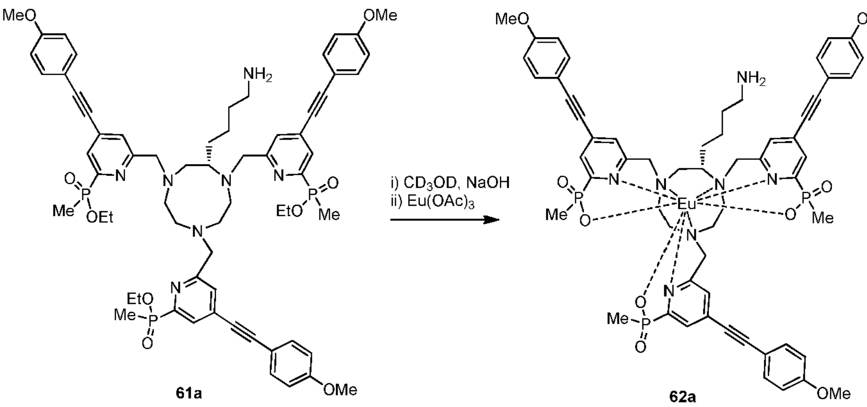


61a 62a

A une solution du ligand **61a** (25 mg, 21 μmol) dans du $CD_3OD$ (3 mL) a été additionné une solution aqueuse deutérée d'hydroxyde de sodium (1 mL, 0,1 M). La solution a été chauffée à 60°C pendant 5 h sous atmosphère inerte. L'avancement de la réaction a été suivi par RMN $^1H$ et $^{31}P$. Après cette période la réaction était totale. Le solvant a été éliminé sous pression réduite et le pH de la solution a été ajusté à 7 par addition d'une solution aqueuse d'acide chlorhydrique 1 M. A cette solution a été additionné de l'acétate d'europium (7,6 mg, 23 μmol). Le mélange réactionnel a été chauffé à 65°C sous atmosphère inerte pendant 12 h puis a été refroidi à température ambiante et le solvant a été éliminé sous pression réduite. Le produit brut a été purifié par chromatographie sur colonne de silice (dichlorométhane - méthanol - ammoniaque : 89,9: 10 :0,1) pour conduire au composé **62a** sous forme d'un solide blanc (8 mg, 30%). HRMS (ESI+) calculée pour $C_{58}H_{64}EuN_7O_9P_3$ $[M+H]^+$, *m/z* 1248,3191 trouvée: 1248,3. $R_t$ = 9,69 min (colonne Waters XBridge RP-$C_{18}$, 3,5 μm, 4,6 × 100 mm) avec une solution aqueuse d'acide trifluoroacetique à 0,2% (pH 1 - MeCN (v/v) comme éluant, gradient linéaire de 10 à 100% MeCN (20 min), avec un débit de 1 mL $min^{-1}$ et une détection UV à 320 nm.

**Composés 62b-h, 62j-l**

Ces composés ont été préparés selon la même procédure que celle utilisée pour le composé **62a** en utilisant les précurseurs correspondants.

## Composé 62i

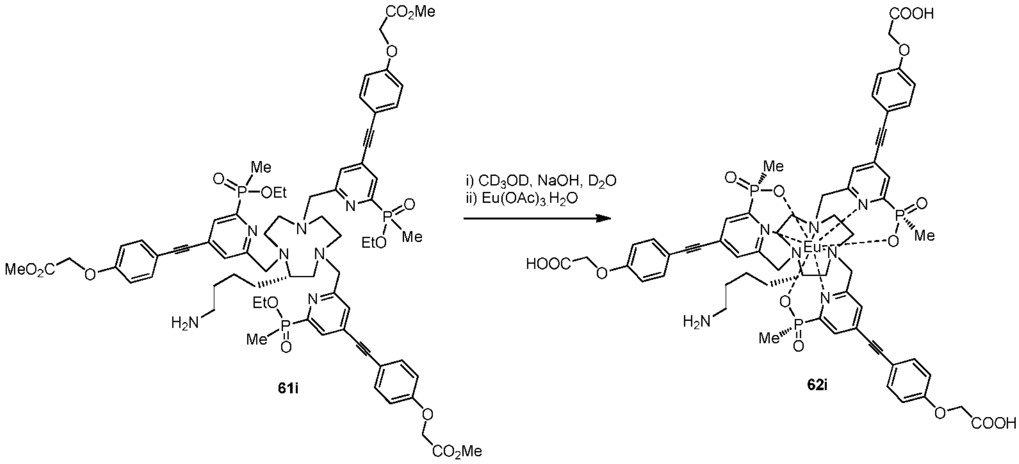


**61i** → **62i**

A une solution de composé **61i** (27 mg, 20 μmol) dans du méthanol deutéré (3 mL) a été ajoutée une solution aqueuse deutérée d'hydroxyde de sodium (0,1 M, 1,5 mL). Le mélange a été chauffé à 60°C pendant 5 h puis refroidi à température ambiante. Le pH de la solution a été ajusté à 7 par addition d'acide chlorhydrique. A ce mélange a été additionné de l'acétate d'europium hydrate (7 mg, 21 μmol) puis la solution a été chauffée à 65°C pendant 18 h sous atmosphère inerte. Après cette période la réaction a été refroidie à température ambiante et le solvant a été éliminé sous pression réduite. Le produit brut a été purifié par HPLC préparative pour conduire au composé **62i** (11 mg, 40%); LRMS (ESI+) calculée pour $C_{61}H_{65}N_7O_{15}P_3Eu$ $[M+2H]^{2+}$, *m*/*z* 690,6482 trouvée: 690,6.

**Composés 63a-c**

Ces composés ont été préparés selon la même procédure que celle utilisée pour le composé **67a** en utilisant les précurseurs correspondants.

**Composés 64a-c**

Ces composés ont été préparés selon la même procédure que celle utilisée pour le composé **68c** en utilisant les précurseurs correspondants.

**Composés 66a-c**

Ces composés ont été préparés selon la même procédure que celle utilisée pour le composé **70c** en utilisant les précurseurs correspondants.

## Composé 67a

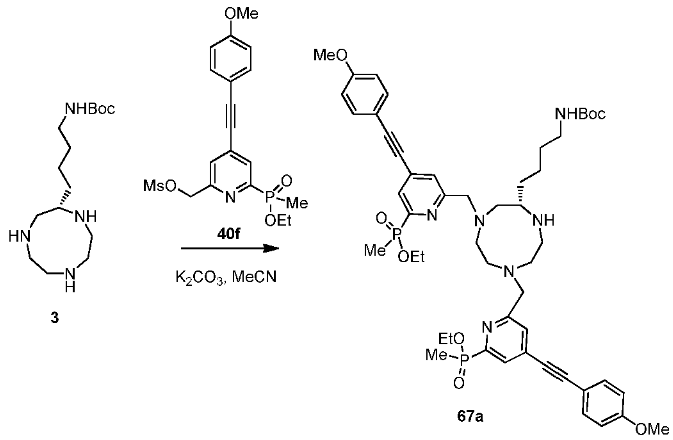


3

67a

A une solution du macrocycle **3** (21 mg, 0,071 mmol) dans l'acétonitrile anhydre (4 mL), a été ajouté du carbonate de potassium (30 mg, 0,21 mmol) sous atmosphère inerte. A cette suspension, a été ajoutée une solution de chromophore mésylé **40f** (90 mg, 0,21 mmol) dans l'acétonitrile anhydre (2 mL). Le mélange a été chauffé à 60°C pendant 24 h. L'avancement de la réaction a été suivi par LC-MS. Après cette période les analyses montraient la formation du composé dialkylé accompagné du composé trialkylé. Le milieu réactionnel a été refroidi à température ambiante. Les sels ont été séparés par décantation et le surnageant a été concentré sous pression réduite. Le brut réactionnel a été purifié par chromatographie sur colonne de silice (dichlorométhane / méthanol 98 :2 jusqu'à 80 :20 par incréments de 1%) pour conduire au composé **67a** sous forme d'une huile (14 mg, 20%). RMN $^1H$ (400 MHz, $CDCl_3$) $\delta$ : 7,87 (m, 2H), 7,40 (m, 6H), 6,82 (m, 4H), 5,04 (s l, 1H), 4,09-3,98 (m, 8H), 3,75 (m, 6H), 3,08-2,60 (m, 13H), 1,82-1,69 (m, 6H), 1,35 (s, 9H), 1,51-1,20 (m, 7H), 1,22 (m, 6H); RMN $^{31}P$ (162 MHz, $CDCl_3$) $\delta$ : +39,4. HRMS (ESI+) calculée pour $C,_{51}H_{69}N_6O_8P_2$ $[M+H]^+$, *m/z* 955,4652 trouvée: 955,4622. $R_f$ = 0,51 (silice; dichlorométhane - méthanol 80:20).

### Composés 67b-f

Ces composés ont été préparés selon la même procédure que celle utilisée pour le composé **67a** en utilisant les précurseurs correspondants.

## Composé 68c

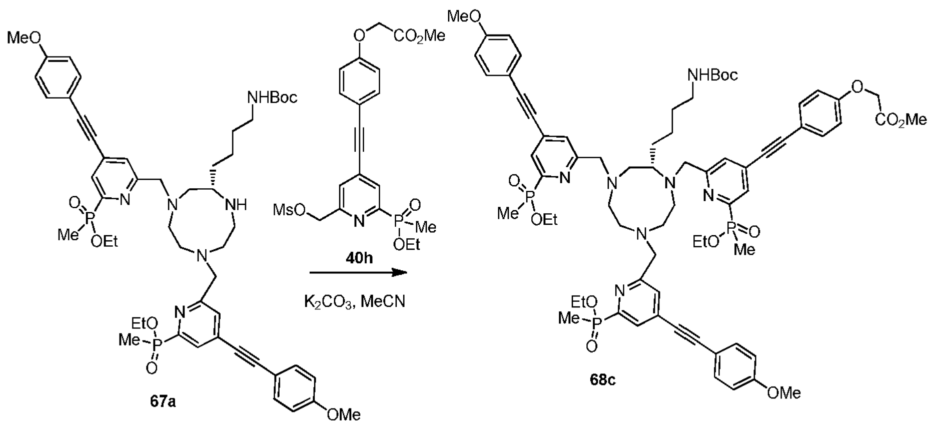


67a

68c

A une solution de TACN dibras **67a** (14 mg, 15 µmol) dans l'acétonitrile anhydre (0,7 mL), a été ajouté du carbonate de potassium (2 mg, 17 µmol) sous atmosphère inerte. A cette suspension, a été ajoutée le chromophore **40h** (8 mg, 17 µmol). Le mélange réactionnel a été chauffé à 60°C pendant 24 h sous atmosphère inerte. L'avancement de la réaction a été suivi par HPLC. Après cette période la réaction était totale. La suspension a été refroidie à température ambiante et les sels ont été séparés par décantation. Le solvant du surnageant a été éliminé sous pression pour obtenir une huile jaune correspondant au composé **68c** (19 mg, 95 %). RMN $^1H$ (700 MHz, $CDCl_3$) $\delta$: 7,92 (m, 3H); 7,43 (m, 9H); 6,83 (m, 6H); 5,04 (s l, 1H); 4,59 (s, 2H); 4,06-3,77 (m, 12H); 3,75 (m, 9H); 3,00-2,55 (m, 13H); 1,74-1,66 (m, 9H);

1,33 (s, 9H); 1,35-1,16 (m, 6H); 1,19 (m, 9H). RMN $^{31}P$ (284 MHz, $CDCl_3$) $\delta$ : +41,1. HRMS (ESI+) calculée pour $C_{71}H_{89}N_7O_{13}P_3$ $[M+H]^+$, *m/z* 1340,5730 trouvée: 1340,5730.

**Composés 68a-b,d-l**

Ces composés ont été préparés selon la même procédure que celle utilisée pour le composé **68c** en utilisant les précurseurs correspondants.

## Composé 70c

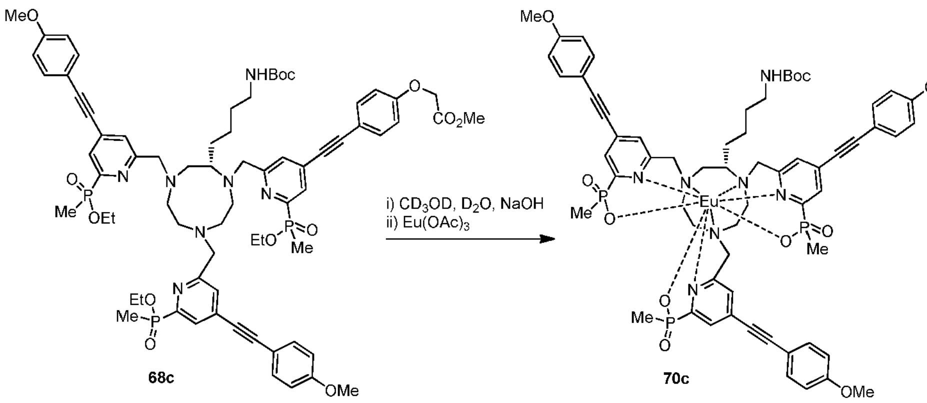


68c

70c

A une solution du ligand **68c** (7 mg, 5,2 μmol) dans du $CD_3OD$ (1,5 mL) a été additionné une solution aqueuse deutérée d'hydroxyde de sodium (0,5 mL, 0,1 M). La solution a été chauffée à 60°C pendant 16 h sous atmosphère inerte. L'avancement de la réaction a été suivi par RMN $^1H$ et $^{31}P$. Après cette période la réaction était totale. Le solvant a été éliminé sous pression réduite et le pH de la solution a été ajusté à 7 par addition d'une solution aqueuse d'acide chlorhydrique 1 M. A cette solution a été additionné de l'acétate d'europium (1,8 mg, 5,7 μmol). Le mélange réactionnel a été chauffé à 65°C sous atmosphère inerte pendant 12 h puis a été refroidi à température ambiante et le solvant a été éliminé sous pression réduite. Le produit brut a été purifié par HPLC. LRMS (ESI+) calculée pour $C_{64}H_{71}EuN_7O_{13}P_3$ $[M+H]^+$, *m/z* 1390,4 trouvée: 1390,4. $R_t$ = 8,6 min (colonne Waters XBridge RP-$C_{18}$, 5 μm, 10 × 100 mm) avec une solution de tampon bicarbonate d'ammonium 0,1 M (pH 9 - MeOH (v/v) comme éluant, [isocratique 10% MeOH (2 min)], gradient linéaire de 10 à 100% MeOH (15 min), avec un débit de 4,4 mL $min^{-1}$ et une détection UV à 330 nm.

**Composés 70a-b,d-l**

Ces composés ont été préparés selon la même procédure que celle utilisée pour le composé **70c** en utilisant les précurseurs correspondants.

## Composé 79

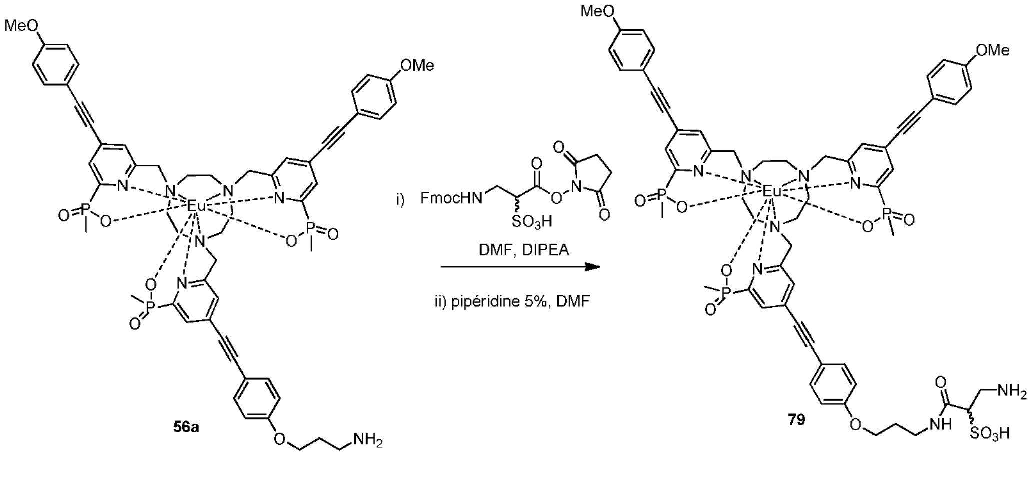


56a

79

A une solution de complexe d'europium $NH_2$ **56a** (500 nmol) dans du diméthylformamide anhydre (200 μL) a été ajoutée de la diisopropyléthylamine (0,2 μL, 1 μmol). A ce mélange a été additionnée une solution de Fmoc-sulfo-β-alanine (500 nmol) dans du diméthylformamide anhydre (61 μL). Le mélange a été agité à température ambiante pendant 1 h. L'avancement de la réaction a été suivi par HPLC. Après cette période, la réaction était totale. A ce mélange a été ajoutée une solution de pipéridine à 5% dans du diméthylformamide (200 μL) et le mélange a été agité à température ambiante pendant 30 min. L'avancement de la réaction a été suivi par HPLC. Après cette période la réaction était totale. Le brut réactionnel a été purifié par HPLC préparative en utilisant le gradient suivant : colonne Waters XBridge RP-$C_{18}$, 5 μm, 19 × 100 mm) avec une solution aqueuse d'acide trifluoroacétique 0,2% pH 1 - MeCN (v/v) comme éluant [isocratique 5% MeCN (2 min), gradient linéaire de 5 jusqu'à 100% MeCN (23 min) avec un débit de 20 mL $min^{-1}$ et une détection UV à 320 nm pour conduire au produit désiré **79** (442 nmol, 88%). HRMS (ESI+) calculée pour $C_{59}H_{66}EuN_8O_{13}P_3S$ $[M+2H]^{2+}$, *m/z* 685,1435 trouvée: 685,1439. $R_t$ = 11,5 min (colonne Waters XBridge RP-$C_{18}$, 3,5 μm, 4,6 × 100 mm) avec une solution aqueuse d'acide trifluoroacetique à 0,2% (pH 1 - MeCN (v/v) comme éluant [isocratique 5% MeCN (2 min)], gradient linéaire de 5 à 100% MeCN (20 min), avec un débit de 1 mL $min^{-1}$ et une détection UV à 320 nm.

## Composé 80

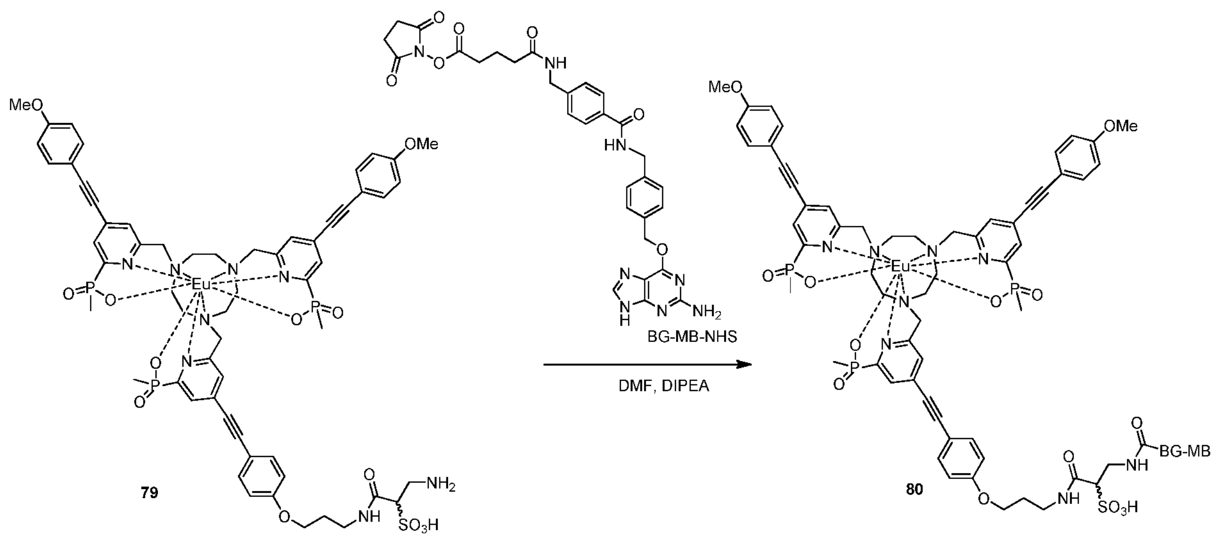


A une solution de complexe d'europium $NH_2$ **79** (25 nmol) dans du diméthylformamide anhydre (38 μL) ont été ajoutée de la diisopropyléthylamine (0,3 μL, 1,72 μmol) et une solution de BG-MB-NHS dans du diméthylformamide (16 μL, 25 nmol). La réaction est agitée à température ambiante pendant 1 h. L'avancement de la réaction a été suivi par HPLC. Après cette période, la réaction était totale. Le mélange réactionnel a été purifié par HPLC semi-préparative en utilisant le gradient suivant : colonne Waters XBridge RP-$C_{18}$, 5 μm, 10 × 250 mm) avec une solution aqueuse d'acide trifluoroacétique 0,2% pH 1 - MeCN (v/v) comme éluant [isocratique 5% MeCN (2 min), gradient linéaire de 5 jusqu'à 100% MeCN (23 min) avec un débit de 5 mL $min^{-1}$ et une détection UV à 320 nm pour conduire au produit désiré **80** (16 nmoles, 64%). LRMS (ESI+) calculée pour $C_{85}H_{91}E_uN_{15}O_{17}P_3S$ $[M+2H]^{2+}$, *m/z* 935,7432 trouvée: 936,12. $R_t$ = 8,42 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composé 81**

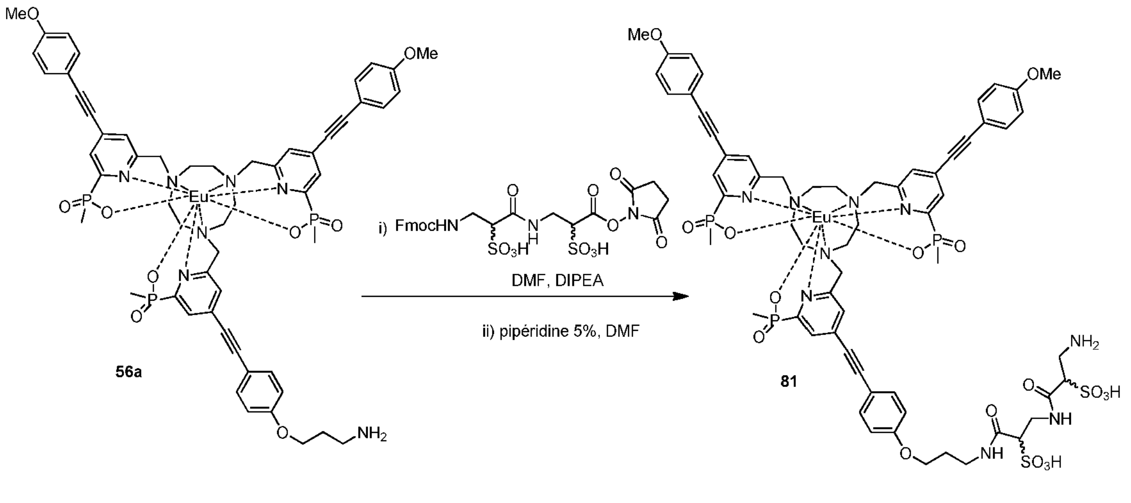


56a

81

A une solution de complexe d'europium $NH_2$ **56a** (5 μmol) dans du diméthylformamide anhydre (500 μL) a été ajoutée de la diisopropyléthylamine (1,7 μL). A ce mélange a été additionnée une solution de N-Fmoc di-sulfo-di-β-alanine NHS (5 μmol) dans du diméthylformamide anhydre (283 μL). Le mélange a été agité à température ambiante pendant 2 h. L'avancement de la réaction a été suivi par HPLC. Après cette période, la réaction était totale. A ce mélange a été ajoutée une solution de pipéridine à 5% (10 μmol) dans du diméthylformamide (20 μL) et le mélange a été agité à température ambiante pendant 12 h. L'avancement de la réaction a été suivi par HPLC. Après cette période la réaction était totale. Le brut réactionnel a été purifié par HPLC préparative en utilisant le gradient suivant : colonne Waters XBridge RP-$C_{18}$, 5 μm, 19 × 100 mm) avec une solution aqueuse d'acide formique 0,1% pH 2 - MeCN (v/v) comme éluant [isocratique 5% MeCN (2 min), gradient linéaire de 5 jusqu'à 100% MeCN (18 min) avec un débit de 20 mL $min^{-1}$ et une détection UV à 320 nm pour conduire au produit désiré **81** (3,6 μmol, 72%). HRMS (ESI+) calculée pour $C_{62}H_{71}EuN_9O_{17}P_3S_2$ $[M+2H]^{2+}$, *m/z* 760,6405 trouvée: 760,6402. $R_t$ = 8,15 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min)], gradient linéaire de 5 à 100% MeCN (14 min), avec un débit de 1 mL $min^{-1}$.

**Composé 82**

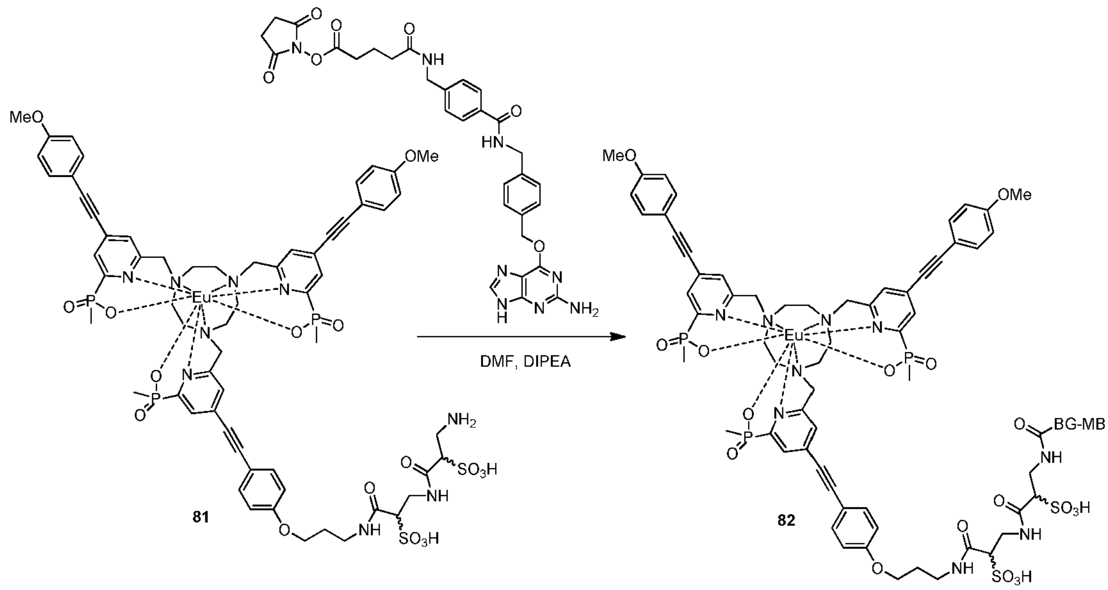


81

82

A une solution de complexe d'europium $NH_2$ **81** (200 nmol) dans du diméthylformamide anhydre (50 μL) ont été ajoutée de la diisopropyléthylamine (1,5 μL) et une solution de BG-MB-NHS (200 nmol) dans du diméthylformamide anhydre (100 μL). La réaction est agitée à température ambiante pendant 12 h. L'avancement de la réaction a été suivi par HPLC. Après cette période, la réaction était totale. Le mélange réactionnel a été purifié par HPLC préparative en

utilisant le gradient suivant : colonne Waters XBridge RP-$C_{18}$, 5 μm, 19 × 100 mm) avec une solution aqueuse d'acide trifluoroacétique 0,2% pH 1 - MeCN (v/v) comme éluant [isocratique 5% MeCN (2 min)], gradient linéaire de 5 jusqu'à 100% MeCN (18 min) avec un débit de 20 mL $min^{-1}$ et une détection UV à 320 nm pour conduire au composé **82** (15 nmol, 7%). LRMS (ESI-) calculée pour $C_{88}H_{92}EuN_{16}O_{21}P_3S_2$ $[M-2H]^{2-}$, *m/z* 1009,2245 trouvée: 1009,29. $R_t$ = 11,65 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (2 min)], gradient linéaire de 5 à 100% MeCN (18 min), avec un débit de 1 mL $min^{-1}$ et une détection UV de 320 nm.

## Composé 83

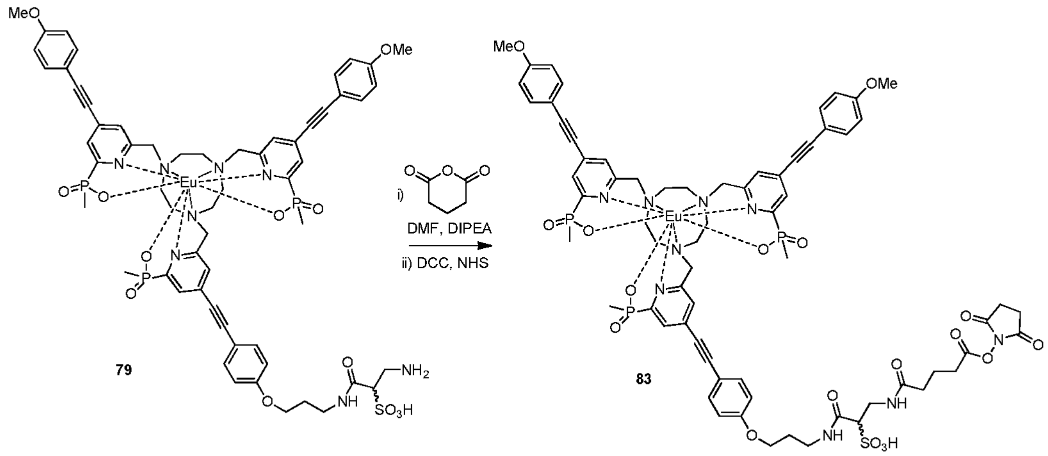


A une solution de complexe d'europium $NH_2$ 79 (1 μmol) dans du DMF anhydre (200 μL) a été ajoutée de la diisopropyléthylamine (0,3 μL). A ce mélange a été additionnée de l'anhydride glutarique (3 μmol). Le mélange a été agité à température ambiante pendant 30 min. L'avancement de la réaction a été suivi par HPLC. Après cette période, la réaction était totale. Le milieu réactionnel a été purifié par HPLC préparative pour conduire à l'acide correspondant (668 nmol, 67%). LRMS (ESI+) calculée pour $C_{64}H_{72}EuN_8O_{16}P_3S$ $[M+2H]^{2+}$, *m/z* 743,1606 trouvée: 742,67. $R_t$ = 7,02 min (colonne Waters XBridge RP-$C_{18}$, 3,5 μm, 4,6 × 100 mm) avec une solution aqueuse d'acide formique à 0,1% (pH 2 - MeCN (v/v) comme éluant [isocratique 5% MeCN (1 min) gradient linéaire de 5 à 100% MeCN (14 min), avec un débit de 1 mL $min^{-1}$. A une solution de complexe fonctionnalisé acide (200 nmol) dans du diméthylformamide anhydre (15 μL) ont été additionnés de la dicyclohexylcarbodiimide (2,2 mg, 11,4 μmol) et du N-hydroxysuccinimide (1,5 mg, 13 μmol). Le mélange réactionnel a été agité à température ambiante pendant 28 h. L'avancement de la réaction a été suivi par HPLC. Après cette période, la réaction était totale. Le milieu réactionnel a été purifié directement par HPLC semi-préparative pour conduire au composé NHS **83** (69 nmol, 35%). LRMS (ESI+) calculée pour $C_{68}H_{75}EuN_9O_{18}P_3S$ $[M+2H]^{2+}$, *m/z* 791,6688 trouvée: 791,83. $R_t$ = 7,35 min (colonne Waters XBridge RP-$C_{18}$, 3,5 μm, 4,6 × 100 mm) avec une solution aqueuse d'un tampon de formiate d'ammonium 10 mM pH 4,7 - MeCN (v/v) comme éluant [isocratique 15% MeCN (1 min) gradient linéaire de 15 à 100% MeCN (14 min), avec un débit de 1 mL $min^{-1}$.

## Composé 84

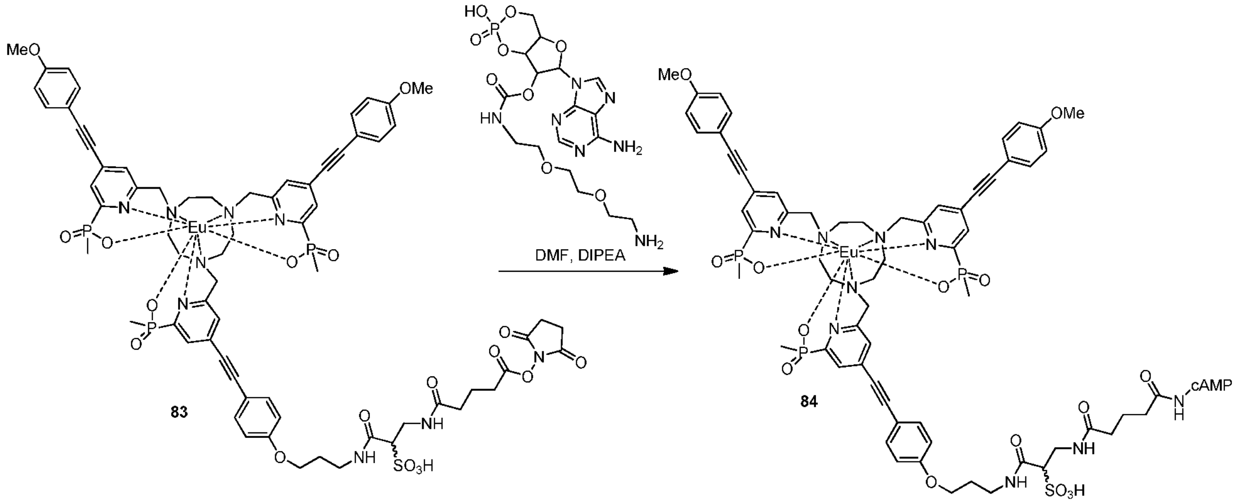


83

84

A une solution de complexe d'europium NHS **83** (69 nmol) dans du diméthylformamide anhydre (200 µL) ont été ajoutés de la diisopropyléthylamine (0,9 µL) et une solution d'AMPc-$NH_2$ (90 nmol) dans du diméthylformamide anhydre (9 µL). Le milieu réactionnel a été agité pendant une nuit. L'avancement de la réaction a été suivi par HPLC. Le milieu réactionnel a été purifié par HPLC semi-préparative en utilisant le gradient suivant : colonne Waters XBridge RP-$C_{18}$, 5 µm, 10 × 250 mm) avec une solution aqueuse d'acide trifluoroacétique 0,2% pH 1 - MeCN (v/v) comme éluant [isocratique 5% MeCN (2 min)], gradient linéaire de 5 jusqu'à 100% MeCN (23 min) avec un débit de 5 mL $min^{-1}$ et une détection UV à 320 nm pour conduire au produit désiré **84** (10 nmoles, 14%). LRMS (ESI+) calculée pour $C_{81}H_{95}EuN_{15}O_{24}P_4S$ $[M+2H]^{2+}$, *m/z* 985,2279 trouvée: 986,30. $R_f$ = 6,58 min (colonne Waters XBridge RP-$C_{18}$, 3,5 µm, 4,6 × 100 mm) avec une solution aqueuse d'acide formique à 0,1% (pH 2 - MeCN (v/v) comme éluant [isocratique 5% MeCN (1 min) gradient linéaire de 5 à 100% MeCN (14 min), avec un débit de 1 mL $min^{-1}$.

## Composé 85

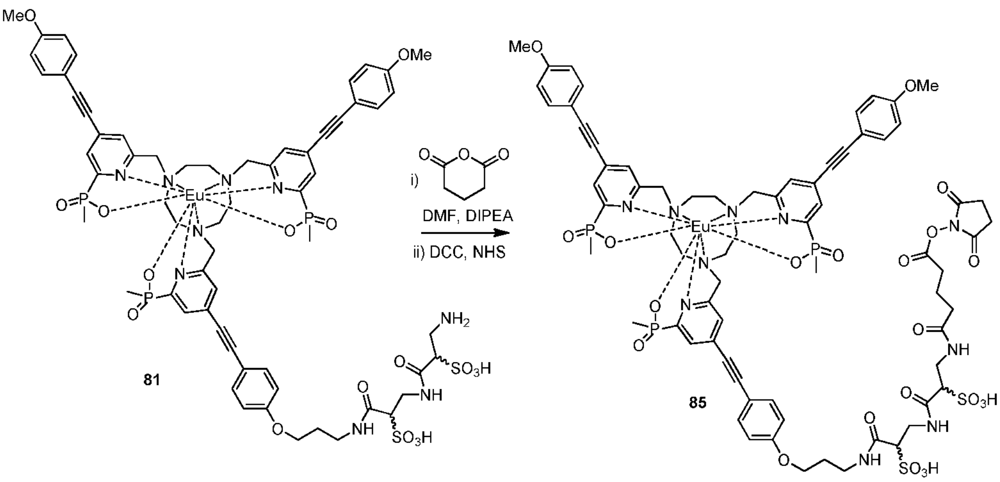


81

85

A une solution de complexe d'europium $NH_2$ **81** (2 µmol) dans du DMF anhydre (500 µL) a été ajoutée de la diisopropyléthylamine (2,6 µL, 15 µmol). A ce mélange a été additionnée de l'anhydride glutarique (6 µmol). Le mélange a été agité à température ambiante pendant 16 h. L'avancement de la réaction a été suivi par HPLC. Après cette période, la réaction était totale. Le milieu réactionnel a été purifié par HPLC préparative en utilisant le gradient suivant : colonne Waters XBridge RP-$C_{18}$, 5 µm, 19 × 100 mm) avec une solution d'un tampon d'acétate de triéthyl ammonium 25 mM pH 5 - MeCN (v/v) comme éluant [isocratique 5% MeCN (2 min)], gradient linéaire de 5 jusqu'à 100% MeCN (25 min) avec un débit de 20 mL $min^{-1}$ et une détection UV à 320 nm pour conduire à l'acide correspondant (1,8 µmol, 98%). LRMS (ESI+) calculée pour $C_{67}H_{77}EuN_9O_{20}P_3S_2$ $[M+2H]^{2+}$, *m/z* 818,6576 trouvée: 818,16. $R_t$ = 6,73 min (colonne Waters XBridge RP-$C_{18}$, 3,5 µm, 4,6 × 100 mm) avec une solution aqueuse d'un tampon de formiate d'ammonium 10 mM pH 4,7 - MeCN (v/v) comme éluant [isocratique 15% MeCN (1 min)], gradient linéaire de 15 à 100% MeCN (14

min), avec un débit de 1 mL min$^{-1}$. A une solution de complexe acide (480 nmol) dans du diméthylformamide anhydre (120 μL) ont été additionnés de la dicyclohexylcarbodiimide (1,9 mg, 9,89 μmol) et du N-hydroxysuccinimide (1,7 mg, 14,8 μmol). Le mélange réactionnel a été agité à température ambiante pendant 28 h. L'avancement de la réaction a été suivi par HPLC. Après cette période, la réaction était totale. Le milieu réactionnel a été purifié directement par HPLC préparative en utilisant le gradient suivant : colonne Waters XBridge RP-$C_{18}$, 5 μm, 19 × 100 mm) avec une solution d'un tampon d'acétate de triéthyl ammonium 25 mM pH 5 - MeCN (v/v) comme éluant [isocratique 5% MeCN (2 min), gradient linéaire de 5 jusqu'à 100% MeCN (25 min) avec un débit de 20 mL min$^{-1}$ et une détection UV à 320 nm pour conduire au composé souhaité **85** (40 nmol, 8%). LRMS (ESI+) calculée pour $C_{71}H_{80}EuN_{10}O_{22}P_3S_2$ $[M+2H]^{2+}$, *m/z* 867,1658 trouvée: 866,60. $R_t$ = 6,93 min (colonne Waters XBridge RP-$C_{18}$, 3,5 μm, 4,6 × 100 mm) avec une solution aqueuse d'un tampon de formiate d'ammonium 10 mM pH 4,7 - MeCN (v/v) comme éluant [isocratique 15% MeCN (1 min) gradient linéaire de 15 à 100% MeCN (14 min), avec un débit de 1 mL min$^{-1}$.

**Composé 86**

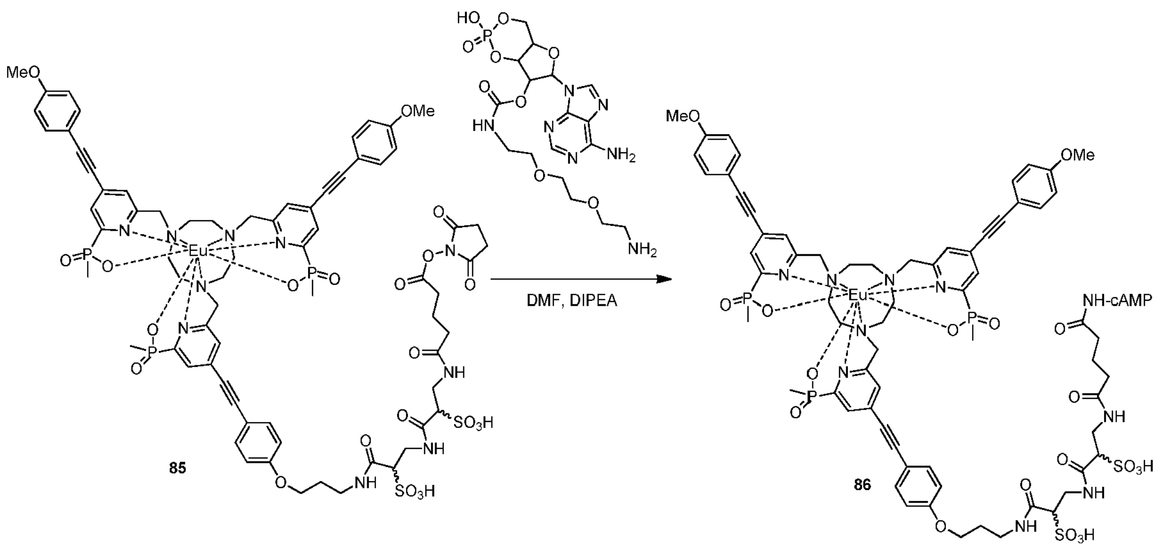


A une solution de complexe d'europium NHS **85** (40 nmol) dans du diméthylformamide anhydre (200 μL) ont été ajoutés de la diisopropyléthylamine (0,1 μL, 575 nmol) et une solution d'AMPc-$NH_2$ (50 nmol) dans du diméthylformamide anhydre (4,8 μL). Le milieu réactionnel a été agité pendant 16 h à température ambiante et sous atmosphère inerte. L'avancement de la réaction a été suivi par HPLC. Après cette période la réaction était totale. Le milieu réactionnel a été purifié par HPLC semi-préparative : colonne Waters XBridge RP-$C_{18}$, 5 μm, 5 × 150 mm) avec une solution d'un tampon d'acétate de triéthyl ammonium 25 mM pH 5 - MeCN (v/v) comme éluant [isocratique 5% MeCN (2 min), gradient linéaire de 5 jusqu'à 100% MeCN (23 min) avec un débit de 5 mL min$^{-1}$ et une détection UV à 320 nm pour conduire au composé souhaité **86** (10 nmol, 25%). LRMS (ESI+) calculée pour $C_{84}H_{101}EuN_{16}O_{28}P_4S_2$ $[M+2H]^{2+}$, *m/z* 1061,2288 trouvée: 1062,07. $R_t$ = 6,33 min (colonne Waters XBridge RP-$C_{18}$, 3,5 μm, 4,6 × 100 mm) avec une solution aqueuse d'un tampon de formiate d'ammonium 10 mM pH 4,7 - MeCN (v/v) comme éluant [isocratique 15% MeCN (1 min) gradient linéaire de 15 à 100% MeCN (14 min), avec un débit de 1 mL min$^{-1}$.

## Composé 87

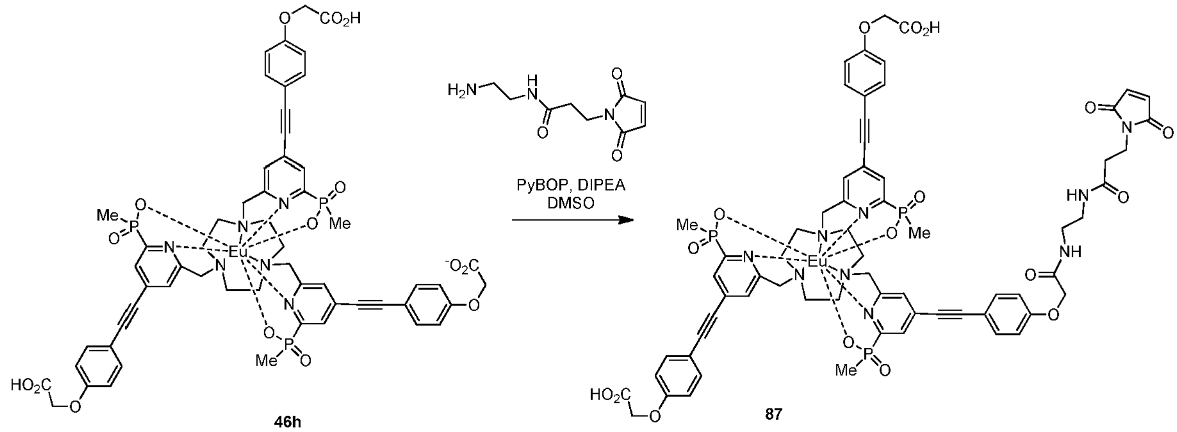


46h 87

A une solution du complexe **46h** (1 mg, 760 nmol) dans du diméthylsufoxyde anhydre (50 μL) ont été additionnés de l'hexafluorophosphate de benzotriazole-1-yl-oxy-tris-(diméthylamino)-phosphonium (0,13 mg, 250 nmol) et de la diisopropyléthylamine (0,13 μL, 760 nmol). Le mélange a été agité pendant à température ambiante pendant 5 min. A cette solution a été ajoutée une solution de dérivé maléimide (0,16 mg, 760 nmol) dans du diméthylsulfoxyde anhydre (50 μL) puis la solution a été agité à température ambiante sous atmosphère inerte pendant 72 h. L'avancement de la réaction a été suivi par LCMS. Après cette période la réaction était totale. Le mélange réactionnel a été purifié par HPLC semi-préparative au produit souhaité **87** (0,41 mg, 41%). HRMS (ESI+) calculée pour $C_{66}H_{67}EuN_9O_{17}P_3$ $[M+2H]^{2+}$, *m/z* 751,6540 trouvée: 751,6664.

## Composé 88

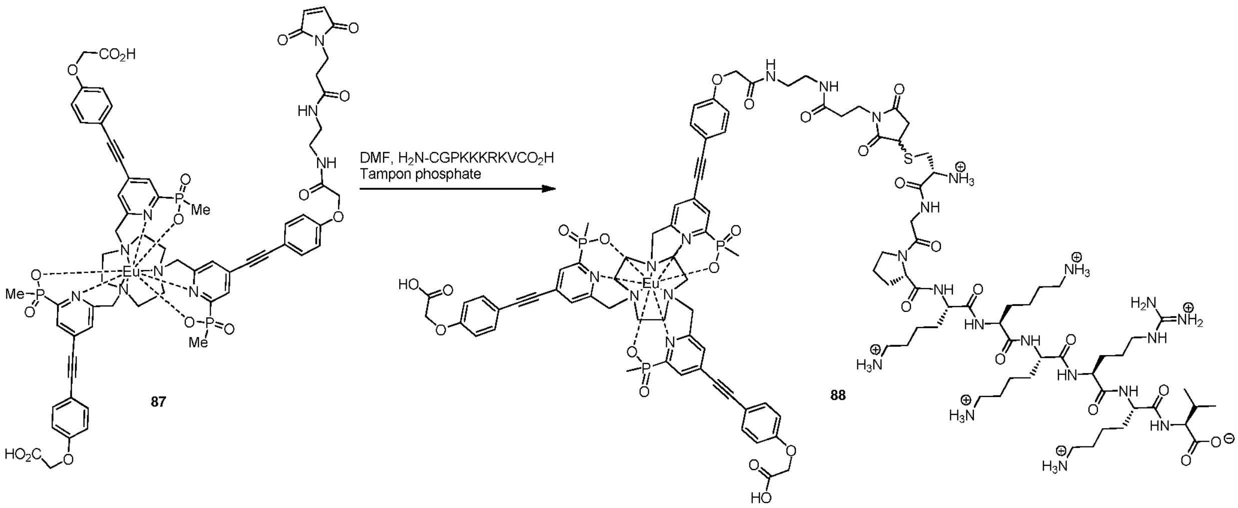


87 88

A une solution de maléimide **87** (0,20 mg, 133 nmol) dans du diméthylformamide anhydre (50 μL) a été additionné une solution de peptide $H_2N$-CGPKKKRKV-$CO_2H$ (0,26 mg, 200 μmol) dans du tampon phosphate 50 mM, pH 7 (20μL). Le mélange a été agité à température ambiante sous atmosphère inerte pendant 15 h. L'avancement de la réaction a été suivi par HPLC. Après cette période, la réaction était totale. Le mélange réactionnel a été purifié par HPLC semi-préparative pour conduire au conjugué **88** (0,21 mg, 62%). HRMS (ESI+) calculée pour $C_{111}H_{154}EuN_{25}O_{27}P_3S$ $[M]^{3+}$, *m/z* 848,9871 trouvée: 848,9963. $R_t$ = 9,9 min (colonne Waters XBridge RP-$C_{18}$, 5 μm, 10 × 100 mm) avec une solution aqueuse d'acide formique à 0,1% (pH 2 - MeOH (v/v) comme éluant gradient linéaire de 5 à 100% MeCN (15 min), avec un débit de 4,4 mL $min^{-1}$ et une détection UV à 330 nm.

## Composé 89

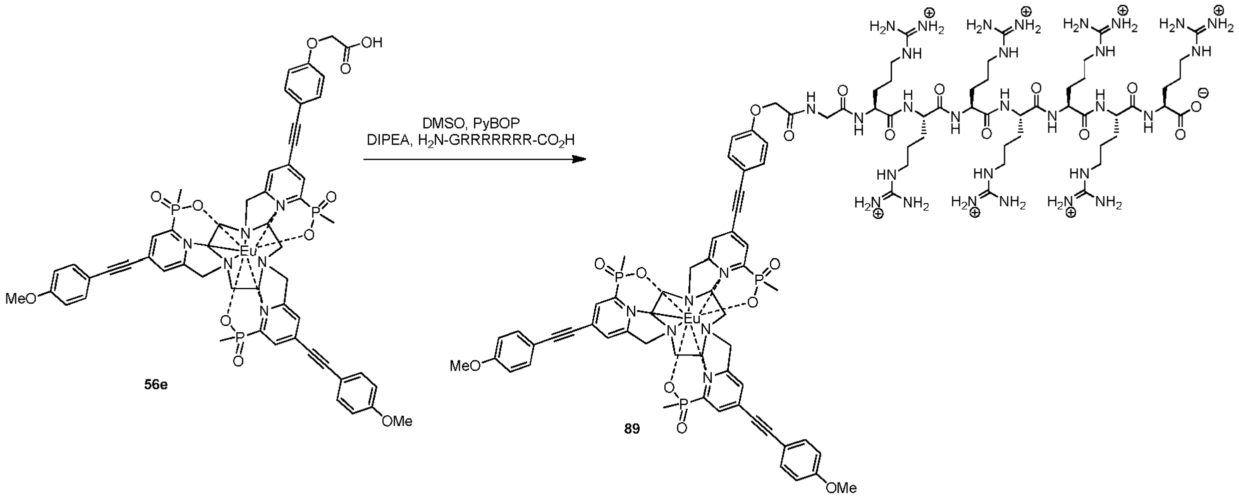


56e

89

A une solution du complexe **56e** (1 mg, 0,82 µmol) dans du diméthylsufoxyde anhydre (150 µL) ont été additionnés de l'hexafluorophosphate de benzotriazole-1-yl-oxy-tris-(diméthylamino)-phosphonium (0,65 mg, 1,25 µmol) et de la diisopropyléthylamine (0,3 µL, 1,65 µmol). Le mélange a été agité à température ambiante pendant 5 min. A cette solution a été ajoutée une solution de $H_2N$-GRRRRRRR-$CO_2H$ (1 mg, 0,86 µmol) dans du diméthylsulfoxyde anhydre (50 µL) puis la solution a été agitée à température ambiante sous atmosphère inerte pendant 24 h. L'avancement de la réaction a été suivi par LC-MS. Après cette période la réaction était totale. Le mélange réactionnel a été purifié par HPLC semi-préparative pour conduire au conjugué **89** (1,2 mg, 62%). HRMS (ESI+) calculée pour $C_{99}H_{144}EuN_{35}O_{19}P_3$ $[M+3H]^{3+}$, *m/z* 790,9940 trouvée: 790,9938.

## Composé 90

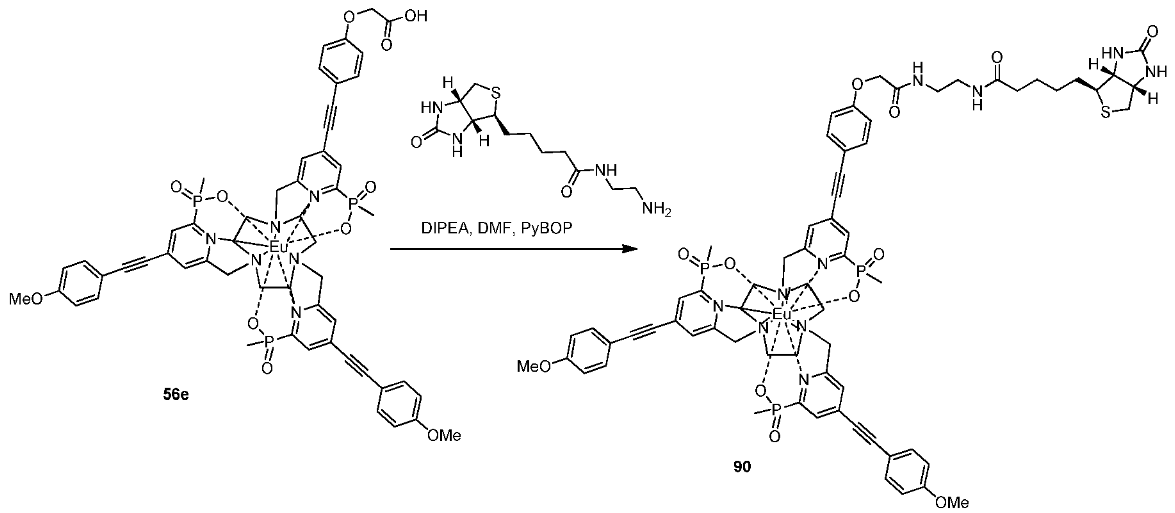


56e

90

A une solution du complexe **56e** (1 mg, 0,82 µmol) dans du diméthylsufoxyde anhydre (150 µL) ont été additionnés de l'hexafluorophosphate de benzotriazole-1-yl-oxy-tris-(diméthylamino)-phosphonium (0,65 mg, 1,25 µmol) et de la diisopropyléthylamine (0,3 µL, 1,65 µmol). Le mélange a été agité à température ambiante pendant 5 min. A cette solution a été ajoutée une solution de d'éthylène diamine-biotine (0,35 mg, 1,23 µmol) dans du diméthylformamide anhydre (50 µL) puis la solution a été agitée à température ambiante sous atmosphère inerte pendant 3 h. L'avancement de la réaction a été suivi par LC-MS. Après cette période la réaction était totale. Le mélange réactionnel a été purifié par HPLC semi-préparative pour conduire au conjugué biotinylé **90** (1,0 mg, 82%). HRMS (ESI+) calculée pour $C_{67}H_{76}EuN_{10}O_{12}P_3$ $[M+2H]^{2+}$, *m/z* 745,1899 trouvée: 745,1907.

**Composé 91**

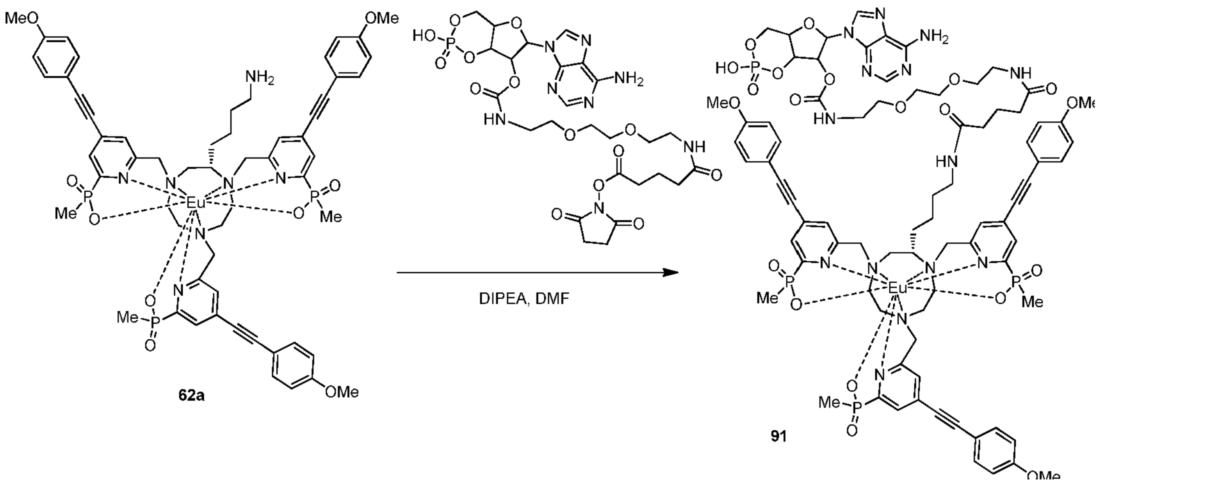


A une solution de complexe d'europium $NH_2$ **62a** (250 nmol) dans du diméthylformamide (50 µL) ont été ajoutés de la diisopropyléthylamine (5 µL, 28 µmol) et une solution AMPc-NHS (250 nmol) dans du diméthylformamide (50 µL). Le mélange a été agité à température ambiante et sous atmosphère inerte pendant 1h. L'avancement de la réaction a été suivi par HPLC. Après cette période la réaction était totale. A cette solution a été ajoutée une solution aqueuse d'acide trifluoroacétique à 0,2% (8 mL) et ce mélange a été purifié par HPLC préparative en utilisant le gradient suivant : (colonne Waters XBridge RP-$C_{18}$, 5 µm, 19 × 100 mm) avec une solution aqueuse d'acide trifluoroacétique 0,2% pH 1 - MeCN (v/v) comme éluant, gradient linéaire de 5 jusqu'à 60% MeCN (19 min) avec un débit de 20 mL $min^{-1}$ et une détection UV à 320 nm pour conduire au produit désiré **91** (82 nmol, 33%). LRMS (ESI+) calculée pour $C_{80}H_{96}EuN_{14}O_{20}P_4$ $[M+3H]^{2+}$, *m/z* 924,7544 trouvée: 924,63. $R_t$ = 9,10 min (colonne Waters XBridge RP-$C_{18}$, 3,5 µm, 4,6 × 100 mm) avec une solution aqueuse d'acide trifluoroacétique à 0,2% (pH 1 - MeCN (v/v) comme éluant gradient linéaire de 10 à 100% MeCN (20 min), avec un débit de 1 mL $min^{-1}$ et une détection UV à 320 nm.

**Composé 92**

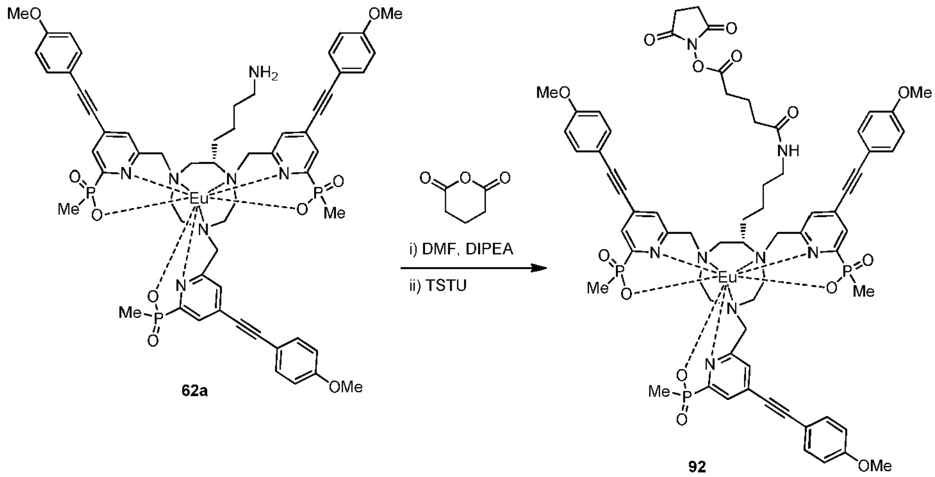


A une solution de complexe d'europium $NH_2$ **62a** (650 nmol) dans du diméthylformamide anhydre (400 µL) a été ajoutée de la diisopropyléthylamine (4 µL, 22 µmol). A ce mélange a été additionnée une solution d'anhydride glutarique (780 nmol) dans du diméthylformamide (80 µL). Le mélange a été agité à température ambiante pendant 1 h. L'avancement de la réaction a été suivi par HPLC. Après cette période, la réaction était totale. A cette solution a été ajoutée une solution aqueuse d'acide formique à 0,1% (8 mL) et ce mélange a été purifié par HPLC préparative en utilisant le gradient suivant : (colonne Waters XBridge RP-$C_{18}$, 5 µm, 19 × 100 mm) avec une solution aqueuse d'acide formique 0,1% pH 2 - MeCN (v/v) comme éluant, gradient linéaire de 10 jusqu'à 60% MeCN (19 min) avec un débit de 20 mL $min^{-1}$ et une détection UV à 320 nm pour conduire à l'acide désiré (400 nmol, 61%). LRMS (ESI+) calculée pour $C_{63}H_{70}EuN_7O_{12}P_3$ $[M+H]^+$, *m/z* 1362,3508 trouvée: 1362,91. $R_t$ = 9,14 min (colonne Waters XBridge RP-$C_{18}$, 3,5 µm,

4,6 × 100 mm) avec une solution aqueuse d'acide formique à 0,1% (pH 2 - MeCN (v/v) comme éluant gradient linéaire de 15 à 100% MeCN (15 min), avec un débit de 1 mL $min^{-1}$ et une détection UV à 320 nm. A une solution de complexe acide (400 nmol) dans du diméthylformamide anhydre (400 μL) ont été additionnés de la diisopropyléthylamine (4 μL, 22 μmol) puis du tétrafluoroborate de O-(N-succinimidyl)-1,1,3,3-tétraméthyluronium (0,14 mg, 480 nmol). Le mélange réactionnel a été agité à température ambiante pendant 15 min. L'avancement de la réaction a été suivi par HPLC. Après cette période, la réaction était totale. A cette solution a été ajoutée une solution aqueuse d'acide formique à 0,1% (8 mL) et ce mélange a été purifié par HPLC préparative en utilisant le gradient suivant : (colonne Waters XBridge RP-$C_{18}$, 5 μm, 19 × 100 mm) avec une solution aqueuse d'acide formique 0,1% pH 2 - MeCN (v/v) comme éluant, gradient linéaire de 10 jusqu'à 60% MeCN (19 min) avec un débit de 20 mL $min^{-1}$ et une détection UV à 320 nm pour conduire à l'ester de NHS désiré **92** (263 nmol, 65%). LRMS (ESI+) calculée pour $C_{67}H_{74}EuN_8O_{14}P_3$ $[M+H]^+$, *m/z* 1460,3750 trouvée: 1460,17. $R_t$ = 9,52 min (colonne Waters XBridge RP-$C_{18}$, 3,5 μm, 4,6 × 100 mm) avec une solution aqueuse d'acide formique à 0,1% (pH 2 - MeCN (v/v) comme éluant gradient linéaire de 15 à 100% MeCN (15 min), avec un débit de 1 mL $min^{-1}$ et une détection UV à 320 nm.

## Composé 93

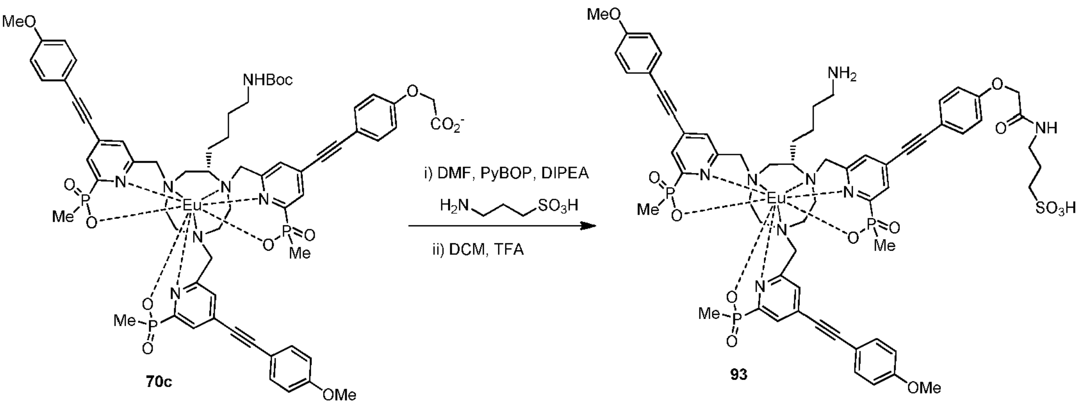


A une solution du complexe **70c** (0,35 mg, 0,25 μmol) dans du diméthylformamide (50 μL) ont été additionnés de l'hexafluorophosphate de benzotriazole-1-yl-oxy-tris-(diméthylamino)-phosphonium (0,2 mg, 0,37 gmol) et de la diisopropyléthylamine (0,11 μL, 0,65 μmol). Le mélange a été agité pendant à température ambiante pendant 5 min. A cette solution a été ajoutée une solution d'homotaurine (0,05 mg, 0,37 μmol) dans de l'eau (10 μL) puis la solution a été agitée à température ambiante sous atmosphère inerte pendant 16 h. L'avancement de la réaction a été suivi par LC-MS. Après cette période la réaction était totale. Le solvant a été éliminé sous pression réduite. Le résidu a été dissous dans du dichlorométhane (95 μL) sous atmosphère inerte à 4°C et à cette solution a été ajouté de l'acide trifluoroacétique. Le mélange a été agité pendant 20 min puis le solvant a été éliminé sous pression réduite. Le mélange réactionnel a été purifié par HPLC semi-préparative pour conduire au composé **93** (0,3 mg, 85%). HRMS (ESI-) calculée pour $C_{62}H_{69}EuN_8O_{13}P_3S$ $[M]^-$, *m/z* 1409,3120 trouvée: 1409,3110.

**Composés 95a-b**

Ces composés ont été préparés selon la même procédure que celle utilisée pour le composé **95c** en utilisant les précurseurs correspondants.

## Composé 95c

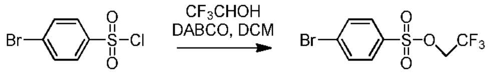


**94c** **95c**

A une solution de chlorure de 4-bromo-benzene-sulfonyle **94c** (3 g, 11,7 mmol) dissous dans du dichlorométhane (20 mL) ont été ajoutés du trifluoroéthanol (840 μL, 11,7 mmol) et une solution de 1,4-diazabicyclo[2.2.2]octane (1,5 g, 14,4 mmol) dans du dichlorométhane (10 mL). Un précipité a été observé. La réaction a été agitée à température ambiante pendant 1 h. A ce mélange a été ajoutée une solution aqueuse d'hydroxyde de sodium 1 M (3 mL). La solution a été diluée dans l'acétate d'éthyle (100 mL) et lavée successivement avec une solution aqueuse de bicarbonate de

sodium 0,5 M puis une solution aqueuse d'acide chlorhydrique 0,1 M, de l'eau et de la saumure. La phase organique a été séchée sur sulfate de magnésium, filtrée et concentrée sous pression réduite pour conduire à un solide blanc correspondant au composé **95c** (3,4 g, 91%). PtF 105-107 °C. RMN $^1$H (600 MHz, $CDCl_3$) δ: 7,77 (m, 4H), 4,40 (q, *J* = 8 Hz, 2H); RMN $^{13}$C (151 MHz, $CDCl_3$) δ: 134,1; 133,1; 130,3; 129,7; 122,0 (q, *J* = 278 Hz); 65,0 (q, *J* = 38 Hz); RMN $^{19}$F (564 MHz, $CDCl_3$) δ: -74,2 (t, $^3J$ = 7,0 Hz); HRMS (ESI+) calculée pour $C_8H_6O_3SBrF_3Na$ [M+Na]$^+$, *m/z* 340,9071, trouvée: 340,9077. $R_f$ = 0,58 (silice; cyclohexane - acétate d'éthyle 8:2).

**Composés 96a-b**

Ces composés ont été préparés selon la même procédure que celle utilisée pour le composé **96c** en utilisant les précurseurs correspondants.

## Composé 96c

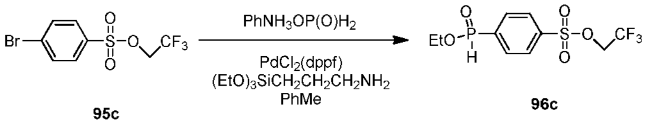


95c 96c

A une solution d'anilinium hypophosphite (1 g, 6,3 mmol) dans du toluène anhydre a été ajouté le composé **95c** (1,6 g, 5 mmol). A cette solution dégazée avec un courant d'argon pendant 30 min, a été ajoutée de l'aminopropyltriéthoxysilane (1,48 mL, 6,3 mmol) sous atmosphère inerte et cette solution a été dégazée encore pendant 30 min supplémentaires. A cette solution a été ajouté du chlorure de bis(diphénylphosphino)ferrocène]palladium(II) (220 mg, 0,2 mmol) et le mélange a été chauffé à 100°C pendant 45 min. L'avancement de la réaction a été suivi par RMN du phosphore. Après cette période la réaction était totale. Le mélange réactionnel a été refroidi à température ambiante et le solvant a été éliminé sous pression réduite. A ce résidu a été ajoutée une solution d'acide chlorhydrique 1 M (10 mL). La phase aqueuse a été extraite avec de l'acétate d'éthyle (3 x 20 mL), les phases organiques ont été réunies, séchées sur sulfate de magnésium, filtrées et concentrées sous pression réduite pour conduire à une huile orange correspondant au composé **96c** attendu. Le composé était suffisamment pur pour être utilisé dans l'étape suivante sans purification complémentaire. RMN $^1$H (600 MHz, $CDCl_3$) δ: 8,03 (m, 4H), 7,76 (d, *J* = 576 Hz, 1H), 4,43 (q, $^3J$ = 10 Hz, 2H), 4,21 (m, 2H), 1,40 (t, $^3J$ = 7,0 Hz, 3H); RMN $^{19}$F (564 MHz, $CDCl_3$) δ: -74,2 (t, $^3J$ = 8 Hz); RMN $^{31}$P (242 MHz, $CDCl_3$) δ: +20,8; HRMS (ESI+) calculée pour $C_{10}H_{13}O_5SF_3P$ [M+H]$^+$, *m/z* 333,0173, trouvée: 333,0167.

**Composés 97a-b**

Ces composés ont été préparés selon la même procédure que celle utilisée pour le composé **97c** en utilisant les précurseurs correspondants.

## Composé 97c

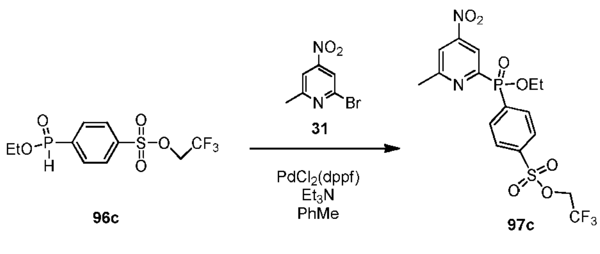


96c 97c

A une solution de composé **96c** (1,6 g, 4,8 mmol) dans du toluène préalablement dégazé (40 mL) ont été ajoutés du 2-bromo-6-méthyl-4-nitropyridine **31** (1 g, 4,8 mmol) et de la triéthylamine fraîchement distillée (2,3 mL, 16,8 mmol). A cette solution jaune préalablement dégazée par un courant d'argon pendant 30 min, a été ajouté du chlorure de bis(diphénylphosphino)ferrocène]palladium(II) (110 mg, 0,1 mmol) puis le mélange réactionnel a été chauffé à 120°C pendant 2 h. Au cours du temps, la couleur de la solution est devenue marron. La solution a été refroidie à température ambiante et le solvant a été éliminé sous pression réduite. Le résidu a été purifié par flash chromatographie en utilisant un gradient d'éluant (hexane-acétate d'éthyle 1 : 3 jusqu'à 1:1) pour conduire à une huile incolore correspondant au composé attendu **97c** (0,9 g, 40%). RMN $^1$H (600 MHz, $CDCl_3$) δ: 8,63 (dd, *J* = 6,5; 1,5 Hz, 1H), 8,24 (dd, *J* = 11,5; 8,5 Hz, 2H), 8,02 (dd, *J* = 8,5; 3 Hz, 2H), 7,79 (s, 1H), 4,42 (q, *J* = 8 Hz, 2H), 4,27-4,09 (m, 2H), 2,74 (s, 3H), 1,40 (t, *J* = 7

Hz, 3H); RMN $^{13}C$ (151 MHz, $CDCl_3$) δ: 163,5 (d, $J$ = 21,5 Hz), 156,5 (d, $J$ = 171 Hz), 154,2 (d, $J$ = 13,5 Hz), 138,9 (d, $J$ = 3,5 Hz), 136,5 (d, $J$ = 138 Hz), 133,7 (d, $J$ = 10 Hz), 127,7 (d, $J$ = 13,5 Hz), 122.1 (q, $J$ = 281 Hz), 118,2; 118,0 (d, $J$ = 9 Hz), 64,8 (q, $J$ = 38,5 Hz), 62,9 (d, $J$ = 6,5 Hz), 24,8; 16,5 (d, $J$ = 6 Hz); RMN $^{19}F$ (564 MHz, $CDCl_3$) -74,2 (t, $J$ = 7 Hz); RMN $^{31}P$ (242 MHz, $CDCl_3$) + 20,7; HRMS (ESI+) calculée pour $C_{16}H_{17}N_2O_7SF_3P$ $[M+H]^+$, $m/z$ 469,0446, trouvée: 469,0444. $R_f$ = 0,7 (silice; hexane - acétate d'éthyle 1:2).

**Composés 98a-b**

Ces composés ont été préparés selon la même procédure que celle utilisée pour le composé **98c** en utilisant les précurseurs correspondants.

**Composé 98c**

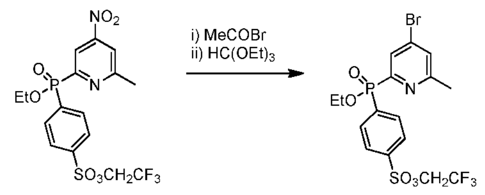


97c 98c

A une solution de bromure d'acétyle (3 mL, 39 mmol) a été additionné le composé 97c (600 mg, 1,3 mmol). Le mélange a été chauffé à 70°C sous atmosphère inerte pendant 16 h. La solution de couleur marron a été refroidie à température ambiante puis versée prudemment sous agitation dans du méthanol (30 mL) refroidi préalablement à 0°C. Le solvant a été éliminé sous pression réduite pour conduire à un solide légèrement marron identifié comme le dérivé d'acide bromo-phosphinique. Ce composé contenant quelques impuretés non identifiées a été utilisé dans la suite de la synthèse sans purification complémentaire et en supposant que la conversion en dérivé d'acide bromo-phosphinique était quantitative. RMN $^1H$ (600 MHz, $CDCl_3$) δ: 8,42 (d, $J$ = 7 Hz, 1H), 8,31 (s, 1H), 8,24 (dd, $J$ = 12,5, 8,5 Hz, 2H), 8,09 (dd, $J$ = 8,5, 2 Hz, 2H), 4,66 (q, $J$ = 8 Hz, 2H), 2,81 (s, 3H); RMN $^{13}C$ (151 MHz, $CDCl_3$) δ: 158,0 (d, $J$ = 8 Hz), 151,0 (d, $J$ = 134 Hz), 143,5 (d, $J$ = 10,5 Hz), 138,9, 138,8 (d, $J$ = 155 Hz), 133,4, 133,3 (d, $J$ = 11 Hz), 131,5 (d, $J$ = 12 Hz), 128,0 (d, $J$ = 14 Hz), 122,2 (q, $J$ = 277 Hz), 65,1 (q, $J$ = 37,5 Hz), 19,0; RMN $^{19}F$ (564 MHz, $CDCl_3$) -76,0 (t, $J$ = 7 Hz); RMN $^{31}P$ (242 MHz, $CDCl_3$) + 8,1. HRMS (ESI+) calculée pour $C_{14}H_{13}NO_5F_3PSBr$ $[M+H]^+$, $m/z$ 473,9388, trouvée: 473,9389. A l'acide bromo-phosphinique obtenu précédemment (600 mg, 1,3 mmol) a été additionné du triéthylorthoformiate (25 mL) et la solution a été chauffée à 140 °C pendant 16 h sous atmosphère inerte. Après cette période, le mélange réactionnel a été refroidi à température ambiante et le solvant a été éliminé sous pression réduite et le résidu a été purifié par chromatographie flash sur colonne de silice en utilisant un mélange d'éluants dichlorométhaneméthanol (9 : 1) pour conduire au composé **98c** attendu comme une huile jaunâtre (530 mg, 81%). RMN $^1H$ (600 MHz, $CDCl_3$) δ: 8,22 (dd, $J$ = 11,5, 8,5 Hz, 2H), 8,11 (dd, $J$ = 6,5; 1,5 Hz, 1H), 8,00 (dd, J = 8,5, 3 Hz, 2H), 7,45 (s, 1H), 4,40 (q, $J$ = 8 Hz, 2H), 4,23 - 4,08 (m, 2H), 2,54 (s, 3H), 1,37 (t, $J$= 7,0 Hz, 3H); RMN $^{13}C$ (151 MHz, $CDCl_3$) δ: 161,3 (d, $J$ = 22 Hz), 153,8 (d, $J$ = 169 Hz), 138,5 (d, $J$ = 3 Hz), 137,3 (d, $J$ = 136 Hz), 133,7, 133,6 (d, $J$ = 10 Hz), 129,2 (d, $J$ = 3 Hz), 129,0 (d, $J$ = 24 Hz), 127,5 (d, $J$ = 13 Hz), 121,7 (q, $J$ = 278 Hz), 64,8 (q, $J$ = 38,5 Hz), 62,6 (d, $J$ = 6,5 Hz), 24,3, 16,4 (d, $J$ = 6,5 Hz); RMN $^{19}F$ (564 MHz, $CDCl_3$) δ: -74.2 (t, $J$ = 7 Hz); RMN $^{31}P$ (242 MHz, $CDCl_3$) δ: + 21,5; HRMS (ESI+) calculée pour $C_{16}H_{17}NO_5F_3PSBr$ $[M+H]^+$, $m/z$ 501,9701, trouvée: 501,9690. $R_f$ = 0,50 (silice; dichlorométhane - méthanol 96:4).

**Composés 99a-b**

Ces composés ont été préparés selon la même procédure que celle utilisée pour le composé **99c** en utilisant les précurseurs correspondants.

## Composé 99c

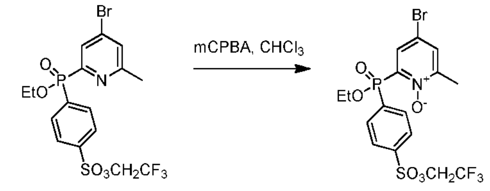


98c 99c

A une solution du composé **98c** (530 mg, 1 mmol) dans du chloroforme (15 mL) 365 mg, 2 mmol) a été additionné de l'acide 3-chloroperbenzoïque (365 mg, 2 mmol) et le mélange a été chauffé à 65°C pendant 16 h. La solution a ensuite été refroidie à température ambiante et le solvant a été éliminé sous pression réduite. Le résidu a été dissous dans du dichlorométhane (15 mL) et lavé avec une solution aqueuse de bicarbonate de sodium (0,5 M, 10 mL). La phase aqueuse a été extraite par du dichlorométhane (3 x 10 mL) et les phases organiques ont été réunies, séchées sur sulfate de magnésium, filtrées puis concentrées sous pression réduite pour conduire à une huile jaune identifiée comme le composé **99c** (475 mg, 91%). RMN $^1H$ (600 MHz, $CDCl_3$) $\delta$: 8,25 (dd, $^3J$ = 13, 8,5 Hz, 2H), 8,12 (dd, $^3J$ = 8 Hz, $^4J$ = 3 Hz, 1H), 7,99 (dd, $^3J$ = 8,5 Hz, $^4J$ = 3 Hz, 2H), 7,55 (d, $^5J$ = 2,5 Hz, 1H), 4,40 (q, $^3J$ = 8 Hz, 2H), 4,26 -4,16 (m, 2H), 2,37 (s, 3H), 1,40 (t, $^3J$ = 7,0 Hz, 3H); RMN $^{13}C$ (151 MHz, $CDCl_3$) $\delta$: 150,9, 142,8 (d, $J$ = 153 Hz), 138,7, 136,3 (d, $J$ = 151 Hz), 134,2 (d, $J$ = 11,5 Hz), 133,2 (d, $J$ = 11 Hz), 132,7, 127,4 (d, $J$ = 14,5 Hz), 124,1, 121,6 (q, $J$ = 274 Hz), 64,8 (q, $J$ = 38,5 Hz), 63,0 (d, $J$ = 6 Hz), 17,2, 16,5 (d, $J$ = 6 Hz); RMN $^{19}F$ (564 MHz, $CDCl_3$) $\delta$: -74.2 (t, $^3J$ = 8 Hz); RMN $^{31}P$ (242 MHz, $CDCl_3$) $\delta$: + 17,2; HRMS (ESI+) calculée pour $C_{16}H_{17}NO_6F_3PSBr$ $[M+H]^+$, *m/z* 517,9650; trouvée: 517,9650 $R_f$ = 0,49 (silice; dichlorométhane - méthanol 96:4).

### Composés 100a-b

Ces composés ont été préparés selon la même procédure que celle utilisée pour le composé **100c** en utilisant les précurseurs correspondants.

## Composé 100c

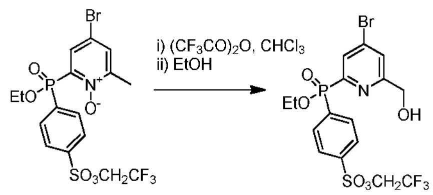


99c 100c

A une solution de composé **99c** (475 mg, 0,91 mmol) dans du chloroforme anhydre (20 mL) a été additionné de l'anhydride trifluoroacétique (2,5 mL). Le mélange réactionnel a été chauffé à 60°C pendant 3 h sous argon puis refroidi à température ambiante. Le solvant a été éliminé sous pression réduite pour conduire à une huile qui a été dissoute dans un mélange éthanol - eau (1/1, v/v), (30 mL). La solution a été agitée à température ambiante pendant 1 h puis le volume de la solution a été réduit à 15 mL sous pression réduite. La solution aqueuse résultante a été extraite avec du dichlorométhane (3 x 30 mL). Les phases organiques ont été réunies, séchées sur sulfate de magnésium, filtrées et concentrées sous pression réduite pour conduire à une huile incolore identifiée comme le composé **100c** (430 mg, 91%). RMN $^1H$ (600 MHz, $CDCl_3$) $\delta$: 8,19-8,13 (m, 3H), 8,00 (dd, $^3J$ = 8,5 Hz, $^4J$ = 2,5 Hz, 2H), 7,72 (s, 1H), 6,83 (sl, 1H), 4,76 (s, 2H), 4,41 (q, $^3J$ $R_f$ = 8 Hz, 2H), 4,29-4,04 (m, 2H), 1,37 (t, $^3J$ = 7 Hz, 3H); RMN $^{13}C$ (151 MHz, $CDCl_3$) $\delta$: 163,1 (d, $J$ = 20,5 Hz), 152,8 (d, $J$ = 169 Hz), 139,0 (d, $J$ = 3 Hz), 136,2 (d, $J$ = 138,5 Hz), 134,6 (d, $J$ = 15 Hz), 133,5 (d, $J$ = 10,5 Hz), 130,3 (d, = 23,5 Hz), 127,8 (d, $J$ = 13,5 Hz), 126,8 (d, $J$ = 3 Hz), 122,8 (q, $J$ = 274 Hz), 64,9 (q, $J$ = 38,5 Hz), 64,0, 63,2 (d, $J$ = 6,5 Hz), 16,4 (d, $J$ = 6 Hz); RMN $^{19}F$ (564 MHz, $CDCl_3$) $\delta$: -74,2 (t, $^3J$ = 8 Hz); RMN $^{31}P$ (242 MHz, $CDCl_3$) $\delta$: + 22,1; HRMS (ESI+) calculée pour $C_{16}H_{17}NO_6F_3PSBr$ $[M+H]^+$, *m/z* 517,9650, trouvée: 517,9647 $R_f$ = 0,56 (silice; dichlorométhane - méthanol 96:4).

### Composés 101a-b

Ces composés ont été préparés selon la même procédure que celle utilisée pour le composé **101c** en utilisant les précurseurs correspondants.

## Composé 101c

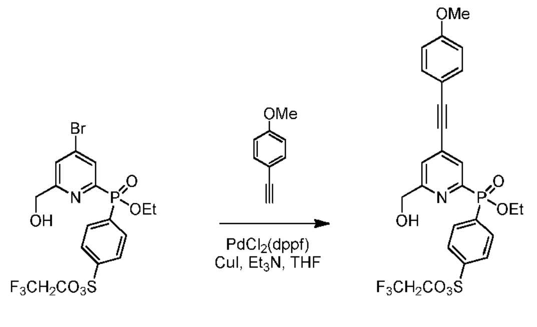


100c 101c

A une solution de composé **100c** (200 mg, 0,39 mmol) dissous dans du tétrahydrofurane anhydre (2,5 mL) préalablement dégazée sous un courant d'argon, ont été additionnés du 4-ethynylanisole (76 mg, 0,56 mmol) et de la triéthylamine (1 mL). Le mélange a été à nouveau dégazé puis à ce mélange ont été additionnés du chlorure de bis(diphénylphosphino)ferrocène]palladium(II) (45 mg, 0,04 mmol) et de l'iodure de cuivre (7 mg, 0,04 mmol). Le mélange a été chauffé à 65°C sous argon pendant 16 h puis a été refroidi à température ambiante et concentré sous pression réduite. Le produit brut a été purifié par chromatographie sur colonne de silice en utilisant un gradient de solvant (dichlorométhane - méthanol 100/0 jusqu'à 98/2 par incrément de 0,2 %) pour donner une huile orange identifiée comme le composé **101c** (153 mg, 69%) RMN $^1H$ (600 MHz, $CDCl_3$) $\delta$: 8,19 (d, $^3J$ = 8,5 Hz, 2H), 8,10 (s, 1H), 8,01 (d, $^3J$ = 7 Hz, 2H), 7,47 (m, 3H), 6,90 (d, $^3J$ = 8,5 Hz, 2H), 4,78 (s, 2H), 4,40 (q, $^3J$ = 7,5 Hz, 2H), 4,17 (m, 2H), 3,84 (s, 3H), 3,52 (si, 1H), 1,39 (t, $^3J$ = 6,5 Hz, 3H); RMN $^{13}C$ (151 MHz, $CDCl_3$) $\delta$: 161,2, 160,7, 151,0 (d, *J* =183 Hz), 138,6, 137,4 (d, *J* = 136 Hz), 133,7, 133,5, 133,4 (d, *J* = 9 Hz), 128,9, 127,7 (d, *J* = 12,5 Hz), 124,4, 121,7 (q, *J* = 327 Hz), 114,3, 113,5, 96,7, 85,0, 64,9 (q, *J* = 38 Hz), 64,1, 62,6, 55,4, 16,5; RMN $^{19}F$ (564 MHz, $CDCl_3$) $\delta$: - 74,2 (t, *J* = 8 Hz); RMN $^{31}P$ (242 MHz, $CDCl_3$) $\delta$: + 22,5; HRMS (ESI+) calculée pour $C_{25}H_{24}NO_7F_3PS$ $[M+H]^+$, *m/z* 570,0963; trouvée: 570,0973. $R_f$ $R_f$ = 0,61 (silice; dichlorométhane - méthanol 95:5).

## Composés 102a-b

Ces composés ont été préparés selon la même procédure que celle utilisée pour le composé **102c** en utilisant les précurseurs correspondants.

## Composé 102c

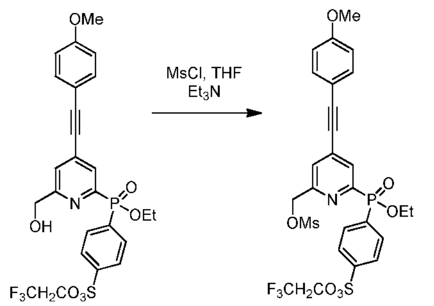


101c 102c

A une solution de composé **101c** (93 mg, 0,16 mmol) dans du tétrahydrofurane anhydre (4 mL) a été additionné de la triéthylamine (0,7 mL, 0,5 mmol). Le mélange a été refroidi à 5°C puis à cette solution a été ajouté du chlorure de méthane sulfonyle (19 µL, 0,24 mmol) puis la solution a été agitée pendant 30 min à la même température. Le solvant a été éliminé sous pression réduite, puis le résidu a été dissous dans du dichlorométhane (15 mL) et la solution a été lavée avec une solution aqueuse de saumure saturée (10 mL). La phase aqueuse a été extraite à nouveau avec du dichlorométhane (3 x 10 mL) puis les phases organiques ont été réunies, séchées sur sulfate de magnésium, filtrées et concentrées sous pression réduite pour conduire à une huile incolore correspondant au composé **102c** (95 mg, 92%). RMN $^1H$ (600 MHz, $CDCl_3$) $\delta$: 8,22 (dd, $^3J$ = 11,5, 8,5 Hz, 2H), 8,17 (d, $^3J$ = 6,5 Hz, 1H), 8,02 (dd, $^3J$ = 8,5 Hz, $^4J$ = 2,5 Hz, 2H), 7,62 (s, 1H), 7,50 (d, $^3J$ = 9 Hz, 2H), 6,91 (d, $^3J$ = 9 Hz, 2H), 5,32 (m, 2H), 4,42 (q, $^3J$ = 8 Hz, 2H), 4,18 (m, 2H), 3,85 (s, 3H), 3,08 (s, 3H), 1,40 (t, $^3J$ = 7 Hz, 3H); RMN $^{19}F$ (564 MHz, $CDCl_3$) $\delta$: -74,1 (t, *J* = 8 Hz); RMN $^{31}P$ (242

MHz, $CDCl_3$) δ: + 21,9; HRMS (ESI+) calculée pour $C_{26}H_{26}NO_9F_3PS_2$ $[M+H]^+$, *m/z* 648,0739, trouvée: 648,0728. $R_f$ = 0,75 (silice; dichlorométhane - méthanol 95:5).

**Composés 103a-b**

Ces composés ont été préparés selon la même procédure que celle utilisée pour le composé **103c** en utilisant les précurseurs correspondants.

## Composé 103c

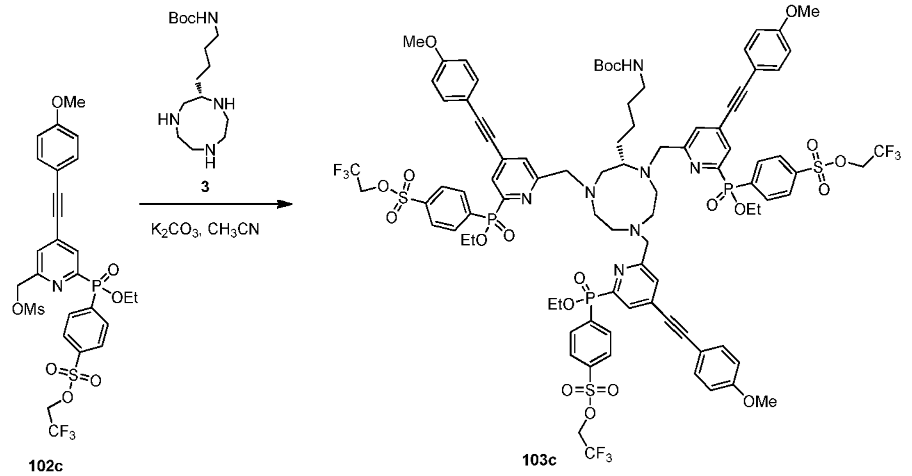


102c

103c

A une solution de macrocycle 3 (15 mg, 0,05 mmol) dans de l'acétonitrile anhydre (2 mL) ont été ajoutés du composé **102c** (95 mg, 0,15 mmol) et du carbonate de potassium (21 mg, 0,15 mmol). Le mélange a été chauffé à 60°C pendant 16 h puis a été refroidi à température ambiante. Les sels en suspension ont été décantés puis séparés de la solution qui a ensuite été concentrée sous pression réduite. Le résidu a été purifié par HPLC préparative pour conduire à une huile jaune identifiée comme le composé **103c** (32 mg, 33%). RMN $^1H$ (600 MHz, $CDCl_3$) δ: 8,18 (m, 6H), 8,04 (m, 3H), 7,96 (m, 6H), 7,48 (m, 3H), 7,44 (m, 6H), 6,88 (m, 6H), 5,08 (si, 1H), 4,40 (q, *J* = 7,5 Hz, 6H), 4,14 (m, 6H), 3,83 (m, 15H), 3,00-2,56 (m, 13H), 1,38 (s, 9H), 1,45-1,30 (m, 6H), 1,35 (m, 9H); RMN $^{19}F$ (564 MHz, $CDCl_3$) δ: -74,2 (t, *J* = 8 Hz); RMN $^{31}P$ (242 MHz, $CDCl_3$) δ: + 22,6 ; HRMS (ESI+) calculée pour $C_{90}H_{97}N_7O_{20}F_9P_3S_3$ $[M+2H]^{2+}$, *m/z* 977,7510, trouvée: 977,7515.

**Composés 104a-b**

Ces composés ont été préparés selon la même procédure que celle utilisée pour le composé **104c** en utilisant les précurseurs correspondants.

## Composé 104c

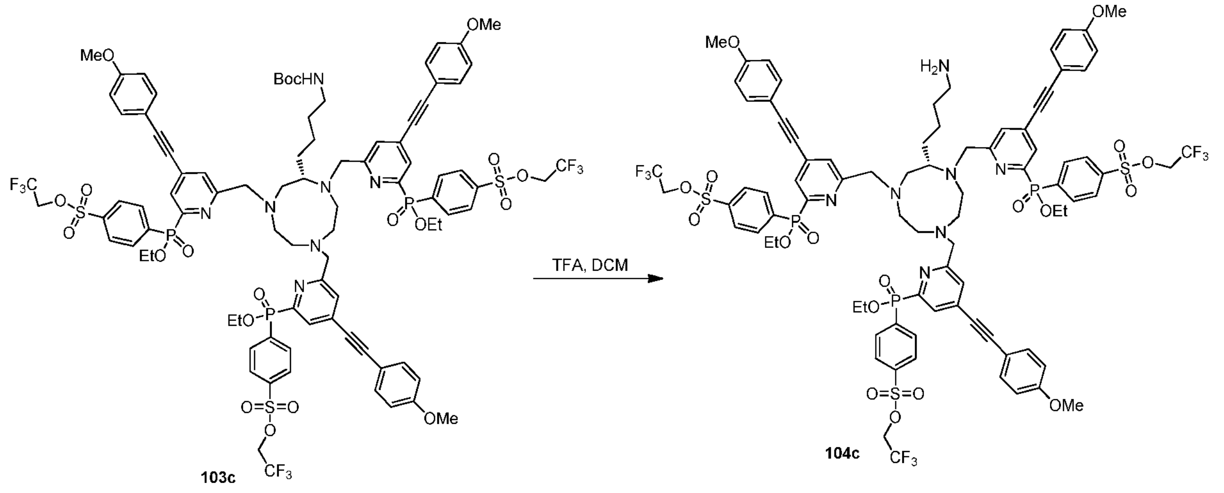


103c 104c

A une solution de composé **103c** (30 mg, 15 μmol) dans du dichlorométhane (1,8 mL) préalablement dégazée par un courant d'argon pendant 10 min a été additionné de l'acide trifluoroacétique (0,2 mL). La solution a été agitée à température ambiante pendant 20 min puis le solvant a été éliminé sous pression réduite pour conduire à huile jaune identifiée comme le composé **104c** (16 mg, 60%). RMN $^{1}$H (600 MHz, $CD_3OD$) δ: 8,11 (m, 6H), 8,04 (m, 3H), 8,01 (m, 6H), 7,61 (m, 3H), 7,57 (m, 6H), 6,93 (m, 6H), 4,65 (m, 6H), 4,17 (m, 6H), 3,96 (m, 15H), 3,82-3,60 (m, 13H), 1,61-1,47 (m, 6H), 1,46 (m, 9H); RMN $^{19}$F (564 MHz, $CD_3OD$) δ: -76,0; RMN $^{31}$P (242 MHz, $CD_3OD$) δ:+ 23,6 ; HRMS (ESI+) calculée pour $C_{85}H_{88}N_7O_{18}F_9P_3S_3$ $[M+H]^+$, *m/z* 1854,4420, trouvée: 1854,4370.

### Composés 105a-b

Ces composés ont été préparés selon la même procédure que celle utilisée pour le composé **105c** en utilisant les précurseurs correspondants.

## Composé 105c

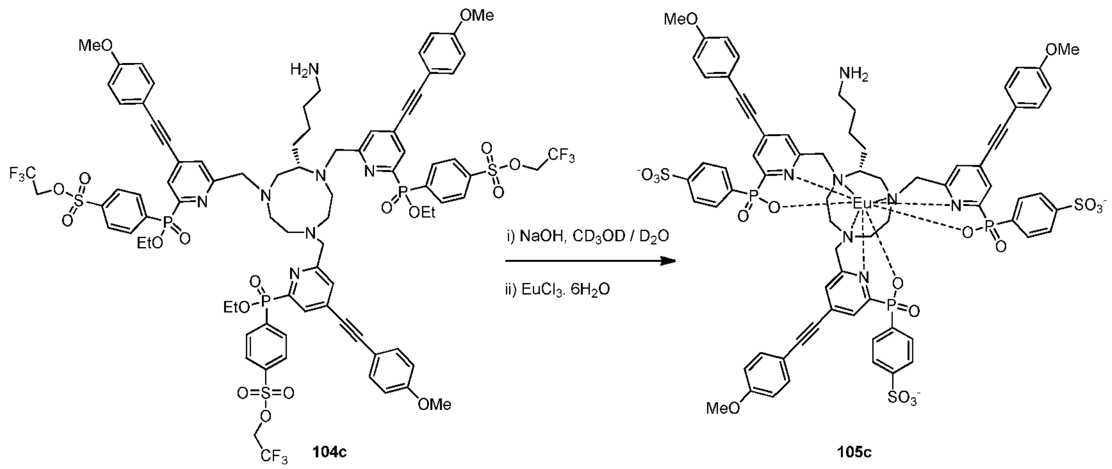


104c 105c

A une solution de composé **104c** (5 mg, 2,7 μmol) dans du méthanol deutéré (1 mL) a été ajoutée une solution aqueuse deutérée d'hydroxyde de sodium (0,1 M, 0,5 mL). Le mélange a été chauffé à 60°C pendant 3 h puis refroidi à température ambiante. Le pH de la solution a été ajusté à 7 par addition d'acide chlorhydrique. A ce mélange a été additionné du chlorure d'europium hexahydrate (1 mg, 2,7 μmol) puis la solution a été chauffée à 65°C pendant 18 h sous atmosphère inerte. Après cette période la réaction a été refroidie à température ambiante et le solvant a été éliminé sous pression réduite. Le produit brut a été purifié par HPLC préparative pour conduire au composé **105c** (2,8 mg, 63%); HRMS (ESI+) calculée pour $C_{73}H_{68}N_7O_{18}P_3S_3Eu$ $[M+H]^+$, *m/z* 1671,2380, trouvée: 1671,2370.

## Composés 107a-b

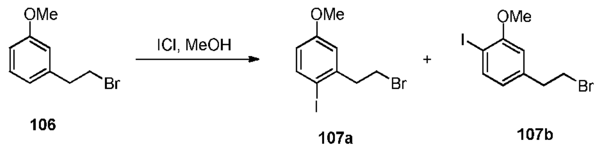


106 107a 107b

A une solution de bromure de 2-bromo-5-méthoxyphénéthyle **106** (1 g, 4,6 mmol) dans du méthanol (20 mL), a été ajouté du chlorure d'iode (1,5 g, 9,2 mmol). Le mélange réactionnel a été agité à température ambiante. Après 4 h, la réaction était totale. Le chlorure d'iode n'ayant pas réagi a été éliminé par une réaction d'oxydo-réduction grâce à l'ajout d'une solution de métabisulfite de sodium à 10% massique (40 mL). Les solvants ont été évaporés sous pression réduite. Le résidu a été solubilisé dans du dichlorométhane (30 mL). Cette solution a été lavée par une solution de métabisulfite de sodium à 10% massique (2 x 50 mL). La phase organique a été séchée sur sulfate de magnésium, filtrée et évaporée sous pression réduite. Le produit brut a été purifié par chromatographie flash sur colonne de silice (cyclohexane / acétate d'éthyle de 0 à 50% par incrément de 5%) pour obtenir une huile jaunâtre correspondant au mélange de composé **107a** et **107b** en proportion 8/2 (1,22 g, 78%). **107a** : RMN $^1$H (400 MHz, DMSO-$d_6$) $\delta$: 7,70 (d, *J* = 8,7 Hz, 1H), 7,02 (d, *J* = 3,0 Hz, 1H), 6,66 (dd, *J* = 8,7 ; 3,0 Hz, 1H), 3,74 (s, 3H), 3,65 (t, *J* = 7,6 Hz, 2H), 3,17 (t, *J* = 7,6 Hz, 2H) ; RMN $^{13}$C (100 MHz, DMSO-$d_6$) $\delta$: 160,08; 142,67; 140,08; 116,87; 115,50; 89,58; 55,76; 43,43; 32,77. HRMS (ESI+) calculée pour $C_9H_{14}BrINO$ $[M+NH_4]^+$, m/z : 357,9303 trouvée : 357,9300. $R_f$ = 0,67 (silice ; cyclohexane - acétate d'éthyle 1:1).

**107b** : RMN $^1$H (400 MHz, DMSO-$d_6$) $\delta$: 7,67 (d, *J* = 7,9 Hz, 1H), 6,96 (d, *J* = 3,0 Hz, 1H), 6,66 (dd, *J* = 7,9 ; 3,0 Hz, 1H), 3,82 (s, 3H), 3,74 (t, *J* = 7,6 Hz, 2H), 3,10 (t, *J* = 7,6 Hz, 2H).

## Composés 108a-b

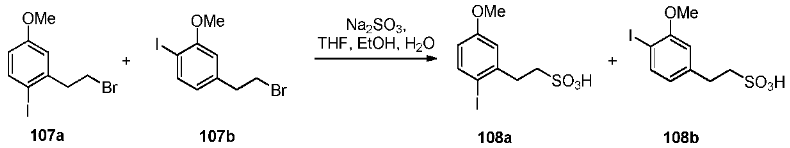


107a 107b 108a 108b

A une solution de bromure de iodo-5-méthoxyphénéthyle **107a-b** (1,4 g, 4,1 mmol) dans un mélange tétrahydrofurane/éthanol/eau en proportion 1/2/2 (150 mL), a été ajouté du sulfite de sodium (0,93 g, 7,4mmol). Le mélange réactionnel a été chauffé à reflux sous agitation magnétique. Après 18 h, la réaction était totale. Le mélange réactionnel a été refroidi à température ambiante et les solvants ont été évaporés sous pression réduite. Le brut réactionnel a été solubilisé dans du méthanol (100 mL), porté à ébullition, et filtré à chaud. Le filtrat a été refroidi à température ambiante, évaporé sous pression réduite et recristallisé dans le méthanol (50 mL) pour conduire à un solide blanc correspondant au mélange des composés **108a** et **108b** en proportion 8/2 (1,06 g, 71%). PtF = 282 °C (déc). **108a** : RMN $^1$H (400 MHz, DMSO-$d_6$) $\delta$ : 7,65 (d, *J* = 8,7 Hz, 1H), 6,86 (d, *J* = 3,0 Hz), 6,58 (dd, *J* = 8,7 ; 3,0 Hz), 3,73 (s, 3H), 2,94-2,89 (m, 2H), 2,67-2,62 (m, 2H); RMN $^{13}$C (400 MHz, DMSO-$d_6$) $\delta$ : 159,67; 144,39; 139,47; 115,24; 114,36; 88,86; 55,17; 51,26; 36,57. HRMS (ESI+) calculée pour $C_9H_{15}INO_4S$ $[M+NH_4]^+$, *m/z*: 359,9766 trouvée : 359,9763. $R_f$ = 0,52 (silice ; dichlorométhane - méthanol - triéthylamine 85:10:5).

**108b** : RMN $^1$H (400 MHz, DMSO-$d_6$) $\delta$: 7,60 (d, *J* = 8,7 Hz, 1H), 6,86 (d, *J* = 3,0 Hz), 6,60 (dd, *J* = 8,7 ; 3,0 Hz), 3,81 (s, 3H), 2,87-2,83 (m, 2H), 2,71-2,68 (m, 2H); RMN $^{13}$C (400 MHz, DMSO-$d_6$) $\delta$ : 157,57; 143,33; 138,57; 123,53; 111,86; 82,58; 56,17; 52,61; 31,35.

## Composés 109a-b

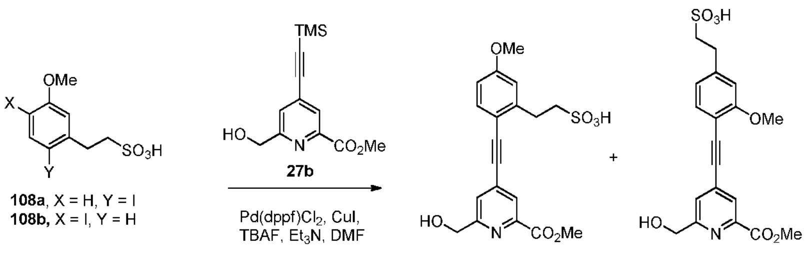


**108a**, X = H, Y = I
**108b,** X = I, Y = H
27b 109a 109b

A une solution de dérivé sulfoné **108a-b** (1 mmol) dans du diméthylformamide anhydre (25 mL), ont été additionnés sous atmosphère inerte du dérivé acétylénique **27b** (263 mg, 1 mmol) et de la triéthylamine (12,5 mL). Le mélange a été dégazé pendant 15 min à l'aide d'un courant d'argon. Puis, du [1,1-Bis(diphénylphosphino)ferrocène]dichloropalladium(II) (73,2 mg, 0,1 mmol) et de l'iodure de cuivre (38 mg, 0,2 mmol) ont été ajoutés. La réaction a été chauffée à 75°C, sous agitation magnétique, dans un réacteur clos et à l'abri de la lumière. Après 4 h, la réaction était totale. Le mélange réactionnel a été refroidi à température ambiante et le solvant a été évaporé sous pression réduite. Le résidu a été solubilisé dans du méthanol (30 mL) et à cette solution a été ajouté du carbonate de calcium (200 mg, 2 mmol). Le mélange a été agité à température ambiante pendant 15 min. La solution a été éluée avec du méthanol (3 x 50 mL) sur une cartouche de résine échangeuse de cation Porapak™Rxn CX. Les fractions ont été réunies, et le solvant a été évaporé sous pression réduite. Le résidu a été purifié par HPLC préparative permettant de séparer les deux isomères **109a** et **109b** sous forme de solides blancs PtF $R_f$ = 218-221 °C. **109a** (77%) : RMN $^1$H (400 MHz, DMSO-$d_6$) δ: 8,01 (s, 1H), 7,83 (s, 1H), 7,55 (d, *J* = 8,5 Hz), 6,95 (d, *J* = 2,2 Hz), 6,86 (dd, *J* = 8,5 ; 2,2 Hz), 4,61 (s, 2H), 3,89 (s, 3H), 3,80 (s, 3H), 3,16-3,11 (m, 2H), 2,79-2,75 (m, 2H); RMN $^{13}$C (100 MHz, DMSO-$d_6$) δ: 164,67; 162,90; 160,51; 146,86; 145,53; 134,21; 132,76; 124,91; 124,20; 114,68; 112,47; 112,33; 93,60; 89,06; 63,70; 55,30; 51,93; 30,53. HRMS (ESI+) calculée pour $C_{19}H_{20}NO_7S$ [M+H]$^+$, *m/z* : 406,0960 trouvée : 406,0954. $R_f$ = 0,45 (silice ; dichlorométhane - méthanol - triéthylamine 85:10:5).

**109b** (11%): RMN $^1$H (400 MHz, DMSO-$d_6$) δ: 7,90 (s, 1H), 7,72 (s, 1H), 7,47 (d, *J* = 8,5 Hz), 7,02 (d, *J* = 2,2 Hz), 6,87 (dd, *J* = 8,5 ; 2,2 Hz), 4,64 (s, 2H), 3,90 (s, 3H), 3,89 (s, 3H), 2,96-2,91 (m, 2H), 2,75-2,71 (m, 2H).

## Composé 110a

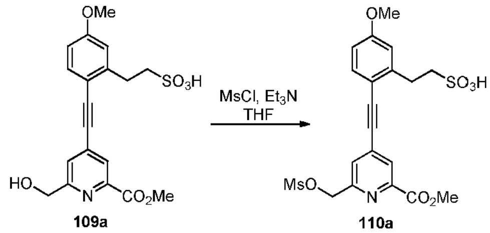


A une solution de dérivé alcool **109a** (90 mg, 0,22 mmol) dans du tétrahydrofurane anhydre (10 mL), ont été ajoutés, sous atmosphère inerte et à 5°C, de la triéthylamine (102 mg, 140 μL, 1 mmol) et du chlorure de mésyle (58,6 mg, 40 μL, 0,51 mmol). La réaction a été agitée à température ambiante. Après 10 min, la réaction était totale. Le solvant a été évaporé sous pression réduite. Le brut réactionnel a été purifié par HPLC préparative pour conduire à un solide blanc correspondant au composé **110a** (88 mg, 84%). PtF $R_f$ = 143-145 °C. RMN $^1$H (400 MHz, DMSO-$d_6$) δ : 8,12 (s, 1H), 8,02 (s, 1H), 7,55 (d, *J* = 8,5 Hz), 6,96 (d, *J* = 2,5 Hz), 6,86 (dd, *J* = 8,5 ; 2,5 Hz), 5,36 (s, 2H), 3,91 (s, 3H), 3,80 (s, 3H), 3,40 (s, 3H), 3,16-3,06 (m, 2H), 2,78-2,74 (m, 2H); RMN $^{13}$C (100 MHz, DMSO-$d_6$) δ : 164,89; 161,15; 155,25; 148,17; 146,60; 134,64; 133,85; 127,63; 125,96; 115,32; 112,92; 112,65; 94,98; 89,23; 71,09; 55,82; 53,14; 52,62; 37,77; 31,41. HRMS (ESI+) calculée pour $C_{20}H_{22}NO_9S_2$ [M+H]$^+$, *m/z* :484,0736 trouvée : 484,0733. $R_f$ = 0,26 (silice ; dichlorométhane - méthanol 9:1).

## Composé 111a

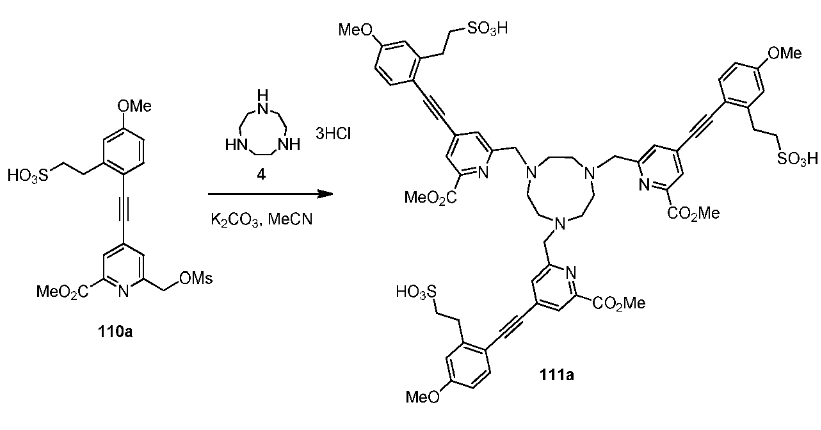


A une solution de TACN sous forme chlorhydrate 4 (8,6 mg, 36 μmol) dans de l'acétonitrile anhydre (4 mL), a

été ajouté, sous atmosphère inerte, du carbonate de potassium (30 mg, 216 μmol). Le mélange réactionnel a été chauffé à 65°C pendant 2 h, sous forte agitation magnétique, puis le dérivé mésylé **110a** (69,7 mg, 144 μmol) a été ajouté. Après 5 h, la réaction était totale. Le solvant a été évaporé sous pression réduite. Le produit brut a été purifié par HPLC préparative pour conduire à un solide blanc correspondant au composé **111a** (27 mg, 58%). HRMS (ESI+) calculée pour $C_{63}H_{68}N_6O_{18}S_3$ $[M+2H]^{2+}$, *m/z* : 646,1876 trouvée : 646,1870.

## Composé 112a

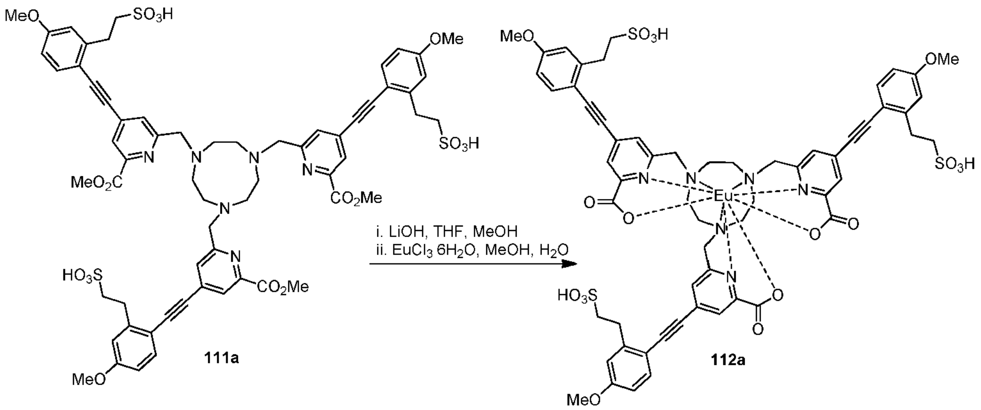


111a

112a

A une solution de ligand **111a** (5,8 mg, 4,5 μmol) dans du tétrahydrofurane (1 mL), a été ajouté une solution aqueuse d'hydroxyde de lithium à 1 M (0,5 mL). Le mélange a été placé sous agitation magnétique à température ambiante. Après 20 min, la réaction était totale. Le solvant a été évaporé sous pression réduite puis le brut réactionnel a été solubilisé dans un mélange de méthanol (1,5 mL) et d'eau pure (1 mL). Le pH du milieu a été neutralisé (pH 7) par ajout d'une solution d'acide chlorhydrique à 1 M et du chlorure d'europium hexahydrate (1,8 mg, 4,95 μmol) a été ajouté. La réaction a été placée sous agitation magnétique à température ambiante. Après 1 h, la réaction était totale. Le solvant a été évaporé sous pression réduite. Le produit brut a été solubilisé avec une solution tampon d'acétate de triéthylammonium pH 7 (4 mL), puis a été purifié par HPLC préparative. Le solvant des fractions collectées a été évaporé sous pression réduite. Le tampon d'acétate de triéthylammonium résiduel a été éliminé par des co-évaporations successives grâce à un mélange méthanol/toluène 1/4 (3 x 6 mL) pour conduire à un solide blanc correspondant au complexe **112a** (2,4 μmol, 53%). HRMS (ESI+) calculée pour $C_{60}H_{59}EuN_6O_{18}S_3$ $[M+2H]^{2+}$, m/z = 700,1130 trouvée 700,1122.

## Composé 113

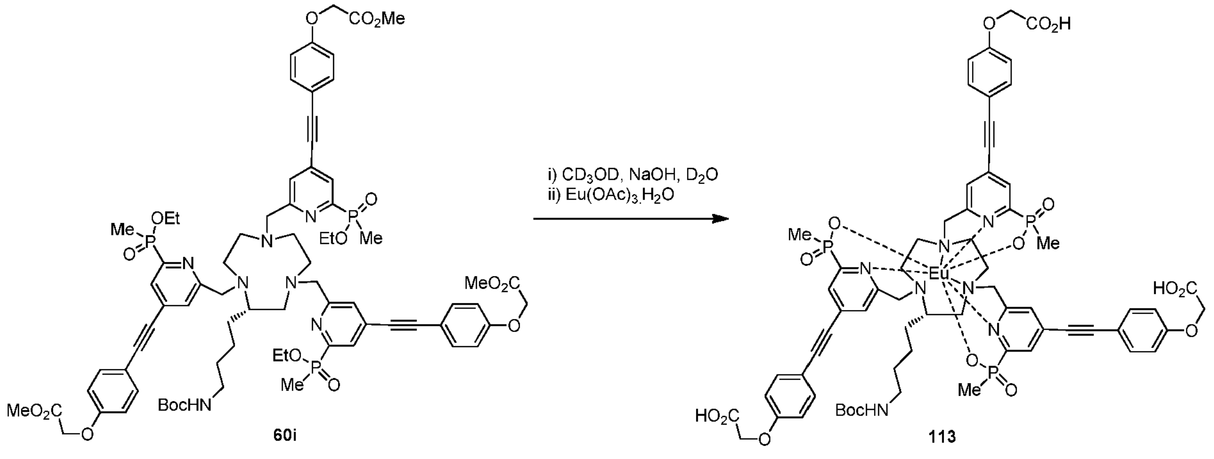


60i

113

A une solution de composé **60i** (10 mg, 6,8 μmol) dans du méthanol deutéré (2 mL) a été ajoutée une solution aqueuse deutérée d'hydroxyde de sodium (0,1 M, 1 mL). Le mélange a été chauffé à 60°C pendant 5 h puis refroidi à température ambiante. Le pH de la solution a été ajusté à 7 par addition d'acide chlorhydrique. A ce mélange a été additionné de l'acétate d'europium hydrate (1,8 mg, 5,7 μmol) puis la solution a été chauffée à 65°C pendant 18 h sous atmosphère inerte. Après cette période la réaction a été refroidie à température ambiante et le solvant a été éliminé sous pression réduite. Le produit brut a été purifié par HPLC préparative pour conduire au composé **113** comme un

solide blanc (6 mg, 59%); LRMS (ESI+) calculée pour $C_{68}H_{77}N_7O_{17}P_3Eu$ $[M+2H]^{2+}$, *m/z* 754,6900 trouvée: 754,6905.

## Composé 114

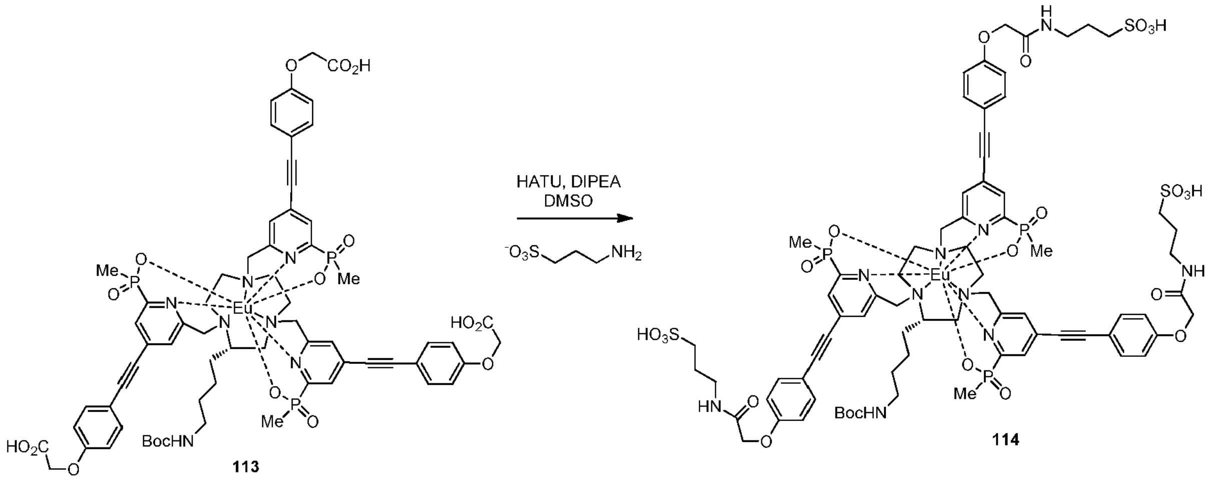


113

114

A une solution de composé **113** (7,1 mg, 4,8 µmol) dans du diméthylsulfoxyde anhydre (1 mL) ont été additionnés successivement de l'homotaurine (4,0 mg, 28,8 µmol), du (O-(7-azabenzotriazol-1-yl)-N,N,N',N'-tétraméthyluronium hexafluorophosphate) (5,4 mg, 14,4 µmol) et de la diisopropyléthylamine (9 µL, 48 µmol). Le mélange a été agité à température ambiante pendant 1 h sous atmosphère inerte. Le produit brut a été purifié par HPLC préparative pour conduire au composé **114** sous la forme d'un solide blanc (6,6 mg, 70 %); HRMS (ESI+) calculée pour $C_{75}H_{94}N_{10}O_{23}P_3S_3Eu$ $[M+2H]^{2+}$, *m/z* 922,2040 trouvée: 922,2045.

## Composé 115

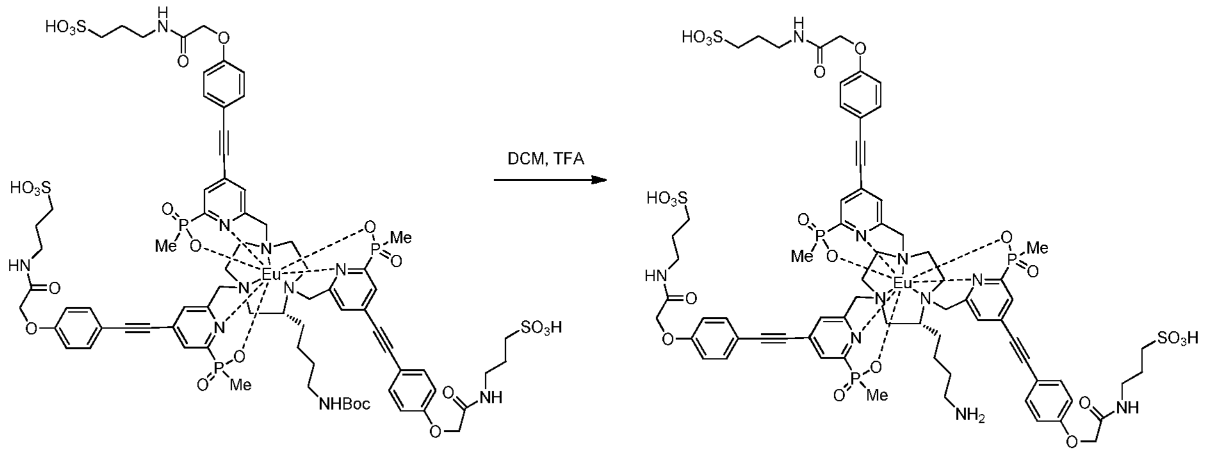


114

115

A une solution de composé **114** (6,6 mg, 3,6 µmol) dans du dichlorométhane (0,8 mL) refroidie à 4°C et préalablement dégazée sous un courant d'argon pendant 10 min, a été additionné de l'acide trifluoroacétique (0,2 mL). La solution est réchauffée à température ambiante et agitée à cette température pendant 20 min. Le solvant est éliminé sous pression réduite pour conduire à une huile jaune identifiée comme le composé **115** (6,4 mg, quantitatif). RMN $^1H$ (600 MHz, $CDCl_3$) δ: 7,91 (m, 3H), 7,51 (m, 9H), 6,92 (m, 6H), 4,67 (s, 6H), 4,16-3,97 (m, 12H), 3,81 (m, 9H), 3,12-2,90 (m, 13H), 1,89-1,75 (m, 9H), 1,45-1,32 (m, 6H), 1,29 (m, 9H); RMN $^{31}P$ (242 MHz, $CDCl_3$) δ: +41,2; HRMS (ESI+) calculée pour $C_{70}H_{86}EuN_{10}O_{21}P_3S_3$ $[M+2H]^{2+}$, *m/z* 872,1778, trouvée: 872,1781.

## Solubilité et propriétés spectroscopiques des composés selon l'invention

Le LogP est une mesure de la solubilité différentielle de composés chimiques dans deux solvants (coefficient de partage octanol/eau). Le LogP est égal au logarithme du rapport des concentrations de la substance étudiée dans

l'octanol et dans l'eau. LogP = Log($C_{oct}/_{eau}$). Cette valeur permet d'appréhender le caractère hydrophile ou hydrophobe (lipophile) d'une molécule. En effet, si le LogP est positif et très élevé, cela exprime le fait que la molécule considérée est bien plus soluble dans l'octanol que dans l'eau, ce qui reflète son caractère lipophile, et inversement. Un LogP nul ou négatif signifie que la molécule possède un caractère hydrophile. Dans le schéma 26, quelques exemples de structures de complexes sont représentés pour lesquelles le logP est fourni dans le tableau 2 et ceci à titre d'exemple pour illustrer la bonne solubilité dans l'eau des composés selon l'invention.

Schéma 26 : structures des complexes du tableau 2 *(les complexes 116-120 ne sont pas des complexes selon l'invention)*

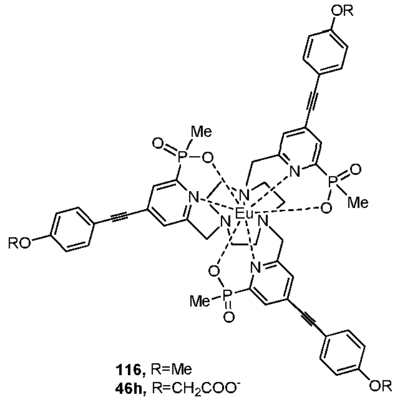

**116,** R=Me
**46h,** $R=CH_2COO^-$

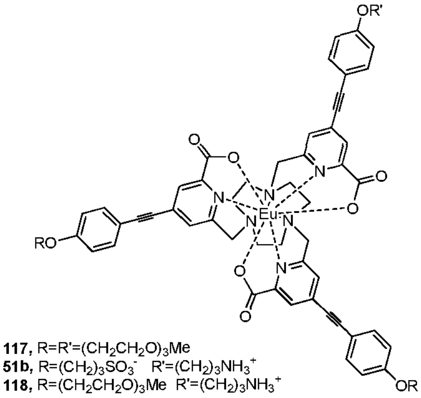

**117,** $R=R'=(CH_2CH_2O)_3Me$
**51b,** $R=(CH_2)_3SO_3^-$ $R'=(CH_2)_3NH_3^+$
**118,** $R=(CH_2CH_2O)_3Me$ $R'=(CH_2)_3NH_3^+$

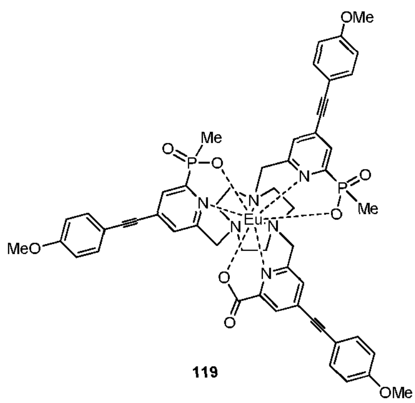

**119**

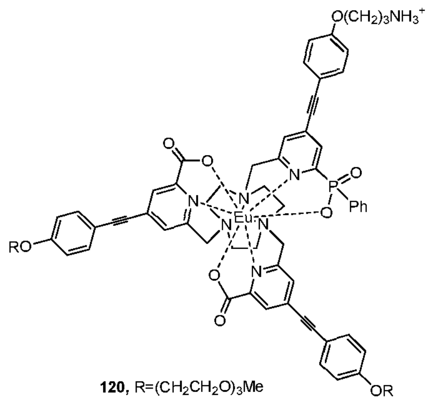

**120,** $R=(CH_2CH_2O)_3Me$

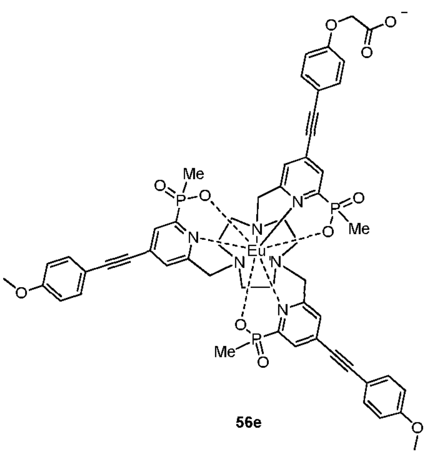

**56e**

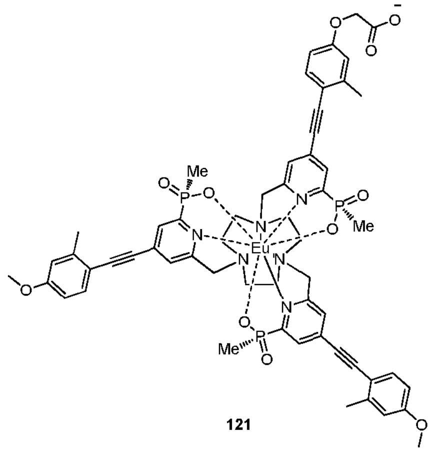

**121**

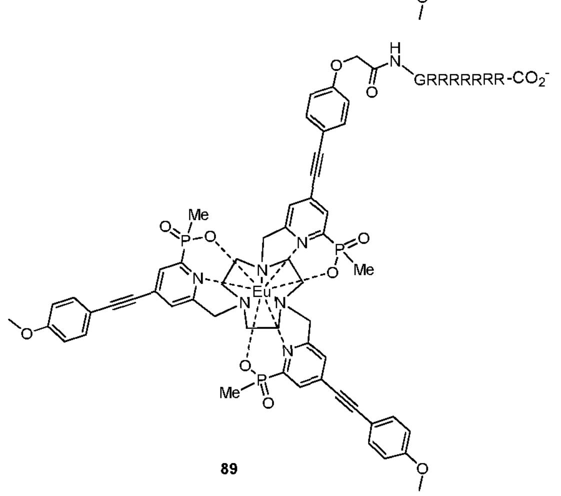

**89**

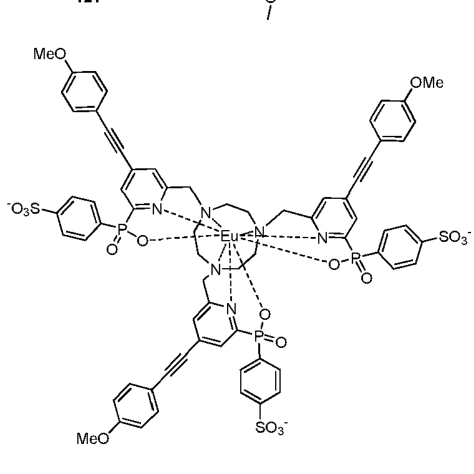

**105d**

**Tableau 2 : Valeurs des logP des complexes du schéma 26**

| Complexes | Fonctions complexantes | Fonction solubilisante | LogP (± 20 %) |
|---|---|---|---|
| **116** | 3($Me-PO_2^-$) | - | 1,4 |
| **117** | 3($CO_2^-$) | 3 $PEG_3$ | 1,1 |
| **118** | 3($CO_2^-$) | 2 $PEG_3$ | 0,7 |
| **119** | 1($CO_2^-$)2($Me-PO_2^-$) | - | 1,1 |
| **120** | 2($CO_2^-$)1($Ph-PO_2^-$) | 2 $PEG_3$ | 1,2 |
| **46h** | 3($Me-PO_2^-$) | 3 Carboxylates | -2,2 |
| **51b** | 3($CO_2^-$) | 2 Sulfonates | -1,1 |
| **56e** | 3($Me-PO_2^-$) | 1 Carboxylate | 0,3 |
| **89** | 3($Me-PO_2^-$) | Polyarginine | 0,3 |
| **105d** | 3($SO_3^-$-Ph-$PO_2^-$) | 3 Sulfonates | -0,7 |
| **121** | 3($Me-PO_2^-$) | 1 Carboxylate | 0,8 |

Les complexes correspondants aux structures **116-120** ont été décrits dans la demande internationale WO 2013/011236. Les valeurs de LogP des complexes selon l'invention sont soit très proches de 0 soit négatives ce qui reflète une parfaire solubilité dans les tampons aqueux, contrairement aux composés de la demande WO 2013/011236, dont certains sont également décrits dans Chem Commun 2013, 49, 1600-1602.

Méthode expérimentale pour détermination du LogP

Trois solutions équimolaires de complexe d'europium ont été préparées dans du méthanol. Le solvant a été éliminé sous pression réduite et le solide restant a été dissous et agité pendant 24 h dans un mélange eau/octanol (2 :1, 1 :1, 1 :2), (0,9 mL) donnant une concentration totale d'environ 2 μM. Après équilibration, un spectre d'émission de chaque phase a été enregistré dans du méthanol (50 μL de solution dans 1 mL de méthanol). Pour chaque mélange, la valeur du logP a été calculée en utilisant l'équation suivante :

$$LogP = Log\left(\frac{\int \Delta J = 2\ (oct)}{\int \Delta J = 2\ (H_2O)}\right)$$

Schéma 27 : structures des complexes du tableau 3 qui ne sont pas mentionnées dans le schéma 26

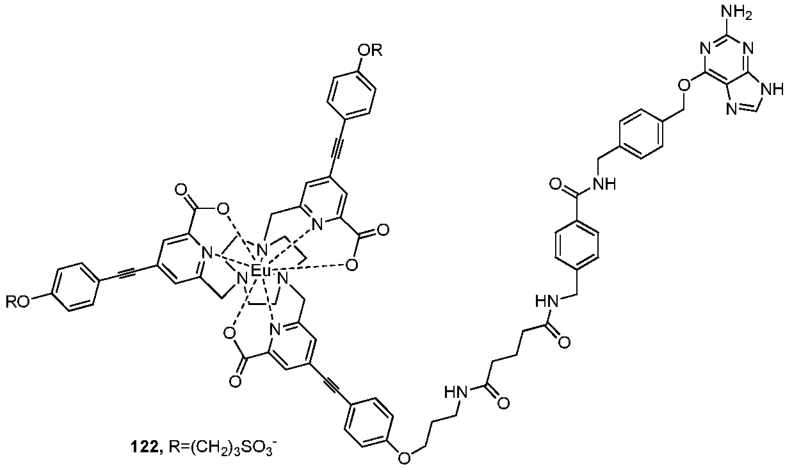

**122,** R=$(CH_2)_3SO_3^-$

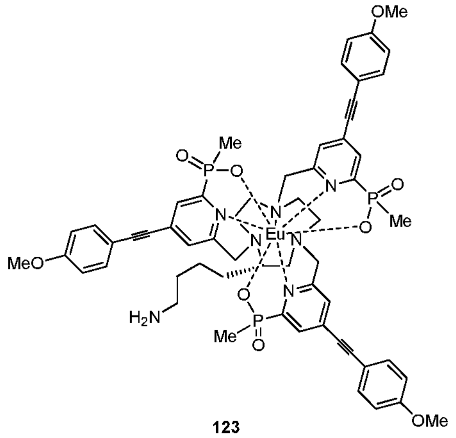

**123**

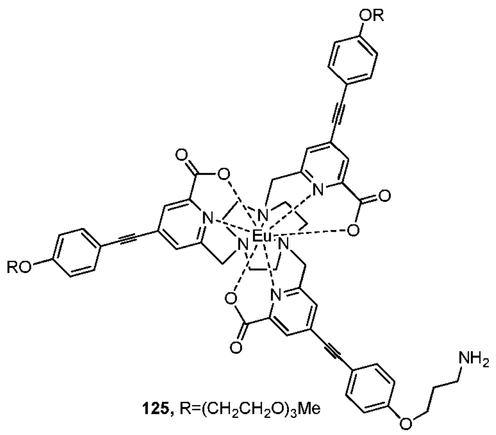

**125,** R=$(CH_2CH_2O)_3Me$

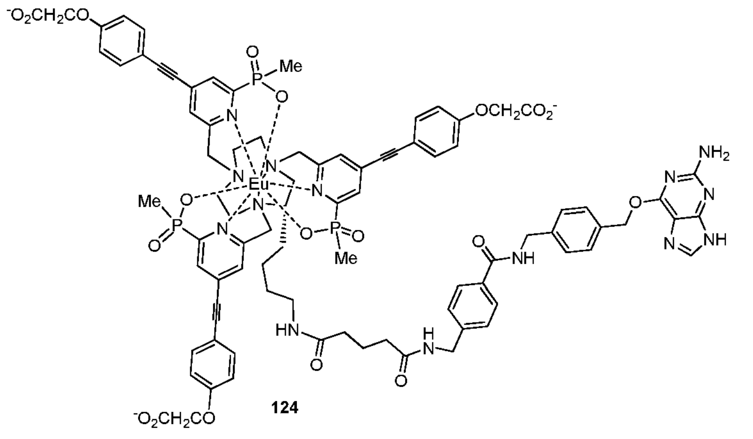

**124**

**Tableau 3: Propriétés photo-physiques de différents complexes**

| Complexe | λ max (nm) | $\Phi_{em}$ (%) | $T_0$ (ms) | Solvant |
|---|---|---|---|---|
| **46j** | 328 | 50 | 1,07 | $H_2O$ |
| **46h** | 330 | 44 | 1,05 | $H_2O$ |

(suite)

| Complexe | λ max (nm) | $\Phi_{em}$ (%) | $T_0$ (ms) | Solvant |
|---|---|---|---|---|
| **51b** | 339 | nd | 0,63 | Tampon Hepes 50 mM, pH 7,4 |
| **56e** | 330 | 49 | 1,04 | $H_2O$ |
| **62i** | 332 | 44 | 1,18 | MeOH |
| **62i** | 328 | 28 | 1,05 | $H_2O$ |
| **80** | 330 | nd | 0,96 | MeOH |
| **82** | 330 | nd | 0,98 | $H_2O$ |
| **84** | 330 | nd | nd | $H_2O$ |
| **86** | 330 | nd | nd | MeOH |
| **88** | 328 | 44 | 1,14 | MeOH |
| **89** | 330 | 50 | 1,12 | MeOH |
| **93** | 328 | 29 | 1,08 | $H_2O$ |
| **105d** | 332 | 31 | 1,11 | $H_2O$ |
| **105d** | 336 | 56 | 1,23 | MeOH |
| **112a** | 341 | 15 | 0,69 | Tampon Hepes 50 mM, pH 7,4 |
| **112a** | 341 | nd | 0,78 | Tampon Hepes 50 mM, pH 7,4 + 0,1% BSA |
| **115*** | 329 | 16 | 1,00 | Tampon Hepes 50 mM, pH 7,4 |
| **116*** | 331 | 39 | 1,03 | $H_2O$ |
| **116*** | 331 | 43 | 1,18 | MeOH |
| **117*** | 338 | 25 | 1,06 | MeOH |
| **121** | 342 | 55 | 1,15 | MeOH |
| **122** | 330 | nd | 0,97 | $H_2O$ |
| **123** | 332 | 43 | 1,18 | MeOH |
| **124** | 328 | 14 | 1,08 | Tampon Hepes 50 mM, pH 7,4 |
| **125** | 338 | nd | nd | MeOH |
| nd : non déterminée<br>* les complexes 115, 116 et 117 ne sont pas des complexes selon l'invention | | | | |

Les caractéristiques photo-physiques de quelques complexes sont décrites dans le tableau 3. L'ensemble de ces complexes possèdent les propriétés photo-physiques attendues à savoir une longueur d'onde d'absorption maximale (λ max) comprise entre 328 et 342 nm correspondant à une excitation au laser à azote (337 nm). Les valeurs des rendements quantiques ($\Phi_{em}$) doivent être considérées avec une marge d'erreur de ±15%. Tous ces complexes présentent des rendements quantiques supérieurs à 10% et sans ajout de fluorures de potassium. Les durées de vie ($T_0$) sont au moins de 0,7 ms ce qui est favorable à des expériences de FRET. Enfin les complexes comportant des groupes solubilisant de type sulfonate ou carboxylate sont hautement solubles dans les tampons biologiques aqueux. La présence de charges permet de solubiliser dans l'eau des complexes qui ne l'étaient pas initialement. De façon inattendue, ces charges ou plus largement les groupements solubilisants n'affectent pas les propriétés photo-physiques des complexes d'europium de l'invention, qui ont une structure similaire à celles des complexes 115, 116 et 117 de l'art antérieur.

## Revendications

**1.** Agent complexant de formule (I') :

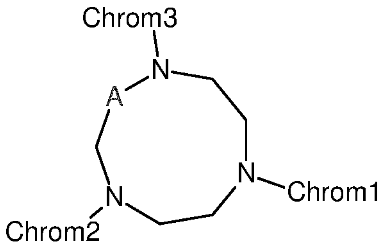

(I')

dans laquelle :

- A représente $-CH_2-$ ou $-CH(L_2-G)-$
- chrom1, chrom2 sont identiques et sont choisis parmi les groupes de formule :

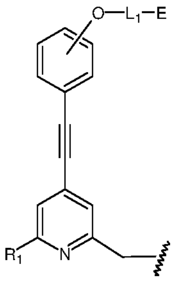

;

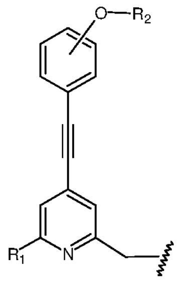

- chrom 3 est soit identique à chrom1 et chrom2, soit un groupe de formule :

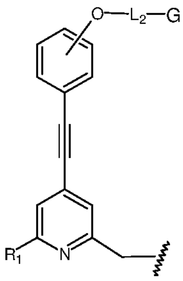

;

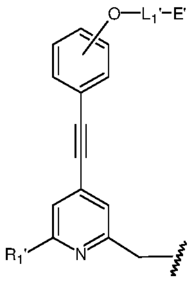

;

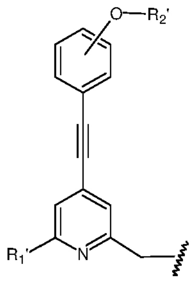

$R_2$, $R_2$', qui peuvent être identiques ou différents, sont choisis parmi : H ; -Alk ; - phényle ; $-CH_2-CO-N-Alk$ ; $-CH_2-CO-O-Alk$ ; $-CH_2-CO-NH_2$ ; $-CH_2-CO-OH$ ;

◦ $L_1$, $L_1$', $L_2$ sont choisis parmi les groupes divalents de formules suivantes :

$-(CH2)_n-;-(CH_2)_n-O-(CH_2)_m-O-(CH_2)_p-;$

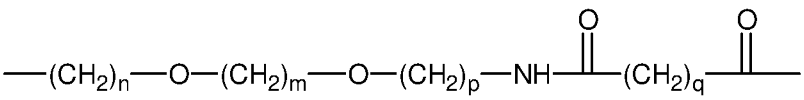

;

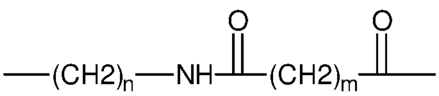

;

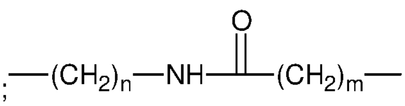

;

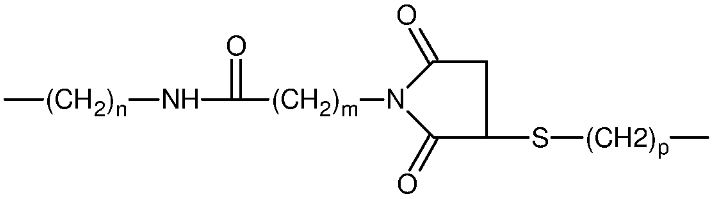

;

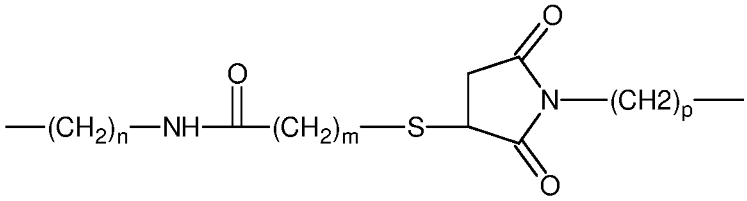

;

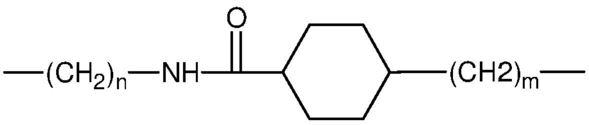

;

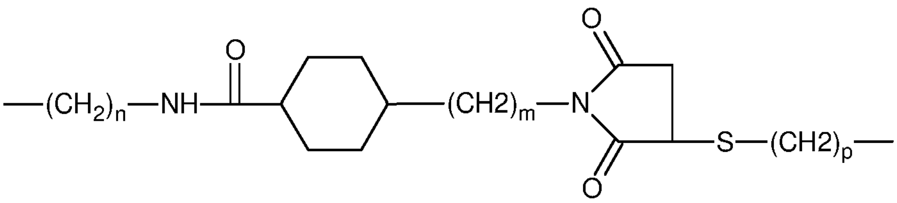

;

$—(CH_2)_n—NH—$ ; $—(CH2)_n—NH—(CH2)_m—$ ; 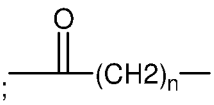 ;

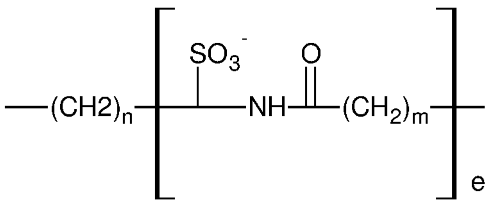

dans lesquelles n, m, p, q sont des nombres entiers de 1 à 16, de préférence de 1 à 5 et e est un nombre entier allant de 1 à 6, de préférence de 1 à 4 ;

- E, E', qui peuvent être identiques ou différents, sont des groupes augmentant l'hydrosolubilité de l'agent complexant, choisis parmi : $-SO_3H$, $-PO(OH)_2$, -COOH, $-N^+Alk_1Alk_2Alk_3$, un résidu carbohydrate de formule $-(CHOH)_k-CH_2OH$, k étant un nombre entier allant de 3 à 12, de préférence, égal à 4 ;
- $R_1$, $R_1$', qui peuvent être identiques ou différents, sont choisis parmi : -COOH, $-PO(OH)R_6$, $R_6$ étant choisi parmi les groupes : phényle, phényle substitué par un groupe $-SO_3H$, de préférence en position ortho ou para, benzyle, méthyle, éthyle, propyle, n-butyle, sec-butyle, isobutyle, tert-butyle ;
- G est une amine éventuellement protégée sous forme -NHBoc, un ester de succinimidyle, un haloacétamide, une hydrazine, un isothiocyanate, un groupe maléimide, ou un acide carboxylique éventuellement protégé sous la forme d'un groupe -COOMe ou, -COOtBu ;
- Alk, $Alk_1$, $Alk_2$, $Alk_3$, qui peuvent être identiques ou différents, représentent un $(C_1-C_6)$alkyle ;

sous réserve que :

- lorsque $R_1$ ou $R_1$' représentent un groupe -COOH, E ou E' ne représentent pas un groupe -COOH ;
- l'agent complexant comporte au moins un groupe E ou $-SO_3H$ ;
- lorsque chrom1, chrom2 et chrom3 comportent chacun un groupe $R_2$ ou $R_2$', alors A est un groupe $-CH(L_2-G)-$ dans lequel $L_2$ comprend au moins un groupe $-SO_3H$ ou bien $R_6$ est un groupe phényle substitué par un groupe $-SO_3H$.

**2.** Agent complexant selon la revendication 1, **caractérisé en ce que** A est le groupe $-CH_2-$et **en ce que** chrom1, chrom2, chrom3 sont identiques et représentent un groupe de formule :

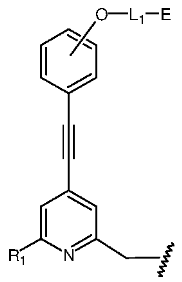

**3.** Agent complexant selon la revendication 1, **caractérisé en ce que** A est le groupe $-CH_2-$et **en ce que** chrom3 est différent de chrom1 et chrom2.

**4.** Agent complexant selon la revendication 1, caractérisé en ce A est le groupe $-CH(L_2-G)-$ et en ce que chrom1, chrom2, chrom3 sont identiques.

**5.** Agent complexant selon la revendication 1, caractérisé en ce A est le groupe $-CH(L_2-G)-$ et en ce que chrom3 est différent de chrom1 et chrom2.

**6.** Agent complexant selon l'une quelconque des revendications précédentes, **caractérisé en ce que** $R_1$ et $R_1$' représentent un groupe $-PO(OH)CH_3$.

**7.** Agent complexant selon l'une quelconque des revendications précédentes, **caractérisé en ce que** E et E' lorsqu'ils sont présents, représentent un groupe $-SO_3H$.

**8.** Agent complexant selon l'une quelconque des revendications 1 à 5, **caractérisé en ce que** $R_1$ et $R_1$' représentent un groupe $-PO(OH)R_6$ et **en ce que** E et E' représentent un groupe - $SO_3H$.

**9.** Agent complexant selon l'une quelconque des revendications précédentes, **caractérisé en ce qu'**il comprend un groupe $-L_2-G$.

**10.** Agent complexant selon l'une quelconque des revendications précédentes, **caractérisé en ce que** le groupe $-L_2-G$ est constitué d'un groupement réactif G tel que défini à la revendication 1, et d'un bras d'espacement $L_2$ constitué d'une chaîne alkylène comprenant de 1 à 5 atomes de carbone ou d'un groupe choisi parmi les groupes de formule :

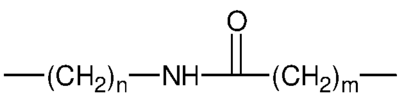 ; 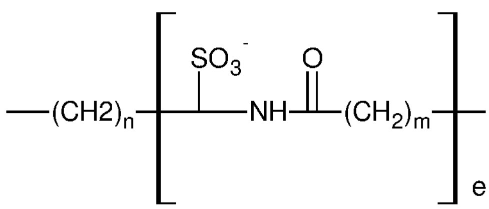

où n, m sont des nombres entiers de 1 à 16, de préférence de 1 à 5 et e est un nombre entier allant de 1 à 6, de préférence de 1 à 4, le groupe G étant lié à l'une ou l'autre extrémité de ces groupes divalents.

**11.** Agent complexant selon l'une quelconque des revendications précédentes, **caractérisé en ce que** les $L_1$ ou $L_1$', lorsqu'ils sont présents, représentent une chaîne alkylène comprenant de 1 à 5 atomes de carbone ou un groupe choisi parmi les groupes de formule :

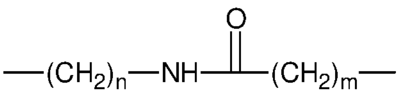 ; 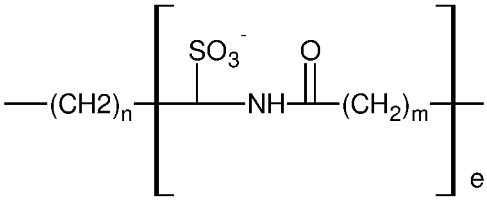

où n, m sont des nombres entiers de 1 à 16, de préférence de 1 à 5 et e est un nombre entier allant de 1 à 6, de préférence de 1 à 4, le groupe E ou E' étant lié à l'une ou l'autre extrémité de ces groupes divalents.

**12.** Complexe de lanthanide comprenant un agent complexant selon l'une quelconque des revendications précédentes et un lanthanide.

**13.** Complexe de lanthanide selon la revendication 12, **caractérisé en ce que** le lanthanide est choisi parmi : $Eu^{3+}$, $Tb^{3+}$, $Gd^{3+}$, $Dy^{3+}$, $Nd^{3+}$, $Er^{3+}$ ,de préférence le lanthanide est $Eu^{3+}$.

**14.** Conjugué fluorescent d'une molécule d'intérêt obtenu par la mise en contact d'un complexe de lanthanide selon l'une des revendications 12 et 13, ledit complexe comprenant un groupe $-L_2-G$, avec une molécule d'intérêt choisie parmi un acide aminé, un peptide, une protéine, un anticorps, un sucre, une chaîne glucidique, un nucléoside, un nucléotide, un oligonucléotide, un substrat d'enzyme, un chloroalcane, ou le coenzyme A.

## Patentansprüche

**1.** Komplexbildner der Formel (I') :

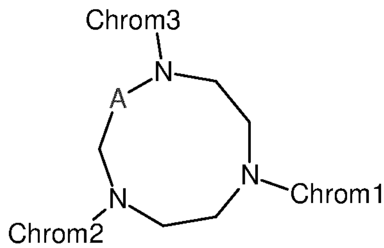

(I')

wobei:

- A für $-CH_2-$ oder $-CH(L_2-G)-$ steht
- chrom1, chrom2 gleich sind und ausgewählt sind aus den Gruppen der Formel:

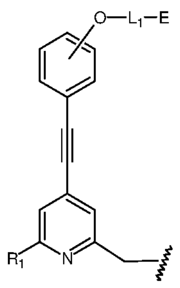 ; 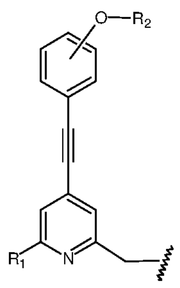

- chrom3 ist entweder identisch mit chrom1 und chrom2 oder eine Gruppe der Formel:

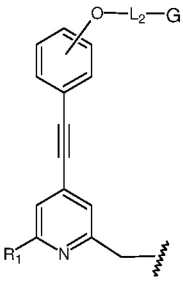

;

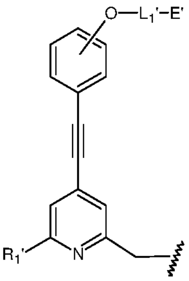

;

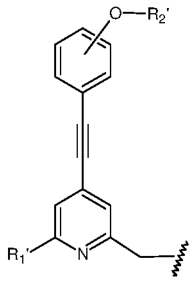

- $R_2$, $R_2$', die gleich oder verschieden sein können, sind ausgewählt aus: H; -Alk;
- Phenyl; $-CH_2-CO-N-Alk$; $-CH_2-CO-O-Alk$; $-CH_2-CO-NH_2$; $-CH_2-CO-OH$;
- $L_1$, $L_1$', $L_2$ sind ausgewählt aus zweiwertigen Gruppen der folgenden Formeln:

$-(CH2)_n-;-(CH_2)_n-O-(CH_2)_m-O-(CH_2)_p-;$

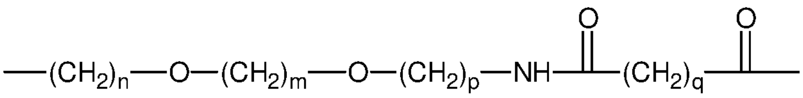

;

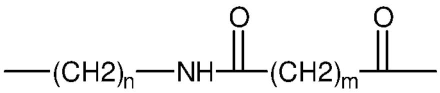

;

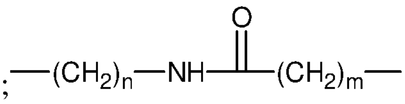

;

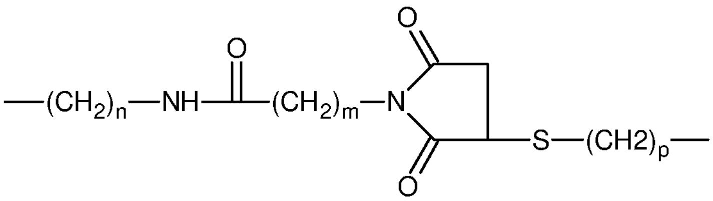

;

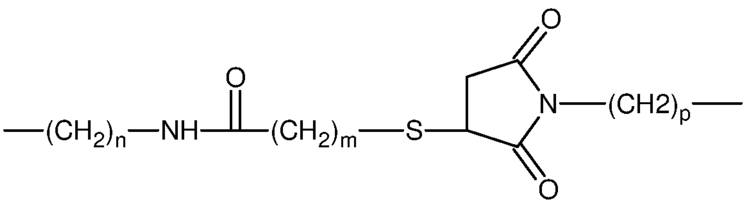

;

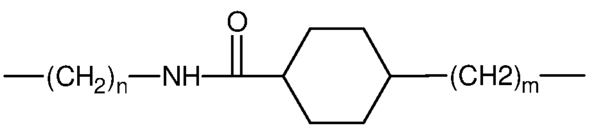

;

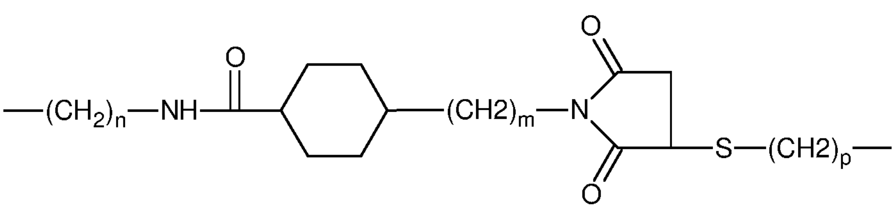

;

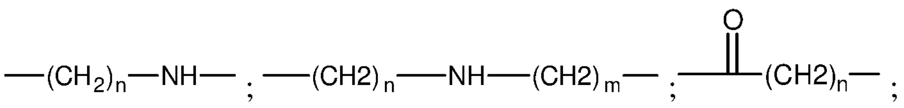

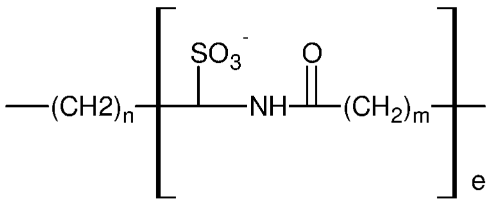

wobei n, m, p, q ganze Zahlen von 1 bis 16, vorzugsweise 1 bis 5, sind und e eine ganze Zahl von 1 bis 6, vorzugsweise 1 bis 4, ist;
- E, E', die gleich oder verschieden sein können, sind Gruppen, die die Wasserlöslichkeit des Komplexbildners erhöhen, ausgewählt aus: $-SO_3H$, $-PO(OH)_2$,
- COOH, $-N^+Alk_1Alk_2Alk_3$, einem Kohlenhydrat-Rest der Formel $-(CHOH)_k-CH_2OH$, wobei k eine ganze Zahl von 3 bis 12, vorzugsweise gleich 4, ist;
- $R_1$, $R_1$', die gleich oder verschieden sein können, sind ausgewählt aus: -COOH,
- $PO(OH)R_6$, wobei $R_6$ ausgewählt ist aus den Gruppen: Phenyl, Phenyl, substituiert mit einer $-SO_3H$-Gruppe, vorzugsweise in ortho- oder para-Position, Benzyl, Methyl, Ethyl, Propyl, n-Butyl, sec-Butyl, Isobutyl, tert-Butyl;
- G ein Amin, das gegebenenfalls in der -NHBoc-Form geschützt ist, ein Succinimidylester, ein Haloacetamid, ein Hydrazin, ein Isothiocyanat, eine Maleimidgruppe oder eine Carbonsäure ist, die gegebenenfalls in der Form einer - COOMe- oder -COOtBu-Gruppe geschützt ist;
- Alk, $Alk_1$, $Alk_2$, $Alk_3$, die gleich oder verschieden sein können, $(C_1-C_6)$-Alkyl darstellen;

unter der Bedingung, dass:

- wenn $R_1$ oder $R_1$' eine -COOH-Gruppe darstellen, E oder E' nicht eine -COOH-Gruppe darstellen;
- der Komplexbildner mindestens eine Gruppe E oder $-SO_3H$ aufweist;
- wenn chrom1, chrom2 und chrom3 jeweils eine Gruppe $R_2$ oder $R_2$' aufweisen, dann ist A eine Gruppe $-CH(L_2-G)-$, in der $L_2$ mindestens eine Gruppe $-SO_3H$ umfasst, oder $R_6$ ist eine Phenylgruppe, die mit einer Gruppe $-SO_3H$ substituiert ist.

2. Komplexbildner nach Anspruch 1, **dadurch gekennzeichnet, dass** A die Gruppe $-CH_2-$ ist und dass chrom1, chrom2, chrom3 gleich sind und eine Gruppe der Formel darstellen:

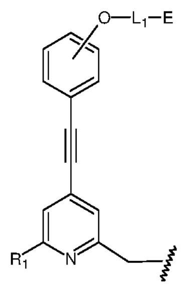

3. Komplexbildner nach Anspruch 1, **dadurch gekennzeichnet, dass** A die Gruppe $-CH_2-$ ist, und dass chrom3 von chrom1 und chrom2 verschieden ist.

4. Komplexbildner nach Anspruch 1, **dadurch gekennzeichnet, dass** A die Gruppe $-CH(L_2-G)-$ ist, und dass chrom1, chrom2 und chrom3 gleich sind.

5. Komplexbildner nach Anspruch 1, **dadurch gekennzeichnet, dass** A die Gruppe $-CH(L_2-G)-$ ist, und dass chrom3 von chrom1 und chrom2 verschieden ist.

6. Komplexbildner nach einem der vorhergehenden Ansprüche, **dadurch gekennzeichnet, dass** $R_1$ und $R_1$' eine

-PO(OH)$CH_3$-Gruppe darstellen.

7. Komplexbildner nach einem der vorhergehenden Ansprüche, **dadurch gekennzeichnet, dass** E und E', wenn sie vorhanden sind, eine -$SO_3$H-Gruppe darstellen.

8. Komplexbildner nach einem der Ansprüche 1 bis 5, **dadurch gekennzeichnet, dass** $R_1$ und $R_1$' eine -PO(OH)$R_6$-Gruppe darstellen und dass E und E' eine -$SO_3$H-Gruppe darstellen.

9. Komplexbildner nach einem der vorhergehenden Ansprüche, **dadurch gekennzeichnet, dass** er eine -$L_2$-G-Gruppe umfasst.

10. Komplexbildner nach einem der vorhergehenden Ansprüche, **dadurch gekennzeichnet, dass** die -$L_2$-G-Gruppe aus einer reaktiven Gruppe G, wie in Anspruch 1 definiert, und einem Spacer-Arm $L_2$ besteht, der aus einer Alkylenkette mit 1 bis 5 Kohlenstoffatomen oder einer Gruppe besteht, ausgewählt aus den Gruppen der Formel:

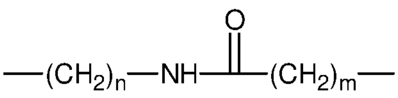 ; 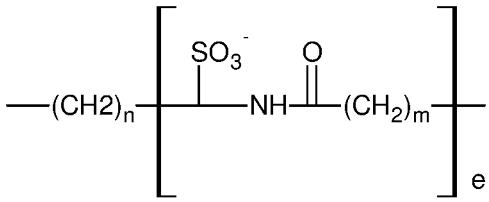

wobei n, m ganze Zahlen von 1 bis 16, vorzugsweise 1 bis 5, sind und e eine ganze Zahl von 1 bis 6, vorzugsweise 1 bis 4, ist, wobei die Gruppe G an das eine oder andere Ende dieser zweiwertigen Gruppen gebunden ist.

11. Komplexbildner nach einem der vorhergehenden Ansprüche, **dadurch gekennzeichnet, dass** $L_1$ oder $L_1$', wenn sie vorhanden sind, eine Alkylenkette mit 1 bis 5 Kohlenstoffatomen oder eine Gruppe, ausgewählt aus den Gruppen der Formel:

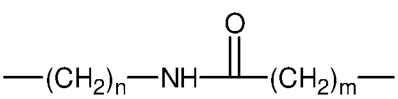 ; 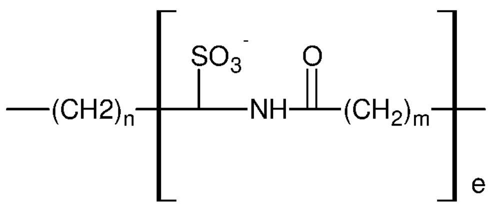

wobei n, m ganze Zahlen von 1 bis 16, vorzugsweise 1 bis 5, sind und e eine ganze Zahl von 1 bis 6, vorzugsweise 1 bis 4, ist, wobei die Gruppe E oder E' an das eine oder andere Ende dieser zweiwertigen Gruppen gebunden ist.

12. Lanthanidkomplex, umfassend einen Komplexbildner gemäß einem der vorhergehenden Ansprüche und ein Lanthanid.

13. Lanthanidkomplex nach Anspruch 12, **dadurch gekennzeichnet, dass** das Lanthanid ausgewählt ist aus: $Eu^{3+}$, $Tb^{3+}$, $Gd^{3+}$, $Dy^{3+}$, $Nd^{3+}$, $Er^{3+}$, vorzugsweise ist das Lanthanid $Eu^{3+}$.

14. Fluoreszierendes Konjugat eines Moleküls von Interesse, erhalten durch Inkontaktbringen eines Lanthanidkomplexes nach einem der Ansprüche 12 und 13, wobei der Komplex eine -$L_2$-G-Gruppe umfasst, mit einem Molekül von Interesse, ausgewählt aus einer Aminosäure, einem Peptid, einem Protein, einem Antikörper, einem Zucker, einer Kohlenhydratkette, einem Nukleosid, einem Nukleotid, einem Oligonukleotid, einem Enzymsubstrat, einem Chloroalkan oder Coenzym A.

## Claims

1. A complexing agent of formula (I'):

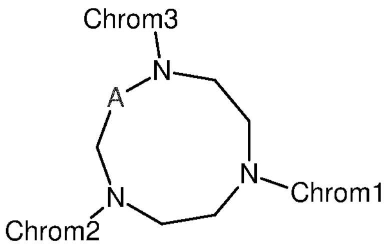

(I)

wherein:

- A represents $-CH_2-$ or $-CH(L_2-G)-$
- chrom1, chrom2 are identical and are selected from the groups of formula:

\- 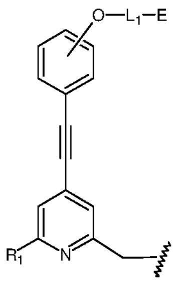 ; 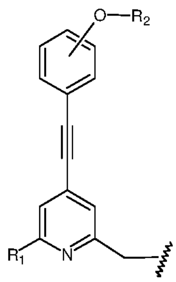

- chrom3 is either identical to chrom1 and chrom2, or is a group of formula:

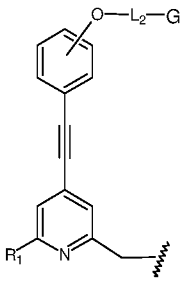 ; 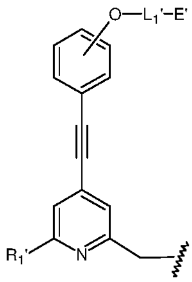 ; 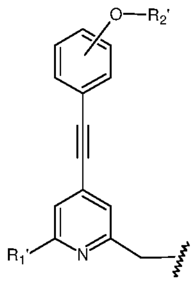

$R_2$, $R_2$', which may be identical or different, are selected from: H; -Alk; -phenyl; $-CH_2-CO-N-Alk$; $-CH_2-CO-O-Alk$; $-CH_2-CO-NH_2$; $-CH_2-CO-OH$;
$L_1$, $L_1$', $L_2$ are selected from the divalent groups having the following formulas:

$-(CH2)_n-$;$-(CH_2)_n-O-(CH_2)_m-O-(CH_2)_p-$;

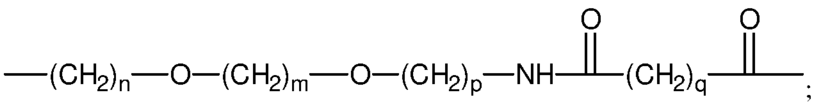;

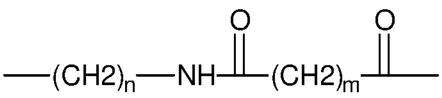; 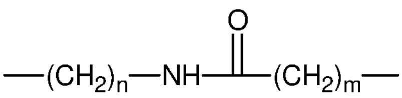;

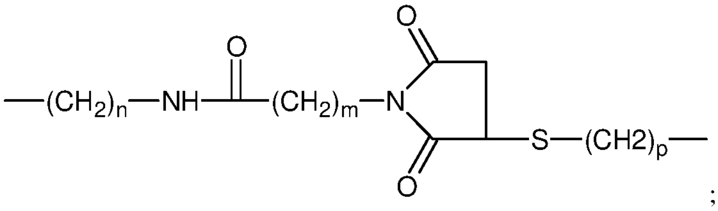

;

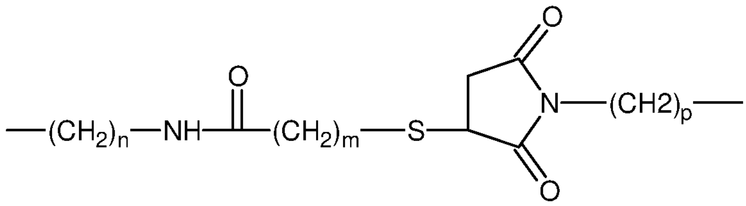

;

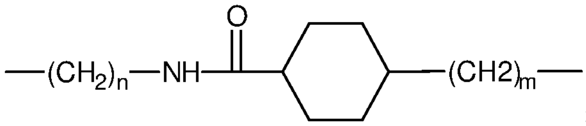

;

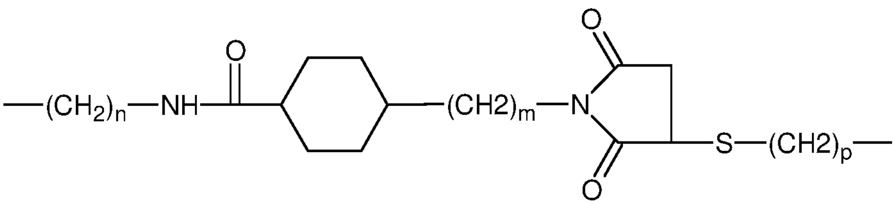

;

$-(CH_2)_n-NH-$; $-(CH2)_n-NH-(CH2)_m-$; 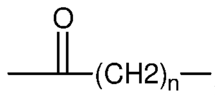;

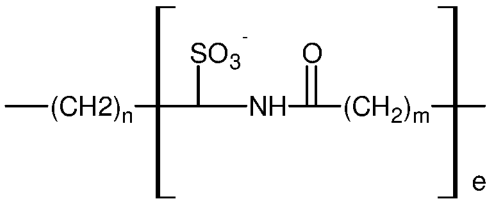

wherein n, m, p, q are integers from 1 to 16, preferably from 1 to 5 and e is an integer from 1 to 6, preferably from 1 to 4;

- E, E', which may be identical or different, are groups increasing the water-solubility of the complexing agent, selected from: $-SO_3H$, $-PO(OH)_2$, -COOH, $-N^+Alk_1Alk_2Alk_3$, a carbohydrate residue of formula $-(CHOH)_k-CH_2OH$, k being an integer from 3 to 12, preferably equal to 4.;
- $R_1$, $R_1$', which may be identical or different, are selected from: -COOH, $-PO(OH)R_6$, $R_6$ being selected from the groups: phenyl, phenyl substituted with a group $-SO_3H$, preferably in the ortho or para position, benzyl, methyl, ethyl, propyl, n-butyl, sec-butyl, isobutyl, tert-butyl;
- G is an amine optionally protected in the form -NHBoc, a succinimidyl ester, a haloacetamide, a hydrazine, an isothiocyanate, a maleimide group, or a carboxylic acid optionally protected in the form of a group -COOMe or -COOtBu;
- Alk, $Alk_1$, $Alk_2$, $Alk_3$, which may be identical or different, represent a $(C_1-C_6)$alkyl;

provided that:

- when $R_1$ or $R_1$' represent a -COOH group, E or E' do not represent a -COOH group;
- the complexing agent comprises at least one group E or $-SO_3H$;
- when chrom1, chrom2 and chrom3 each comprise a group $R_2$ or $R_2$', then A is a group $-CH(L_2-G)$-in which $L_2$ comprises at least one group $-SO_3H$ or else $R_6$ is a phenyl group substituted with a group $-SO_3H$.

2. The complexing agent of claim 1, **characterized in that** A is the group $-CH_2-$ and **in that** chrom1, chrom2, chrom3 are identical and represent a group of formula:

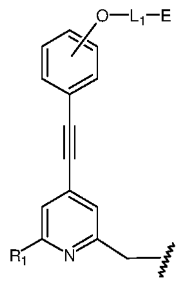

3. The complexing agent of claim 1, **characterized in that** A is the group $-CH_2-$ and **in that** chrom3 is different from chrom1 and chrom2.

4. The complexing agent of claim 1, **characterized in that** A is the group $-CH(L_2-G)-$ and **in that** chrom1, chrom2, chrom3 are identical.

5. The complexing agent of claim 1, **characterized in that** A is the group $-CH(L_2-G)-$ and **in that** chrom3 is different from chrom1 and chrom2.

6. The complexing agent of any one of the preceding claims, **characterized in that** $R_1$ and $R_1$' represent a group $-PO(OH)CH_3$.

7. The complexing agent of any one of the preceding claims, **characterized in that** E and E' when they are present, represent a group $-SO_3H$.

8. The complexing agent of any one of claims 1 to 5, **characterized in that** $R_1$ and $R_1$' represent a group $-PO(OH)R_6$ and **in that** E and E' represent a group $-SO_3H$.

9. The complexing agent of any one of the preceding claims, **characterized in that** it comprises a group $-L_2-G$.

10. The complexing agent of any one of the preceding claims, **characterized in that** the group $-L_2-G$ consists of a reactive group G as defined in claim 1, and a spacer arm $L_2$ consisting of an alkylene chain comprising 1 to 5 carbon atoms or a group selected from the groups of formula:

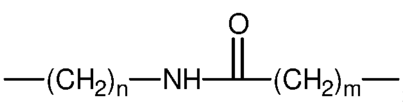 ; 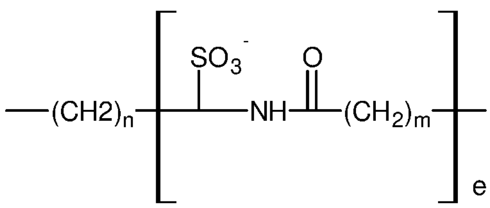

where n, m are integers from 1 to 16, preferably from 1 to 5 and e is an integer from 1 to 6, preferably from 1 to 4, group G being bound to one or other end of these divalent groups.

11. The complexing agent of any one of the preceding claims, **characterized in that** $L_1$ or $L_1$', when they are present, represent an alkylene chain comprising 1 to 5 carbon atoms or a group selected from the groups of formula:

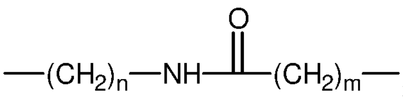 ; 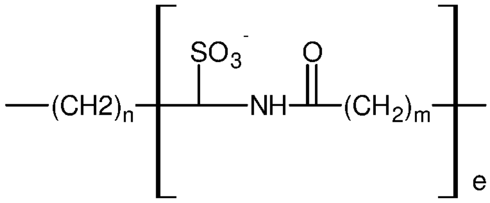

where n, m are integers from 1 to 16, preferably from 1 to 5 and e is an integer from 1 to 6, preferably from 1 to 4, the group E or E' being bound to one or other end of these divalent groups.

**12.** A lanthanide complex comprising a complexing agent of any one of the preceding claims and a lanthanide.

**13.** The lanthanide complex of claim 12, **characterized in that** the lanthanide is selected from: $Eu^{3+}$, $Tb^{3+}$, $Gd^{3+}$, $Dy^{3+}$, $Nd^{3+}$, $Er^{3+}$; preferably the lanthanide is $Eu^{3+}$.

**14.** A fluorescent conjugate of a molecule of interest obtained by bringing a lanthanide complex of one of claims 12 and 13, said complex comprising a group $-L_2-G$, into contact with a molecule of interest selected from an amino acid, a peptide, a protein, an antibody, a sugar, a carbohydrate chain, a nucleoside, a nucleotide, an oligonucleotide, an enzyme substrate, a chloroalkane or coenzyme A.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005058877 A
- EP 0180492 A
- EP 0321353 A
- EP 0601113 A
- WO 200196877 A
- WO 2008063721 A
- US 5622821 A
- US 4920195 A
- US 4761481 A
- US 5216134 A
- US 4859777 A
- US 5202423 A
- US 5324825 A
- WO 8901475 A
- WO 2013011236 A
- WO 02095412 A


### Littérature non-brevet citée dans la description


- *Journal of Luminescence,* Septembre 1997, vol. 75 (2), 149-169
- *Inorg Chem.,* 17 Novembre 2008, vol. 47 (22), 10258-68
- **D'ALÉO et al.** *Angew. Chem. Int. Ed.,* 2012, vol. 51, 6622-6625
- **PLACIDE et al.** *Tetrahedron Letters,* 2014, vol. 55, 1357-1361
- **WALTON et al.** *Chem. Comm.,* 2013, vol. 49, 1600-1602
- **COX et al.** *J. Chem. Soc., Perkin Trans.,* 1990, vol. 1, 2567-2576
- **CRAIG et al.** *J. Chem. Soc., Chem. Commun.,* 1989, 794-796
- **G.T. HERMANSON.** Bioconjugate Techniques. Academic Press, 2008, 169-211
- **PARKER et al.** *J. Chem. Soc. Perkin Trans,* vol. 1, 1990-2567
- **SONOGASHIRA et al.** *Tetrahedron Letters,* 1975, vol. 50, 4467-4470
- **ROSSI et al.** *Organic Préparation and Procedure International,* 1995, vol. 27, 129-160
- *J. Am. Chem. Soc,* 2011, vol. 133, 958
- **MAURY et al.** *Inorganic Chemistry,* 2011, vol. 50, 4987-4999
- **GINSBURG et al.** *Journal American Chemical Society,* 1957, vol. 79, 481-485
- *Analytical Chemistry,* 2006, vol. 78, 4175-4183
- *European Journal of Chemistry,* 2001, 349-352
- *Bioconjugate Chemistry,* 2008, vol. 19, 279-289
- *J. Chem. Soc Perkin Trans,* 1990, vol. 1, 2567
- **S. MACHIDA et al.** *Journal American Chemical Society,* 2011, 133-958, 963
- *Chem Commun,* 2013, vol. 49, 1600-1602